# EUROPEAN PATENT SPECIFICATION

(11) **EP 2 493 916 B1**
(45) Date of publication and mention of the grant of the patent: **08.06.2016**
(21) Application number: 10785003.4
(22) Date of filing: 26.10.2010
(51) Int. Cl.: C07K 14/47, C12Q 1/68, G01N 33/50, G01N 33/574

(54) **LUNG TUMOR MARKERS AND METHODS OF USE THEREOF**
MARKER FÜR LUNGENTUMORE UND DEREN VERWENDUNG
MARQUEURS ASSOCIÉS AVEC DES TUMEURS PULMONAIRES ET LEUR UTILISATION

(30) Priority: 26.10.2009 EP 09174057
(43) Date of publication of application: 05.09.2012
(73) Proprietor: Externautics S.p.A., 53100 Siena (IT)
(72) Inventor: GRIFANTINI, Renata, 53100 Siena (IT); PILERI, Piero, 53100 Siena (IT); CAMPAGNOLI, Susanna, 53100 Siena (IT); GRANDI, Alberto, 53100 Siena (IT); PARRI, Matteo, 53100 Siena (IT); PIERLEONI, Andrea, 53100 Siena (IT); NOGAROTTO, Renzo, 53100 Siena (IT)
(74) Representative: Banfi, Paolo
(86) International application number: PCT/EP2010/066147
(87) International publication number: WO 2011/051278

(56) References cited:
- WO-A1-2004/058288
- WO-A2-02/086443
- KAGARA NAOFUMI ET AL: "Zinc and its transporter ZIP10 are involved in invasive behavior of breast cancer cells" CANCER SCIENCE, vol. 98, no. 5, May 2007 (2007-05), pages 692-697, XP002616766 ISSN: 1347-9032
- ALBRECHT AMY L ET AL: "Zinc transporter mRNA expression in the RWPE-1 human prostate epithelial cell line" BIOMETALS, vol. 21, no. 4, August 2008 (2008-08), pages 405-416, XP019600312 ISSN: 0966-0844

## Description

The present invention relates to newly identified proteins as markers for the detection of lung tumors, or as therapeutic targets for their treatment. Also provided are affinity ligands capable of selectively interacting with the newly identified markers, as well as methods for tumor diagnosis and therapy using such ligands.

### Background of the invention

### Tumor markers (or biomarkers)

Tumor markers are substances that can be produced by tumor cells or by other cells of the body in response to cancer. In particular, a protein biomarker is either a single protein or a panel of different proteins, that could be used to unambiguously distinguish a disease state. Ideally, a biomarker would have both a high specificity and sensitivity, being represented in a significant percentage of the cases of given disease and not in healthy state.

Biomarkers can be identified in different biological samples, like tissue biopsies or preferably biological fluids (saliva, urine, blood-derivatives and other body fluids), whose collection does not necessitate invasive treatments. Tumor marker levels may be categorized in three major classes on the basis of their clinical use. Diagnostic markers can be used in the detection and diagnosis of cancer. Prognostics markers are indicative of specific outcomes of the disease and can be used to define predictive models that allow the clinicians to predict the likely prognosis of the disease at time of diagnosis. Moreover, prognosis markers are helpful to monitor the patient response to a drug therapy and facilitate a more personalized patient management. A decrease or return to a normal level may indicate that the cancer is responding to therapy, whereas an increase may indicate that the cancer is not responding. After treatment has ended, tumor marker levels may be used to check for recurrence of the tumor. Finally, therapeutic markers can be used to develop tumor-specific drugs or affinity ligand (i.e. antibodies) for a prophylactic intervention.

Currently, although an abnormal tumor marker level may suggest cancer, this alone is usually not enough to accurately diagnose cancer and their measurement in body fluids is frequently combined with other tests, such as a biopsy and radioscopic examination. Frequently, tumor marker levels are not altered in all of people with a certain cancer disease, especially if the cancer is at early stage. Some tumor marker levels can also be altered in patients with noncancerous conditions. Most biomarkers commonly used in clinical practice do not reach a sufficiently high level of specificity and sensitivity to unambiguously distinguish a tumor from a normal state.

To date the number of markers that are expressed abnormally is limited to certain types/subtypes of cancer, some of which are also found in other diseases. (http://www.cancer.gov/cancertopics/factsheet).

For instance, the human epidermal growth factor receptor (HER2) is a marker protein overproduced in about 20% of breast cancers, whose expression is typically associated with a more aggressive and recurrent tumors of this class.

### Routine screening test for tumor diagnosis

Screening tests are a way of detecting cancer early, before there are any symptoms. For a screening test to be helpful, it should have high sensitivity and specificity. Sensitivity refers to the test's ability to identify people who have the disease. Specificity refers to the test's ability to identify people who do not have the disease. Different molecular biology approaches such as analysis of DNA sequencing, small nucleotide polymorphyms, in situ hybridization and whole transcriptional profile analysis have done remarkable progresses to discriminate a tumor state from a normal state and are accelerating the knowledge process in the tumor field. However so far different reasons are delaying their use in the common clinical practice, including the higher analysis complexity and their expensiveness. Other diagnosis tools whose application is increasing in clinics include in situ hybridization and gene sequencing.

Currently, Immuno-HistoChemistry (IHC), a technique that allows the detection of proteins expressed in tissues and cells using specific antibodies, is the most commonly used method for the clinical diagnosis of tumor samples. This technique enables the analysis of cell morphology and the classification of tissue samples on the basis of their immunoreactivity. However, at present, IHC can be used in clinical practice to detect cancerous cells of tumor types for which protein markers and specific antibodies are available. In this context, the identification of a large panel of markers for the most frequent cancer classes would have a great impact in the clinical diagnosis of the disease.

### Anti-cancer therapies

In the last decades, an overwhelming number of studies remarkably contributed to the comprehension of the molecular mechanisms leading to cancer. However, this scientific progress in the molecular oncology field has not been paralleled by a comparable progress in cancer diagnosis and therapy. Surgery and/or radiotherapy are the still the main modality of local treatment of cancer in the majority of patients. However, these treatments are effective only at initial phases of the disease and in particular for solid tumors of epithelial origin, as is the case of colon, lung, breast, prostate and others, while they are not effective for distant recurrence of the disease. In some tumor classes, chemotherapy treatments have been developed, which generally relies on drugs, hormones and antibodies, targeting specific biological processes used by cancers to grow and spread. However, so far many cancer therapies had limited efficacy due to severity of side effects and overall toxicity. Indeed, a major effort in cancer therapy is the development of treatments able to target specifically tumor cells causing limited damages to surrounding normal cells thereby decreasing adverse side effects. Recent developments in cancer therapy in this direction are encouraging, indicating that in some cases a cancer specific therapy is feasible. In particular, the development and commercialization of humanized monoclonal antibodies that recognize specifically tumor-associated markers and promote the elimination of cancer is one of the most promising solutions that appears to be an extremely favorable market opportunity for pharmaceutical companies. However, at present the number of therapeutic antibodies available on the market or under clinical studies is very limited and restricted to specific cancer classes. So far licensed monoclonal antibodies currently used in clinics for the therapy of specific tumor classes, show only a partial efficacy and are frequently associated with chemotherapies to increase their therapeutic effect. Administration of Trastuzumab (Herceptin), a commercial monoclonal antibody targeting HER2, a protein overproduced in about 20% of breast cancers, in conjunction with Taxol adjuvant chemotherapy induces tumor remission in about 42% of the cases. Bevacizumab (Avastin) and Cetuximab (Erbitux) are two monoclonal antibodies recently licensed for use in humans, targeting the endothelial and epithelial growth factors respectively that, combined with adjuvant chemotherapy, proved to be effective against different tumor diseases. Bevacizumab proved to be effective in prolonging the life of patients with metastatic colorectal, breast and lung cancers. Cetuximab demostrated efficacy in patients with tumor types refractory to standard chemotherapeutic treatments (Adams G.P. and Weiner L.M. (2005) Monoclonal antibody therapy cancer. Nat Biotechnol. 23:1147-57).

In summary, available screening tests for tumor diagnosis are uncomfortable or invasive and this sometimes limits their applications. Moreover tumor markers available today have a limited utility in clinics due to either their incapability to detect all tumor subtypes of the defined cancers types and/or to distinguish unambiguously tumor vs. normal tissues. Similarly, licensed monoclonal antibodies combined with standard chemotherapies are not effective against the majority of cases. Therefore, there is a great demand for new tools to advance the diagnosis and treatment of cancer.

### Experimental approaches commonly used to identify tumor markers

Most popular approaches used to discover new tumor markers are based on genome-wide transcription profile or total protein content analyses of tumor. These studies usually lead to the identification of groups of mRNAs and proteins which are differentially expressed in tumors. Validation experiments then follow to eventually single out, among the hundreds of RNAs/proteins identified, the very few that have the potential to become useful markers. Although often successful, these approaches have several limitations and often, do not provide firm indications on the association of protein markers with tumor. A first limitation is that, since frequently mRNA levels not always correlate with corresponding protein abundance (approx. 50% correlation), studies based on transcription profile do not provide solid information regarding the expression of protein markers in tumor.

A second limitation is that neither transcription profiles nor analysis of total protein content discriminate post-translation modifications, which often occur during oncogenesis. These modifications, including phosphorylations, acetylations, and glycosylations, or protein cleavages influence significantly protein stability, localization, interactions, and functions (5).

As a consequence, large scale studies generally result in long lists of differentially expressed genes that would require complex experimental paths in order to validate the potential markers. However, large scale genomic/proteomic studies reporting novel tumor markers frequently lack of confirmation data on the reported potential novel markers and thus do not provide solid demonstration on the association of the described protein markers with tumor.

The approach that we used to identify the protein markers included in the present invention is based on an innovative immuno-proteomic technology. In essence, a library of recombinant human proteins has been produced from E. coli and is being used to generate polyclonal antibodies against each of the recombinant proteins.

The screening of the antibodies library on Tissue microarrays (TMAs) carrying clinical samples from different patients affected by the tumor under investigation lead to the identification of specific tumor marker proteins. Therefore, by screening TMAs with the antibody library, the tumor markers are visualized by immuno-histochemistry, the classical technology applied in all clinical pathology laboratories. Since TMAs also include healthy tissues, the specificity of the antibodies for the tumors can be immediately appreciated and information on the relative level of expression and cellular localization of the markers can be obtained. In our approach the markers are subjected to a validation process consisting in a molecular and cellular characterization.

Altogether, the detection the marker proteins disclosed in the present invention selectively in tumor samples and the subsequent validation experiments lead to an unambiguous confirmation of the marker identity and confirm its association with defined tumor classes. Moreover this process provides an indication of the possible use of the proteins as tools for diagnostic or therapeutic intervention. For instance, markers showing a surface cellular localization could be both diagnostic and therapeutic markers against which both chemical and antibody therapies can be developed. Differently, markers showing a cytoplasmic expression could be more likely considered for the development of tumor diagnostic tests and chemotherapy/small molecules treatments.

### Prior art

WO02/086443 discloses a method of detecting and monitoring lung cancer by determining the levels of a lung cancer-associated polynucleotide or polypeptide in a biological sample. One of the markers (SEQ ID NO:415) is approximately 37% identical to SLC39A10 (SEQ ID NO:15 according to the present invention).

WO2004/058288 discloses a SLC39A10 sequence (first entry in Table 29) containing 4 extra amino acids at the N-terminal end relative to SEQ ID NO:15 according to the present invention and is identical in the overlapping region. There are also provided methods for detecting cancer, antibodies to SLC39A10 and their use to detect the marker on a cancer cell surface and for cancer therapy. The tumor markers are associated with cancers, especially lymphoma, breast cancer or prostate cancer. Lung cancer is mentioned among many cancer types but it is not correlated to SLC39A10.

Kagara Naofumi et al (Cancer Science, vol. 98, bo. 5, May 2007 pages 692-697) discloses that SLC39A10 (ZIP10) is associated with the metastasis of breast cancer to the lymph node (6).

### Summary of the invention

The present invention provides new means for the detection and treatment of lung tumors, based on the identification of a protein marker specific for these tumor types, namely the solute carrier family 39 (zinc transporter), member 10 (SLC39A10).

In a preferred embodiment, the invention provides the use of SLC39A10 as marker or target for lung tumor.

The invention also provides a method for the diagnosis of these cancer types, comprising a step of detecting the above-identified markers in a biological sample, e.g. in a tissue sample of a subject suspected of having or at risk of developing malignancies or susceptible to cancer recurrences.

In addition, the tumor marker identify novel targets for affinity ligands, which can be used for therapeutic applications.

### Detailed disclosure of the invention

The present invention is based on the surprising finding of antibodies that are able to specifically stain lung tumor tissues from patients, while negative or very poor staining is observed in normal lung tissues from the same patients. These antibodies have been found to specifically bind to proteins for which no previous association with tumor has been reported.

Hence, in a first aspect, the invention provides a lung tumor marker, which is SLC39A10 in one of its variant isoforms SEQ ID NO:15, SEQ ID NO:16 or a different isoform having sequence identity of at least 80%, preferably at least 90%, more preferably at least 95% to SEQ ID NO:15 or SEQ ID NO:16, or a nucleic acid molecule containing a sequence coding for a SLC39A10 protein, said encoding sequence being preferably selected from SEQ ID NO:17, SEQ ID NO:18.

As used herein, "Percent (%) amino acid sequence identity" with respect to the marker protein sequences identified herein indicates the percentage of amino acid residues in a full-length protein variant or isoform according to the invention, or in a portion thereof, that are identical with the amino acid residues in the specific marker sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. Identity between nucleotide sequences is preferably determined by the Smith-Waterman homology search algorithm as implemented in the MPSRCH program (Oxford Molecular), using an affine gap search with parameters gap open penalty = 12 and gap extension penalty = 1.

Solute carrier family 39 member 10 (SLC39A10, synonyms: Zinc transporter ZIP10 Precursor, Zrt- and Irt-like protein 10, ZIP-10, Solute carrier family 39 member 10; gene ID: ENSG00000196950; transcript IDs: ENST00000359634, ENST00000409086; protein ID: ENSP00000352655, ENSP00000386766) belongs to a subfamily of proteins that show structural characteristics of zinc transporters. It is an integral membrane protein likely involved in zinc transport. While other members of the zinc transport family have been at least partially studied in tumors, little is known about the association of SLC39A10 with this disease. SLC39A10 mRNA has been shown be moderately upregulated in breast cancer tissues as compared to normal samples (approximately 1.5 fold). In the same sudy, loss of SLC39A10 transcription in breast cell lines has been reported to reduce cell migratory activity (7). However, published studies on the expression of SLC39A10 in breast tumor cells are limited to the analysis of SLC39A10 transcript whilst, to the best of our knowledge, no data have been reported documenting the presence of SLC39A10 protein in these tumor cells. SLC39A10 is also mentioned in a patent application reporting long lists of differentially transcribed genes in tumor cells by using genome-scale transcription profile analysis (e.g. in Publication Number: US20070237770A1). Again, no data are given documenting the expression of SLC39A10 in tumor at protein level. The lack of correlation between mRNA and protein expression, besides being a general fact, has been specifically demonstrated for LIV-1, another member of the zinc transporter family, suggesting that a similar phenomenon could be extended to other proteins of this class (7).

Finally, no evidence exists on the association of SLC39A10 protein with other tumors, such as lung tumor classes.

In the present invention we disclose SLC39A10 as a protein without previous known association with lung tumor classes and preferably used as a marker lung tumors and in general for cancers of these types. As described below, an antibody generated towards the SLC39A10 protein shows a selective immunoreactivity in histological preparation of lung cancer tissues which indicates the presence of SLC39A10 in these cancer samples and makes SLC39A10 protein and its antibody highly interesting tools for specifically distinguishing these cancer types from a normal state.

By localization analysis of cell lines transfected with a SLC39A10 encoding plasmid we show that the protein is exposed on the cell surface and accessible to the binding of specific antibodies. This piece of data indicates that the protein is a target for anticancer therapy being accessible to the action of affinity ligands.

A further aspect of this invention is a method of screening a tissue sample for malignancy, which comprises determining the presence in said sample of at least one of the above-mentioned tumor markers. This method includes detecting either the marker protein, e.g. by means of labeled monoclonal or polyclonal antibodies that specifically bind to the target protein, or the respective mRNA, e.g. by means of polymerase chain reaction techniques such as RT-PCR. The methods for detecting proteins in a tissue sample are known to one skilled in the art and include immunoradiometric, immunoenzymatic or immunohistochemical techniques, such as radioimmunoassays, immunofluorescent assays or enzyme-linked immunoassays. Other known protein analysis techniques, such as polyacrylamide gel electrophoresis (PAGE), Western blot or Dot blot are suitable as well. Preferably, the detection of the protein marker is carried out with the immune-histochemistry technology, particularly by means of High Through-Put methods that allow the analyses of the antibody immune-reactivity simultaneously on different tissue samples immobilized on a microscope slide. Briefly, each Tissue Micro Array (TMA) slide includes tissue samples suspected of malignancy taken from different patients, and an equal number of normal tissue samples from the same patients as controls. The direct comparison of samples by qualitative or quantitative measurement, e.g. by enzimatic or colorimetric reactions, allows the identification of tumors.

In one embodiment, the invention provides a method of screening a sample of lung tissue for malignancy, which comprises determining the presence in said sample of the SLC39A10 protein tumor marker, variants or isoforms thereof as described above.

A further aspect of the invention is a method *in vitro* for determining the presence of a tumor in a subject, which comprises the steps of:
- providing a sample of the tissue suspected of containing tumor cells;
- determining the presence of a tumor marker as above defined, or a combination thereof in said tissue sample by detecting the expression of the marker protein or the presence of the respective mRNA transcript;
wherein the detection of one or more tumor markers in the tissue sample is indicative of the presence of tumor in said subject.

The methods and techniques for carrying out the assay are known to one skilled in the art and are preferably based on immunoreactions for detecting proteins and on PCR methods for the detection of mRNAs. The same methods for detecting proteins or mRNAs from a tissue sample as disclosed above can be applied.

Herein disclosed is an antibody or a fragment thereof, which is able to specifically recognize and bind to one of the tumor-associated proteins described above. The term "antibody" as used herein refers to all types of immunoglobulins, including IgG, IgM, IgA, IgD and IgE. Such antibodies may include polyclonal, monoclonal, chimeric, single chain, antibodies or fragments such as Fab or scFv. The antibodies may be of various origin, including human, mouse, rat, rabbit and horse, or chimeric antibodies. The production of antibodies is well known in the art. For the production of antibodies in experimental animals, various hosts including goats, rabbits, rats, mice, and others, may be immunized by injection with polypeptides of the present invention or any fragment or oligopeptide or derivative thereof which has immunogenic properties or forms a suitable epitope. Monoclonal antibodies may be produced following the procedures described in Kohler and Milstein, Nature 265:495 (1975) or other techniques known in the art.

The antibodies to the tumor markers of the invention can be used to detect the presence of the marker in histologic preparations or to distinguish tumor cells from normal cells. To that purpose, the antibodies may be labeled with radioactive, fluorescent or enzyme labels.

Herein disclosed is also a diagnostic kit containing suitable means for detection, in particular the polypeptides or polynucleotides, antibodies or fragments or derivatives thereof described above, reagents, buffers, solutions and materials needed for setting up and carrying out the immunoassays, nucleic acid hybridization or PCR assays described above. Parts of the kit of the invention can be packaged individually in vials or bottles or in combination in containers or multicontainer units.

### Description of the Figures

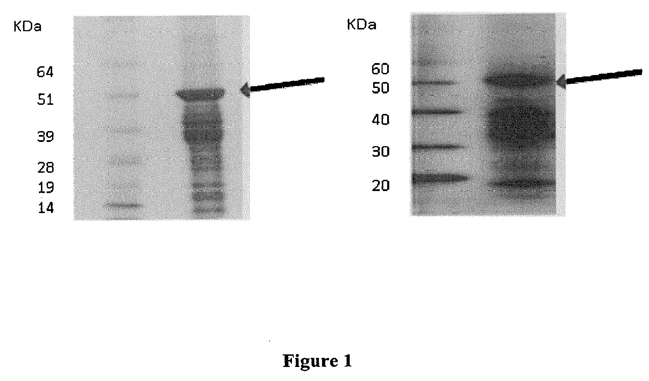

**Figure 1****. Analysis of purified SLC39A10 recombinant protein**
   Left panel: Comassie staining of purified His-tag SLC39A10 fusion protein espressed in *E. coli* separated by SDS-PAGE; Right panel: WB on the purified recombinant SLC39A10 protein stained with anti-SLC39A10 antibody. Arrow marks the protein band of the expected size. The low molecular weight bands correspond to partially degraded forms of SLC39A10 protein. Molecular weight markers are reported on the left
**Figure 2****. Staining of lung tumor TMA with anti-SLC39A10 antibodies**
   Examples of TMA of tumor (lower panel) and normal tissue samples (upper panel) stained with anti- SLC39A10 antibodies. The antibody stains specifically tumor cells (in dark gray).
**Figure 3****. Confocal microscopy analysis of expression and localization of SLC39A10.** HeLa cells transfected with the empty pcDNA3 vector (upper panels) or with the plasmid construct encoding the SLC39A10 gene (lower panels) stained with the secondary antibody (left panels) and with anti-SLC39A10 antibodies (right panels). Arrowheads mark surface specific localization.
**Figure 4****. Expression and localization of SLC39A10 in tumor cells**
   Flow cytometry analysis of SLC39A10 cell surface localization in HOP-92 tumor cells stained with negative control antibody (filled curve or with anti-SLC39A10 antibody (empty curve). X axis, Fluorescence scale; Y axis, Cells (expressed as % relatively to major peaks).

The following examples further illustrate the invention.

### EXAMPLES

### Example 1. Generation of recombinant human protein antigens and antibodies to identify tumor markers

### Methods

The entire coding region or suitable fragments of the genes encoding the target proteins, were designed for cloning and expression using bioinformatic tools with the human genome sequence as template (Lindskog M et al (2005). Where present, the leader sequence for secretion was replaced with the ATG codon to drive the expression of the recombinant proteins in the cytoplasm of E. coli. For cloning, genes were PCR-amplified from templates derived from the Mammalian Gene Collection (http://mgc.nci.nih.gov/) clones or from cDNAs mixtures generated from pools of total RNA derived from Human testis, Human placenta, Human bone marrow, Human fetal brain, using specific primers. Clonings were designed so as to fuse a 10 histidine tag sequence at the 5' end, annealed to in house developed vectors, derivatives of vector pSP73 (Promega) adapted for the T4 ligation independent cloning method (Nucleic Acids Res. 1990 October 25; 18(20): 6069-6074) and used to transform *E.coli* NovaBlue cells recipient strain. *E. coli* tranformants were plated onto selective LB plates containing 100 µg/ml ampicillin (LB Amp) and positive E.coli clones were identified by restriction enzyme analysis of purified plasmid followed by DNA sequence analysis. For expression, plasmids were used to transform BL21-(DE3) *E.coli* cells and BL21-(DE3) E. coli cells harbouring the plasmid were inoculated in ZYP-5052 growth medium (Studier, 2005) and grown at 37°C for 24 hours. Afterwards, bacteria were collected by centrifugation, lysed into B-Per Reagent containing 1 mM MgCl2, 100 units DNAse I (Sigma), and 1 mg/ml lysozime (Sigma). After 30 min at room temperature under gentle shaking, the lysate was clarified by centrifugation at 30.000 g for 40 min at 4°C. All proteins were purified from the inclusion bodies by resuspending the pellet coming from lysate centrifugation in 40 mM TRIS-HCl, 1 mM TCEP {Tris(2-carboxyethyl)-phosphine hydrochloride, Pierce} and 6M guanidine hydrochloride, pH 8 and performing an IMAC in denaturing conditions. Briefly, the resuspended material was clarified by centrifugation at 30.000 g for 30 min and the supernatant was loaded on 0.5 ml columns of Ni-activated Chelating Sepharose Fast Flow (Pharmacia). The column was washed with 50 mM TRIS-HCl buffer, 1 mM TCEP, 6M urea, 60 mM imidazole, 0.5M NaCl, pH 8. Recombinant proteins were eluted with the same buffer containing 500 mM imidazole. Proteins were analysed by SDS-Page and their concentration was determined by Bradford assay using the BIORAD reagent (BIORAD) with a bovine serum albumin standard according to the manufacturer's recommendations.

To generate antisera, the purified proteins were used to immunize CD1 mice (6 week-old females, Charles River laboratories, 5 mice per group) intraperitoneally, with 3 protein doses of 20 micrograms each, at 2 week-interval. Freund's complete adjuvant was used for the first immunization, while Freund's incomplete adjuvant was used for the two booster doses. Two weeks after the last immunization animals were bled and sera collected from each animal was pooled.

### Results

Gene fragments of the expected size were obtained by PCR from specific clones of the Mammalian Gene Collection or, alternatively, from cDNA generated from pools of total RNA derived from Human testis, Human placenta, Human bone marrow, Human fetal brain using primers specific for each gene.

For the SLC39A10 gene, a DNA fragment corresponding to nucleotides 154-1287 of the transcript ENST00000359634 and encoding a protein of 378 residues, corresponding to the amino acid region from 26 to 403 of ENSP00000352656 sequence was obtained.

A clone encoding the correct amino acid sequence was identified for each gene/gene fragment and, upon expression in *E. coli*, a protein of the correct size was produced and subsequently purified using affinity chromatography (Figure 1, left panel). Antibodies generated by immunization specifically recognized their target proteins in Western blot (WB) (Figure 1 right panel).

### Example 2. Tissue profiling by immune-histochemistry

### Methods

The analysis of the antibodies' capability to recognize their target proteins in tumor samples was carried out by Tissue Micro Array (TMA), a miniaturized immuno-histochemistry technology suitable for HTP analysis that allows to analyse the antibody immuno-reactivity simultaneously on different tissue samples immobilized on a microscope slide.

Since the TMAs include both tumor and healthy tissues, the specificity of the antibodies for the tumors can be immediately appreciated. The use of this technology, differently from approaches based on transcription profile, has the important advantage of giving a first-hand evaluation on the potential of the markers in clinics. Conversely, since mRNA levels not always correlate with protein levels (approx. 50% correlation), studies based on transcription profile do not provide solid information regarding the expression of protein markers.

A tissue microarray was prepared containing formalin-fixed paraffin-embedded cores of human tissues from patients affected by lung cancer and corresponding normal tissues as controls and analyzed using the specific antibody sample. In total, a TMA design consisted in 10 tumor lung tumor samples and 10 normal tissues from 5 well pedigreed patients (equal to two tumor samples and 2 normal tissues from each patient) to identify promising target molecules differentially expressed in cancer and normal cells. The direct comparison between tumor and normal tissues of each patient allowed the identification of antibodies that stain specifically tumor cells and provided indication of target expression in lung tumor. The association of each protein with lung tumor was confirmed on a tissue microarray containing 100 formalin-fixed paraffin-embedded cores of human lung tumor tissues from 50 patients (equal to two tissue samples from each patient).

All formalin fixed, paraffin embedded tissues used as donor blocks for TMA production were selected from the archives at the IEO (Istituto Europeo Oncologico, Milan). Corresponding whole tissue sections were examined to confirm diagnosis and tumor classification, and to select representative areas in donor blocks. Normal tissues were defined as microscopically normal (non- neoplastic) and were generally selected from specimens collected from the vicinity of surgically removed tumors. The TMA production was performed essentially as previously described (Kononen J et al (1998) Nature Med. 4:844-847; Kallioniemi OP et al (2001) Hum. MoI. Genet. 10:657-662). Briefly, a hole was made in the recipient TMA block. A cylindrical core tissue sample (1 mm in diameter) from the donor block was acquired and deposited in the recipient TMA block. This was repeated in an automated tissue arrayer "Galileo TMA CK 3500" (BioRep, Milan) until a complete TMA design was produced. TMA recipient blocks were baked at 42 <0>C for 2 h prior to sectioning. The TMA blocks were sectioned with 2-3 mm thicknes using a waterfall microtome (Leica), and placed onto poli-L-lysinated glass slides for immunohistochemical analysis. Automated immunohistochemistry was performed as previously described (Kampf C. et al (2004) Clin. Proteomics 1:285-300). In brief, the glass slides were incubated for 30' min in 60°C, de-paraffinized in xylene (2 x 15 min) using the Bio-Clear solution (Midway. Scientific, Melbourne, Australia), and re-hydrated in graded alcohols. For antigen retrieval, slides were immersed 0.01 M Na-citrate buffer, pH 6.0 at 99°C for 30 min Slides were placed in the Autostainer (R) (DakoCytomation) and endogenous peroxidase was initially blocked with 3% H2O2, for 5 min. Slides were then blocked in Dako Cytomation Wash Buffer containing 5% Bovine serum albumin (BSA) and subsequently incubated with mouse antibodies for 30' (dilution 1:200 in Dako Real ^{™} dilution buffer). After washing with DakoCytomation wash buffer, slides were incubated with the goat anti-mouse peroxidase conjugated Envision(R) for 30 min each at room temperature (DakoCytomation). Finally, diaminobenzidine (DakoCytomation) was used as chromogen and Harris hematoxylin (Sigma- Aldrich) was used for counterstaining. The slides were mounted with Pertex(R) (Histolab).

The staining results have been evaluated by a trained pathologist at the light microscope, and scored according to both the percentage of immunostained cells and the intensity of staining. The individual values and the combined score (from 0 to 300) were recorded in a custom-tailored database. Digital images of the immunocytochemical findings have been taken at a Leica DM LB light microscope, equipped with a Leica DFC289 color camera.

### Results

TMA designs were obtained representing lung tumor tissue samples and normal controls derived from patients affected by lung tumor. The results from tissue profiling showed that the antibodies specific for the recombinant proteins (see Example 1) are strongly immunoreactive on lung cancer tissues, while no or poor reactivity was detected in normal tissues, indicating the presence of the target proteins in lung tumors. Based on this finding, the detection of target proteins in tissue samples can be associated with lung tumor. In some cases IHC staining accumulates at the plasma membrane of tumor cells, providing a first-hand indication on the localization of the target proteins.

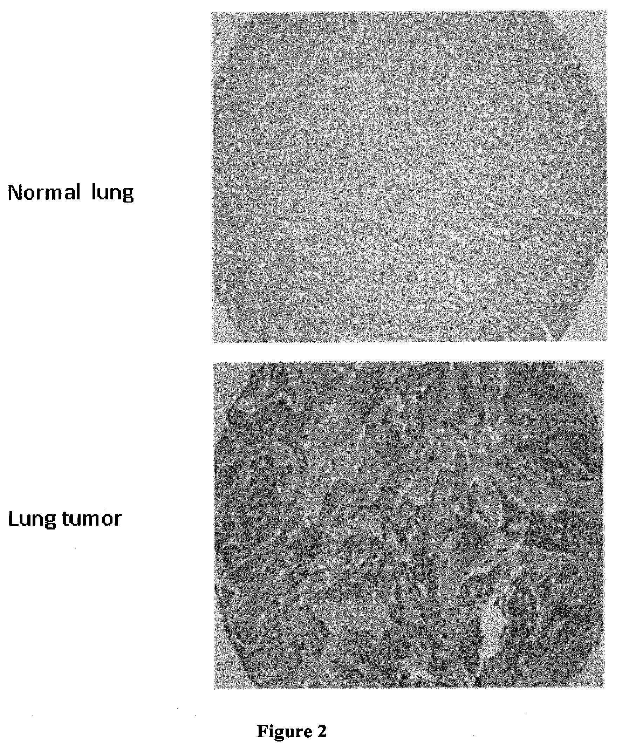

The capability of target-specific antibodies to stain lung tumor tissues is summarized in Table I. Representative examples of microscopic enlargements of antibody-stained tissue samples are reported in Figure 2).

The table reports the number of positive lung tumor tissue samples after staining with the target specific antibodies.

| **Table I** | |
|---|---|
| **Marker name** | **Percentage of lung tumor samples showing positive IHC staining** |
| SLC39A10 | 49 |

### Example 3. Expression of target protein in transfected mammalian cells

### Methods

The expression of target proteins was assessed on eukaryotic cells transiently transfected with plasmid constructs containing the complete coding sequences of the genes encoding the target proteins by Western blot or confocal microscopy. Examples of this type of experiments are given for SLC39A10 (cloned sequence corresponding to Transcript ENST00000359634).

For clonings, cDNA were generated from pools of total RNA derived from Human testis, Human placenta, Human bone marrow, Human fetal brain, in reverse transcription reactions and the entire coding regions were PCR-amplified with specific primers pairs. PCR products were cloned into plasmid pcDNA3 (Invitrogen). HeLa cells were grown in DMEM-10% FCS supplemented with 1 mM Glutamine were transiently transfected with preparation of the resulting plasmids and with the empty vector as negative control using the Lipofectamine-2000 transfection reagent (Invitrogen). After 48 hours, cells were collected and analysed by Western blot or confocal microscopy. For Western blot, cells were lysed with PBS buffer containing 1% Triton X100 and total cell extracts (corresponding to 1x10⁶ cells) were separated on pre-cast SDS-PAGE gradient gels (NuPage 4-12% Bis-Tris gel, Invitrogen) under reducing conditions, followed by electro-transfer to nitrocellulose membranes (Invitrogen) according to the manufacturer's recommendations. The membranes were blocked in blocking buffer composed of 1x PBS-0.1% Tween 20 (PBST) added with 10% dry milk, for 1 h at room temperature, incubated with the antibody diluted 1:2500 in blocking buffer containing 1% dry milk and washed in PBST-1%. The secondary HRP-conjugated antibody (goat anti-mouse immunoglobulin/HRP, Perkin Elmer) was diluted 1:5000 in blocking buffer and chemiluminescence detection was carried out using a Chemidoc-IT UVP CCD camera (UVP) and the Western lightning^{Tm} cheminulescence Reagent Plus (Perkin Elmer), according to the manufacturer's protocol.

For confocal microscopy analysis, the cells were plated on glass cover slips and after 48 h were washed with PBS and fixed with 3% *p*-formaldheyde solution in PBS for 20 min at RT. For surface staining, cells were incubated overnight at 4°C with polyclonal antibodies (1:200). The cells were then stained with Alexafluor 488-labeled goat anti-mouse antibodies (Molecular Probes). DAPI (Molecular Probes) was used to visualize nuclei; Live/Dead® red fixable (Molecular Probes) was used to visualize membrane. The cells were mounted with glycerol plastine and observed under a laser-scanning confocal microscope (LeicaSP5).

### Results

The complete coding sequence for the target proteins were cloned in a eukaryotic expression vector and used for transient transfection of HeLa or HEK-293T cells.

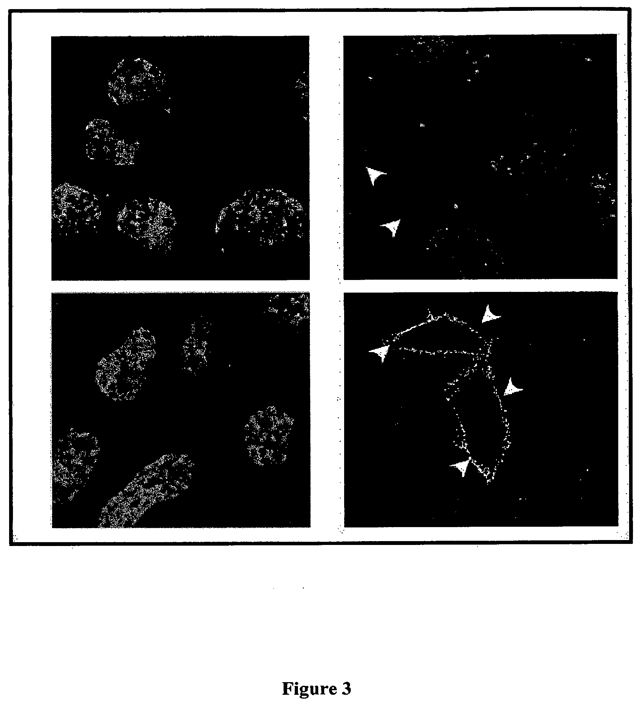

Expression and localization of protein SLC3910 was carried on transfected cells by confocal microscopy. The analysis showed that the anti-SLC39A10 antibodies specifically detected the protein on the surface of transfected cells, while marginal staining was visible in cells transfected with empty vector (Figure 3).

### Example 4. Expression of target proteins in tumor cell lines

Expression of target proteins was assessed by WB and/or flow cytometry analysis of lung tumor cell lines. Cells were cultured in under ATCC recommended conditions, and sub-confluent cell monolayers were detached with PBS-0.5 mM EDTA for subsequent analysis. For Western blot, cells were lysed by several freeze-thaw passages in PBS-1% Triton. Total protein extracts were loaded on SDS-PAGE (1x10⁶ cells/lane), and subjected to WB with specific antibodies as described above.

For flow cytometry analysis cells (2x10⁴ per well) were pelletted in 96 U-bottom microplates by centrifugation at 200 x g for 5 min at 4°C and incubated for 1 hour at 4°C with the appropriate dilutions of the marker-specific antibodies. The cells were washed twice in PBS-5% FCS and incubated for 20 min with the appropriate dilution of R-Phycoerythrin (PE)-conjugated secondary antibodies (Jackson Immuno Research, PA, USA) at 4°C. After washing, cells were analysed by a FACS Canto II flow cytometer (Becton Dickinson). Data were analyzed with FlowJo 8.3.3 program.

### Results

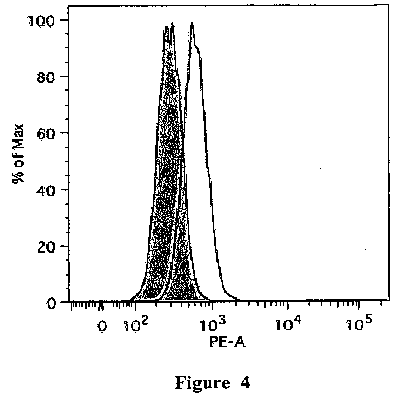

For SLC39A10, expression and localization analysis was performed by surface staining and flow cytometry analysis of tumor cells lines, showing that the proteins are detected on the cell surface. Figure 4 shows surface staining of the HOP-92 cell line with anti-SLC39A10 antibodies.

### Example 5. Expression of the marker proteins confer malignant cell phenotype

To verify that the proteins included in the present invention can be exploited as targets for therapeutic applications, the effect of marker depletion was evaluated *in vitro* in cellular studies generally used to define the role of newly discovered proteins in tumor development. Marker-specific knock-down and control tumor cell lines were assayed for proliferation and migration/invasiveness properties using the MTT and the Boyden in vitro invasion assays, respectively.

### Method

Expression of marker genes were silenced in tumor cell lines by the siRNA technology and the influence of the reduction of marker expression on cell parameters relevant for tumor development was assessed in *in vitro* assays.

The expression of marker genes was knocked down in a panel of epithelial tumor cell lines previously shown to express the tumor markers using a panel of marker-specific siRNAs using the HiPerfect transfection reagent (QIAGEN) following the manufacturer's protocol. As control, cells treated with irrelevant siRNA (scrambled siRNA) were analysed in parallel. At different time points (ranging from 24 to 72 hours) post transfection, the reduction of gene transcription was assessed by quantitative RT-PCR (Q-RT-PCR) on total RNA, by evaluating the relative marker transcript level, using the beta-actin, GAPDH or MAPK genes as internal normalization control. Afterwards, cell proliferation and migration/invasiveness assays were carried out to assess the effect of the reduced marker expression. Cell proliferation was determined using the MTT assay, a colorimetric assay based on the cellular conversion of a tetrazolium salt into a purple colored formazan product. Absorbance of the colored solution can be quantified using a spectrophotometer to provide an estimate of the number of attached living cells. Approximately 5 x 10³ cells/100µl were seeded in 96-well plates in DMEM with 10% FCS to allow cell attachment. After overnight incubation with DMEM without FCS, the cells were treated with 2,5% FBS for 72 hours. Four hours before harvest 15 µL of the MTT dye solution (Promega) were added to each well. After 4-hour incubation at 37°C, the formazan precipitates were solubilized by the addition of 100 µL of solubilization solution (Promega) for 1h at 37°C. Absorbance at 570 nm was determined on a multiwell plate reader (SpectraMax, Molecular Devices).

Cell migration/invasiveness was tested using the Boyden *in vitro* invasion assay, as compared to control cell lines treated with a scramble siRNA. This assay is based on a chamber of two medium-filled compartments separated by a microporous membrane. Cells are placed in the upper compartment and are allowed to migrate through the pores of the membrane into the lower compartment, in which chemotactic agents are present. After an appropriate incubation time, the membrane between the two compartments is fixed and stained, and the number of cells that have migrated to the lower side of the membrane is determined. For this assay, a transwell system, equipped with 8-µm pore polyvinylpirrolidone-free polycarbonate filters, was used. The upper sides of the porous polycarbonate filters were coated with 50 µg/cm² of reconstituted Matrigel basement membrane and placed into six-well culture dishes containing complete growth medium. Cells (1x10⁴ cells/well) were loaded into the upper compartment in serum-free growth medium. After 16 h of incubation at 37°C, non-invading cells were removed mechanically using cotton swabs, and the microporous membrane was stained with Diff-Quick solution. Chemotaxis was evaluated by counting the cells migrated to the lower surface of the polycarbonate filters (six randomly chosen fields, mean± SD).

### References

1) Anderson, L., and Seilhamer, J. (1997). A comparison of selected mRNA and protein abundances in human liver. Electrophoresis 18, 533 - 537;
2) Chen, G., Gharib, T. G., Wang, H., Huang, C. C., Kuick, R., Thomas, D. G., Shedden, K. A., Misek, D. E., Taylor, J. M., Giordano, T. J., Kardia, S. L., Iannettoni, M. D., Yee, J., Hogg, P. J., Orringer, M. B., Hanash, S. M., and Beer, D. G. (2003) Protein profiles associated with survival in lung adenocarcinoma. Proc. Natl. Acad. Sci. U. S. A 100, 13537 - 13542;
3) Ginestier, C., Charafe-Jauffret, E., Bertucci, F., Eisinger, F., Geneix, J., Bechlian, D., Conte, N., Adelaide, J., Toiron, Y., Nguyen, C., Viens, P., Mozziconacci, M. J., Houlgatte, R., Birnbaum, D., and Jacquemier, J. (2002) Distinct and complementary information provided by use of tissue and DNA microarrays in the study of breast tumor markers. Am. J. Pathol. 161, 1223 -1233;
4) Gygi, S. P., Rochon, Y., Franza, B. R., and Aebersold, R. (1999) Correlation between protein and mRNA abundance in yeast. Mol. Cell. Biol. 19, 1720 -1730; Nishizuka, S., Charboneau, L., Young, L., Major, S., Reinhold, W. C., Waltham, M., Kouros-Mehr, H., Bussey, K. J., Lee, J. K., Espina, V., Munson, P. J., Petricoin, E., III, Liotta, L. A., and Weinstein, J. N. (2003) Proteomic profiling of the NCI-60 cancer cell lines using new high-density reverse-phase lysate microarrays. Proc. Natl. Acad. Sci. U. S. A 100, 14229 - 14234;
5) Tyers, M., and Mann, M. (2003); From genomics to proteomics. Nature 422, 193 - 197;
   Nguyen ST, Hasegawa S, Tsuda H, Tomioka H, Ushijima M, Noda M, Omura K, Miki Y, (2007) Identification of a predictive gene expression signature of cervical lymph node metastasis in oral squamous cell carcinoma., Cancer Sci. 98:740-746;
6) Kagara N, Tanaka N, Noguchi S, Hirano T. (2007) Zinc and its transporter ZIP 10 are involved in invasive behavior of breast cancer cells. Cancer Sci. 98:692-697;
7) Kasper G, Weiser AA, Rump A, Sparbier K, Dahl E, Hartmann A, Wild P, Schwidetzky U, Castaños-Vélez E, Lehmann K. (2005) Expression levels of the putative zinc transporter LIV-1 are associated with a better outcome of breast cancer patients. Int J Cancer. 117:961-973;

## Claims

1. The use of:
SLC39A10, in one of its variant isoforms SEQ ID NO:15, SEQ ID NO:16 or a different isoform having sequence identity of at least 80% to SEQ ID NO:15 or SEQ ID NO:16, or a nucleic acid molecule containing a sequence coding for a SLC39A10 protein,
as a tumor marker for *in vitro* detection of lung cancer.

2. The use according to claim 1, wherein said isoform has a sequence identity of at least 90% to SEQ ID NO:15 or SEQ ID NO:16.

3. The use according to claim 1, wherein said isoform has a sequence identity of at least 95% to SEQ ID NO:15 or SEQ ID NO:16.

4. The use according to claim 1, wherein said encoding sequence is selected from SEQ ID NO:17 and SEQ ID NO:18.

5. A method of screening a sample of lung tissue for malignancy, said method comprising determining the presence in said sample of a tumor marker according to claims 1-4.

6. A method according to claim 5, wherein the tumor marker is a protein, said method being based on immunoradiometric, immunoenzymatic or immunohistochemical techniques.

7. A method according to claim 5, wherein the tumor marker is a nucleic acid molecule, said method being based on polymerase chain reaction techniques.

8. A method *in vitro* for determining the presence of a lung tumor in a subject, which comprises the steps of:
(a) using a sample of the tissue suspected of containing tumor cells;
(b) determining the presence of a tumor marker according to claim 1 in said tissue sample by detecting the expression of the marker protein or the presence of the respective mRNA transcript;
wherein the detection of the tumor marker in the tissue sample is indicative of the presence of tumor in said subject.

## Patentansprüche

1. Verwendung von:
SLC39A10 in einer seiner varianten Isoformen SEQ ID NO:15, SEQ ID NO:16 oder einer anderen Isoform mit Sequenz-identLität von mindestens 80% mit SEQ ID NO:15 oder SEQ ID NO:16, oder einem Nucleinsäuremolekül, welches eine Sequenz enthält, die für ein SLC39A10-Protein codiert,
als Tumormarker zur In-vitro-Detektion von Lungenkrebs.

2. Verwendung gemäß Anspruch 1, wobei die Isoform eine Sequenzidentität von mindestens 90% mit SEQ ID NO:15 oder SEQ ID NO:16 besitzt.

3. Verwendung gemäß Anspruch 1, wobei die Isoform eine Sequenzidentität von mindestens 95% mit SEQ ID NO:15 oder SEQ ID NO:16 besitzt.

4. Verwendung gemäß Anspruch 1, wobei die codierende Sequenz aus SEQ ID NO:17 und SEQ ID NO:18 ausgewählt wird.

5. Verfahren zum Screenen einer Probe von Lungengewebe auf Malignität, wobei das Verfahren Bestimmen der Gegenwart.eines Tumormarkers gemäß den Ansprüchen 1-4 in der Probe umfasst.

6. Verfahren gemäß Anspruch 5, wobei der Tumormarker ein Protein ist, wobei das Verfahren auf immunradiometrischen, immunenzymatischen oder immunhistochemischen Techniken basiert.

7. Verfahren gemäß Anspruch 5, wobei der Tumormarker ein Nucleinsäuremolekül ist, und das Verfahren auf Polymerasekettenreaktionstechniken basiert.

8. *In-vitro-*Verfahren zur Bestimmung der Gegenwart eines Lungentumors in einem Subjekt, welches die folgenden Schritte umfasst:
(a) Verwenden einer Probe des Gewebes, welches vermutlich Tumorzellen enthält,
(b) Bestimmen der Gegenwart eines Tumormarkers gemäß Anspruch 1 in der Gewebeprobe durch Detektieren der Expression des Markerproteins oder der Gegenwart des entsprechenden mRNA-Transkripts,
wobei die Detektion des Tumormarkers in der Gewebeprobe die Gegenwart eines Tumors in dem Subjekt anzeigt.

## Revendications

1. Utilisation de :
SLC39A10, sous l'un de ses isoformes variants SEQ ID NO: 15, SEQ ID NO: 16 ou un isoforme différent ayant une identité de séquence d'au moins 80 % à SEQ ID NO: 15 ou SEQ ID NO: 16, ou une molécule d'acide nucléique contenant une séquence codant pour une protéine SLC39A10, en tant que marqueur tumoral pour la détection *in vitro* du cancer du poumon.

2. Utilisation selon la revendication 1, dans laquelle ledit isoforme présente une identité de séquence d'au moins 90 % à SEQ ID NO: 15 ou SEQ ID NO: 16.

3. Utilisation selon la revendication 1, dans laquelle ledit isoforme présente une identité de séquence d'au moins 95 % à SEQ ID NO: 15 ou SEQ ID NO: 16.

4. Utilisation selon la revendication 1, dans laquelle ladite séquence codante est choisie parmi SEQ ID NO: 17 et SEQ ID NO: 18.

5. Procédé de criblage d'un échantillon de tissu pulmonaire à la recherche d'une malignité, ledit procédé comprenant la détermination de la présence dans ledit échantillon d'un marqueur tumoral selon les revendications 1 à 4.

6. Procédé selon la revendication 5, dans lequel le marqueur tumoral est une protéine, ledit procédé étant basé sur des techniques immunoradiométriques, immunoenzymatiques ou immunohistochimiques.

7. Procédé selon la revendication 5, dans lequel le marqueur tumoral est une molécule d'acide nucléique, ledit procédé étant basé sur des techniques de réaction en chaîne par polymérase.

8. Procédé *in vitro* pour déterminer la présence d'une tumeur du poumon chez un sujet, qui comprend les étapes de :
(a) utilisation d'un échantillon du tissu soupçonné de contenir des cellules tumorales ;
(b) détermination de la présence d'un marqueur tumoral selon la revendication 1 dans ledit échantillon de tissu par détection de l'expression de la protéine marqueur ou de la présence du transcrit d'ARNm respectif ;
dans lequel la détection du marqueur tumoral dans l'échantillon de tissu est une indication de la présence d'une tumeur chez ledit sujet.


### SEQUENCE LISTING

<110> EXTERNAUTICS S.P.A.
<120> LUNG TUMOR MARKERS AND METHODS OF USE THEREOF
<130> 1299PCT
<160> 100
<170> PatentIn version 3.3
<210> 1
   <211> 157
   <212> PRT
   <213> Homo sapiens
<400> 1
   Met Leu Pro Glu Gln Gly Pro Gln Pro Ser Thr Met Pro Leu Trp Cys
   1               5                   10                  15
   Leu Leu Ala Ala Cys Thr Ser Leu Pro Arg Gln Ala Ala Thr Met Leu
               20                  25                  30
   Glu Glu Ala Ala Ser Pro Asn Glu Ala Val His Ala Ser Thr Ser Gly
           35                  40                  45
   Ser Gly Ala Leu Thr Asp Gln Thr Phe Thr Asp Leu Ser Ala Ala Glu
       50                  55                  60
   Ala Ser Ser Glu Glu Val Pro Asp Phe Met Glu Val Pro His Ser Val
   65                  70                  75                  80
   His His Lys Ile Asn Cys Phe Phe Tyr Leu Glu Lys Gln Leu Cys Gln
                   85                  90                  95
   Leu Pro Ser Pro Leu Cys Leu Ser Ser Leu Leu Thr Leu Lys Leu Lys
               100                 105                 110
   Thr Thr Val Pro Ala Pro Gly Arg Trp Trp Ser Phe Gln Pro His Lys
           115                 120                 125
   Ala Phe Pro Leu Leu Val Gly Thr Pro Gly Ser Trp Gln Ser Thr Ile
       130                 135                 140
   Asp Pro Ala Trp Ala Ala Pro Ser Gln Pro Ser Pro Gly
   145                 150                 155
<210> 2
   <211> 474
   <212> DNA
   <213> Homo sapiens
<400> 2
   atgcttccag agcaagggcc ccagccttcc acgatgccgc tctggtgcct cctcgccgcc      60
   tgcaccagcc tcccaaggca ggcagccacc atgctggagg aagctgcttc tcccaacgag     120
   gctgtccacg catcaacatc aggcagtggc gcactcactg atcagacatt tacagacctc     180
   tcagctgccg aggcctcctc agaggaggtt cctgacttca tggaggtgcc acactctgtt     240
   caccataaaa ttaactgctt tttctactta gaaaaacaac tctgccaact gccgtcccca     300
   ctgtgtctaa gcagcttgct tactttaaaa ttaaaaacaa cggtcccagc tcctggcagg     360
   tggtggagct tccagcctca caaggcattc ccacttctgg tgggcactcc tggaagctgg     420
   cagagcacaa tcgatcccgc gtgggcggcc ccctctcagc caagcccagg gtga           474
<210> 3
   <211> 147
   <212> PRT
   <213> Homo sapiens
<400> 3
   Met Gly Phe Ile Phe Ser Lys Ser Met Asn Glu Ser Met Lys Asn Gln
   1               5                   10                  15
   Lys Glu Phe Met Leu Met Asn Ala Arg Leu Gln Leu Glu Arg Gln Leu
               20                  25                  30
   Ile Met Gln Ser Glu Met Arg Glu Arg Gln Met Ala Met Gln Ile Ala
           35                  40                  45
   Trp Ser Arg Glu Phe Leu Lys Tyr Phe Gly Thr Phe Phe Gly Leu Ala
       50                  55                  60
   Ala Ile Ser Leu Thr Ala Gly Ala Ile Lys Lys Lys Lys Pro Ala Phe
   65                  70                  75                  80
   Leu Val Pro Ile Val Pro Leu Ser Phe Ile Leu Thr Tyr Gln Tyr Asp
                   85                  90                  95
   Leu Gly Tyr Gly Thr Leu Leu Glu Arg Met Lys Gly Glu Ala Glu Asp
               100                 105                 110
   Ile Leu Glu Thr Glu Lys Ser Lys Leu Gln Leu Pro Arg Gly Met Ile
           115                 120                 125
   Thr Phe Glu Ser Ile Glu Lys Ala Arg Lys Glu Gln Ser Arg Phe Phe
       130                 135                 140
   Ile Asp Lys
   145
<210> 4
   <211> 932
   <212> DNA
   <213> Homo sapiens
<400> 4
   gagcgaggcc cggtccctgc agcgggcgaa aggagcccgg gcctggaggt ttgcgtaccg     60
   gtcgcctggt cccggcacca gcgccgccca gtgtggtttc ccataaggaa gctcttcttc    120
   ctgcttggct tccaccttta acccttccac ctgggagcgt cctctaacac attcagacta    180
   caagtccaga cccaggagag caaggcccag aaagaggtca aaatggggtt tatattttca    240
   aaatctatga atgaaagcat gaaaaatcaa aaggagttca tgcttatgaa tgctcgactt    300
   cagctggaaa ggcagctcat catgcagagt gaaatgaggg aaagacaaat ggccatgcag    360
   attgcgtggt ctcgggaatt cctcaaatat tttggaactt tttttggcct tgcagccatc    420
   tctttaacag ctggagcgat taaaaaaaag aagccagcct tcctggtccc gattgttcca    480
   ttaagcttta tcctcaccta ccagtatgac ttgggctatg gaaccctttt agaaagaatg    540
   aaaggtgaag ctgaggacat actggaaaca gaaaagagta aattgcagct gccaagagga    600
   atgatcactt ttgaaagcat tgaaaaagcc agaaaggaac agagtagatt cttcatagac    660
   aaatgaaatc atgcttacca atcaaatctc aaagcacaga attattgact tgaatcatgg    720
   tttttacagt tttttaaatg ctcaagattt tgatattata gattttattt taaaatatta    780
   aaatgcaaga tagttttgag ctattttaaa ataaaattta taacattcaa cacaaaatca    840
   tggaggtgct ctaaataact tttagatttc ctctctctgt gtgcattacc aatatctaag    900
   tgtaaaatta ataaattgtt ttgaattcct gg                                  932
<210> 5
   <211> 543
   <212> PRT
   <213> Homo sapiens
<400> 5
<210> 6
   <211> 938
   <212> PRT
   <213> Homo sapiens
<400> 6
   Met Pro Ala Leu Glu His Met Asn Gln Ile Leu His Ile Leu Phe Val
   1               5                   10                  15
   Phe Leu Pro Phe Leu Trp Ala Leu Gly Thr Leu Pro Pro Pro Asp Ala
               20                  25                  30
   Leu Leu Leu Trp Ala Met Glu Gln Val Leu Glu Phe Gly Leu Gly Gly
           35                  40                  45
   Ser Ser Met Ser Thr His Leu Arg Leu Leu Val Met Phe Ile Met Ser
       50                  55                  60
   Ala Gly Thr Ala Ile Ala Ser Tyr Phe Ile Pro Ser Thr Val Gly Val
   65                  70                  75                  80
   Val Leu Phe Met Thr Gly Phe Gly Phe Leu Leu Ser Leu Asn Leu Ser
                   85                  90                  95
   Asp Met Gly His Lys Ile Gly Thr Lys Ser Lys Asp Leu Pro Ser Gly
               100                 105                 110
   Pro Glu Lys His Phe Ser Trp Lys Glu Cys Leu Phe Tyr Ile Ile Ile
           115                 120                 125
   Leu Val Leu Ala Leu Leu Glu Thr Ser Leu Leu His His Phe Ala Gly
       130                 135                 140
   Phe Ser Gln Ile Ser Lys Ser Asn Ser Gln Ala Ile Val Gly Tyr Gly
   145                 150                 155                 160
   Leu Met Ile Leu Leu Ile Ile Leu Trp Ile Leu Arg Glu Ile Gln Ser
                   165                 170                 175
   Val Tyr Ile Ile Gly Ile Phe Arg Asn Pro Phe Tyr Pro Lys Asp Val
               180                 185                 190
   Gln Thr Val Thr Val Phe Phe Glu Lys Gln Thr Arg Leu Met Lys Ile
           195                 200                 205
   Gly Ile Val Arg Arg Ile Leu Leu Thr Leu Val Ser Pro Phe Ala Met
       210                 215                 220
   Ile Ala Phe Leu Ser Leu Asp Ser Ser Leu Gln Gly Leu His Ser Val
   225                 230                 235                 240
   Ser Val Cys Ile Gly Phe Thr Arg Ala Phe Arg Met Val Trp Gln Asn
                   245                 250                 255
   Thr Glu Asn Ala Leu Leu Glu Thr Val Ile Val Ser Thr Val His Leu
               260                 265                 270
   Ile Ser Ser Thr Asp Ile Trp Trp Asn Arg Ser Leu Asp Thr Gly Leu
           275                 280                 285
   Arg Leu Leu Leu Val Gly Ile Ile Arg Asp Arg Leu Ile Gln Phe Ile
       290                 295                 300
   Ser Lys Leu Gln Phe Ala Val Thr Val Leu Leu Thr Ser Trp Thr Glu
   305                 310                 315                 320
   Lys Lys Gln Arg Arg Lys Thr Thr Ala Thr Leu Cys Ile Leu Asn Ile
                   325                 330                 335
   Val Phe Ser Pro Phe Val Leu Val Ile Ile Val Phe Ser Thr Leu Leu
               340                 345                 350
   Ser Ser Pro Leu Leu Pro Leu Phe Thr Leu Pro Val Phe Leu Val Gly
           355                 360                 365
   Phe Pro Arg Pro Ile Gln Ser Trp Pro Gly Ala Ala Gly Thr Thr Ala
       370                 375                 380
   Cys Val Cys Ala Asp Thr Val Tyr Tyr Tyr Gln Met Val Pro Arg Leu
   385                 390                 395                 400
   Thr Ala Val Leu Gln Thr Ala Met Ala Ala Gly Ser Leu Gly Leu Leu
                   405                 410                 415
   Leu Pro Gly Ser His Tyr Leu Gly Arg Phe Gln Asp Arg Leu Met Trp
               420                 425                 430
   Ile Met Ile Leu Glu Cys Gly Tyr Thr Tyr Cys Ser Ile Asn Ile Lys
           435                 440                 445
   Gly Leu Glu Leu Gln Glu Thr Ser Cys His Thr Ala Glu Ala Arg Arg
       450                 455                 460
   Val Asp Glu Val Phe Glu Asp Ala Phe Glu Gln Glu Tyr Thr Arg Val
   465 470 475 480
   Cys Ser Leu Asn Glu His Phe Gly Asn Val Leu Thr Pro Cys Thr Val
   485 490 495
   Leu Pro Val Lys Leu Tyr Ser Asp Ala Arg Asn Val Leu Ser Gly Ile
   500 505 510
   Ile Asp Ser His Glu Asn Leu Lys Glu Phe Lys Gly Asp Leu Ile Lys
   515 520 525
   Val Leu Val Trp Ile Leu Val Gln Tyr Cys Ser Lys Arg Pro Gly Met
   530 535 540
   Lys Glu Asn Val His Asn Thr Glu Asn Lys Gly Lys Ala Pro Leu Met
   545 550 555 560
   Leu Pro Ala Leu Asn Thr Leu Pro Pro Pro Lys Ser Pro Glu Asp Ile
   565 570 575
   Asp Ser Leu Asn Ser Glu Thr Phe Asn Asp Trp Ser Asp Asp Asn Ile
   580 585 590
   Phe Asp Asp Glu Pro Thr Ile Lys Lys Val Ile Glu Glu Lys His Gln
   595 600 605
   Leu Lys Asp Leu Pro Gly Thr Asn Leu Phe Ile Pro Gly Ser Val Glu
   610 615 620
   Ser Gln Arg Val Gly Asp His Ser Thr Gly Thr Val Pro Glu Asn Asp
   625 630 635 640
   Leu Tyr Lys Ala Val Leu Leu Gly Tyr Pro Ala Val Asp Lys Gly Lys
   645 650 655
   Gln Glu Asp Met Pro Tyr Ile Pro Leu Met Glu Phe Ser Cys Ser His
   660 665 670
   Ser His Leu Val Cys Leu Pro Ala Glu Trp Arg Thr Ser Cys Met Pro
   675 680 685
   Ser Ser Lys Met Lys Glu Met Ser Ser Leu Phe Pro Glu Asp Trp Tyr
   690 695 700
   Gln Phe Val Leu Arg Gln Leu Glu Cys Tyr His Ser Glu Glu Lys Ala
   705 710 715 720
   Ser Asn Val Leu Glu Glu Ile Ala Lys Asp Lys Val Leu Lys Asp Phe
                   725                 730                 735
   Tyr Val His Thr Val Met Thr Cys Tyr Phe Ser Leu Phe Gly Ile Asp
               740                 745                 750
   Asn Met Ala Pro Ser Pro Gly His Ile Leu Arg Val Tyr Gly Gly Val
           755                 760                 765
   Leu Pro Trp Ser Val Ala Leu Asp Trp Leu Thr Glu Lys Pro Glu Leu
       770                 775                 780
   Phe Gln Leu Ala Leu Lys Ala Phe Arg Tyr Thr Leu Lys Leu Met Ile
   785                 790                 795                 800
   Asp Lys Ala Ser Leu Gly Pro Ile Glu Asp Phe Arg Glu Leu Ile Lys
                   805                 810                 815
   Tyr Leu Glu Glu Tyr Glu Arg Asp Trp Tyr Ile Gly Leu Val Ser Asp
               820                 825                 830
   Glu Lys Trp Lys Glu Ala Ile Leu Gln Glu Lys Pro Tyr Leu Phe Ser
           835                 840                 845
   Leu Gly Tyr Asp Ser Asn Met Gly Ile Tyr Thr Gly Arg Val Leu Ser
       850                 855                 860
   Leu Gln Glu Leu Leu Ile Gln Val Gly Lys Leu Asn Pro Glu Ala Val
   865                 870                 875                 880
   Arg Gly Gln Trp Ala Asn Leu Ser Trp Glu Leu Leu Tyr Ala Thr Asn
                   885                 890                 895
   Asp Asp Glu Glu Arg Tyr Ser Ile Gln Ala His Pro Leu Leu Leu Arg
               900                 905                 910
   Asn Leu Thr Val Gln Ala Ala Glu Pro Pro Leu Gly Tyr Pro Ile Tyr
           915                 920                 925
   Ser Ser Lys Pro Leu His Ile His Leu Tyr
       930                 935
<210> 7
   <211> 230
   <212> PRT
   <213> Homo sapiens
<400> 7
   Met Ser Pro Asp Val Pro Leu Leu Asn Asp Tyr Lys Gln Asp Phe Phe
   1               5                   10                  15
   Leu Lys Arg Phe Pro Gln Thr Val Leu Gly Gly Pro Arg Phe Lys Leu
               20                  25                  30
   Gly Tyr Cys Ala Pro Pro Tyr Ile Tyr Val Asn Gln Ile Ile Leu Phe
           35                  40                  45
   Leu Met Pro Trp Val Trp Gly Gly Val Gly Thr Leu Leu Tyr Gln Leu
       50                  55                  60
   Gly Ile Leu Lys Asp Tyr Tyr Thr Ala Ala Leu Ser Gly Gly Leu Met
   65                  70                  75                  80
   Leu Phe Thr Ala Phe Val Ile Gln Phe Thr Ser Leu Tyr Ala Lys Asn
                   85                  90                  95
   Lys Ser Thr Thr Val Glu Arg Ile Leu Thr Thr Asp Ile Leu Ala Glu
               100                 105                 110
   Glu Asp Glu His Glu Phe Thr Ser Cys Thr Gly Ala Glu Thr Val Lys
           115                 120                 125
   Phe Leu Ile Pro Gly Lys Lys Tyr Val Ala Asn Thr Val Phe His Ser
       130                 135                 140
   Ile Leu Ala Gly Leu Ala Cys Gly Leu Gly Thr Trp Tyr Leu Leu Pro
   145                 150                 155                 160
   Asn Arg Ile Thr Leu Leu Tyr Gly Ser Thr Gly Gly Thr Ala Leu Leu
                   165                 170                 175
   Phe Phe Phe Gly Trp Met Thr Leu Cys Ile Ala Glu Tyr Ser Leu Ile
               180                 185                 190
   Val Asn Thr Ala Thr Glu Thr Ala Thr Phe Gln Thr Gln Asp Thr Tyr
           195                 200                 205
   Glu Ile Ile Pro Leu Met Arg Pro Leu Tyr Ile Phe Phe Phe Val Ser
       210                 215                 220
   Val Asp Leu Ala His Arg
   225                 230
<210> 8
   <211> 1172
   <212> PRT
   <213> Homo sapiens
<400> 8
   Met Ser Pro Asp Val Pro Leu Leu Asn Asp Tyr Lys Gln Asp Phe Phe
   1               5                   10                  15
   Leu Lys Arg Phe Pro Gln Thr Val Leu Gly Gly Pro Arg Phe Lys Leu
               20                  25                  30
   Gly Tyr Cys Ala Pro Pro Tyr Ile Tyr Val Asn Gln Ile Ile Leu Phe
           35                  40                  45
   Leu Met Pro Trp Val Trp Gly Gly Val Gly Thr Leu Leu Tyr Gln Leu
       50                  55                  60
   Gly Ile Leu Lys Asp Tyr Tyr Thr Ala Ala Leu Ser Gly Gly Leu Met
   65                  70                  75                  80
   Leu Phe Thr Ala Phe Val Ile Gln Phe Thr Ser Leu Tyr Ala Lys Asn
                   85                  90                  95
   Lys Ser Thr Thr Val Glu Arg Ile Leu Thr Thr Asp Ile Leu Ala Glu
               100                 105                 110
   Glu Asp Glu His Glu Phe Thr Ser Cys Thr Gly Ala Glu Thr Val Lys
           115                 120                 125
   Phe Leu Ile Pro Gly Lys Lys Tyr Val Ala Asn Thr Val Phe His Ser
       130                 135                 140
   Ile Leu Ala Gly Leu Ala Cys Gly Leu Gly Thr Trp Tyr Leu Leu Pro
   145                 150                 155                 160
   Asn Arg Ile Thr Leu Leu Tyr Gly Ser Thr Gly Gly Thr Ala Leu Leu
                   165                 170                 175
   Phe Phe Phe Gly Trp Met Thr Leu Cys Ile Ala Glu Tyr Ser Leu Ile
               180                 185                 190
   Val Asn Thr Ala Thr Glu Thr Ala Thr Phe Gln Thr Gln Asp Thr Tyr
           195                 200                 205
   Glu Ile Ile Pro Leu Met Arg Pro Leu Tyr Ile Phe Phe Phe Val Ser
       210                 215                 220
   Val Asp Leu Ala His Arg Phe Val Val Asn Met Pro Ala Leu Glu His
   225                 230                 235                 240
   Met Asn Gln Ile Leu His Ile Leu Phe Val Phe Leu Pro Phe Leu Trp
                   245                 250                 255
   Ala Leu Gly Thr Leu Pro Pro Pro Asp Ala Leu Leu Leu Trp Ala Met
               260                 265                 270
   Glu Gln Val Leu Glu Phe Gly Leu Gly Gly Ser Ser Met Ser Thr His
           275                 280                 285
   Leu Arg Leu Leu Val Met Phe Ile Met Ser Ala Gly Thr Ala Ile Ala
       290                 295                 300
   Ser Tyr Phe Ile Pro Ser Thr Val Gly Val Val Leu Phe Met Thr Gly
   305                 310                 315                 320
   Phe Gly Phe Leu Leu Ser Leu Asn Leu Ser Asp Met Gly His Lys Ile
                   325                 330                 335
   Gly Thr Lys Ser Lys Asp Leu Pro Ser Gly Pro Glu Lys His Phe Ser
               340                 345                 350
   Trp Lys Glu Cys Leu Phe Tyr Ile Ile Ile Leu Val Leu Ala Leu Leu
           355                 360                 365
   Glu Thr Ser Leu Leu His His Phe Ala Gly Phe Ser Gln Ile Ser Lys
       370                 375                 380
   Ser Asn Ser Gln Ala Ile Val Gly Tyr Gly Leu Met Ile Leu Leu Ile
   385                 390                 395                 400
   Ile Leu Trp Ile Leu Arg Glu Ile Gln Ser Val Tyr Ile Ile Gly Ile
                   405                 410                 415
   Phe Arg Asn Pro Phe Tyr Pro Lys Asp Val Gln Thr Val Thr Val Phe
               420                 425                 430
   Phe Glu Lys Gln Thr Arg Leu Met Lys Ile Gly Ile Val Arg Arg Ile
           435                 440                 445
   Leu Leu Thr Leu Val Ser Pro Phe Ala Met Ile Ala Phe Leu Ser Leu
       450                 455                 460
   Asp Ser Ser Leu Gln Gly Leu His Ser Val Ser Val Cys Ile Gly Phe
   465                 470                 475                 480
   Thr Arg Ala Phe Arg Met Val Trp Gln Asn Thr Glu Asn Ala Leu Leu
                   485                 490                 495
   Glu Thr Val Ile Val Ser Thr Val His Leu Ile Ser Ser Thr Asp Ile
               500                 505                 510
   Trp Trp Asn Arg Ser Leu Asp Thr Gly Leu Arg Leu Leu Leu Val Gly
           515                 520                 525
   Ile Ile Arg Asp Arg Leu Ile Gln Phe Ile Ser Lys Leu Gln Phe Ala
       530                 535                 540
   Val Thr Val Leu Leu Thr Ser Trp Thr Glu Lys Lys Gln Arg Arg Lys
   545                 550                 555                 560
   Thr Thr Ala Thr Leu Cys Ile Leu Asn Ile Val Phe Ser Pro Phe Val
                   565                 570                 575
   Leu Val Ile Ile Val Phe Ser Thr Leu Leu Ser Ser Pro Leu Leu Pro
               580                 585                 590
   Leu Phe Thr Leu Pro Val Phe Leu Val Gly Phe Pro Arg Pro Ile Gln
           595                 600                 605
   Ser Trp Pro Gly Ala Ala Gly Thr Thr Ala Cys Val Cys Ala Asp Thr
       610                 615                 620
   Val Tyr Tyr Tyr Gln Met Val Pro Arg Leu Thr Ala Val Leu Gln Thr
   625                 630                 635                 640
   Ala Met Ala Ala Gly Ser Leu Gly Leu Leu Leu Pro Gly Ser His Tyr
                   645                 650                 655
   Leu Gly Arg Phe Gln Asp Arg Leu Met Trp Ile Met Ile Leu Glu Cys
               660                 665                 670
   Gly Tyr Thr Tyr Cys Ser Ile Asn Ile Lys Gly Leu Glu Leu Gln Glu
           675                 680                 685
   Thr Ser Cys His Thr Ala Glu Ala Arg Arg Val Asp Glu Val Phe Glu
       690                 695                 700
   Asp Ala Phe Glu Gln Glu Tyr Thr Arg Val Cys Ser Leu Asn Glu His
   705                 710                 715                 720
   Phe Gly Asn Val Leu Thr Pro Cys Thr Val Leu Pro Val Lys Leu Tyr
                   725                 730                 735
   Ser Asp Ala Arg Asn Val Leu Ser Gly Ile Ile Asp Ser His Glu Asn
               740                 745                 750
   Leu Lys Glu Phe Lys Gly Asp Leu Ile Lys Val Leu Val Trp Ile Leu
           755                 760                 765
   Val Gln Tyr Cys Ser Lys Arg Pro Gly Met Lys Glu Asn Val His Asn
       770                 775                 780
   Thr Glu Asn Lys Gly Lys Ala Pro Leu Met Leu Pro Ala Leu Asn Thr
   785                 790                 795                 800
   Leu Pro Pro Pro Lys Ser Pro Glu Asp Ile Asp Ser Leu Asn Ser Glu
                   805                 810                 815
   Thr Phe Asn Asp Trp Ser Asp Asp Asn Ile Phe Asp Asp Glu Pro Thr
               820                 825                 830
   Ile Lys Lys Val Ile Glu Glu Lys His Gln Leu Lys Asp Leu Pro Gly
           835                 840                 845
   Thr Asn Leu Phe Ile Pro Gly Ser Val Glu Ser Gln Arg Val Gly Asp
       850                 855                 860
   His Ser Thr Gly Thr Val Pro Glu Asn Asp Leu Tyr Lys Ala Val Leu
   865                 870                 875                 880
   Leu Gly Tyr Pro Ala Val Asp Lys Gly Lys Gln Glu Asp Met Pro Tyr
                   885                 890                 895
   Ile Pro Leu Met Glu Phe Ser Cys Ser His Ser His Leu Val Cys Leu
               900                 905                 910
   Pro Ala Glu Trp Arg Thr Ser Cys Met Pro Ser Ser Lys Met Lys Glu
           915                 920                 925
   Met Ser Ser Leu Phe Pro Glu Asp Trp Tyr Gln Phe Val Leu Arg Gln
       930                 935                 940
   Leu Glu Cys Tyr His Ser Glu Glu Lys Ala Ser Asn Val Leu Glu Glu
   945                 950                 955                 960
   Ile Ala Lys Asp Lys Val Leu Lys Asp Phe Tyr Val His Thr Val Met
   965 970 975
   Thr Cys Tyr Phe Ser Leu Phe Gly Ile Asp Asn Met Ala Pro Ser Pro
   980 985 990
   Gly His Ile Leu Arg Val Tyr Gly Gly Val Leu Pro Trp Ser Val Ala
   995 1000 1005
   Leu Asp Trp Leu Thr Glu Lys Pro Glu Leu Phe Gln Leu Ala Leu
   1010 1015 1020
   Lys Ala Phe Arg Tyr Thr Leu Lys Leu Met Ile Asp Lys Ala Ser
   1025 1030 1035
   Leu Gly Pro Ile Glu Asp Phe Arg Glu Leu Ile Lys Tyr Leu Glu
   1040 1045 1050
   Glu Tyr Glu Arg Asp Trp Tyr Ile Gly Leu Val Ser Asp Glu Lys
   1055 1060 1065
   Trp Lys Glu Ala Ile Leu Gln Glu Lys Pro Tyr Leu Phe Ser Leu
   1070 1075 1080
   Gly Tyr Asp Ser Asn Met Gly Ile Tyr Thr Gly Arg Val Leu Ser
   1085 1090 1095
   Leu Gln Glu Leu Leu Ile Gln Val Gly Lys Leu Asn Pro Glu Ala
   1100 1105 1110
   Val Arg Gly Gln Trp Ala Asn Leu Ser Trp Glu Leu Leu Tyr Ala
   1115 1120 1125
   Thr Asn Asp Asp Glu Glu Arg Tyr Ser Ile Gln Ala His Pro Leu
   1130 1135 1140
   Leu Leu Arg Asn Leu Thr Val Gln Ala Ala Glu Pro Pro Leu Gly
   1145 1150 1155
   Tyr Pro Ile Tyr Ser Ser Lys Pro Leu His Ile His Leu Tyr
   1160 1165 1170
<210> 9
   <211> 873
   <212> PRT
   <213> Homo sapiens
<400> 9
   Met Pro Ala Leu Glu His Met Asn Gln Ile Leu His Ile Leu Phe Val
   1               5                   10                  15
   Phe Leu Pro Phe Leu Trp Ala Leu Gly Thr Leu Pro Pro Pro Asp Ala
               20                  25                  30
   Leu Leu Leu Trp Ala Met Glu Gln Val Leu Glu Phe Gly Leu Gly Gly
           35                  40                  45
   Ser Ser Met Ser Thr His Leu Arg Leu Leu Val Met Phe Ile Met Ser
       50                  55                  60
   Ala Gly Thr Ala Ile Ala Ser Tyr Phe Ile Pro Ser Thr Val Gly Val
   65                  70                  75                  80
   Val Leu Phe Met Thr Gly Phe Gly Phe Leu Leu Ser Leu Asn Leu Ser
                   85                  90                  95
   Asp Met Gly His Lys Ile Gly Thr Lys Ser Lys Asp Leu Pro Ser Gly
               100                 105                 110
   Pro Glu Lys His Phe Ser Trp Lys Glu Cys Leu Phe Tyr Ile Ile Ile
           115                 120                 125
   Leu Val Leu Ala Leu Leu Glu Thr Ser Leu Leu His His Phe Ala Gly
       130                 135                 140
   Phe Ser Gln Ile Ser Lys Ser Asn Ser Gln Ala Ile Val Gly Tyr Gly
   145                 150                 155                 160
   Leu Met Ile Leu Leu Ile Ile Leu Trp Ile Leu Arg Glu Ile Gln Ser
                   165                 170                 175
   Val Tyr Ile Ile Gly Ile Phe Arg Asn Pro Phe Tyr Pro Lys Asp Val
               180                 185                 190
   Gln Thr Val Thr Val Phe Phe Glu Lys Gln Thr Arg Leu Met Lys Ile
           195                 200                 205
   Gly Ile Val Arg Arg Ile Leu Leu Thr Leu Val Ser Pro Phe Ala Met
       210                 215                 220
   Ile Ala Phe Leu Ser Leu Asp Ser Ser Leu Gln Gly Leu His Ser Val
   225                 230                 235                 240
   Ser Val Cys Ile Gly Phe Thr Arg Ala Phe Arg Met Val Trp Gln Asn
   245 250 255
   Thr Glu Asn Ala Leu Leu Glu Thr Val Ile Val Ser Thr Val His Leu
   260 265 270
   Ile Ser Ser Thr Asp Ile Trp Trp Asn Arg Ser Leu Asp Thr Gly Leu
   275 280 285
   Arg Leu Leu Leu Val Gly Ile Ile Arg Asp Arg Leu Ile Gln Phe Ile
   290 295 300
   Ser Lys Leu Gln Phe Ala Val Thr Val Leu Leu Thr Ser Trp Thr Glu
   305 310 315 320
   Lys Lys Gln Arg Arg Lys Thr Thr Ala Thr Leu Cys Ile Leu Asn Ile
   325 330 335
   Val Phe Ser Pro Phe Val Leu Val Ile Ile Val Phe Ser Thr Leu Leu
   340 345 350
   Ser Ser Pro Leu Leu Pro Leu Phe Thr Leu Pro Val Phe Leu Val Gly
   355 360 365
   Phe Pro Arg Pro Ile Gln Ser Trp Pro Gly Ala Ala Gly Thr Thr Ala
   370 375 380
   Cys Val Cys Ala Asp Thr Val Tyr Tyr Tyr Gln Met Val Pro Arg Leu
   385 390 395 400
   Thr Ala Val Leu Gln Thr Ala Met Ala Ala Gly Ser Leu Gly Leu Leu
   405 410 415
   Leu Pro Gly Ser His Tyr Leu Gly Arg Phe Gln Asp Arg Leu Met Trp
   420 425 430
   Ile Met Ile Leu Glu Cys Gly Tyr Thr Tyr Cys Ser Ile Asn Ile Lys
   435 440 445
   Gly Leu Glu Leu Gln Glu Thr Ser Cys His Thr Ala Glu Ala Arg Arg
   450 455 460
   Val Asp Glu Val Phe Glu Asp Ala Phe Glu Gln Glu Tyr Thr Arg Val
   465 470 475 480
   Cys Ser Leu Asn Glu His Phe Gly Asn Val Leu Thr Pro Cys Thr Val
   485 490 495
   Leu Pro Val Lys Leu Tyr Ser Asp Ala Arg Asn Val Leu Ser Gly Ile
   500 505 510
   Ile Asp Ser His Glu Asn Leu Lys Glu Phe Lys Gly Asp Leu Ile Lys
   515 520 525
   Val Leu Val Trp Ile Leu Val Gln Tyr Cys Ser Lys Arg Pro Gly Met
   530 535 540
   Lys Glu Asn Val His Asn Thr Glu Asn Lys Gly Lys Ala Pro Leu Met
   545 550 555 560
   Leu Pro Ala Leu Asn Thr Leu Pro Pro Pro Lys Ser Pro Glu Asp Ile
   565 570 575
   Asp Ser Leu Asn Ser Glu Thr Phe Asn Asp Trp Ser Asp Asp Asn Ile
   580 585 590
   Phe Asp Asp Glu Pro Thr Ile Lys Lys Val Ile Glu Glu Lys His Gln
   595 600 605
   Leu Lys Asp Leu Pro Gly Thr Asn Leu Phe Ile Pro Gly Ser Val Glu
   610 615 620
   Ser Gln Arg Val Gly Asp His Ser Thr Gly Thr Val Pro Glu Asn Asp
   625 630 635 640
   Leu Tyr Lys Ala Val Leu Leu Gly Tyr Pro Ala Val Asp Lys Gly Lys
   645 650 655
   Gln Glu Asp Met Pro Tyr Ile Pro Leu Met Glu Phe Ser Cys Ser His
   660 665 670
   Ser His Leu Val Cys Leu Pro Ala Glu Trp Arg Thr Ser Cys Met Pro
   675 680 685
   Ser Ser Lys Met Lys Glu Met Ser Ser Leu Phe Pro Glu Asp Trp Tyr
   690 695 700
   Gln Phe Val Leu Arg Gln Leu Glu Cys Tyr His Ser Glu Glu Lys Ala
   705 710 715 720
   Ser Asn Val Leu Glu Glu Ile Ala Lys Asp Lys Val Leu Lys Asp Phe
   725 730 735
   Tyr Val His Thr Val Met Thr Cys Tyr Phe Ser Leu Phe Gly Ile Asp
   740 745 750
   Asn Met Ala Pro Ser Pro Gly His Ile Leu Arg Val Tyr Gly Gly Val
   755 760 765
   Leu Pro Trp Ser Val Ala Leu Asp Trp Leu Thr Glu Lys Pro Glu Leu
   770 775 780
   Phe Gln Leu Ala Leu Lys Ala Phe Arg Tyr Thr Leu Lys Leu Met Ile
   785 790 795 800
   Asp Lys Ala Ser Leu Gly Pro Ile Glu Asp Phe Arg Glu Leu Ile Lys
   805 810 815
   Tyr Leu Glu Glu Tyr Glu Arg Asp Trp Tyr Ile Gly Leu Val Ser Asp
   820 825 830
   Glu Lys Trp Lys Glu Ala Ile Leu Gln Glu Lys Pro Tyr Leu Phe Ser
   835 840 845
   Leu Gly Tyr Asp Ser Asn Met Pro Gly Pro Ala Leu Glu Ile Ser Arg
   850 855 860
   Val Asn Arg Asn Leu Trp Ser Gln Ile
   865 870
<210> 10
   <211> 3927
   <212> DNA
   <213> Homo sapiens
<400> 10
   aacgacgctc ttgcgtaaag gcccggccca agggaacgtt cagggcgtct cggctttccc      60
   cgctgctgct tctgctaggc ccagtgcgag accagagcac gagcgactcc cgtcgtcccc     120
   ggccaggcag atgttggcct agtcctggcg cgaacgaagc gcgctatttc cctgcttcct     180
   ctaggccaag cctgctttac ggcagggccc gcctcgggag cgagcacaga ccggggcagc     240
   gaggccagcc aggcgccgac gaggtccccg aacgcgcacg cgctccgttc agctccgggt     300
   ggcggccgcc ggagtagacg ttagccatgg aaaccgagag ctggcccggg cggggccgcg     360
   gtgagctcgt tattcggccg ccgcagcttt tctgcctccg cattcgggca ctaaccaacc     420
   tcccggcggg agcgcccagc ccgagtttac ctgcaaaaat gcggtccctg ggatgccttc     480
   gcgtcttctc ttccctcggg tgacttgagg tttgtggtaa atatgccagc tctagaacac     540
   atgaatcaga ttttacacat cttgtttgta tttttaccct ttctgtgggc acttgggact     600
   ctgcccccac ccgatgcact tctcttatgg gcaatggagc aggttttaga gttcggcctt     660
   ggaggctcat ctatgtcaac ccacttacgg ttattagtaa tgttcatcat gtctgctgga 720
   acagctatag catcatattt cattccaagc actgttggtg tggttctttt catgactgga 780
   tttggtttct tgctgagtct gaacttaagt gatatgggtc acaaaattgg aaccaaatct 840
   aaggatttac ccagtggtcc ggaaaaacat ttttcatgga aggaatgcct tttctacatc 900
   attatattag tcttggctct tttagaaact agcttgcttc atcactttgc tggcttctca 960
   cagatttcta aaagcaattc ccaggctatt gtgggctatg gtttgatgat attacttata 1020
   atactgtgga tacttagaga aattcaaagc gtatatatca ttggaatttt ccgaaatccc 1080
   ttttatccga aggatgtgca aactgtgact gtattctttg agaagcaaac taggctcatg 1140
   aagattggta ttgtcagacg gattttgcta actttagtat caccttttgc catgatagca 1200
   tttctttcat tggacagttc cttacaaggg ctccactcag tgtctgtctg tattggattc 1260
   acaagagcct ttagaatggt atggcagaat acagaaaatg ctttattgga gacagtcatt 1320
   gtatcaacag tacacttgat ctccagtaca gacatatggt ggaacagaag cctggataca 1380
   ggactcagac tcttactggt tggtatcata cgtgatcgtt tgattcagtt catctctaaa 1440
   ttgcagtttg ccgtgactgt gcttttgaca tcatggacag agaaaaaaca acgtcgaaaa 1500
   acaactgcca ctttatgtat actcaacatt gtcttttctc cattcgtgtt ggtcatcata 1560
   gttttttcta cactactctc ttctccctta ctccctcttt tcacccttcc tgtgttcttg 1620
   gtggggtttc cccgacctat tcagagttgg ccaggagcag caggcaccac agcctgtgtg 1680
   tgtgcagata cagtgtacta ctaccaaatg gtgcccaggt tgactgctgt actgcagact 1740
   gcaatggcag ctggaagttt aggtctcctc ctacctggat ctcattactt gggccgtttt 1800
   caggatcgtt taatgtggat aatgattctg gaatgtggct atacttactg ctctattaac 1860
   attaaggggt tagaattgca ggaaacatcc tgtcatactg cagaagctcg cagagttgat 1920
   gaagtttttg aagatgcttt tgagcaagaa tacacaagag tatgttccct taatgaacac 1980
   tttggaaatg tcttgacacc ctgtactgtt ttgcctgtga aattgtattc tgatgccagg 2040
   aatgttctat caggcataat tgattctcat gaaaacttaa aagaatttaa aggtgacctc 2100
   attaaagtac ttgtgtggat acttgttcaa tactgctcca aaaggcctgg catgaaagag 2160
   aatgttcaca acactgaaaa taaagggaaa gcacctctaa tgttgcctgc tttgaacact 2220
   ttgccacctc ccaaatcccc agaagacata gacagtttaa attcagaaac ttttaatgac 2280
   tggtctgatg ataatatttt tgatgatgag ccaactatca aaaaagtaat agaagaaaaa 2340
   catcagttga aagatttgcc aggtacaaat ttgtttattc caggatcagt agaatcacag 2400
   agggttggtg atcattctac aggcactgtt cctgaaaacg atctttacaa agcagttcta 2460
   ttaggatacc ctgctgttga caaaggaaaa caagaggaca tgccatatat tcctctcatg 2520
   gagttcagtt gttcacattc tcacttagta tgcttacccg cagagtggag gactagctgt 2580
   atgcccagtt ccaaaatgaa ggagatgagc tcgttatttc cagaagactg gtaccaattt 2640
   gttctaaggc agttggaatg ttatcattca gaagagaagg cctcaaatgt actggaagaa 2700
   attgccaagg acaaagtttt aaaagacttt tatgttcata cagtaatgac ttgttatttt 2760
   agtttatttg gaatagacaa tatggctcct agtcctggtc atatattgag agtttacggt 2820
   ggtgttttgc cttggtctgt tgctttggac tggctcacag aaaagccaga actgtttcaa 2880
   ctagcactga aagcattcag gtatactctg aaactaatga ttgataaagc aagtttaggt 2940
   ccaatagaag actttagaga actgattaag taccttgaag aatatgaacg tgactggtac 3000
   attggtttgg tatctgatga aaagtggaag gaagcaattt tacaagaaaa gccatacttg 3060
   ttttctctgg ggtatgattc taatatggga atttacactg ggagagtgct tagccttcaa 3120
   gaattattga tccaagtggg aaagttaaat cctgaagctg ttagaggtca gtgggccaat 3180
   ctttcatggg aattacttta tgccacaaac gatgatgaag aacgttatag tatacaagct 3240
   catccactac ttttaagaaa tcttacggta caagcagcag aacctcccct gggatatccg 3300
   atttattctt caaaacctct ccacatacat ttgtattaga gctcattttg actgtaatgt 3360
   catcaaatgc aatgttttta ttttttcatc ctaaaaaagt aactgtgatt cttgtaactt 3420
   gaggacttct ccacaccccc attcagatgc ctgagaacag ctaagctccg taaagttggt 3480
   tctcttagcc atcttaatgg ttctaaaaaa cagcaaaaac atctttatgt ctaagataaa 3540
   agaactattt ggccaatatt tgtgccctct ggactttagt aggctttggt aaatgtgaga 3600
   aaacttttgt agaattatca tataatgaat tttgtaatgc tttcttaaat gtgttatagg 3660
   tgaattgcca tacaaagtta acagctatgt aatttttaca tacttaagag ataaacatat 3720
   cagtgttcta agtagtgata atggatcctg ttgaaggtta acataatgtg tatatatttg 3780
   tttgaaatat aatttatagt attttcaaat gtgctgattt attttgacat ctaatatctg 3840
   aatgtttttg tatcaagtag tttgttttca tagacttcaa ttcataaact ttaaaaaact 3900
   tttaataaaa tattttcctt ccttttc 3927
<210> 11
   <211> 3932
   <212> DNA
   <213> Homo sapiens
<400> 11
   aacgacgctc ttgcgtaaag gcccggccca agggaacgtt cagggcgtct cggctttccc 60
   cgctgctgct tctgctaggc ccagtgcgag accagagcac gagcgactcc cgtcgtcccc 120
   ggccaggcag atgttggcct agtcctggcg cgaacgaagc gcgctatttc cctgcttcct 180
   ctaggccaag cctgctttac ggcagggccc gcctcgggag cgagcacaga ccggggcagc 240
   gaggccagcc aggcgccgac gaggtccccg aacgcgcacg cgctccgttc agctccgggt 300
   ggcggccgcc ggagtagacg ttagccatgg aaaccgagag ctggcccggg cggggccgcg 360
   gtgagctcgt tattcggccg ccgcagcttt tctgcctccg cattcgggca ctaaccaacc 420
   tcccggcggg agcgcccagc ccgagtttac ctgcaaaaat gcggtccctg ggatgccttc 480
   gcgtcttctc ttccctcggg tgacttgagg tttgtggtaa atatgccagc tctagaacac 540
   atgaatcaga ttttacacat cttgtttgta tttttaccct ttctgtgggc acttgggact 600
   ctgcccccac ccgatgcact tctcttatgg gcaatggagc aggttttaga gttcggcctt 660
   ggaggctcat ctatgtcaac ccacttacgg ttattagtaa tgttcatcat gtctgctgga 720
   acagctatag catcatattt cattccaagc actgttggtg tggttctttt catgactgga 780
   tttggtttct tgctgagtct gaacttaagt gatatgggtc acaaaattgg aaccaaatct 840
   aaggatttac ccagtggtcc ggaaaaacat ttttcatgga aggaatgcct tttctacatc 900
   attatattag tcttggctct tttagaaact agcttgcttc atcactttgc tggcttctca 960
   cagatttcta aaagcaattc ccaggctatt gtgggctatg gtttgatgat attacttata 1020
   atactgtgga tacttagaga aattcaaagc gtatatatca ttggaatttt ccgaaatccc 1080
   ttttatccga aggatgtgca aactgtgact gtattctttg agaagcaaac taggctcatg 1140
   aagattggta ttgtcagacg gattttgcta actttagtat caccttttgc catgatagca 1200
   tttctttcat tggacagttc cttacaaggg ctccactcag tgtctgtctg tattggattc 1260
   acaagagcct ttagaatggt atggcagaat acagaaaatg ctttattgga gacagtcatt 1320
   gtatcaacag tacacttgat ctccagtaca gacatatggt ggaacagaag cctggataca 1380
   ggactcagac tcttactggt tggtatcata cgtgatcgtt tgattcagtt catctctaaa 1440
   ttgcagtttg ccgtgactgt gcttttgaca tcatggacag agaaaaaaca acgtcgaaaa 1500
   acaactgcca ctttatgtat actcaacatt gtcttttctc cattcgtgtt ggtcatcata 1560
   gttttttcta cactactctc ttctccctta ctccctcttt tcacccttcc tgtgttcttg 1620
   gtggggtttc cccgacctat tcagagttgg ccaggagcag caggcaccac agcctgtgtg 1680
   tgtgcagata cagtgtacta ctaccaaatg gtgcccaggt tgactgctgt actgcagact 1740
   gcaatggcag ctggaagttt aggtctcctc ctacctggat ctcattactt gggccgtttt 1800
   caggatcgtt taatgtggat aatgattctg gaatgtggct atacttactg ctctattaac 1860
   attaaggggt tagaattgca ggaaacatcc tgtcatactg cagaagctcg cagagttgat 1920
   gaagtttttg aagatgcttt tgagcaagaa tacacaagag tatgttccct taatgaacac 1980
   tttggaaatg tcttgacacc ctgtactgtt ttgcctgtga aattgtattc tgatgccagg 2040
   aatgttctat caggcataat tgattctcat gaaaacttaa aagaatttaa aggtgacctc 2100
   attaaagtac ttgtgtggat acttgttcaa tactgctcca aaaggcctgg catgaaagag 2160
   aatgttcaca acactgaaaa taaagggaaa gcacctctaa tgttgcctgc tttgaacact 2220
   ttgccacctc ccaaatcccc agaagacata gacagtttaa attcagaaac ttttaatgac 2280
   tggtctgatg ataatatttt tgatgatgag ccaactatca aaaaagtaat agaagaaaaa 2340
   catcagttga aagatttgcc aggtacaaat ttgtttattc caggatcagt agaatcacag 2400
   agggttggtg atcattctac aggcactgtt cctgaaaacg atctttacaa agcagttcta 2460
   ttaggatacc ctgctgttga caaaggaaaa caagaggaca tgccatatat tcctctcatg 2520
   gagttcagtt gttcacattc tcacttagta tgcttacccg cagagtggag gactagctgt 2580
   atgcccagtt ccaaaatgaa ggagatgagc tcgttatttc cagaagactg gtaccaattt 2640
   gttctaaggc agttggaatg ttatcattca gaagagaagg cctcaaatgt actggaagaa 2700
   attgccaagg acaaagtttt aaaagacttt tatgttcata cagtaatgac ttgttatttt 2760
   agtttatttg gaatagacaa tatggctcct agtcctggtc atatattgag agtttacggt 2820
   ggtgttttgc cttggtctgt tgctttggac tggctcacag aaaagccaga actgtttcaa 2880
   ctagcactga aagcattcag gtatactctg aaactaatga ttgataaagc aagtttaggt 2940
   ccaatagaag actttagaga actgattaag taccttgaag aatatgaacg tgactggtac 3000
   attggtttgg tatctgatga aaagtggaag gaagcaattt tacaagaaaa gccatacttg 3060
   ttttctctgg ggtatgattc taatatggga atttacactg ggagagtgct tagccttcaa 3120
   gaattattga tccaagtggg aaagttaaat cctgaagctg ttagaggtca gtgggccaat 3180
   ctttcatggg aattacttta tgccacaaac gatgatgaag aacgttatag tatacaagct 3240
   catccactac ttttaagaaa tcttacggta caagcagcag aacctcccct gggatatccg 3300
   atttattctt caaaacctct ccacatacat ttgtattaga gctcattttg actgtaatgt 3360
   catcaaatgc aatgttttta ttttttcatc ctaaaaaagt aactgtgatt cttgtaactt 3420
   gaggacttct ccacaccccc attcagatgc ctgagaacag ctaagctccg taaagttggt 3480
   tctcttagcc atcttaatgg ttctaaaaaa cagcaaaaac atctttatgt ctaagataaa 3540
   agaactattt ggccaatatt tgtgccctct ggactttagt aggctttggt aaatgtgaga 3600
   aaacttttgt agaattatca tataatgaat tttgtaatgc tttcttaaat gtgttatagg 3660
   tgaattgcca tacaaagtta acagctatgt aatttttaca tacttaagag ataaacatat 3720
   cagtgttcta agtagtgata atggatcctg ttgaaggtta acataatgtg tatatatttg 3780
   tttgaaatat aatttatagt attttcaaat gtgctgattt attttgacat ctaatatctg 3840
   aatgtttttg tatcaagtag tttgttttca tagacttcaa ttcataaact ttaaaaaact 3900
   tttaataaaa tattttcctt ccttttcaaa ta 3932
<210> 12
   <211> 4073
   <212> DNA
   <213> Homo sapiens
<400> 12
   tcttgcgtaa aggcccggcc caagggaacg ttcagggcgt ctcggctttc cccgctgctg     60
   cttctgctag gcccagtgcg agaccagagc acgagcgact cccgtcgtcc ccggccaggc    120
   agatgttggc ctagtcctgg cgcgaacgaa gcgcgctatt tccctgcttc ctctaggcca    180
   agcctgcttt acggcagggc ccgcctcggg agcgagcaca gaccggggca gcgaggccag    240
   ccaggcgccg acgaggtccc cgaacgcgca cgcgctccgt tcagctccgg gtggcggccg    300
   ccggagtaga cgttagccat ggaaaccgag agctggcccg ggcggggccg cggtgagctc    360
   gttattcggc cgccgcagct tttctgcctc cgcattcggg cactaaccaa cctcccggcg    420
   ggagcgccca gcccgagttt acctgcaaaa atgcggtccc tgggatgcct tcgcgtcttc    480
   tcttccctcg ggtgacttga gaaactgctg tgttacagaa aagcatgtga ctttcagaat    540
   aatcccgagt gaggatgagt ccagatgtgc ctctactgaa tgattacaag caggacttct    600
   ttctgaagcg ctttccacag actgttcttg gaggccctcg attcaaatta ggctattgtg    660
   cccctcctta catatatgtt aatcaaatta ttctttttct aatgccatgg gtttggggtg    720
   gagtcggaac acttttatac cagttaggca tcctgaaaga ctattataca gcagcacttt    780
   caggtggatt aatgcttttt actgcatttg tcatccagtt cacaagttta tacgccaaaa    840
   acaaatcaac aacagtagaa agaatactaa ccacggatat cttagcagag gaggatgagc    900
   atgaatttac cagttgtact ggtgctgaga ctgtcaaatt tctcattcct ggcaagaaat    960
   atgtagccaa tacagttttt cattctattc ttgctggatt agcgtgtggt cttggaacat   1020
   ggtatctgct cccaaataga ataaccttgc tgtatggcag tacaggaggc actgctctac   1080
   tattcttctt tggatggatg acactatgta tagcagaata ttctttaatt gtaaacacag   1140
   ctacagagac tgcgactttc caaacacagg atacttatga aattattcct cttatgagac   1200
   ctctttatat ttttttcttt gtttctgtgg atctggcaca caggtaaaaa cctaccaaat   1260
   actttgtaac taactttgtt tttaagtata cagagtaaga gagctttcct tttagtgtta   1320
   caaaaaaatg aatccatgga ttaaaaatca tcaaaccatt gggtgacagg ttattttgat   1380
   aattattctt ttaggattaa tctctgtaaa acatactaaa gcaatagtta aaacttatta   1440
   aagagttttt ttaaaaaacc ctttttgaga taaggaactt ttcaattttg tgtttcactt   1500
   taaataagga gctttgagtt tttaagatag cctggctaaa acctgtgtaa ggagatggaa   1560
   ctttcctgtg gggggaaaga agaaattaaa atttatacat ataaatatta tatacagatt   1620
   gaatgaattt aagacaaata caaaatttat ttctaatttt atgatagcaa caatagtaga 1680
   agtaatattg attttttaaa aaccaacttg ttacagaaga aaagtagaaa atagtttttt 1740
   taacagacat aattgttcac aaaattgttt gataacccct tttacttgcc ttttcaagtt 1800
   tgacttttct ttctccgtct ccgtagattg cagctttctt ttcttaggtt agtgtccagg 1860
   tagaaatgtt cagcatgtta tggactgaat atttgtgtct ctccagaatt catatattac 1920
   agtcttaaac cccaatgtga tggtattttg agatgaggcc tttgggaggt aattaggtca 1980
   tgaaggtggg tccttggtat gatgggacta gtccccttat gagaagaggt accagagagc 2040
   ttgatttgtc cctctctgtg ccatctaagg acacagcaaa aatgtggcca cctgcaagtc 2100
   aaagaagaga gctctcatca aaacctgacc atactggcag cctgatcatg gtctttcggc 2160
   cttcagaagt gtgagaaagt aaattgatgt tgtttaagcc actcagctta tggcattttt 2220
   ttatggcagc ccatgctgat taagattttg ctaccaagaa gtgggatgcc tttgtaccaa 2280
   atacctaaaa atgtggaaat ggctttgtaa ctgttggtaa tgggtagagg ctgaaagagg 2340
   tttttttgtt tgtttctttg ttttgttttg tttgagatgg agtcttcact ctcttgccca 2400
   ggctggagtg cagtggcaca atcttggccc actgcaacgt ccgcctcctg gcttcaagtg 2460
   atactcctcc ctgagcctcc tgagtagctg gaattacaga catgcgccac catgcccggc 2520
   taatttttat atttttaata gagacagggt ttcgccatgt tggccaggct ggtcccaaag 2580
   tcctgacctc aagtgatcca cccgcctcgg cctcccaaag ttctgggatt acaggcttga 2640
   gccatcacac ccagccaagg gttttgatgt gcatgctaga aatatggaca ttaagggtga 2700
   ttctgatgaa gtctgaagtc ccctgaaccc agaggaaagg cagtccttgt tacaaagcgt 2760
   caaagaactt agctgaactg tgtcctagtg ttttgtggag ccatgaagtt ggatacctac 2820
   gtaaggagag ttcaaagcag tgttgaaagg agcagttggc ttctcctgtt tctagtaaaa 2880
   tgtgaaagga gagagatgga ttgaagaagg gattgttaag caagagaagg aaccagaact 2940
   tgaagatttg gaaaattttc agcctggatt atccatattg taaaacatga gaaagcatat 3000
   tctgaagaga acaccaaaag tgtggggctg gactgtccct cagtaaagag cttttggcat 3060
   tatgtgagta gaaacactgc cagtttgaat tgaagtggtt ggagacagga agtaatgaag 3120
   gccgacagtt gaacttcttg gatttgacag gatgtaatga tagaactgtt cagctacaaa 3180
   agtgcagtat tcttcaagaa aaggggaaaa ttatgccaaa ggtgatttaa gggtcttgag 3240
   ggctaccacc tgtttcaaca agtcagctag cctctaccca aagcctcggg agcagaactg 3300
   aacttcagag ccacagaagc aggaccctca cctagagcac tggcggtgac ctgccacccc 3360
   agtggcctgg tgggcagagt atggaaccaa acagaattat tctcaagctc taagatctaa 3420
   tggaatttgc cttgctaggt tttgacttcc ttgggatcat cacccttttt ttcctgtttc 3480
   tcctattgga gtggggatgt ctcttctata cctgccctac ccttagattt tggaagcacg 3540
   taactcatct ggtttcacag attcacagct ggagaggaat tttgcctcag gatggattgt 3600
   acctaggtct catccatatc tgatttagat gagactttga attttagcca tcagagttaa 3660
   tgctagaatg agtgaagact ttgggggata tggggatgga atgaatgtct tttgcatttg 3720
   aatttggaaa ggacatgagt tttgaggggc cagggatgga atgttataga ctaattgtgt 3780
   tccctcaaaa atgtttatgt tgaagcccta acccccaatg tgttggtatt tggagatggg 3840
   cctctgggag gtagtttatg aaggtgagac cctagtctga taggattagt gcccttagga 3900
   gagatgccaa agagcttgat gtctctcttt ttgctacaaa aagacacagc aaaaaggcag 3960
   ccatgtgtaa gccaggaaga gagtcttcac cagaacctga ctatactggc agcctgatct 4020
   tgtacttgta gcccccagaa ctgttagaaa ataaatttct gttgtttaag cca 4073
<210> 13
   <211> 4666
   <212> DNA
   <213> Homo sapiens
<400> 13
   tcttgcgtaa aggcccggcc caagggaacg ttcagggcgt ctcggctttc cccgctgctg 60
   cttctgctag gcccagtgcg agaccagagc acgagcgact cccgtcgtcc ccggccaggc 120
   agatgttggc ctagtcctgg cgcgaacgaa gcgcgctatt tccctgcttc ctctaggcca 180
   agcctgcttt acggcagggc ccgcctcggg agcgagcaca gaccggggca gcgaggccag 240
   ccaggcgccg acgaggtccc cgaacgcgca cgcgctccgt tcagctccgg gtggcggccg 300
   ccggagtaga cgttagccat ggaaaccgag agctggcccg ggcggggccg cggtgagctc 360
   gttattcggc cgccgcagct tttctgcctc cgcattcggg cactaaccaa cctcccggcg 420
   ggagcgccca gcccgagttt acctgcaaaa atgcggtccc tgggatgcct tcgcgtcttc 480
   tcttccctcg ggtgacttga gaaactgctg tgttacagaa aagcatgtga ctttcagaat 540
   aatcccgagt gaggatgagt ccagatgtgc ctctactgaa tgattacaag caggacttct 600
   ttctgaagcg ctttccacag actgttcttg gaggccctcg attcaaatta ggctattgtg 660
   cccctcctta catatatgtt aatcaaatta ttctttttct aatgccatgg gtttggggtg 720
   gagtcggaac acttttatac cagttaggca tcctgaaaga ctattataca gcagcacttt 780
   caggtggatt aatgcttttt actgcatttg tcatccagtt cacaagttta tacgccaaaa 840
   acaaatcaac aacagtagaa agaatactaa ccacggatat cttagcagag gaggatgagc 900
   atgaatttac cagttgtact ggtgctgaga ctgtcaaatt tctcattcct ggcaagaaat 960
   atgtagccaa tacagttttt cattctattc ttgctggatt agcgtgtggt cttggaacat 1020
   ggtatctgct cccaaataga ataaccttgc tgtatggcag tacaggaggc actgctctac 1080
   tattcttctt tggatggatg acactatgta tagcagaata ttctttaatt gtaaacacag 1140
   ctacagagac tgcgactttc caaacacagg atacttatga aattattcct cttatgagac 1200
   ctctttatat ttttttcttt gtttctgtgg atctggcaca caggtttgtg gtaaatatgc 1260
   cagctctaga acacatgaat cagattttac acatcttgtt tgtattttta ccctttctgt 1320
   gggcacttgg gactctgccc ccacccgatg cacttctctt atgggcaatg gagcaggttt 1380
   tagagttcgg ccttggaggc tcatctatgt caacccactt acggttatta gtaatgttca 1440
   tcatgtctgc tggaacagct atagcatcat atttcattcc aagcactgtt ggtgtggttc 1500
   ttttcatgac tggatttggt ttcttgctga gtctgaactt aagtgatatg ggtcacaaaa 1560
   ttggaaccaa atctaaggat ttacccagtg gtccggaaaa acatttttca tggaaggaat 1620
   gccttttcta catcattata ttagtcttgg ctcttttaga aactagcttg cttcatcact 1680
   ttgctggctt ctcacagatt tctaaaagca attcccaggc tattgtgggc tatggtttga 1740
   tgatattact tataatactg tggatactta gagaaattca aagcgtatat atcattggaa 1800
   ttttccgaaa tcccttttat ccgaaggatg tgcaaactgt gactgtattc tttgagaagc 1860
   aaactaggct catgaagatt ggtattgtca gacggatttt gctaacttta gtatcacctt 1920
   ttgccatgat agcatttctt tcattggaca gttccttaca agggctccac tcagtgtctg 1980
   tctgtattgg attcacaaga gcctttagaa tggtatggca gaatacagaa aatgctttat 2040
   tggagacagt cattgtatca acagtacact tgatctccag tacagacata tggtggaaca 2100
   gaagcctgga tacaggactc agactcttac tggttggtat catacgtgat cgtttgattc 2160
   agttcatctc taaattgcag tttgccgtga ctgtgctttt gacatcatgg acagagaaaa 2220
   aacaacgtcg aaaaacaact gccactttat gtatactcaa cattgtcttt tctccattcg 2280
   tgttggtcat catagttttt tctacactac tctcttctcc cttactccct cttttcaccc 2340
   ttcctgtgtt cttggtgggg tttccccgac ctattcagag ttggccagga gcagcaggca 2400
   ccacagcctg tgtgtgtgca gatacagtgt actactacca aatggtgccc aggttgactg 2460
   ctgtactgca gactgcaatg gcagctggaa gtttaggtct cctcctacct ggatctcatt 2520
   acttgggccg ttttcaggat cgtttaatgt ggataatgat tctggaatgt ggctatactt 2580
   actgctctat taacattaag gggttagaat tgcaggaaac atcctgtcat actgcagaag 2640
   ctcgcagagt tgatgaagtt tttgaagatg cttttgagca agaatacaca agagtatgtt 2700
   cccttaatga acactttgga aatgtcttga caccctgtac tgttttgcct gtgaaattgt 2760
   attctgatgc caggaatgtt ctatcaggca taattgattc tcatgaaaac ttaaaagaat 2820
   ttaaaggtga cctcattaaa gtacttgtgt ggatacttgt tcaatactgc tccaaaaggc 2880
   ctggcatgaa agagaatgtt cacaacactg aaaataaagg gaaagcacct ctaatgttgc 2940
   ctgctttgaa cactttgcca cctcccaaat ccccagaaga catagacagt ttaaattcag 3000
   aaacttttaa tgactggtct gatgataata tttttgatga tgagccaact atcaaaaaag 3060
   taatagaaga aaaacatcag ttgaaagatt tgccaggtac aaatttgttt attccaggat 3120
   cagtagaatc acagagggtt ggtgatcatt ctacaggcac tgttcctgaa aacgatcttt 3180
   acaaagcagt tctattagga taccctgctg ttgacaaagg aaaacaagag gacatgccat 3240
   atattcctct catggagttc agttgttcac attctcactt agtatgctta cccgcagagt 3300
   ggaggactag ctgtatgccc agttccaaaa tgaaggagat gagctcgtta tttccagaag 3360
   actggtacca atttgttcta aggcagttgg aatgttatca ttcagaagag aaggcctcaa 3420
   atgtactgga agaaattgcc aaggacaaag ttttaaaaga cttttatgtt catacagtaa 3480
   tgacttgtta ttttagttta tttggaatag acaatatggc tcctagtcct ggtcatatat 3540
   tgagagttta cggtggtgtt ttgccttggt ctgttgcttt ggactggctc acagaaaagc 3600
   cagaactgtt tcaactagca ctgaaagcat tcaggtatac tctgaaacta atgattgata 3660
   aagcaagttt aggtccaata gaagacttta gagaactgat taagtacctt gaagaatatg 3720
   aacgtgactg gtacattggt ttggtatctg atgaaaagtg gaaggaagca attttacaag 3780
   aaaagccata cttgttttct ctggggtatg attctaatat gggaatttac actgggagag 3840
   tgcttagcct tcaagaatta ttgatccaag tgggaaagtt aaatcctgaa gctgttagag 3900
   gtcagtgggc caatctttca tgggaattac tttatgccac aaacgatgat gaagaacgtt 3960
   atagtataca agctcatcca ctacttttaa gaaatcttac ggtacaagca gcagaacctc 4020
   ccctgggata tccgatttat tcttcaaaac ctctccacat acatttgtat tagagctcat 4080
   tttgactgta atgtcatcaa atgcaatgtt tttatttttt catcctaaaa aagtaactgt 4140
   gattcttgta acttgaggac ttctccacac ccccattcag atgcctgaga acagctaagc 4200
   tccgtaaagt tggttctctt agccatctta atggttctaa aaaacagcaa aaacatcttt 4260
   atgtctaaga taaaagaact atttggccaa tatttgtgcc ctctggactt tagtaggctt 4320
   tggtaaatgt gagaaaactt ttgtagaatt atcatataat gaattttgta atgctttctt 4380
   aaatgtgtta taggtgaatt gccatacaaa gttaacagct atgtaatttt tacatactta 4440
   agagataaac atatcagtgt tctaagtagt gataatggat cctgttgaag gttaacataa 4500
   tgtgtatata tttgtttgaa atataattta tagtattttc aaatgtgctg atttattttg 4560
   acatctaata tctgaatgtt tttgtatcaa gtagtttgtt ttcatagact tcaattcata 4620
   aactttaaaa aacttttaat aaaatatttt ccttcctttt caaata 4666
<210> 14
   <211> 3513
   <212> DNA
   <213> Homo sapiens
<400> 14
   aaaggcccgg cccaagggaa cgttcagggc gtctcggctt tccccgctgc tgcttctgct 60
   aggcccagtg cgagaccaga gcacgagcga ctcccgtcgt ccccggccag gcagatgttg 120
   gcctagtcct ggcgcgaacg aagcgcgcta tttccctgct tcctctaggc caagcctgct 180
   ttacggcagg gcccgcctcg ggagcgagca cagaccgggg cagcgaggcc agccaggcgc 240
   cgacgaggtc cccgaacgcg cacgcgctcc gttcagctcc gggtggcggc cgccggagta 300
   gacgttagcc atggaaaccg agagctggcc cgggcggggc cgcggtgagc tcgttattcg 360
   gccgccgcag cttttctgcc tccgcattcg ggcactaacc aacctcccgg cgggagcgcc 420
   cagcccgagt ttacctgcaa aaatgcggtc cctgggatgc cttcgcgtct tctcttccct 480
   cgggtgactt gaggtttgtg gtaaatatgc cagctctaga acacatgaat cagattttac 540
   acatcttgtt tgtattttta ccctttctgt gggcacttgg gactctgccc ccacccgatg 600
   cacttctctt atgggcaatg gagcaggttt tagagttcgg ccttggaggc tcatctatgt 660
   caacccactt acggttatta gtaatgttca tcatgtctgc tggaacagct atagcatcat 720
   atttcattcc aagcactgtt ggtgtggttc ttttcatgac tggatttggt ttcttgctga 780
   gtctgaactt aagtgatatg ggtcacaaaa ttggaaccaa atctaaggat ttacccagtg 840
   gtccggaaaa acatttttca tggaaggaat gccttttcta catcattata ttagtcttgg 900
   ctcttttaga aactagcttg cttcatcact ttgctggctt ctcacagatt tctaaaagca 960
   attcccaggc tattgtgggc tatggtttga tgatattact tataatactg tggatactta 1020
   gagaaattca aagcgtatat atcattggaa ttttccgaaa tcccttttat ccgaaggatg 1080
   tgcaaactgt gactgtattc tttgagaagc aaactaggct catgaagatt ggtattgtca 1140
   gacggatttt gctaacttta gtatcacctt ttgccatgat agcatttctt tcattggaca 1200
   gttccttaca agggctccac tcagtgtctg tctgtattgg attcacaaga gcctttagaa 1260
   tggtatggca gaatacagaa aatgctttat tggagacagt cattgtatca acagtacact 1320
   tgatctccag tacagacata tggtggaaca gaagcctgga tacaggactc agactcttac 1380
   tggttggtat catacgtgat cgtttgattc agttcatctc taaattgcag tttgccgtga 1440
   ctgtgctttt gacatcatgg acagagaaaa aacaacgtcg aaaaacaact gccactttat 1500
   gtatactcaa cattgtcttt tctccattcg tgttggtcat catagttttt tctacactac 1560
   tctcttctcc cttactccct cttttcaccc ttcctgtgtt cttggtgggg tttccccgac 1620
   ctattcagag ttggccagga gcagcaggca ccacagcctg tgtgtgtgca gatacagtgt 1680
   actactacca aatggtgccc aggttgactg ctgtactgca gactgcaatg gcagctggaa 1740
   gtttaggtct cctcctacct ggatctcatt acttgggccg ttttcaggat cgtttaatgt 1800
   ggataatgat tctggaatgt ggctatactt actgctctat taacattaag gggttagaat 1860
   tgcaggaaac atcctgtcat actgcagaag ctcgcagagt tgatgaagtt tttgaagatg 1920
   cttttgagca agaatacaca agagtatgtt cccttaatga acactttgga aatgtcttga 1980
   caccctgtac tgttttgcct gtgaaattgt attctgatgc caggaatgtt ctatcaggca 2040
   taattgattc tcatgaaaac ttaaaagaat ttaaaggtga cctcattaaa gtacttgtgt 2100
   ggatacttgt tcaatactgc tccaaaaggc ctggcatgaa agagaatgtt cacaacactg 2160
   aaaataaagg gaaagcacct ctaatgttgc ctgctttgaa cactttgcca cctcccaaat 2220
   ccccagaaga catagacagt ttaaattcag aaacttttaa tgactggtct gatgataata 2280
   tttttgatga tgagccaact atcaaaaaag taatagaaga aaaacatcag ttgaaagatt 2340
   tgccaggtac aaatttgttt attccaggat cagtagaatc acagagggtt ggtgatcatt 2400
   ctacaggcac tgttcctgaa aacgatcttt acaaagcagt tctattagga taccctgctg 2460
   ttgacaaagg aaaacaagag gacatgccat atattcctct catggagttc agttgttcac 2520
   attctcactt agtatgctta cccgcagagt ggaggactag ctgtatgccc agttccaaaa 2580
   tgaaggagat gagctcgtta tttccagaag actggtacca atttgttcta aggcagttgg 2640
   aatgttatca ttcagaagag aaggcctcaa atgtactgga agaaattgcc aaggacaaag 2700
   ttttaaaaga cttttatgtt catacagtaa tgacttgtta ttttagttta tttggaatag 2760
   acaatatggc tcctagtcct ggtcatatat tgagagttta cggtggtgtt ttgccttggt 2820
   ctgttgcttt ggactggctc acagaaaagc cagaactgtt tcaactagca ctgaaagcat 2880
   tcaggtatac tctgaaacta atgattgata aagcaagttt aggtccaata gaagacttta 2940
   gagaactgat taagtacctt gaagaatatg aacgtgactg gtacattggt ttggtatctg 3000
   atgaaaagtg gaaggaagca attttacaag aaaagccata cttgttttct ctggggtatg 3060
   attctaatat gccaggacca gccttggaga taagcagagt gaacagaaat ttatggtctc 3120
   agatttaaga aaaacaaaat tctttcttgc ttcttaaatc atactccatc ccattggctt 3180
   gcaaacatgc tgacactcct cataatttca ctcttcataa accaaagcat aaggtcagag 3240
   gagaacttga catattagaa cacttaggca ttgaaagtgg ttagtctaac taacccattg 3300
   aagttttgga gaacctggga ctcaaatttt ggaagatgtg acagatgata tgttaacata 3360
   cattgcaccg aggctgaagt gggaggattg cttgagactg cctggaaggc agaagttgca 3420
   gtgagccgag actgatggtg tcactgcact ccagcctggg caacagagca agaccctgtc 3480
   ttaaaaaaac aaaacaaaca aacaaaagaa acc 3513
<210> 15
   <211> 831
   <212> PRT
   <213> Homo sapiens
<400> 15
   Met Lys Val His Met His Thr Lys Phe Cys Leu Ile Cys Leu Leu Thr
   1               5                   10                  15
   Phe Ile Phe His His Cys Asn His Cys His Glu Glu His Asp His Gly
               20                  25                  30
   Pro Glu Ala Leu His Arg Gln His Arg Gly Met Thr Glu Leu Glu Pro
           35                  40                  45
   Ser Lys Phe Ser Lys Gln Ala Ala Glu Asn Glu Lys Lys Tyr Tyr Ile
       50                  55                  60
   Glu Lys Leu Phe Glu Arg Tyr Gly Glu Asn Gly Arg Leu Ser Phe Phe
   65                  70                  75                  80
   Gly Leu Glu Lys Leu Leu Thr Asn Leu Gly Leu Gly Glu Arg Lys Val
                   85                  90                  95
   Val Glu Ile Asn His Glu Asp Leu Gly His Asp His Val Ser His Leu
               100                 105                 110
   Asp Ile Leu Ala Val Gln Glu Gly Lys His Phe His Ser His Asn His
           115                 120                 125
   Gln His Ser His Asn His Leu Asn Ser Glu Asn Gln Thr Val Thr Ser
       130                 135                 140
   Val Ser Thr Lys Arg Asn His Lys Cys Asp Pro Glu Lys Glu Thr Val
   145                 150                 155                 160
   Glu Val Ser Val Lys Ser Asp Asp Lys His Met His Asp His Asn His
                   165                 170                 175
   Arg Leu Arg His His His Arg Leu His His His Leu Asp His Asn Asn
               180                 185                 190
   Thr His His Phe His Asn Asp Ser Ile Thr Pro Ser Glu Arg Gly Glu
           195                 200                 205
   Pro Ser Asn Glu Pro Ser Thr Glu Thr Asn Lys Thr Gln Glu Gln Ser
       210                 215                 220
   Asp Val Lys Leu Pro Lys Gly Lys Arg Lys Lys Lys Gly Arg Lys Ser
   225                 230                 235                 240
   Asn Glu Asn Ser Glu Val Ile Thr Pro Gly Phe Pro Pro Asn His Asp
                   245                 250                 255
   Gln Gly Glu Gln Tyr Glu His Asn Arg Val His Lys Pro Asp Arg Val
               260                 265                 270
   His Asn Pro Gly His Ser His Val His Leu Pro Glu Arg Asn Gly His
           275                 280                 285
   Asp Pro Gly Arg Gly His Gln Asp Leu Asp Pro Asp Asn Glu Gly Glu
       290                 295                 300
   Leu Arg His Thr Arg Lys Arg Glu Ala Pro His Val Lys Asn Asn Ala
   305                 310                 315                 320
   Ile Ile Ser Leu Arg Lys Asp Leu Asn Glu Asp Asp His His His Glu
                   325                 330                 335
   Cys Leu Asn Val Thr Gln Leu Leu Lys Tyr Tyr Gly His Gly Ala Asn
               340                 345                 350
   Ser Pro Ile Ser Thr Asp Leu Phe Thr Tyr Leu Cys Pro Ala Leu Leu
           355                 360                 365
   Tyr Gln Ile Asp Ser Arg Leu Cys Ile Glu His Phe Asp Lys Leu Leu
       370                 375                 380
   Val Glu Asp Ile Asn Lys Asp Lys Asn Leu Val Pro Glu Asp Glu Ala
   385                 390                 395                 400
   Asn Ile Gly Ala Ser Ala Trp Ile Cys Gly Ile Ile Ser Ile Thr Val
                   405                 410                 415
   Ile Ser Leu Leu Ser Leu Leu Gly Val Ile Leu Val Pro Ile Ile Asn
               420                 425                 430
   Gln Gly Cys Phe Lys Phe Leu Leu Thr Phe Leu Val Ala Leu Ala Val
           435                 440                 445
   Gly Thr Met Ser Gly Asp Ala Leu Leu His Leu Leu Pro His Ser Gln
       450                 455                 460
   Gly Gly His Asp His Ser His Gln His Ala His Gly His Gly His Ser
   465 470 475 480
   His Gly His Glu Ser Asn Lys Phe Leu Glu Glu Tyr Asp Ala Val Leu
   485 490 495
   Lys Gly Leu Val Ala Leu Gly Gly Ile Tyr Leu Leu Phe Ile Ile Glu
   500 505 510
   His Cys Ile Arg Met Phe Lys His Tyr Lys Gln Gln Arg Gly Lys Gln
   515 520 525
   Lys Trp Phe Met Lys Gln Asn Thr Glu Glu Ser Thr Ile Gly Arg Lys
   530 535 540
   Leu Ser Asp His Lys Leu Asn Asn Thr Pro Asp Ser Asp Trp Leu Gln
   545 550 555 560
   Leu Lys Pro Leu Ala Gly Thr Asp Asp Ser Val Val Ser Glu Asp Arg
   565 570 575
   Leu Asn Glu Thr Glu Leu Thr Asp Leu Glu Gly Gln Gln Glu Ser Pro
   580 585 590
   Pro Lys Asn Tyr Leu Cys Ile Glu Glu Glu Lys Ile Ile Asp His Ser
   595 600 605
   His Ser Asp Gly Leu His Thr Ile His Glu His Asp Leu His Ala Ala
   610 615 620
   Ala His Asn His His Gly Glu Asn Lys Thr Val Leu Arg Lys His Asn
   625 630 635 640
   His Gln Trp His His Lys His Ser His His Ser His Gly Pro Cys His
   645 650 655
   Ser Gly Ser Asp Leu Lys Glu Thr Gly Ile Ala Asn Ile Ala Trp Met
   660 665 670
   Val Ile Met Gly Asp Gly Ile His Asn Phe Ser Asp Gly Leu Ala Ile
   675 680 685
   Gly Ala Ala Phe Ser Ala Gly Leu Thr Gly Gly Ile Ser Thr Ser Ile
   690 695 700
   Ala Val Phe Cys His Glu Leu Pro His Glu Leu Gly Asp Phe Ala Val
   705 710 715 720
   Leu Leu Lys Ala Gly Met Thr Val Lys Gln Ala Ile Val Tyr Asn Leu
                   725                 730                 735
   Leu Ser Ala Met Met Ala Tyr Ile Gly Met Leu Ile Gly Thr Ala Val
               740                 745                 750
   Gly Gln Tyr Ala Asn Asn Ile Thr Leu Trp Ile Phe Ala Val Thr Ala
           755                 760                 765
   Gly Met Phe Leu Tyr Val Ala Leu Val Asp Met Leu Pro Glu Met Leu
       770                 775                 780
   His Gly Asp Gly Asp Asn Glu Glu His Gly Phe Cys Pro Val Gly Gln
   785                 790                 795                 800
   Phe Ile Leu Gln Asn Leu Gly Leu Leu Phe Gly Phe Ala Ile Met Leu
                   805                 810                 815
   Val Ile Ala Leu Tyr Glu Asp Lys Ile Val Phe Asp Ile Gln Phe
               820                 825                 830
<210> 16
   <211> 831
   <212> PRT
   <213> Homo sapiens
<400> 16
   Met Lys Val His Met His Thr Lys Phe Cys Leu Ile Cys Leu Leu Thr
   1               5                   10                  15
   Phe Ile Phe His His Cys Asn His Cys His Glu Glu His Asp His Gly
               20                  25                  30
   Pro Glu Ala Leu His Arg Gln His Arg Gly Met Thr Glu Leu Glu Pro
           35                  40                  45
   Ser Lys Phe Ser Lys Gln Ala Ala Glu Asn Glu Lys Lys Tyr Tyr Ile
       50                  55                  60
   Glu Lys Leu Phe Glu Arg Tyr Gly Glu Asn Gly Arg Leu Ser Phe Phe
   65                  70                  75                  80
   Gly Leu Glu Lys Leu Leu Thr Asn Leu Gly Leu Gly Glu Arg Lys Val
                   85                  90                  95
   Val Glu Ile Asn His Glu Asp Leu Gly His Asp His Val Ser His Leu
               100                 105                 110
   Asp Ile Leu Ala Val Gln Glu Gly Lys His Phe His Ser His Asn His
       115                 120                 125
   Gln His Ser His Asn His Leu Asn Ser Glu Asn Gln Thr Val Thr Ser
       130                 135                 140
   Val Ser Thr Lys Arg Asn His Lys Cys Asp Pro Glu Lys Glu Thr Val
   145                 150                 155                 160
   Glu Val Ser Val Lys Ser Asp Asp Lys His Met His Asp His Asn His
                   165                 170                 175
   Arg Leu Arg His His His Arg Leu His His His Leu Asp His Asn Asn
               180                 185                 190
   Thr His His Phe His Asn Asp Ser Ile Thr Pro Ser Glu Arg Gly Glu
           195                 200                 205
   Pro Ser Asn Glu Pro Ser Thr Glu Thr Asn Lys Thr Gln Glu Gln Ser
       210                 215                 220
   Asp Val Lys Leu Pro Lys Gly Lys Arg Lys Lys Lys Gly Arg Lys Ser
   225                 230                 235                 240
   Asn Glu Asn Ser Glu Val Ile Thr Pro Gly Phe Pro Pro Asn His Asp
                   245                 250                 255
   Gln Gly Glu Gln Tyr Glu His Asn Arg Val His Lys Pro Asp Arg Val
               260                 265                 270
   His Asn Pro Gly His Ser His Val His Leu Pro Glu Arg Asn Gly His
           275                 280                 285
   Asp Pro Gly Arg Gly His Gln Asp Leu Asp Pro Asp Asn Glu Gly Glu
       290                 295                 300
   Leu Arg His Thr Arg Lys Arg Glu Ala Pro His Val Lys Asn Asn Ala
   305                 310                 315                 320
   Ile Ile Ser Leu Arg Lys Asp Leu Asn Glu Asp Asp His His His Glu
                   325                 330                 335
   Cys Leu Asn Val Thr Gln Leu Leu Lys Tyr Tyr Gly His Gly Ala Asn
               340                 345                 350
   Ser Pro Ile Ser Thr Asp Leu Phe Thr Tyr Leu Cys Pro Ala Leu Leu
           355                 360                 365
   Tyr Gln Ile Asp Ser Arg Leu Cys Ile Glu His Phe Asp Lys Leu Leu
       370                 375                 380
   Val Glu Asp Ile Asn Lys Asp Lys Asn Leu Val Pro Glu Asp Glu Ala
   385                 390                 395                 400
   Asn Ile Gly Ala Ser Ala Trp Ile Cys Gly Ile Ile Ser Ile Thr Val
                   405                 410                 415
   Ile Ser Leu Leu Ser Leu Leu Gly Val Ile Leu Val Pro Ile Ile Asn
               420                 425                 430
   Gln Gly Cys Phe Lys Phe Leu Leu Thr Phe Leu Val Ala Leu Ala Val
           435                 440                 445
   Gly Thr Met Ser Gly Asp Ala Leu Leu His Leu Leu Pro His Ser Gln
       450                 455                 460
   Gly Gly His Asp His Ser His Gln His Ala His Gly His Gly His Ser
   465                 470                 475                 480
   His Gly His Glu Ser Asn Lys Phe Leu Glu Glu Tyr Asp Ala Val Leu
                   485                 490                 495
   Lys Gly Leu Val Ala Leu Gly Gly Ile Tyr Leu Leu Phe Ile Ile Glu
               500                 505                 510
   His Cys Ile Arg Met Phe Lys His Tyr Lys Gln Gln Arg Gly Lys Gln
           515                 520                 525
   Lys Trp Phe Met Lys Gln Asn Thr Glu Glu Ser Thr Ile Gly Arg Lys
       530                 535                 540
   Leu Ser Asp His Lys Leu Asn Asn Thr Pro Asp Ser Asp Trp Leu Gln
   545                 550                 555                 560
   Leu Lys Pro Leu Ala Gly Thr Asp Asp Ser Val Val Ser Glu Asp Arg
                   565                 570                 575
   Leu Asn Glu Thr Glu Leu Thr Asp Leu Glu Gly Gln Gln Glu Ser Pro
               580                 585                 590
   Pro Lys Asn Tyr Leu Cys Ile Glu Glu Glu Lys Ile Ile Asp His Ser
           595                 600                 605
   His Ser Asp Gly Leu His Thr Ile His Glu His Asp Leu His Ala Ala
   610 615 620
   Ala His Asn His His Gly Glu Asn Lys Thr Val Leu Arg Lys His Asn
   625 630 635 640
   His Gln Trp His His Lys His Ser His His Ser His Gly Pro Cys His
   645 650 655
   Ser Gly Ser Asp Leu Lys Glu Thr Gly Ile Ala Asn Ile Ala Trp Met
   660 665 670
   Val Ile Met Gly Asp Gly Ile His Asn Phe Ser Asp Gly Leu Ala Ile
   675 680 685
   Gly Ala Ala Phe Ser Ala Gly Leu Thr Gly Gly Ile Ser Thr Ser Ile
   690 695 700
   Ala Val Phe Cys His Glu Leu Pro His Glu Leu Gly Asp Phe Ala Val
   705 710 715 720
   Leu Leu Lys Ala Gly Met Thr Val Lys Gln Ala Ile Val Tyr Asn Leu
   725 730 735
   Leu Ser Ala Met Met Ala Tyr Ile Gly Met Leu Ile Gly Thr Ala Val
   740 745 750
   Gly Gln Tyr Ala Asn Asn Ile Thr Leu Trp Ile Phe Ala Val Thr Ala
   755 760 765
   Gly Met Phe Leu Tyr Val Ala Leu Val Asp Met Leu Pro Glu Met Leu
   770 775 780
   His Gly Asp Gly Asp Asn Glu Glu His Gly Phe Cys Pro Val Gly Gln
   785 790 795 800
   Phe Ile Leu Gln Asn Leu Gly Leu Leu Phe Gly Phe Ala Ile Met Leu
   805 810 815
   Val Ile Ala Leu Tyr Glu Asp Lys Ile Val Phe Asp Ile Gln Phe
   820 825 830
<210> 17
   <211> 5227
   <212> DNA
   <213> Homo sapiens
<210> 18
   <211> 5432
   <212> DNA
   <213> Homo sapiens
<400> 18
   agttgatcac tctgaagctt tttggctaaa gcgtttgggt ttagagcttc cattactcat 60
   tcgccttgcc caaggcctca gcaaccgacg ttcgaaagcc aggagaaaag gcgaatgata    120
   aagggcgctc cacgcatgcg ttaagaagcc gccccaactc ccccgcggcg ttctttcttg    180
   gaacaaaact agcgcggagc cacggaactc cgcagtttgc gtagacttga atttcctatt    240
   cctcggacga tccatgtgga atccgaaaaa tagaaatgaa ggtacatatg cacacaaaat    300
   tttgcctcat ttgtttgctg acatttattt ttcatcattg caaccattgc catgaagaac    360
   atgaccatgg ccctgaagcg cttcacagac agcatcgtgg aatgacagaa ttggagccaa    420
   gcaaattttc aaagcaagct gctgaaaatg aaaaaaaata ctatattgaa aaactttttg    480
   agcgttatgg tgaaaatgga agattatcct tttttggttt ggagaaactt ttaacaaact    540
   tgggccttgg agagagaaaa gtagttgaga ttaatcatga ggatcttggc cacgatcatg    600
   tttctcattt agatattttg gcagttcaag agggaaagca ttttcactca cataaccacc    660
   agcattccca taatcattta aattcagaaa atcaaactgt gaccagtgta tccacaaaaa    720
   gaaaccataa atgtgatcca gagaaagaga cagttgaagt gtctgtaaaa tctgatgata    780
   aacatatgca tgaccataat caccgcctac gtcatcacca tcgtttgcat catcatcttg    840
   atcataacaa cactcaccat tttcataatg attccattac tcccagtgag cgtggggagc    900
   ctagcaatga accttcaaca gagaccaata aaacccagga acaatctgat gttaaactac    960
   cgaaaggaaa gaggaagaaa aaagggagga aaagtaatga aaattctgag gttattacac   1020
   caggttttcc ccctaaccat gatcagggtg aacagtatga gcataatcgg gtccacaaac   1080
   ctgatcgtgt acataaccca ggtcattctc atgtacatct tccagaacgt aatggtcatg   1140
   atcctggtcg tggacaccaa gatcttgatc ctgataatga aggtgaactt cgacatacta   1200
   gaaagagaga agcaccacat gttaaaaata atgcaataat ttctttgaga aaagatctaa   1260
   atgaagatga ccatcatcat gaatgtttga acgtcactca gttattaaaa tactatggtc   1320
   atggtgccaa ctctcccatc tcaactgatt tatttacata cctttgccct gcattgttat   1380
   atcaaatcga cagcagactt tgtattgagc attttgacaa acttttagtt gaagatataa   1440
   ataaggataa aaacctggtt cctgaagatg aggcaaatat aggggcatca gcctggattt   1500
   gtggtatcat ttctatcact gtcattagcc tgctttcctt gctaggcgtg atcttggttc   1560
   ctatcattaa ccaaggatgc ttcaaattcc ttcttacatt ccttgttgca ttagctgtag   1620
   gaacaatgag tggagacgcc cttcttcatc tactgcccca ttctcagggt ggacatgatc   1680
   acagtcacca acatgcacat gggcatggac attctcatgg acatgaatct aacaagtttt   1740
   tggaagaata tgatgctgta ttgaaaggac ttgttgctct aggaggcatt tacttgctat   1800
   ttatcattga acactgcatt agaatgttta agcactacaa acaacaaaga ggaaaacaga   1860
   aatggtttat gaaacagaac acagaagaat caactattgg aagaaagctt tcagatcaca   1920
   agttaaacaa tacaccagat tctgactggc ttcaactcaa gcctcttgcc ggaactgatg 1980
   actcggttgt ttctgaagat cgacttaatg aaactgaact gacagattta gaaggccaac 2040
   aagaatcccc tcctaaaaat tacctttgta tagaagagga gaaaatcata gaccattctc 2100
   acagtgatgg attacatacc attcatgagc atgatctcca tgctgctgca cataaccacc 2160
   acggcgagaa caaaactgtg ctgaggaagc ataatcacca gtggcaccac aagcattctc 2220
   atcattccca tggcccctgt cattctggat ccgatctgaa agaaacagga atagctaata 2280
   tagcctggat ggtgatcatg ggggatggca tccacaactt cagtgatggg ctcgcaattg 2340
   gtgcagcttt cagtgctgga ttgacaggag gaatcagtac ttctatagcc gtcttctgtc 2400
   atgaactgcc acatgaatta ggagattttg cagttcttct taaagcaggc atgactgtaa 2460
   agcaagcaat tgtatacaac ctcctctctg ccatgatggc ttacataggc atgctcatag 2520
   gcacagctgt tggtcagtat gccaataaca tcacactttg gatctttgca gtcactgcag 2580
   gcatgttcct ctatgtagcc ttggtggata tgcttccaga aatgttgcat ggtgatggtg 2640
   acaatgaaga acatggcttt tgtcctgtgg ggcaattcat ccttcagaat ttaggattgc 2700
   tctttggatt tgccattatg ctggtgattg ccctctatga agataaaatt gtgtttgaca 2760
   tccagttttg acctttccca gtaatcactg ttgattacga gaatgttacc atgcagcttt 2820
   gcatctgttc cttgtactgt atgcacattg ctcaaaggaa agtcagtggc ttgcactact 2880
   tacaagtttc atagatttga gcctaaccac aagaggctgg tgcttagtac tgttttccct 2940
   gcacgtaggg gtcttttaaa aatataaagc ttgtgataaa gagaggagaa tatgggactc 3000
   catgaaccag tgttgatatg tttgattaag acttttcaca aaataatcat ataaaacact 3060
   agtctcttta ttagtagaaa cttctgtggc tatgcagaaa tagagatcga accaaaaaaa 3120
   atcatttaaa ctttaaaaat attttaaatg gactttgggg agacattttt tgtgtgtttt 3180
   aagaatgaat tgtagtgctc tttaattcag ctacatatat tcatgtggtg atagggatca 3240
   acttgacaca actttgaaac tgcataaagt agacatagga actagaggaa agctcaggct 3300
   gcattagagt atgaatttag cattgggaaa agcccttatt cttgaatcta gagttactat 3360
   ttttgtatat atttgcatag tgtttaaacc tgcagcctaa actactgaaa tttgtgattg 3420
   tatgtttgtg tgagcttcag tttaatgaaa gattcataat ggttctttgt attattataa 3480
   tacttggtgt tggggtgttc tttctgtttt gttttttact ttaattttgt tttgattttt 3540
   tttttttttt tttggcgggg gtaggtgagg gtttggagca tgtggtcttt ttaaaaaatt 3600
   gtaaccctct agaaaatatc aaagaaatga accagacgtg gtttaaatag ttgattttcc 3660
   tattttaaca gtaccaacta gttaattggg aaatgtaagt tctgaatgtt cacattgctt 3720
   taccagtttg gcactggaac caagagcaca tgtcgtggct ggctacaagg ttgtaaagca 3780
   gaaaatcgaa gtttaccatg tctgtaatgt gtacatgaag tgtcaattta gaacagttac 3840
   taggataaac tccattattg ccatggctgt catggtaccc aagtgacttg gaagatgcat 3900
   ttaaattact cagctgaaat cacttgatca tcttgtgcca agatatgctg ttggtgcctg 3960
   atagggatta gtcttttagg tgccctgttc tcctaccata attgtgaatg atttgtgaga 4020
   agtgcaagcc atgtttatcc tgaattttta cttaataatt tgtattacta gtcatatgca 4080
   tgtagctttc tgtttacatc ctatgccaca tggtcttcat ttatgccagg taaactgtat 4140
   ttgaactatg tgcagctagc tttgttttaa tctgcttggc aaccagtgta gctgctgtaa 4200
   caatctatct tattgttcaa atatataaga gccaaactct tttccattcc atctaaaatg 4260
   ttttcattta gtactcttct ttcctcctac tctatgaact tcaaaacaaa aacaaaactt 4320
   tgagagcagc acatgcatcc aggtatttat agattattgc cagtgtcttt tctgtatgct 4380
   ataagcaagg gagcttaggt gttatttctt taatttatgc ttgaatctga aaaattattt 4440
   ctgacttact ccatggcctc cttataataa gtagaagttt tatatataat taattttcag 4500
   cattgggcac tgaattagga cagtcctcat ctcattgctt ggcccttcaa gcaacctagc 4560
   taaaaggtgc tgatatttta tttagtactg ccaacttcaa gtgatttaga tatctatcta 4620
   tctagatttc tgaaccaaga tatatttata gttcactttt gggtttttat acccacggta 4680
   ggattctgca ttccagcatt aaatctgctt cattttagaa cctttataaa agcaatagct 4740
   ggaatatact cccagtttta aaataaatgc ctgattgatt taaagcaagt aggttatgct 4800
   gaagtatata aagaagtttt atattctctc aaaaatggta ttatctttct ttatttgcta 4860
   gattcttaca aatcttttaa gagggctgta acagttgctg ctagtattag ggttccacat 4920
   cattctaatg tatagtttca agtcttaata gacaatctga attccactac atttcttttg 4980
   gctccaacat tccttttagc ttgaccagtc taatttaaaa tgtgtttgtt ggaggtcatt 5040
   aacgttactt gtacaatgct gtcactgtgt gacatccata tgaattttgg tatatatcaa 5100
   tcaatcaatc aatcacattg cattcaatca atcagctgtg attgattgat tatgcttaga 5160
   aatactatag taactagatg cagtgtgaat tttttccatt aacaaacaaa caagtcagtg 5220
   gcttaaatgt gattatggtc ctgcaaggtg attcttgcta aaatatctaa acttttgttt 5280
   tgttttaact gaatcatttt ttaacttaaa aagctggaaa atatcaaatg ctgttttttt 5340
   tttttcattg tcaacagtgg tgtgtcattt tatgtatgtt cctaatgctt atggaactcc 5400
   tccaaaataa agttactcaa agagagcaaa ta 5432
<210> 19
   <211> 132
   <212> PRT
   <213> Homo sapiens
<400> 19
   Met Ser Gln Gly Arg His Leu Leu Glu Phe Leu Pro Leu Tyr Ile Ala
   1               5                   10                  15
   Phe Met Leu Arg Gly Val Cys Arg Ile Asp Ala Gly Ser Leu Asn Pro
               20                  25                  30
   Glu Leu Phe Leu Pro Met Leu His Glu Glu Asp Trp Cys Trp Glu Ile
           35                  40                  45
   Ala Gly His Val Asp Ser Gln Glu Leu Phe Val Gly Leu Phe Ser Ser
       50                  55                  60
   Thr Ser Thr Gly His Ala Glu Leu Asp Lys Lys Val Asn Gly Leu Tyr
   65                  70                  75                  80
   Tyr Asp Ser Val Phe Gln Leu Ser Leu Asp Arg Met Arg His Thr Arg
                   85                  90                  95
   Ser Met Ala Arg Val Glu Arg Leu Arg His Arg Lys Ala Ile Gln Lys
               100                 105                 110
   Lys Thr Gln Leu Val His His Leu Leu Phe Lys Gly Trp Ala Ser Asp
           115                 120                 125
   Glu Thr Glu Ile
       130
<210> 20
   <211> 399
   <212> DNA
   <213> Homo sapiens
<400> 20
   atgtcacaag gcaggcatct tcttgagttt cttccattgt acatagcttt catgttacgt     60
   ggggtttgta ggatagacgc tggaagcctt aatccagaac tgtttttgcc aatgttacat    120
   gaagaggatt ggtgttggga gatagctggc catgtggact cccaagagtt attcgttggt    180
   ttgttttcta gtacctctac tgggcatgca gagctggaca aaaaggttaa tggactttat    240
   tatgactctg tattccagtt gtctctggac cgtatgcgtc atacaaggag tatggctaga    300
   gtagagaggc tgagacacag gaaagcgatc cagaaaaaga ctcagttagt ccatcatctg    360
   ctatttaaag gatgggcttc tgatgaaact gaaatttag                           399
<210> 21
   <211> 316
   <212> PRT
   <213> Homo sapiens
<400> 21
   Met Ala Ser Ala Asp Glu Leu Thr Phe His Glu Phe Glu Glu Ala Thr
   1               5                   10                  15
   Asn Leu Leu Ala Asp Thr Pro Asp Ala Ala Thr Thr Ser Arg Ser Asp
               20                  25                  30
   Gln Leu Thr Pro Gln Gly His Val Ala Val Ala Val Gly Ser Gly Gly
           35                  40                  45
   Ser Tyr Gly Ala Glu Asp Glu Val Glu Glu Glu Ser Asp Lys Ala Ala
       50                  55                  60
   Leu Leu Gln Glu Gln Gln Gln Gln Gln Gln Pro Gly Phe Trp Thr Phe
   65                  70                  75                  80
   Ser Tyr Tyr Gln Ser Phe Phe Asp Val Asp Thr Ser Gln Val Leu Asp
                   85                  90                  95
   Arg Ile Lys Gly Ser Leu Leu Pro Arg Pro Gly His Asn Phe Val Arg
               100                 105                 110
   His His Leu Arg Asn Arg Pro Asp Leu Tyr Gly Pro Phe Trp Ile Cys
           115                 120                 125
   Ala Thr Leu Ala Phe Val Leu Ala Val Thr Gly Asn Leu Thr Leu Val
       130                 135                 140
   Leu Ala Gln Arg Arg Asp Pro Ser Ile His Tyr Ser Pro Gln Phe His
   145                 150                 155                 160
   Lys Val Thr Val Ala Gly Ile Ser Ile Tyr Cys Tyr Ala Trp Leu Val
                   165                 170                 175
   Pro Leu Ala Leu Trp Gly Phe Leu Arg Trp Arg Lys Gly Val Gln Glu
               180                 185                 190
   Arg Met Gly Pro Tyr Thr Phe Leu Glu Thr Val Cys Ile Tyr Gly Tyr
           195                 200                 205
   Ser Leu Phe Val Phe Ile Pro Met Val Val Leu Trp Leu Ile Pro Val
       210                 215                 220
   Pro Trp Leu Gln Trp Leu Phe Gly Ala Leu Ala Leu Gly Leu Ser Ala
   225 230 235 240
   Ala Gly Leu Val Phe Thr Leu Trp Pro Val Val Arg Glu Asp Thr Arg
   245 250 255
   Leu Val Ala Thr Val Leu Leu Ser Val Val Val Leu Leu His Ala Leu
   260 265 270
   Leu Ala Met Gly Cys Lys Leu Tyr Phe Phe Gln Ser Leu Pro Pro Glu
   275 280 285
   Asn Val Ala Pro Pro Pro Gln Ile Thr Ser Leu Pro Ser Asn Ile Ala
   290 295 300
   Leu Ser Pro Thr Leu Pro Gln Ser Leu Ala Pro Ser
   305 310 315
<210> 22
   <211> 316
   <212> PRT
   <213> Homo sapiens
<400> 22
   Met Ala Ser Ala Asp Glu Leu Thr Phe His Glu Phe Glu Glu Ala Thr
   1 5 10 15
   Asn Leu Leu Ala Asp Thr Pro Asp Ala Ala Thr Thr Ser Arg Ser Asp
   20 25 30
   Gln Leu Thr Pro Gln Gly His Val Ala Val Ala Val Gly Ser Gly Gly
   35 40 45
   Ser Tyr Gly Ala Glu Asp Glu Val Glu Glu Glu Ser Asp Lys Ala Ala
   50 55 60
   Leu Leu Gln Glu Gln Gln Gln Gln Gln Gln Pro Gly Phe Trp Thr Phe
   65 70 75 80
   Ser Tyr Tyr Gln Ser Phe Phe Asp Val Asp Thr Ser Gln Val Leu Asp
   85 90 95
   Arg Ile Lys Gly Ser Leu Leu Pro Arg Pro Gly His Asn Phe Val Arg
   100 105 110
   His His Leu Arg Asn Arg Pro Asp Leu Tyr Gly Pro Phe Trp Ile Cys
   115 120 125
   Ala Thr Leu Ala Phe Val Leu Ala Val Thr Gly Asn Leu Thr Leu Val
       130                 135                 140
   Leu Ala Gln Arg Arg Asp Pro Ser Ile His Tyr Ser Pro Gln Phe His
   145                 150                 155                 160
   Lys Val Thr Val Ala Gly Ile Ser Ile Tyr Cys Tyr Ala Trp Leu Val
                   165                 170                 175
   Pro Leu Ala Leu Trp Gly Phe Leu Arg Trp Arg Lys Gly Val Gln Glu
               180                 185                 190
   Arg Met Gly Pro Tyr Thr Phe Leu Glu Thr Val Cys Ile Tyr Gly Tyr
           195                 200                 205
   Ser Leu Phe Val Phe Ile Pro Met Val Val Leu Trp Leu Ile Pro Val
       210                 215                 220
   Pro Trp Leu Gln Trp Leu Phe Gly Ala Leu Ala Leu Gly Leu Ser Ala
   225                 230                 235                 240
   Ala Gly Leu Val Phe Thr Leu Trp Pro Val Val Arg Glu Asp Thr Arg
                   245                 250                 255
   Leu Val Ala Thr Val Leu Leu Ser Val Val Val Leu Leu His Ala Leu
               260                 265                 270
   Leu Ala Met Gly Cys Lys Leu Tyr Phe Phe Gln Ser Leu Pro Pro Glu
           275                 280                 285
   Asn Val Ala Pro Pro Pro Gln Ile Thr Ser Leu Pro Ser Asn Ile Ala
       290                 295                 300
   Leu Ser Pro Thr Leu Pro Gln Ser Leu Ala Pro Ser
   305                 310                 315
<210> 23
   <211> 1210
   <212> DNA
   <213> Homo sapiens
<400> 23
   aggtgagggg gggcggagcg cacctgtggg gacgggacga cgagttcaag cctccgtggg     60
   tgcagttggt cgccagcgag ggatgcggag acgcccctga acgaccatgg catcggccga    120
   cgagctgacc ttccatgaat tcgaggaggc cactaatctt ctggctgaca ccccagatgc    180
   agccaccacc agcagaagcg atcagctgac cccacaaggg cacgtggctg tggccgtggg    240
   ctcaggtggc agctatggag ccgaggatga ggtggaggag gagagtgaca aggccgcgct    300
   cctgcaggag cagcagcagc agcagcagcc gggattctgg accttcagct actatcagag    360
   cttctttgac gtggacacct cacaggtcct ggaccggatc aaaggctcac tgctgccccg    420
   gcctggccac aactttgtgc ggcaccatct gcggaatcgg ccggatctgt atggcccctt    480
   ctggatctgt gccacgttgg cctttgtcct ggccgtcact ggcaacctga cgctggtgct    540
   ggcccagagg agggacccct ccatccacta cagcccccag ttccacaagg tgaccgtggc    600
   aggcatcagc atctactgct atgcgtggct ggtgcccctg gccctgtggg gcttcctgcg    660
   gtggcgcaag ggtgtccagg agcgcatggg gccctacacc ttcctggaga ctgtgtgcat    720
   ctacggctac tccctctttg tcttcatccc catggtggtc ctgtggctca tccctgtgcc    780
   ttggctgcag tggctctttg gggcgctggc cctgggcctg tcagccgccg ggctggtatt    840
   caccctctgg cccgtggtcc gtgaggacac caggctggtg gccacagtgc tgctgtccgt    900
   ggtcgtgctg ctccacgccc tcctggccat gggctgtaag ttgtacttct tccagtcgct    960
   gcctccggag aacgtggctc ctccacccca aatcacatct ctgccctcaa acatcgcgct   1020
   gtcccctacc ttgccgcagt ccctggcccc ctcctaggaa ggcccgggtc ccacaggcaa   1080
   cacctaagtg gaccaacccc tctgcctgtc ctgcccccca gacgatgact gaaggctcct   1140
   ttgacacctt gagatgattc tgctactttc cagacttttc ttacaaagca aacactttta   1200
   ttttctatgc                                                          1210
<210> 24
   <211> 1502
   <212> DNA
   <213> Homo sapiens
<400> 24
   ggatgttgct gtcaggtctg agtcggttgg agtctgacgg gtaggcgaga cgcgcaggcg 60
   cagagagccc cagccacgcc ggcccaggtg gcctcagcga gggatgcgga gacgcccctg 120
   aacgaccatg gcatcggccg acgagctgac cttccatgaa ttcgaggagg ccactaatct 180
   tctggctgac accccagatg cagccaccac cagcagaagc gatcagctga ccccacaagg 240
   gcacgtggct gtggccgtgg gctcaggtgg cagctatgga gccgaggatg aggtggagga 300
   ggagagtgac aaggccgcgc tcctgcagga gcagcagcag cagcagcagc cgggattctg 360
   gaccttcagc tactatcaga gcttctttga cgtggacacc tcacaggtcc tggaccggat 420
   caaaggctca ctgctgcccc ggcctggcca caactttgtg cggcaccatc tgcggaatcg 480
   gccggatctg tatggcccct tctggatctg tgccacgttg gcctttgtcc tggccgtcac 540
   tggcaacctg acgctggtgc tggcccagag gagggacccc tccatccact acagccccca 600
   gttccacaag gtgaccgtgg caggcatcag catctactgc tatgcgtggc tggtgcccct 660
   ggccctgtgg ggcttcctgc ggtggcgcaa gggtgtccag gagcgcatgg ggccctacac 720
   cttcctggag actgtgtgca tctacggcta ctccctcttt gtcttcatcc ccatggtggt 780
   cctgtggctc atccctgtgc cttggctgca gtggctcttt ggggcgctgg ccctgggcct 840
   gtcagccgcc gggctggtat tcaccctctg gcccgtggtc cgtgaggaca ccaggctggt 900
   ggccacagtg ctgctgtccg tggtcgtgct gctccacgcc ctcctggcca tgggctgtaa 960
   gttgtacttc ttccagtcgc tgcctccgga gaacgtggct cctccacccc aaatcacatc 1020
   tctgccctca aacatcgcgc tgtcccctac cttgccgcag tccctggccc cctcctagga 1080
   aggcccgggt cccacaggca acacctaagt ggaccaaccc ctctgcctgt cctgcccccc 1140
   agacgatgac tgaaggctcc tttgacacct tgagatgatt ctgctacttt ccagactttt 1200
   cttacaaagc aaacactttt attttctatg caaaggtgat tcagagaatt tatataaagg 1260
   cgggcgaggg gcagccgagc agggagcttt gggacagggc tggggccccc atatcccccc 1320
   cgggccacct gctttccctc ctatggctcc cctggaacag gagggagagc caagggggcg 1380
   gcccagcctg gacagcgccc gctcctgcct gggtgcacac acggcgggcc tgagctccag 1440
   catctgagtt tgggggtatg agaaacaggg gagcagaagg agaagaaaac tgcctgtgct 1500
   gc 1502
<210> 25
   <211> 131
   <212> PRT
   <213> Homo sapiens
<400> 25
   Met Phe Leu Gly Leu Val Gly Leu Arg Thr Lys Gly Arg Arg Trp Ile
   1               5                   10                  15
   Ser Ser Trp Ser Glu Gly Glu Asp Arg Gly Gln Ser Pro Glu Gly Val
               20                  25                  30
   Leu Leu Thr Trp Val Phe Gly Thr Lys Cys Val Met His Pro Cys Glu
           35                  40                  45
   Glu Thr Thr Lys Gln Ala Leu Cys Glu Gln Gln Gly Cys Leu Phe His
       50                  55                  60
   Leu Gly Ala Asp Glu Leu Ser Pro Lys Arg Glu Ser Ala Gln Ser Ile
   65                  70                  75                  80
   Ser Phe Lys Trp Glu Asn Ser Ile Tyr Leu His Ala Thr Leu Phe Leu
                   85                  90                  95
   Ile Gly Glu Tyr Leu His Leu Ala Phe Tyr Tyr Phe Leu Leu Val Leu
               100                 105                 110
   Tyr Ile Leu Cys Ser Phe Leu Ser Tyr Cys Leu Leu Leu Trp Leu Gly
           115                 120                 125
   Ser Phe Leu
       130
<210> 26
   <211> 2950
   <212> DNA
   <213> Homo sapiens
<400> 26
   attatctagg tctcggagga tggagaaatc aaaagtgcca ttttctggcc atttagaacc 60
   attgtcgagt ttgtattggg gccaagcagt gttgcagaag aaaataagac atttagattt 120
   tagttcaggt gatagttgaa gaaattttaa gttcttgaga acacaggcta agggagaaga 180
   aggaggaatg gagggtggaa gtttgcccat agtgaaggag gcaagtttaa agagaaaggt 240
   agagacatgg agaaagggtt ggggagcagc cctgggctgc aatgtgggtg agcagccaaa 300
   gcaggcatcc ccgcaattga cttgccacca agggaatgtg gttgaatgac caaggcaggc 360
   atccctgaag atatcagacg ccaatggaat gtgggtgaat aatcaggcag gcatccccgg 420
   aatgattaaa cactaaggga aggctgcctt cctgagtaca tgaccagcac cagagttttg 480
   ggtccatgga taaaatgtgt ctcctttgtc tctactagaa aatgaaagga attgaaatta 540
   agagaagaga gggagtgaag ggtggcacca agaatgaaag gagaaagagg ttgagggata 600
   gtgagaaagg ttggagaaga gagtaaaaag aggccactta cccgatttaa aatttgtgag 660
   atgttccttg ggctggttgg tctgaggacc aaaggtcgta ggtggatctc ttcatggagt 720
   gagggtgagg acaggggaca gtctcctgaa ggagtcctgc tgacctgggt ctttggcacc 780
   aaatgtgtca tgcatccatg tgaagagacc accaaacagg ctttgtgtga gcaacagggc 840
   tgtttgtttc acctgggtgc agacgagttg agtccaaaaa gagagtcagc ccagtctata 900
   tcttttaaat gggaaaattc aatctattta catgcaacgt tattcttgat aggtgagtac 960
   ttacatctag cattctatta ttttctgctt gttttgtata tcctttgttc cttcctctct 1020
   tactgtttac ttttgtggtt gggcagtttt ctgtagggat aagatttgag tcttatctct 1080
   ttctccctat gtgttagctt taccagtgag tttttatagt ttcacatatt tttatgatgc 1140
   tggttatcat cttctctgtg gggaacaggc cccccaaaac ctggccataa actggcccca 1200
   aaactggcca taaacaaaat ctctgcagca ctgtgacatg tacatgatgg tcttaacgcc 1260
   cacgctggaa ggttgtgggt ttaccagaat gagggcaagg aacacctggc ccacccaggg 1320
   tggaaaaccg cttaaaggca ttcttaaacc acaaacaata gcatgagtga tctgtgcctt    1380
   aaggccatgc tcctgctgca gatagctagt ccaacccatc cctttatttc agcccatctc    1440
   ttcatttccc ataaggaata attttagtta atctaatatc tatagaaaga atgctaatga    1500
   ctagcttgct gttaataaat acatgggtaa acctctgttg gaggctctca gctctgaagg    1560
   ctgtgagacc cttgatttcc tacttcactc ctctatattt ctgtgtcttt aattcctcta    1620
   gtgccactgg gttagagtct ccccgaccaa gctggtctca gcaagtggtc tccatcatgg    1680
   gggctcgaat ccaggttgaa gggtcaccag agtgatggtt ggagaacatg gaactagctg    1740
   gaggacacct gagtactctt aaagcaaacc ccgtggtgag taagaagggg agctcagaag    1800
   catcagggta acaatgggac aagtgtgggg tctggttcgt tccatcttgg aactttttca    1860
   cactgatgat gaggaagaag gagagtataa tgaagtaaca gaagaggtta tagagcaggt    1920
   ttatttgcca gctaaagcta aagtggcaaa ggagggagag gttcatccct acccttctgc    1980
   accccctcat tattattttg aagaaaaaga gtggcctgac cctccagatc tttcttttcc    2040
   agaggacagt gggcaaaaat tagttgcccc agtgactgtt caagcagcac ctcgagcgac    2100
   tgctcttagt tctattcagt caggaattca gcaagctaga tgagaaggtg attaagaggc    2160
   ttggcagttc cctgttagac tacactgccc agaccaacag ggaaatattg tagctacatt    2220
   tgagcctttt tgttttaaat tactcaaaga atttaaacaa gctattaatc agtatggacc    2280
   aggttctcct tttgtaatgg gactattaaa gaacattgct gtttccagtc agatgattcc    2340
   tactgactgg gacgctctta ctcaagcttg tctaactcct gcttagttct tacaatttaa    2400
   aacttggtgg gcagatgaag cttccattca ggcttctcac aacacgcagg accaacctca    2460
   aattaatata actgcagacc aacttttggg ggttggcagt tgggctggtt tagatgcaca    2520
   aatggtcatg caggatgatg ccatagaaca gcttagagga gcgtgcatta gagcttgggg    2580
   aaaaaaaatc acttcaagtg gagaacaata ccctttcttt agtgctataa aacagggacc    2640
   agaagaatca tatgtggatt ttatagctca gttacaggag tctcttaaaa agatgactgc    2700
   agatttggct gctcaggata tagtgttgca attattagct ttcaacaatg ctaatcctga    2760
   ttgccaggct gctctgtgac ctatcagagg gaaagcacat ttagttgatt atatcaaggc    2820
   ctgtggtggt atcagaggta atctgcatca ggccacctgc tagcacgggc aatggcagga    2880
   ctgagagtgg atacagaaag tactccattt cctggagctt gttttaactg tgggaagcat    2940
   ggtcatactg                                                           2950
<210> 27
   <211> 173
   <212> PRT
   <213> Homo sapiens
<400> 27
   Met Thr Leu Cys His Arg Asp Ser Phe Gly Ser Trp His Leu Phe His
   1               5                   10                  15
   Leu Leu Leu Leu Glu Tyr Met Ile His Ile Leu Gln Ser Cys Leu Glu
               20                  25                  30
   Glu Glu Glu Glu Glu Glu Asp Met Gly Thr Val Lys Glu Met Leu Pro
           35                  40                  45
   Asp Asp Pro Thr Leu Gly Gln Pro Asp Gln Ala Leu Phe His Ser Leu
       50                  55                  60
   Asn Ser Ser Leu Ser Gln Ala Cys Ala Ser Pro Ser Met Glu Pro Leu
   65                  70                  75                  80
   Gly Val Met Pro Thr His Met Gly Gln Gly Arg Tyr Pro Val Gly Val
                   85                  90                  95
   Ser Asn Met Val Leu Arg Ile Leu Gly Phe Leu Val Asp Thr Ala Met
               100                 105                 110
   Gly Asn Lys Leu Ile Gln Val Leu Leu Glu Asp Glu Thr Thr Glu Ser
           115                 120                 125
   Ala Val Lys Leu Ser Leu Pro Met Gly Gln Glu Ala Leu Ile Thr Leu
       130                 135                 140
   Lys Asp Gly Gln Gln Phe Val Ile Gln Ile Ser Asp Val Pro Gln Ser
   145                 150                 155                 160
   Ser Glu Asp Ile Tyr Phe Arg Glu Asn Asn Ala Asn Val
                   165                 170
<210> 28
   <211> 1907
   <212> DNA
   <213> Homo sapiens
<400> 28
   ataactccaa cgctcaagca agtcaaggac acccacggac tcaacaccgc gaccagattg      60
   gaaaaggtgt tggtcgacaa cttctgcatt tgcgaagagt gcagcgtccc tcgctgtctc     120
   atgtatgaga tttacgtgga gacctgtggg caaaacactg agaaccaagt caacccggcc     180
   acctttggga agcttgtgag attggttttt ccggaccttg gcacccggag gctgggcact     240
   agaggaagtg ccaggtatca ttatgatgga atctgtatca agaaaagctc tttcttctat     300
   gcccagtatt gctacctgat aggtgaaaaa aggtatcaca gtggagatgc cattgccttt 360
   gaaaaatcta ctaattataa cagcattatc caacaagaag caacatgtga agatcattca 420
   ccgatgaaga cagacccagt tggatcccct ttgtctgaat tcaggagatg tccatttctg 480
   gagcaagaac aggcaaagaa atactcctgt aatatgatgg ccttccttgc tgacgaatac 540
   tgcaactatt gtcgagacat tttacgaaat gtgaggaact gagaacttga gagggtggag 600
   gacttgctta cttccttctg gaagtctctg cagcaagaca cagtcatgct gatgtcattg 660
   cctgacgtgt gccagctctt taaatgctac gacgtccagc tgtacaaggg aattgaggat 720
   gttctccttc atgacttctt ggaagatgtt tctattcagt acctgaaatc tgtgcagtta 780
   tttagtaaga aatttaagct gtggctcctt aatgctttgg aaggtgttcc agccctcttg 840
   cagatctcca aactcaaaga ctatgcgaat ggtattgaaa agtaagaggc gtgtcagcgt 900
   tttgaagtca gatctacagg ccatcatcaa tcaaggcact ttggctactt ctaagaaagc 960
   cctggcaagt gaccggagtg gcgcagatga actggagaac aacccagaga tgaaatgttt 1020
   aagaaactta atttctttgc tgggaacatc aacagatctc agggtattcc tcagctgtct 1080
   gtcttcacat ctccaagcat ttgtgttcca gacaagcaga agcaaagaag agtttaccaa 1140
   attggccgcc agcttccagc tgagatggaa tcttcttctc actgctgtaa gcaaagccat 1200
   gaccctctgc cacagagata gttttggctc ctggcatctg tttcacttgt tgcttttgga 1260
   atatatgatt catatacttc agtcatgcct agaggaggaa gaggaggagg aggacatggg 1320
   gactgtcaag gaaatgctac cagatgaccc gactctcggc cagccagacc aggcactttt 1380
   ccattctctg aattcctcac tgtcgcaggc gtgtgccagc cccagcatgg agccactggg 1440
   ggtgatgccc acacacatgg gccagggccg atatcccgtg ggtgtgagca acatggtcct 1500
   caggatcctg ggcttcctgg tggacactgc catgggcaat aagctcatcc aggtgctgtt 1560
   ggaagatgaa accactgaaa gcgcagttaa actcagcctt cctatgggac aagaagccct 1620
   cataacccta aaagatggac aacaatttgt gattcagata tcagatgtac cccaaagctc 1680
   tgaagatatt tatttcagag aaaacaatgc taatgtgtga gattatttat ttgaatagag 1740
   aataagaaaa ctgatagact tgcattctta aaaatattaa atactaaagt ttttctattg 1800
   acgaaagatg atgttatgta tataatagat gtagcattgt ctattttatg tttatatgta 1860
   tttcaaggag gtggtttcga taaaatatgt aaactgattt ggagaat 1907
<210> 29
   <211> 458
   <212> PRT
   <213> Homo sapiens
<400> 29
   Met Glu Gly Ser Ala Ser Pro Pro Glu Lys Pro Arg Ala Arg Pro Ala
   1               5                   10                  15
   Ala Ala Val Leu Cys Arg Gly Pro Val Glu Pro Leu Val Phe Leu Ala
               20                  25                  30
   Asn Phe Ala Leu Val Leu Gln Gly Pro Leu Thr Thr Gln Tyr Leu Trp
           35                  40                  45
   His Arg Phe Ser Ala Asp Leu Gly Tyr Asn Gly Thr Arg Gln Arg Gly
       50                  55                  60
   Gly Cys Ser Asn Arg Ser Ala Asp Pro Thr Met Gln Glu Val Glu Thr
   65                  70                  75                  80
   Leu Thr Ser His Trp Thr Leu Tyr Met Asn Val Gly Gly Phe Leu Val
                   85                  90                  95
   Gly Leu Phe Ser Ser Thr Leu Leu Gly Ala Trp Ser Asp Ser Val Gly
               100                 105                 110
   Arg Arg Pro Leu Leu Val Leu Ala Ser Leu Gly Leu Leu Leu Gln Ala
           115                 120                 125
   Leu Val Ser Val Phe Val Val Gln Leu Gln Leu His Val Gly Tyr Phe
       130                 135                 140
   Val Leu Gly Arg Ile Leu Cys Ala Leu Leu Gly Asp Phe Gly Gly Leu
   145                 150                 155                 160
   Leu Ala Ala Ser Phe Ala Ser Val Ala Asp Val Ser Ser Ser Arg Ser
                   165                 170                 175
   Arg Thr Phe Arg Met Ala Leu Leu Glu Ala Ser Ile Gly Val Ala Gly
               180                 185                 190
   Met Leu Ala Ser Leu Leu Gly Gly His Trp Leu Arg Ala Gln Gly Tyr
           195                 200                 205
   Ala Asn Pro Phe Trp Leu Ala Leu Ala Leu Leu Ile Ala Met Thr Leu
       210                 215                 220
   Tyr Ala Ala Phe Cys Phe Gly Glu Thr Leu Lys Glu Pro Lys Ser Thr
   225                 230                 235                 240
   Arg Leu Phe Thr Phe Arg His His Arg Ser Ile Val Gln Leu Tyr Val
   245 250 255
   Ala Pro Ala Pro Glu Lys Ser Arg Lys His Leu Ala Leu Tyr Ser Leu
   260 265 270
   Ala Ile Phe Val Val Ile Thr Val His Phe Gly Ala Gln Asp Ile Leu
   275 280 285
   Thr Leu Tyr Glu Leu Ser Thr Pro Leu Cys Trp Asp Ser Lys Leu Ile
   290 295 300
   Gly Tyr Gly Ser Ala Ala Gln His Leu Pro Tyr Leu Thr Ser Leu Leu
   305 310 315 320
   Ala Leu Lys Leu Leu Gln Tyr Cys Leu Ala Asp Ala Trp Val Ala Glu
   325 330 335
   Ile Gly Leu Ala Phe Asn Ile Leu Gly Met Val Val Phe Ala Phe Ala
   340 345 350
   Thr Ile Thr Pro Leu Met Phe Thr Gly Tyr Gly Leu Leu Phe Leu Ser
   355 360 365
   Leu Val Ile Thr Pro Val Ile Arg Ala Lys Leu Ser Lys Leu Val Arg
   370 375 380
   Glu Thr Glu Gln Gly Ala Leu Phe Ser Ala Val Ala Cys Val Asn Ser
   385 390 395 400
   Leu Ala Met Leu Thr Ala Ser Gly Phe Asn Ser Leu Tyr Pro Ala Thr
   405 410 415
   Leu Asn Phe Met Lys Gly Phe Pro Phe Leu Leu Gly Ala Gly Leu Leu
   420 425 430
   Leu Ile Pro Ala Val Leu Ile Gly Met Leu Glu Lys Ala Asp Pro His
   435 440 445
   Leu Glu Phe Gln Gln Phe Pro Gln Ser Pro
   450 455
<210> 30
   <211> 430
   <212> PRT
   <213> Homo sapiens
<400> 30
   Met Glu Gly Ser Ala Ser Pro Pro Glu Lys Pro Arg Ala Arg Pro Ala
   1 5 10 15
   Ala Ala Val Leu Cys Arg Gly Pro Val Glu Pro Leu Val Phe Leu Ala
   20 25 30
   Asn Phe Ala Leu Val Leu Gln Gly Pro Leu Thr Thr Gln Tyr Leu Trp
   35 40 45
   His Arg Phe Ser Ala Asp Leu Gly Tyr Asn Gly Thr Arg Gln Arg Gly
   50 55 60
   Gly Cys Ser Asn Arg Ser Ala Asp Pro Thr Met Gln Glu Val Glu Thr
   65 70 75 80
   Leu Thr Ser His Trp Thr Leu Tyr Met Asn Val Gly Gly Phe Leu Val
   85 90 95
   Gly Leu Phe Ser Ser Thr Leu Leu Gly Ala Trp Ser Asp Ser Val Gly
   100 105 110
   Arg Arg Pro Leu Leu Val Leu Ala Ser Leu Gly Leu Leu Leu Gln Ala
   115 120 125
   Leu Val Ser Val Phe Val Val Gln Leu Gln Leu His Val Gly Tyr Phe
   130 135 140
   Val Leu Gly Arg Ile Leu Cys Ala Leu Leu Gly Asp Phe Gly Gly Leu
   145 150 155 160
   Leu Ala Ala Ser Phe Ala Ser Val Ala Asp Val Ser Ser Ser Arg Ser
   165 170 175
   Arg Thr Phe Arg Met Ala Leu Leu Glu Ala Ser Ile Gly Val Ala Gly
   180 185 190
   Met Leu Ala Ser Leu Leu Gly Gly His Trp Leu Arg Ala Gln Gly Tyr
   195 200 205
   Ala Asn Pro Phe Trp Leu Ala Leu Ala Leu Leu Ile Ala Met Thr Leu
   210 215 220
   Tyr Ala Ala Phe Cys Phe Gly Glu Thr Leu Lys Glu Pro Lys Ser Thr
   225 230 235 240
   Arg Leu Phe Thr Phe Arg His His Arg Ser Ile Val Gln Leu Tyr Val
   245 250 255
   Ala Pro Ala Pro Glu Lys Ser Arg Lys His Leu Ala Leu Tyr Ser Leu
               260                 265                 270
   Ala Ile Phe Val Val Ile Thr Val His Phe Gly Ala Gln Asp Ile Leu
           275                 280                 285
   Thr Leu Tyr Glu Leu Ser Thr Pro Leu Cys Trp Asp Ser Lys Leu Ile
       290                 295                 300
   Gly Tyr Gly Ser Ala Ala Gln His Leu Pro Tyr Leu Thr Ser Leu Leu
   305                 310                 315                 320
   Ala Leu Lys Leu Leu Gln Tyr Cys Leu Ala Asp Ala Trp Val Ala Glu
                   325                 330                 335
   Ile Gly Leu Ala Phe Asn Ile Leu Gly Met Val Val Phe Ala Phe Ala
               340                 345                 350
   Thr Ile Thr Pro Leu Met Phe Thr Gly Ala Leu Phe Ser Ala Val Ala
           355                 360                 365
   Cys Val Asn Ser Leu Ala Met Leu Thr Ala Ser Gly Phe Asn Ser Leu
       370                 375                 380
   Tyr Pro Ala Thr Leu Asn Phe Met Lys Gly Phe Pro Phe Leu Leu Gly
   385                 390                 395                 400
   Ala Gly Leu Leu Leu Ile Pro Ala Val Leu Ile Gly Met Leu Glu Lys
                   405                 410                 415
   Ala Asp Pro His Leu Glu Phe Gln Gln Phe Pro Gln Ser Pro
               420                 425                 430
<210> 31
   <211> 6484
   <212> DNA
   <213> Homo sapiens
<400> 31
   acagcgcaag ccccacgcca cgcgtcgctg gtcccaggca gcgagtcgct cgcgcgcccc      60
   gccgcccgcc tggcgacagc tccgccgcgc acgcacatgg aggggagcgc gagccccccg     120
   gaaaagcccc gcgcccgccc tgcggctgcc gtgctgtgcc ggggcccggt agagccgctg     180
   gtcttcctgg ccaactttgc cttggtcctg cagggcccgc tcaccacgca gtatctgtgg     240
   caccgcttca gcgccgacct cggctacaat ggcacccgcc aaaggggggg ctgcagcaac     300
   cgcagcgcgg accccaccat gcaggaagtg gagaccctta cctcccactg gaccctctac 360
   atgaacgtgg gcggcttcct ggtggggctc ttctcgtcca ccctgctggg agcttggagc 420
   gacagtgtgg gccgccgccc gctgctagtg ctggcctcgc tgggcctgct gctccaggcc 480
   ctagtgtccg tttttgtggt gcagctgcag ctccacgtcg gctacttcgt gctgggtcgc 540
   atcctttgtg ccctcctcgg cgacttcggt ggccttctgg ctgctagctt tgcgtccgtg 600
   gcagatgtca gctccagtcg cagccgcacc ttccggatgg ccctgctgga agccagcatc 660
   ggggtggctg ggatgctggc aagcctcctc gggggccact ggctccgggc ccagggttat 720
   gccaacccct tctggctggc cttggccttg ctgatagcca tgactctcta tgcagctttc 780
   tgctttggtg agaccttaaa ggagccaaag tccacccggc tcttcacgtt ccgtcaccac 840
   cgatccattg tccagctcta tgtggctccc gccccagaga agtccaggaa acatttagcc 900
   ctctactcac tggccatctt cgtggtgatc actgtgcact ttggggccca ggacatctta 960
   accctttatg aactaagcac acccctctgc tgggactcca aactaatcgg ctatggttct 1020
   gcagctcagc atctccccta cctcaccagc ctgctggccc tgaagctcct gcagtactgc 1080
   ctggccgatg cctgggtagc tgagatcggc ctggccttca acatcctggg gatggtggtc 1140
   tttgcctttg ccactatcac gcctctcatg ttcacaggat atgggttgct tttcctgtca 1200
   ttagtcatca cacctgtcat ccgggctaaa ctctccaagc tggtgagaga gacagagcag 1260
   ggtgctctct tttctgctgt ggcctgtgtg aatagcctgg ccatgctgac ggcctccggc 1320
   ttcaactcac tctacccagc cactctgaac tttatgaagg ggttcccctt cctcctggga 1380
   gctggcctcc tgctcatccc ggctgttctg attgggatgc tggaaaaggc tgatcctcac 1440
   ctcgagttcc agcagtttcc ccagagcccc tgatctgcct ggaccagaag acagagggca 1500
   agaggagcaa agtgaacacc aagcaactgg aggtctgcag ctggaagccc agcccacagc 1560
   aggacaagca actcttgtct aagggcagtg ctctctttgg acgaggtagt caagagagac 1620
   caaggcacca ccccatccac agctgaccca gcctctgtct aggatctaga atcataaccc 1680
   acacaggccc actgcaggac aggtggcaga ggagctattt gggacaggag tcagttctcc 1740
   ctttctgcta tccatcactt ataaccccac aggccagagg agaggtcctg agagaggtga 1800
   cacttcaggg accagaggca gcacgagggc tggcatctct ccttccagcc caaactgcac 1860
   agccccaacc aggttcccaa cacgtgtagc agtcatcagc cattccttaa caatgaatgt 1920
   ggtacctggt tagtgccagc cttgggaagg agggagggag gtaagagggg cttggtgatc 1980
   tctggaggaa gagtgttgcc tttgctgtgg ttggggaatc gatccttggc tatggcctac 2040
   ccactcccct ggctgaagag tgccaatcat tacaaatcag cttcagcaaa ctgaaacaaa 2100
   ccttctggtg ccttgggctt tggggccctc tgttttcctg ctcccagtct gcctgccata 2160
   cgttagacaa cagaagctgc tgggctgggc aggagccctg agctgcctgg tggattggta   2220
   ccaagtgggg ccagccaggg cttctactga ctcgctgtgc aaccttgagc aagccccact   2280
   aggaacggct agatcagcaa tgccccaaat tgttgaatcg tggtgatcac atttggatgg   2340
   acaagttagg aaacttagct gattctctga gatacttccc acttcctcat acattctaca   2400
   atgagatgcc tctcttcatg ggcctgggga aaggatgaat gagtgtggct ggacttttga   2460
   cctttaatgt ccctgagcag ttcttggccc ttggggagaa gtagggggag gatctggctg   2520
   actcagcccc aaccgagggg tagcagctgc agcccagcca agcagaacac cctggcccta   2580
   cagacctgtg tctgagcagg ctttggagga aaggccactt ctgagtcctc tgtctcagga   2640
   gaagccaggc ctaggtgctt gacctggtga tctgagctgt gtttgtccta gtatgggtgg   2700
   cagtcacctc tgaacacact ggccttggct tagaggaggc ttgtgacttc agctgttcca   2760
   gctggggctg gatgctggac tctgattccc tcagagagac agaagatcgg gcagaggtca   2820
   accagaaggc cacacagggc agtggacaac agtggagggt ataccaaata gaaataaaac   2880
   gcaggaaacc tctctgaata ctgcccttgg ggacctcagg agggagtggg actgattgga   2940
   gacaaagagc cagaaatcat cccctggaga tactcagaca ctgtattgtt attggcgctg   3000
   tcctgtgagg gcagtgtcca ggctgccctg cttgtcatat ccatgccctg gctctggccc   3060
   cggtgagcac ttgtgctgca tgaggaaagg aggatcaggt ggggtcaaaa ccaaagcatc   3120
   gaggctggca ctggccaaga cagactctgg cagggagggg agtggaagga aggagcccca   3180
   aacctggagt gagacctttg actttctgat gatgtggcca agaactagga gtcacctggc   3240
   cagcacagcc aagagtgggg ctgggggcgc cagctttaag gtgtcaacac ctggcccttg   3300
   cggggaaaga gccttcagca gcccccacgg cctggagcct gggtttattg gagggtgctt   3360
   tcttaccctc tgaacccagt ggaggagggg gcaatgggat attcctgctc tgtatggcct   3420
   ctgggtcagc tttttgttgc aaaatcctat cccccgctgc tagattcttt gaagcaaggg   3480
   agaagcaata gcccagcctc agaatgattc tcaggaaagc aattccactg ccagtggact   3540
   gggtgccagg aggcctaggt ctggatctgg ggctgccact aatgacagga aagtgggctt   3600
   atcctcaacc tttctgagcc tcagtgtcat tatctgtaaa atggggatga taataccagg   3660
   tagagttgct ctgatgactg gagaccatgg agcggagtgt ttgggaaatg gcaaagattt   3720
   gtgagggctt cgtgctttag agatttgcta cccttaacca ccagcatcag ctcacctggg   3780
   aatctgtaag aaatgcagag tcctaggccc caccccagat ctcctgattt taacaacatt   3840
   cccaggtgac ttggatgcat gttaaagttg gagaagctgc cctaagtgac aaccctttgc  3900
   cttttcagga ctttgcctct ttccacagag ctgcaggttc tgtctaggct gcttttactg   3960
   ccatgggggc tgcctggtcc ctgagtaagc agtgggtagc cctggatggc tgaggggctg   4020
   gggaagccag ctgggccaga ccaggaagga gcctgaggca cagcaccaag gcatggggta 4080
   gggtgggctt tgaggcacgg cctccccatg cccagaccca ggcccaaaca ggaggctcct 4140
   gtgcaccagc aagtgcagca aagctggctg cagtgacaag gaagtcagag ggagggctgg 4200
   tttgcctcaa ccctcgccct ggatgtggca aaagatacca caccgaagga agctgtaggg 4260
   gatctgggac ttagtcaggc tagctaaggg cactgagtga ttacagggca aggccacaga 4320
   gcggcaggaa gttgtaagtg caggggccag gcacagtctg agtggagagc catgatggca 4380
   ctcatccctg gctggctcct gggcccaggc ctttgctcag aatacagtgc ccaggcccgc 4440
   ctgggcattg ccttaattcc tgtctgaggc tgccttcctt catcctcctc aagctgactg 4500
   ctatcagcag gagaagggaa tgcagaacca cccccacccc aactccagtg tctccagaac 4560
   tgagcaggga ccctccccat gacggagaca gacaacccaa tggcagggcc tgggggagtt 4620
   ctaacctccc gtgcccaagc ctgacttccc ttccctctcc agatactgag gaaagtgggt 4680
   tggaggtggg ccatgagggt gggggacagg gggagggaga gccatttcct aagaagagcc 4740
   caggttgtct cagcccaggg agactggttt caggagctgt tccttcagaa aggacgatgg 4800
   aaatggaagt cacagcaggg ctggggaact ggggactggt taggttggac ccatgggtgc 4860
   agcaccctcc aaactggtgt cagagcctgc agatgagtcc cgggaggagc ggccctgcag 4920
   gaagcggatg atcttctcaa tggtggcctc ctggtattcg tgggaccacc tgtgggcaca 4980
   ggagccaatg tggttcagac agctgggcag agggagctgc tagtcagctg tttgcccaca 5040
   ctctgcctgt catggtgatt tgtaccctac ccccctggtc ccagggccct ggggctcact 5100
   tgataccgtt gaaagtaagc acagcctgca tgtcctcagg caggacctgg ggctcaggcc 5160
   actcgaagcc atcaatgatg ggcacaatgt tcttgccgca gcttaaagca gtcacaatct 5220
   cctggagaaa gaaaggaaag gaggaagata cccctgtacc catctaggga cccttgtagc 5280
   caggcaggtg ctgggaggcc cactggcccg aggctgggcc cagggcagga caccagtcct 5340
   gatgtccacc ttgatgtcta ccttaaccct tgtagtgtaa taaaaaataa atatttttcc 5400
   tttgcccaga gttcctggca cagagctgct aaaacccttg gaatctcttg agtgatgagt 5460
   gtctttgtat gctaaagaga tgattcccca ctgggggccc ggggggtgct atgggggtag 5520
   cctagatagt ttcaggaaga gggctggtca ccagggagac cagggcatga ctagaggatt 5580
   ggaactttca gccccacctc ccagcctcca gagaggtgct ggagattgag ttcaatcacc 5640
   aatggccaat gatttaatca atcatgccta cataatggaa cctccataaa aacccccaaa 5700
   caaggggttt tgaagagcct ccaggttgat aaacaggtgt aggtgctggg aaggtggtag 5760
   aaggcatggc atccacacct ccaccctcca tacgtagaga tcctatgtat ctcttccatt 5820
   tggctattct tgagctgtgt cctttataac tgtgaaatta aataattcag ctgggcatgg 5880
   tgggtcacgc ctgtaatccc aacactttgg gaggctgagg caggaggatc acttgagccc   5940
   aggattttga gattaataat attatttatt actattattc aagctttgtt aaatcaaacc   6000
   taaagctctt ggaactttaa gttattctga gccttgacag gaattggtct ctgcagcctg   6060
   agtcatgggg catgcagctg caacttccac ctattttttt tttttctgta attacttagg   6120
   aagaccaaat ggcaccagag ataagactcc cttagatccc cacttagatc accacccttt   6180
   ctcagggggt aataaagtca tcttccttgg aatgtagcaa tctataacca atcaaagcac   6240
   tgtaacatac gcactggtct tgtatggaaa atgttgtaat cctgctaaaa ttcctctctc   6300
   tttgcctgtg gaagtgaaac cttaacttct ccagtttgga atgctcaccc catgcctttg   6360
   gagtcaatgc ttactgggtg gctattctca aactttgcac tcaaagaaac tctatactta   6420
   gtcttatttt ctgaatctca ttatttaagg ttgacataat aaacaactaa tagtaagtaa   6480
   agtg                                                                 6484
<210> 32
   <211> 6400
   <212> DNA
   <213> Homo sapiens
<400> 32
   acagcgcaag ccccacgcca cgcgtcgctg gtcccaggca gcgagtcgct cgcgcgcccc     60
   gccgcccgcc tggcgacagc tccgccgcgc acgcacatgg aggggagcgc gagccccccg    120
   gaaaagcccc gcgcccgccc tgcggctgcc gtgctgtgcc ggggcccggt agagccgctg    180
   gtcttcctgg ccaactttgc cttggtcctg cagggcccgc tcaccacgca gtatctgtgg    240
   caccgcttca gcgccgacct cggctacaat ggcacccgcc aaaggggggg ctgcagcaac    300
   cgcagcgcgg accccaccat gcaggaagtg gagaccctta cctcccactg gaccctctac    360
   atgaacgtgg gcggcttcct ggtggggctc ttctcgtcca ccctgctggg agcttggagc    420
   gacagtgtgg gccgccgccc gctgctagtg ctggcctcgc tgggcctgct gctccaggcc    480
   ctagtgtccg tttttgtggt gcagctgcag ctccacgtcg gctacttcgt gctgggtcgc    540
   atcctttgtg ccctcctcgg cgacttcggt ggccttctgg ctgctagctt tgcgtccgtg    600
   gcagatgtca gctccagtcg cagccgcacc ttccggatgg ccctgctgga agccagcatc    660
   ggggtggctg ggatgctggc aagcctcctc gggggccact ggctccgggc ccagggttat    720
   gccaacccct tctggctggc cttggccttg ctgatagcca tgactctcta tgcagctttc    780
   tgctttggtg agaccttaaa ggagccaaag tccacccggc tcttcacgtt ccgtcaccac    840
   cgatccattg tccagctcta tgtggctccc gccccagaga agtccaggaa acatttagcc    900
   ctctactcac tggccatctt cgtggtgatc actgtgcact ttggggccca ggacatctta    960
   accctttatg aactaagcac acccctctgc tgggactcca aactaatcgg ctatggttct   1020
   gcagctcagc atctccccta cctcaccagc ctgctggccc tgaagctcct gcagtactgc 1080
   ctggccgatg cctgggtagc tgagatcggc ctggccttca acatcctggg gatggtggtc 1140
   tttgcctttg ccactatcac gcctctcatg ttcacaggtg ctctcttttc tgctgtggcc 1200
   tgtgtgaata gcctggccat gctgacggcc tccggcttca actcactcta cccagccact 1260
   ctgaacttta tgaaggggtt ccccttcctc ctgggagctg gcctcctgct catcccggct 1320
   gttctgattg ggatgctgga aaaggctgat cctcacctcg agttccagca gtttccccag 1380
   agcccctgat ctgcctggac cagaagacag agggcaagag gagcaaagtg aacaccaagc 1440
   aactggaggt ctgcagctgg aagcccagcc cacagcagga caagcaactc ttgtctaagg 1500
   gcagtgctct ctttggacga ggtagtcaag agagaccaag gcaccacccc atccacagct 1560
   gacccagcct ctgtctagga tctagaatca taacccacac aggcccactg caggacaggt 1620
   ggcagaggag ctatttggga caggagtcag ttctcccttt ctgctatcca tcacttataa 1680
   ccccacaggc cagaggagag gtcctgagag aggtgacact tcagggacca gaggcagcac 1740
   gagggctggc atctctcctt ccagcccaaa ctgcacagcc ccaaccaggt tcccaacacg 1800
   tgtagcagtc atcagccatt ccttaacaat gaatgtggta cctggttagt gccagccttg 1860
   ggaaggaggg agggaggtaa gaggggcttg gtgatctctg gaggaagagt gttgcctttg 1920
   ctgtggttgg ggaatcgatc cttggctatg gcctacccac tcccctggct gaagagtgcc 1980
   aatcattaca aatcagcttc agcaaactga aacaaacctt ctggtgcctt gggctttggg 2040
   gccctctgtt ttcctgctcc cagtctgcct gccatacgtt agacaacaga agctgctggg 2100
   ctgggcagga gccctgagct gcctggtgga ttggtaccaa gtggggccag ccagggcttc 2160
   tactgactcg ctgtgcaacc ttgagcaagc cccactagga acggctagat cagcaatgcc 2220
   ccaaattgtt gaatcgtggt gatcacattt ggatggacaa gttaggaaac ttagctgatt 2280
   ctctgagata cttcccactt cctcatacat tctacaatga gatgcctctc ttcatgggcc 2340
   tggggaaagg atgaatgagt gtggctggac ttttgacctt taatgtccct gagcagttct 2400
   tggcccttgg ggagaagtag ggggaggatc tggctgactc agccccaacc gaggggtagc 2460
   agctgcagcc cagccaagca gaacaccctg gccctacaga cctgtgtctg agcaggcttt 2520
   ggaggaaagg ccacttctga gtcctctgtc tcaggagaag ccaggcctag gtgcttgacc 2580
   tggtgatctg agctgtgttt gtcctagtat gggtggcagt cacctctgaa cacactggcc 2640
   ttggcttaga ggaggcttgt gacttcagct gttccagctg gggctggatg ctggactctg 2700
   attccctcag agagacagaa gatcgggcag aggtcaacca gaaggccaca cagggcagtg 2760
   gacaacagtg gagggtatac caaatagaaa taaaacgcag gaaacctctc tgaatactgc 2820
   ccttggggac ctcaggaggg agtgggactg attggagaca aagagccaga aatcatcccc 2880
   tggagatact cagacactgt attgttattg gcgctgtcct gtgagggcag tgtccaggct 2940
   gccctgcttg tcatatccat gccctggctc tggccccggt gagcacttgt gctgcatgag 3000
   gaaaggagga tcaggtgggg tcaaaaccaa agcatcgagg ctggcactgg ccaagacaga 3060
   ctctggcagg gaggggagtg gaaggaagga gccccaaacc tggagtgaga cctttgactt 3120
   tctgatgatg tggccaagaa ctaggagtca cctggccagc acagccaaga gtggggctgg 3180
   gggcgccagc tttaaggtgt caacacctgg cccttgcggg gaaagagcct tcagcagccc 3240
   ccacggcctg gagcctgggt ttattggagg gtgctttctt accctctgaa cccagtggag 3300
   gagggggcaa tgggatattc ctgctctgta tggcctctgg gtcagctttt tgttgcaaaa 3360
   tcctatcccc cgctgctaga ttctttgaag caagggagaa gcaatagccc agcctcagaa 3420
   tgattctcag gaaagcaatt ccactgccag tggactgggt gccaggaggc ctaggtctgg 3480
   atctggggct gccactaatg acaggaaagt gggcttatcc tcaacctttc tgagcctcag 3540
   tgtcattatc tgtaaaatgg ggatgataat accaggtaga gttgctctga tgactggaga 3600
   ccatggagcg gagtgtttgg gaaatggcaa agatttgtga gggcttcgtg ctttagagat 3660
   ttgctaccct taaccaccag catcagctca cctgggaatc tgtaagaaat gcagagtcct 3720
   aggccccacc ccagatctcc tgattttaac aacattccca ggtgacttgg atgcatgtta 3780
   aagttggaga agctgcccta agtgacaacc ctttgccttt tcaggacttt gcctctttcc 3840
   acagagctgc aggttctgtc taggctgctt ttactgccat gggggctgcc tggtccctga 3900
   gtaagcagtg ggtagccctg gatggctgag gggctgggga agccagctgg gccagaccag 3960
   gaaggagcct gaggcacagc accaaggcat ggggtagggt gggctttgag gcacggcctc 4020
   cccatgccca gacccaggcc caaacaggag gctcctgtgc accagcaagt gcagcaaagc 4080
   tggctgcagt gacaaggaag tcagagggag ggctggtttg cctcaaccct cgccctggat 4140
   gtggcaaaag ataccacacc gaaggaagct gtaggggatc tgggacttag tcaggctagc 4200
   taagggcact gagtgattac agggcaaggc cacagagcgg caggaagttg taagtgcagg 4260
   ggccaggcac agtctgagtg gagagccatg atggcactca tccctggctg gctcctgggc 4320
   ccaggccttt gctcagaata cagtgcccag gcccgcctgg gcattgcctt aattcctgtc 4380
   tgaggctgcc ttccttcatc ctcctcaagc tgactgctat cagcaggaga agggaatgca 4440
   gaaccacccc caccccaact ccagtgtctc cagaactgag cagggaccct ccccatgacg 4500
   gagacagaca acccaatggc agggcctggg ggagttctaa cctcccgtgc ccaagcctga 4560
   cttcccttcc ctctccagat actgaggaaa gtgggttgga ggtgggccat gagggtgggg 4620
   gacaggggga gggagagcca tttcctaaga agagcccagg ttgtctcagc ccagggagac 4680
   tggtttcagg agctgttcct tcagaaagga cgatggaaat ggaagtcaca gcagggctgg 4740
   ggaactgggg actggttagg ttggacccat gggtgcagca ccctccaaac tggtgtcaga 4800
   gcctgcagat gagtcccggg aggagcggcc ctgcaggaag cggatgatct tctcaatggt 4860
   ggcctcctgg tattcgtggg accacctgtg ggcacaggag ccaatgtggt tcagacagct 4920
   gggcagaggg agctgctagt cagctgtttg cccacactct gcctgtcatg gtgatttgta 4980
   ccctaccccc ctggtcccag ggccctgggg ctcacttgat accgttgaaa gtaagcacag 5040
   cctgcatgtc ctcaggcagg acctggggct caggccactc gaagccatca atgatgggca 5100
   caatgttctt gccgcagctt aaagcagtca caatctcctg gagaaagaaa ggaaaggagg 5160
   aagatacccc tgtacccatc tagggaccct tgtagccagg caggtgctgg gaggcccact 5220
   ggcccgaggc tgggcccagg gcaggacacc agtcctgatg tccaccttga tgtctacctt 5280
   aacccttgta gtgtaataaa aaataaatat ttttcctttg cccagagttc ctggcacaga 5340
   gctgctaaaa cccttggaat ctcttgagtg atgagtgtct ttgtatgcta aagagatgat 5400
   tccccactgg gggcccgggg ggtgctatgg gggtagccta gatagtttca ggaagagggc 5460
   tggtcaccag ggagaccagg gcatgactag aggattggaa ctttcagccc cacctcccag 5520
   cctccagaga ggtgctggag attgagttca atcaccaatg gccaatgatt taatcaatca 5580
   tgcctacata atggaacctc cataaaaacc cccaaacaag gggttttgaa gagcctccag 5640
   gttgataaac aggtgtaggt gctgggaagg tggtagaagg catggcatcc acacctccac 5700
   cctccatacg tagagatcct atgtatctct tccatttggc tattcttgag ctgtgtcctt 5760
   tataactgtg aaattaaata attcagctgg gcatggtggg tcacgcctgt aatcccaaca 5820
   ctttgggagg ctgaggcagg aggatcactt gagcccagga ttttgagatt aataatatta 5880
   tttattacta ttattcaagc tttgttaaat caaacctaaa gctcttggaa ctttaagtta 5940
   ttctgagcct tgacaggaat tggtctctgc agcctgagtc atggggcatg cagctgcaac 6000
   ttccacctat tttttttttt tctgtaatta cttaggaaga ccaaatggca ccagagataa 6060
   gactccctta gatccccact tagatcacca ccctttctca gggggtaata aagtcatctt 6120
   ccttggaatg tagcaatcta taaccaatca aagcactgta acatacgcac tggtcttgta 6180
   tggaaaatgt tgtaatcctg ctaaaattcc tctctctttg cctgtggaag tgaaacctta 6240
   acttctccag tttggaatgc tcaccccatg cctttggagt caatgcttac tgggtggcta 6300
   ttctcaaact ttgcactcaa agaaactcta tacttagtct tattttctga atctcattat 6360
   ttaaggttga cataataaac aactaatagt aagtaaagtg 6400
<210> 33
   <211> 402
   <212> PRT
   <213> Homo sapiens
<400> 33
   Met Met Val Ala Leu Arg Gly Ala Ser Ala Leu Leu Val Leu Phe Leu
   1               5                   10                  15
   Ala Ala Phe Leu Pro Pro Pro Gln Cys Thr Gln Asp Pro Ala Met Val
               20                  25                  30
   His Tyr Ile Tyr Gln Arg Phe Arg Val Leu Glu Gln Gly Leu Glu Lys
           35                  40                  45
   Cys Thr Gln Ala Thr Arg Ala Tyr Ile Gln Glu Phe Gln Glu Phe Ser
       50                  55                  60
   Lys Asn Ile Ser Val Met Leu Gly Arg Cys Gln Thr Tyr Thr Ser Glu
   65                  70                  75                  80
   Tyr Lys Ser Ala Val Gly Asn Leu Ala Leu Arg Val Glu Arg Ala Gln
                   85                  90                  95
   Arg Glu Ile Asp Tyr Ile Gln Tyr Leu Arg Glu Ala Asp Glu Cys Ile
               100                 105                 110
   Glu Ser Glu Asp Lys Thr Leu Ala Glu Met Leu Leu Gln Glu Ala Glu
           115                 120                 125
   Glu Glu Lys Lys Ile Arg Thr Leu Leu Asn Ala Ser Cys Asp Asn Met
       130                 135                 140
   Leu Met Gly Ile Lys Ser Leu Lys Ile Val Lys Lys Met Met Asp Thr
   145                 150                 155                 160
   His Gly Ser Trp Met Lys Asp Ala Val Tyr Asn Ser Pro Lys Val Tyr
                   165                 170                 175
   Leu Leu Ile Gly Ser Arg Asn Asn Thr Val Trp Glu Phe Ala Asn Ile
               180                 185                 190
   Arg Ala Phe Met Glu Asp Asn Thr Lys Pro Ala Pro Arg Lys Gln Ile
           195                 200                 205
   Leu Thr Leu Ser Trp Gln Gly Thr Gly Gln Val Ile Tyr Lys Gly Phe
       210                 215                 220
   Leu Phe Phe His Asn Gln Ala Thr Ser Asn Glu Ile Ile Lys Tyr Asn
   225                 230                 235                 240
   Leu Gln Lys Arg Thr Val Glu Asp Arg Met Leu Leu Pro Gly Gly Val
                   245                 250                 255
   Gly Arg Ala Leu Val Tyr Gln His Ser Pro Ser Thr Tyr Ile Asp Leu
               260                 265                 270
   Ala Val Asp Glu His Gly Leu Trp Ala Ile His Ser Gly Pro Gly Thr
           275                 280                 285
   His Ser His Leu Val Leu Thr Lys Ile Glu Pro Gly Thr Leu Gly Val
       290                 295                 300
   Glu His Ser Trp Asp Thr Pro Cys Arg Ser Gln Asp Ala Glu Ala Ser
   305                 310                 315                 320
   Phe Leu Leu Cys Gly Val Leu Tyr Val Val Tyr Ser Thr Gly Gly Gln
                   325                 330                 335
   Gly Pro His Arg Ile Thr Cys Ile Tyr Asp Pro Leu Gly Thr Ile Ser
               340                 345                 350
   Glu Glu Asp Leu Pro Asn Leu Phe Phe Pro Lys Arg Pro Arg Ser His
           355                 360                 365
   Ser Met Ile His Tyr Asn Pro Arg Asp Lys Gln Leu Tyr Ala Trp Asn
       370                 375                 380
   Glu Gly Asn Gln Ile Ile Tyr Lys Leu Gln Thr Lys Arg Lys Leu Pro
   385                 390                 395                 400
   Leu Lys
<210> 34
   <211> 2786
   <212> DNA
   <213> Homo sapiens
<400> 34
   gggggaaggg aacgggggga agccatcttc accccccacc ccaatgcaca cacaattaag      60
   ccaggaagca gcttgcaacc actagcctgg ggagggtccg catgtgtcaa gggtgagggc     120
   aacagatgct ggacccaggg agctctctgc cacaggtcag tctacaaggc ctcagggacc     180
   aacttgccaa cagctggact tgatcactag ctggcaaact gagctcacgt atcgggtgga     240
   ataacaagcg gactttgctc tctgctgtgc aaaacgctgt ttttagagga tttgccacag     300
   cagcggatag agcaggagag caccaccgga gcccttgaga catccttgag aagagccaca     360
   gcataagaga ctgccctgct tggtgttttg caggatgatg gtggcccttc gaggagcttc 420
   tgcattgctg gttctgttcc ttgcagcttt tctgcccccg ccgcagtgta cccaggaccc 480
   agccatggtg cattacatct accagcgctt tcgagtcttg gagcaagggc tggaaaaatg 540
   tacccaagca acgagggcat acattcaaga attccaagag ttctcaaaaa atatatctgt 600
   catgctggga agatgtcaga cctacacaag tgagtacaag agtgcagtgg gtaacttggc 660
   actgagagtt gaacgtgccc aacgggagat tgactacata caataccttc gagaggctga 720
   cgagtgcatc gaatcagagg acaagacact ggcagaaatg ttgctccaag aagctgaaga 780
   agagaaaaag atccggactc tgctgaatgc aagctgtgac aacatgctga tgggcataaa 840
   gtctttgaaa atagtgaaga agatgatgga cacacatggc tcttggatga aagatgctgt 900
   ctataactct ccaaaggtgt acttattaat tggatccaga aacaacactg tttgggaatt 960
   tgcaaacata cgggcattca tggaggataa caccaagcca gctccccgga agcaaatcct 1020
   aacactttcc tggcagggaa caggccaagt gatctacaaa ggttttctat tttttcataa 1080
   ccaagcaact tctaatgaga taatcaaata taacctgcag aagaggactg tggaagatcg 1140
   aatgctgctc ccaggagggg taggccgagc attggtttac cagcactccc cctcaactta 1200
   cattgacctg gctgtggatg agcatgggct ctgggccatc cactctgggc caggcaccca 1260
   tagccatttg gttctcacaa agattgagcc gggcacactg ggagtggagc attcatggga 1320
   taccccatgc agaagccagg atgctgaagc ctcattcctc ttgtgtgggg ttctctatgt 1380
   ggtctacagt actgggggcc agggccctca tcgcatcacc tgcatctatg atccactggg 1440
   cactatcagt gaggaggact tgcccaactt gttcttcccc aagagaccaa gaagtcactc 1500
   catgatccat tacaacccca gagataagca gctctatgcc tggaatgaag gaaaccagat 1560
   catttacaaa ctccagacaa agagaaagct gcctctgaag taatgcatta cagctgtgag 1620
   aaagagcact gtggctttgg cagctgttct acaggacagt gaggctatag ccccttcaca 1680
   atatagtatc cctctaatca cacacaggaa gagtgtgtag aagtggaaat acgtatgcct 1740
   cctttcccaa atgtcactgc cttaggtatc ttccaagagc ttagatgaga gcatatcatc 1800
   aggaaagttt caacaatgtc cattactccc ccaaacctcc tggctctcaa ggatgaccac 1860
   attctgatac agcctacttc aagccttttg ttttactgct ccccagcatt tactgtaact 1920
   ctgccatctt ccctcccaca attagagttg tatgccagcc cctaatattc accactggct 1980
   tttctctccc ctggcctttg ctgaagctct tccctctttt tcaaatgtct attgatattc 2040
   tcccattttc actgcccaac taaaatacta ttaatatttc tttcttttct tttctttttt 2100
   ttgagacaag gtctcactat gttgcccagg ctggtctcaa actccagagc tcaagagatc 2160
   ctcctgcctc agcctcctaa gtacctggga ttacaggcat gtgccaccac acctggctta 2220
   aaatactatt tcttattgag gtttaacctc tatttcccct agccctgtcc ttccactaag 2280
   cttggtagat gtaataataa agtgaaaata ttaacatttg aatatcgctt tccaggtgtg 2340
   gagtgtttgc acatcattta attctcgttt cacctttgtg aaacatgcac aagtctttac 2400
   agctgtcatt ctagagttta ggtgagtaac acaattacaa agtgaaagat acagctagaa 2460
   aatactacaa atcccatagt ttttccattg cccaaggaag catcaaatac gtatgtttgt 2520
   tcacctactc ttatagtcaa tgcgttcatc gtttcagcct aaaaataata gtctgtccct 2580
   ttagccagtt ttcatgtctg cacaagacct ttcaataggc ctttcaaatg ataattcctc 2640
   cagaaaacca gtctaagggt gaggacccca actctagcct cctcttgtct tgctgtcctc 2700
   tgtttctctc tttctgcttt aaattcaata aaagtgacac tgagcaaata acctcatcag 2760
   gttatatttg cccacatacc ctaagc 2786
<210> 35
   <211> 1328
   <212> PRT
   <213> Homo sapiens
<400> 35
   Met Ser Ser Gly Asp Pro Ala His Leu Gly Leu Cys Leu Trp Leu Trp
   1 5 10 15
   Leu Gly Ala Thr Leu Gly Arg Glu Gln Val Gln Ala Ser Gly Leu Leu
   20 25 30
   Arg Leu Ala Val Leu Pro Glu Asp Arg Leu Gln Met Lys Trp Arg Glu
   35 40 45
   Ser Glu Gly Ser Gly Leu Gly Tyr Leu Val Gln Val Lys Pro Met Ala
   50 55 60
   Gly Asp Ser Glu Gln Glu Val Ile Leu Thr Thr Lys Thr Pro Lys Ala
   65 70 75 80
   Thr Val Gly Gly Leu Ser Pro Ser Lys Gly Tyr Thr Leu Gln Ile Phe
   85 90 95
   Glu Leu Thr Gly Ser Gly Arg Phe Leu Leu Ala Arg Arg Glu Phe Val
   100 105 110
   Ile Glu Asp Leu Lys Ser Ser Ser Leu Asp Arg Ser Ser Gln Arg Pro
   115 120 125
   Leu Gly Ser Gly Ala Pro Glu Pro Thr Pro Ser His Thr Gly Ser Pro
   130 135 140
   Asp Pro Glu Gln Ala Ser Glu Pro Gln Val Ala Phe Thr Pro Ser Gln
   145 150 155 160
   Asp Pro Arg Thr Pro Gly Gly Ser Glu Trp Arg Glu Thr Gly Pro Gln
   165 170 175
   Phe Arg Cys Leu Pro Pro Val Pro Ala Asp Met Val Phe Leu Val Asp
   180 185 190
   Gly Ser Trp Ser Ile Gly His Ser His Phe Gln Gln Val Lys Asp Phe
   195 200 205
   Leu Ala Ser Val Ile Ala Pro Phe Glu Ile Gly Pro Asp Lys Val Gln
   210 215 220
   Val Gly Leu Thr Gln Tyr Ser Gly Asp Ala Gln Thr Glu Trp Asp Leu
   225 230 235 240
   Asn Ser Leu Ser Thr Lys Glu Gln Val Leu Ala Ala Val Arg Arg Leu
   245 250 255
   Arg Tyr Lys Gly Gly Asn Thr Phe Thr Gly Leu Ala Leu Thr His Val
   260 265 270
   Leu Gly Gln Asn Leu Gln Pro Ala Ala Gly Leu Arg Pro Glu Ala Ala
   275 280 285
   Lys Val Val Ile Leu Val Thr Asp Gly Lys Ser Gln Asp Asp Val His
   290 295 300
   Thr Ala Ala Arg Val Leu Lys Asp Leu Gly Val Asn Val Phe Ala Val
   305 310 315 320
   Gly Val Lys Asn Ala Asp Glu Ala Glu Leu Arg Leu Leu Ala Ser Pro
   325 330 335
   Pro Arg Asp Ile Thr Val His Ser Val Leu Asp Phe Leu Gln Leu Gly
   340 345 350
   Ala Leu Ala Gly Leu Leu Ser Arg Leu Ile Cys Gln Arg Leu Gln Gly
   355 360 365
   Gly Ser Pro Arg Gln Gly Pro Ala Ala Ala Pro Ala Leu Asp Thr Leu
   370 375 380
   Pro Ala Pro Thr Ser Leu Val Leu Ser Gln Val Thr Ser Ser Ser Ile
   385                 390                 395                 400
   Arg Leu Ser Trp Thr Pro Ala Pro Arg His Pro Leu Lys Tyr Leu Ile
                   405                 410                 415
   Val Trp Arg Ala Ser Arg Gly Gly Thr Pro Arg Glu Val Val Val Glu
               420                 425                 430
   Gly Pro Ala Ala Ser Thr Glu Leu His Asn Leu Ala Ser Arg Thr Glu
           435                 440                 445
   Tyr Leu Val Ser Val Phe Pro Ile Tyr Glu Gly Gly Val Gly Glu Gly
       450                 455                 460
   Leu Arg Gly Leu Val Thr Thr Ala Pro Leu Pro Pro Pro Arg Ala Leu
   465                 470                 475                 480
   Thr Leu Ala Ala Val Thr Pro Arg Thr Val His Leu Thr Trp Gln Pro
                   485                 490                 495
   Ser Ala Gly Ala Thr His Tyr Leu Val Arg Cys Ser Pro Ala Ser Pro
               500                 505                 510
   Lys Gly Glu Glu Glu Glu Arg Glu Val Gln Val Gly Arg Pro Glu Val
           515                 520                 525
   Leu Leu Asp Gly Leu Glu Pro Gly Arg Asp Tyr Glu Val Ser Val Gln
       530                 535                 540
   Ser Leu Arg Gly Pro Glu Gly Ser Glu Ala Arg Gly Ile Arg Ala Arg
   545                 550                 555                 560
   Thr Pro Thr Leu Ala Pro Pro Arg His Leu Gly Phe Ser Asp Val Ser
                   565                 570                 575
   His Asp Ala Ala Arg Val Phe Trp Glu Gly Ala Pro Arg Pro Val Arg
               580                 585                 590
   Leu Val Arg Val Thr Tyr Val Ser Ser Glu Gly Gly His Ser Gly Gln
           595                 600                 605
   Thr Glu Ala Pro Gly Asn Ala Thr Ser Ala Thr Leu Gly Pro Leu Ser
       610                 615                 620
   Ser Ser Thr Thr Tyr Thr Val Arg Val Thr Cys Leu Tyr Pro Gly Gly
   625                 630                 635                 640
   Gly Ser Ser Thr Leu Thr Gly Arg Val Thr Thr Lys Lys Ala Pro Ser
                   645                 650                 655
   Pro Ser Gln Leu Ser Met Thr Glu Leu Pro Gly Asp Ala Val Gln Leu
               660                 665                 670
   Ala Trp Val Ala Ala Ala Pro Ser Gly Val Leu Val Tyr Gln Ile Thr
           675                 680                 685
   Trp Thr Pro Leu Gly Glu Gly Lys Ala His Glu Ile Ser Val Pro Gly
       690                 695                 700
   Asn Leu Gly Thr Ala Val Leu Pro Gly Leu Gly Arg His Thr Glu Tyr
   705                 710                 715                 720
   Asp Val Thr Ile Leu Ala Tyr Tyr Arg Asp Gly Ala Arg Ser Asp Pro
                   725                 730                 735
   Val Ser Leu Arg Tyr Thr Pro Ser Thr Val Ser Arg Ser Pro Pro Ser
               740                 745                 750
   Asn Leu Ala Leu Ala Ser Glu Thr Pro Asp Ser Leu Gln Val Ser Trp
           755                 760                 765
   Thr Pro Pro Leu Gly Arg Val Leu His Tyr Trp Leu Thr Tyr Ala Pro
       770                 775                 780
   Ala Ser Gly Leu Gly Pro Glu Lys Ser Val Ser Val Pro Gly Ala Arg
   785                 790                 795                 800
   Ser His Val Thr Leu Pro Asp Leu Gln Ala Ala Thr Lys Tyr Arg Val
                   805                 810                 815
   Leu Val Ser Ala Ile Tyr Ala Ala Gly Arg Ser Glu Ala Val Ser Ala
               820                 825                 830
   Thr Gly Gln Thr Ala Cys Pro Ala Leu Arg Pro Asp Gly Ser Leu Pro
           835                 840                 845
   Gly Phe Asp Leu Met Val Ala Phe Ser Leu Val Glu Lys Ala Tyr Ala
       850                 855                 860
   Ser Ile Arg Gly Val Ala Met Glu Pro Ser Ala Phe Gly Gly Thr Pro
   865                 870                 875                 880
   Thr Phe Thr Leu Phe Lys Asp Ala Gln Leu Thr Arg Arg Val Ser Asp
                   885                 890                 895
   Val Tyr Pro Ala Pro Leu Pro Pro Glu His Thr Ile Val Phe Leu Val
               900                 905                 910
   Arg Leu Leu Pro Glu Thr Pro Arg Glu Ala Phe Ala Leu Trp Gln Met
           915                 920                 925
   Thr Ala Glu Asp Phe Gln Pro Leu Leu Gly Val Leu Leu Asp Ala Gly
       930                 935                 940
   Lys Lys Ser Leu Thr Tyr Phe His Arg Asp Pro Arg Ala Ala Leu Gln
   945                 950                 955                 960
   Glu Ala Thr Phe Asp Pro Gln Glu Val Arg Lys Ile Phe Phe Gly Ser
                   965                 970                 975
   Phe His Lys Val His Val Ala Val Gly Arg Ser Lys Val Arg Leu Tyr
               980                 985                 990
   Val Asp Cys Arg Lys Val Ala Glu  Arg Pro Leu Gly Glu  Met Gly Ser
           995                 1000                 1005
   Pro Pro  Ala Ala Gly Phe Val  Thr Leu Gly Arg Leu  Ala Lys Ala
       1010                 1015                 1020
   Arg Gly  Pro Arg Ser Ser Ser  Ala Ala Phe Gln Leu  Gln Met Leu
       1025                 1030                 1035
   Gln Ile  Val Cys Ser Asp Thr  Trp Ala Asp Glu Asp  Arg Cys Cys
       1040                 1045                 1050
   Glu Leu  Pro Ala Ser Arg Asp  Gly Glu Thr Cys Pro  Ala Phe Val
       1055                 1060                 1065
   Ser Ala  Cys Ser Cys Ser Ser  Glu Thr Pro Gly Pro  Pro Gly Pro
       1070                 1075                 1080
   Gln Gly  Pro Pro Gly Leu Pro  Gly Arg Asn Gly Thr  Pro Gly Glu
       1085                 1090                 1095
   Gln Gly  Phe Pro Gly Pro Arg  Gly Glu Pro Gly Pro  Pro Gly Gln
       1100                 1105                 1110
   Met Gly  Pro Glu Gly Pro Gly  Gly Gln Gln Gly Ser  Pro Gly Thr
   1115 1120 1125
   Gln Gly Arg Ala Val Gln Gly Pro Val Gly Pro Pro Gly Val Lys
   1130 1135 1140
   Gly Glu Lys Gly Asp His Gly Leu Pro Gly Leu Gln Gly His Pro
   1145 1150 1155
   Gly His Gln Gly Ile Pro Gly Arg Val Gly Leu Gln Gly Pro Lys
   1160 1165 1170
   Gly Met Arg Gly Leu Glu Gly Thr Ala Gly Leu Pro Gly Pro Gly
   1175 1180 1185
   Pro Arg Gly Phe Gln Gly Met Ala Gly Ala Arg Gly Thr Ser Gly
   1190 1195 1200
   Glu Arg Gly Pro Pro Gly Thr Val Gly Pro Thr Gly Leu Pro Gly
   1205 1210 1215
   Pro Lys Gly Glu Arg Gly Glu Lys Gly Glu Pro Gln Ser Leu Ala
   1220 1225 1230
   Thr Leu Tyr Gln Leu Val Ser Gln Ala Cys Glu Ser Ala Ile Gln
   1235 1240 1245
   Thr His Val Ser Lys Phe Asp Ser Phe His Glu Asn Thr Arg Pro
   1250 1255 1260
   Pro Met Pro Ile Leu Glu Gln Lys Leu Glu Pro Gly Thr Glu Pro
   1265 1270 1275
   Leu Gly Ser Pro Gly Thr Arg Ser Lys Ala Leu Val Pro Gly Glu
   1280 1285 1290
   Trp Gly Arg Gly Gly Arg His Leu Glu Gly Arg Gly Glu Pro Gly
   1295 1300 1305
   Ala Val Gly Gln Met Gly Ser Pro Gly Gln Gln Gly Ala Ser Thr
   1310 1315 1320
   Gln Gly Leu Trp Glu
   1325
<210> 36
   <211> 1278
   <212> PRT
   <213> Homo sapiens
<400> 36
   Met Ser Ser Gly Asp Pro Ala His Leu Gly Leu Cys Leu Trp Leu Trp
   1 5 10 15
   Leu Gly Ala Thr Leu Gly Arg Glu Gln Val Gln Ala Ser Gly Leu Leu
   20 25 30
   Arg Leu Ala Val Leu Pro Glu Asp Arg Leu Gln Met Lys Trp Arg Glu
   35 40 45
   Ser Glu Gly Ser Gly Leu Gly Tyr Leu Val Gln Val Lys Pro Met Ala
   50 55 60
   Gly Asp Ser Glu Gln Glu Val Ile Leu Thr Thr Lys Thr Pro Lys Ala
   65 70 75 80
   Thr Val Gly Gly Leu Ser Pro Ser Lys Gly Tyr Thr Leu Gln Ile Phe
   85 90 95
   Glu Leu Thr Gly Ser Gly Arg Phe Leu Leu Ala Arg Arg Glu Phe Val
   100 105 110
   Ile Glu Asp Leu Lys Ser Ser Ser Leu Asp Arg Ser Ser Gln Arg Pro
   115 120 125
   Leu Gly Ser Gly Ala Pro Glu Pro Thr Pro Ser His Thr Gly Ser Pro
   130 135 140
   Asp Pro Glu Gln Ala Ser Glu Pro Gln Val Ala Phe Thr Pro Ser Gln
   145 150 155 160
   Asp Pro Arg Thr Pro Gly Gly Ser Glu Trp Arg Glu Thr Gly Pro Gln
   165 170 175
   Phe Arg Cys Leu Pro Pro Val Pro Ala Asp Met Val Phe Leu Val Asp
   180 185 190
   Gly Ser Trp Ser Ile Gly His Ser His Phe Gln Gln Val Lys Asp Phe
   195 200 205
   Leu Ala Ser Val Ile Ala Pro Phe Glu Ile Gly Pro Asp Lys Val Gln
   210 215 220
   Val Gly Leu Thr Gln Tyr Ser Gly Asp Ala Gln Thr Glu Trp Asp Leu
   225 230 235 240
   Asn Ser Leu Ser Thr Lys Glu Gln Val Leu Ala Ala Val Arg Arg Leu
                   245                 250                 255
   Arg Tyr Lys Gly Gly Asn Thr Phe Thr Gly Leu Ala Leu Thr His Val
               260                 265                 270
   Leu Gly Gln Asn Leu Gln Pro Ala Ala Gly Leu Arg Pro Glu Ala Ala
           275                 280                 285
   Lys Val Val Ile Leu Val Thr Asp Gly Lys Ser Gln Asp Asp Val His
       290                 295                 300
   Thr Ala Ala Arg Val Leu Lys Asp Leu Gly Val Asn Val Phe Ala Val
   305                 310                 315                 320
   Gly Val Lys Asn Ala Asp Glu Ala Glu Leu Arg Leu Leu Ala Ser Pro
                   325                 330                 335
   Pro Arg Asp Ile Thr Val His Ser Val Leu Asp Phe Leu Gln Leu Gly
               340                 345                 350
   Ala Leu Ala Gly Leu Leu Ser Arg Leu Ile Cys Gln Arg Leu Gln Gly
           355                 360                 365
   Gly Ser Pro Arg Gln Gly Pro Ala Ala Ala Pro Ala Leu Asp Thr Leu
       370                 375                 380
   Pro Ala Pro Thr Ser Leu Val Leu Ser Gln Val Thr Ser Ser Ser Ile
   385                 390                 395                 400
   Arg Leu Ser Trp Thr Pro Ala Pro Arg His Pro Leu Lys Tyr Leu Ile
                   405                 410                 415
   Val Trp Arg Ala Ser Arg Gly Gly Thr Pro Arg Glu Val Val Val Glu
               420                 425                 430
   Gly Pro Ala Ala Ser Thr Glu Leu His Asn Leu Ala Ser Arg Thr Glu
           435                 440                 445
   Tyr Leu Val Ser Val Phe Pro Ile Tyr Glu Gly Gly Val Gly Glu Gly
       450                 455                 460
   Leu Arg Gly Leu Val Thr Thr Ala Pro Leu Pro Pro Pro Arg Ala Leu
   465                 470                 475                 480
   Thr Leu Ala Ala Val Thr Pro Arg Thr Val His Leu Thr Trp Gln Pro
   485 490 495
   Ser Ala Gly Ala Thr His Tyr Leu Val Arg Cys Ser Pro Ala Ser Pro
   500 505 510
   Lys Gly Glu Glu Glu Glu Arg Glu Val Gln Val Gly Arg Pro Glu Val
   515 520 525
   Leu Leu Asp Gly Leu Glu Pro Gly Arg Asp Tyr Glu Val Ser Val Gln
   530 535 540
   Ser Leu Arg Gly Pro Glu Gly Ser Glu Ala Arg Gly Ile Arg Ala Arg
   545 550 555 560
   Thr Pro Thr Leu Ala Pro Pro Arg His Leu Gly Phe Ser Asp Val Ser
   565 570 575
   His Asp Ala Ala Arg Val Phe Trp Glu Gly Ala Pro Arg Pro Val Arg
   580 585 590
   Leu Val Arg Val Thr Tyr Val Ser Ser Glu Gly Gly His Ser Gly Gln
   595 600 605
   Thr Glu Ala Pro Gly Asn Ala Thr Ser Ala Thr Leu Gly Pro Leu Ser
   610 615 620
   Ser Ser Thr Thr Tyr Thr Val Arg Val Thr Cys Leu Tyr Pro Gly Gly
   625 630 635 640
   Gly Ser Ser Thr Leu Thr Gly Arg Val Thr Thr Lys Lys Ala Pro Ser
   645 650 655
   Pro Ser Gln Leu Ser Met Thr Glu Leu Pro Gly Asp Ala Val Gln Leu
   660 665 670
   Ala Trp Val Ala Ala Ala Pro Ser Gly Val Leu Val Tyr Gln Ile Thr
   675 680 685
   Trp Thr Pro Leu Gly Glu Gly Lys Ala His Glu Ile Ser Val Pro Gly
   690 695 700
   Asn Leu Gly Thr Ala Val Leu Pro Gly Leu Gly Arg His Thr Glu Tyr
   705 710 715 720
   Asp Val Thr Ile Leu Ala Tyr Tyr Arg Asp Gly Ala Arg Ser Asp Pro
   725 730 735
   Val Ser Leu Arg Tyr Thr Pro Ser Thr Val Ser Arg Ser Pro Pro Ser
   740 745 750
   Asn Leu Ala Leu Ala Ser Glu Thr Pro Asp Ser Leu Gln Val Ser Trp
   755 760 765
   Thr Pro Pro Leu Gly Arg Val Leu His Tyr Trp Leu Thr Tyr Ala Pro
   770 775 780
   Ala Ser Gly Leu Gly Pro Glu Lys Ser Val Ser Val Pro Gly Ala Arg
   785 790 795 800
   Ser His Val Thr Leu Pro Asp Leu Gln Ala Ala Thr Lys Tyr Arg Val
   805 810 815
   Leu Val Ser Ala Ile Tyr Ala Ala Gly Arg Ser Glu Ala Val Ser Ala
   820 825 830
   Thr Gly Gln Thr Ala Cys Pro Ala Leu Arg Pro Asp Gly Ser Leu Pro
   835 840 845
   Gly Phe Asp Leu Met Val Ala Phe Ser Leu Val Glu Lys Ala Tyr Ala
   850 855 860
   Ser Ile Arg Gly Val Ala Met Glu Pro Ser Ala Phe Gly Gly Thr Pro
   865 870 875 880
   Thr Phe Thr Leu Phe Lys Asp Ala Gln Leu Thr Arg Arg Val Ser Asp
   885 890 895
   Val Tyr Pro Ala Pro Leu Pro Pro Glu His Thr Ile Val Phe Leu Val
   900 905 910
   Arg Leu Leu Pro Glu Thr Pro Arg Glu Ala Phe Ala Leu Trp Gln Met
   915 920 925
   Thr Ala Glu Asp Phe Gln Pro Leu Leu Gly Val Leu Leu Asp Ala Gly
   930 935 940
   Lys Lys Ser Leu Thr Tyr Phe His Arg Asp Pro Arg Ala Ala Leu Gln
   945 950 955 960
   Glu Ala Thr Phe Asp Pro Gln Glu Val Arg Lys Ile Phe Phe Gly Ser
   965 970 975
   Phe His Lys Val His Val Ala Val Gly Arg Ser Lys Val Arg Leu Tyr
   980 985 990
   Val Asp Cys Arg Lys Val Ala Glu Arg Pro Leu Gly Glu Met Gly Ser
   995 1000 1005
   Pro Pro Ala Ala Gly Phe Val Thr Leu Gly Arg Leu Ala Lys Ala
   1010 1015 1020
   Arg Gly Pro Arg Ser Ser Ser Ala Ala Phe Gln Leu Gln Met Leu
   1025 1030 1035
   Gln Ile Val Cys Ser Asp Thr Trp Ala Asp Glu Asp Arg Cys Cys
   1040 1045 1050
   Glu Leu Pro Ala Ser Arg Asp Gly Glu Thr Cys Pro Ala Phe Val
   1055 1060 1065
   Ser Ala Cys Ser Cys Ser Ser Glu Thr Pro Gly Pro Pro Gly Pro
   1070 1075 1080
   Gln Gly Pro Pro Gly Pro Pro Gly Val Lys Gly Glu Lys Gly Asp
   1085 1090 1095
   His Gly Leu Pro Gly Leu Gln Gly His Pro Gly His Gln Gly Ile
   1100 1105 1110
   Pro Gly Arg Val Gly Leu Gln Gly Pro Lys Gly Met Arg Gly Leu
   1115 1120 1125
   Glu Gly Thr Ala Gly Leu Pro Gly Pro Gly Pro Arg Gly Phe Gln
   1130 1135 1140
   Gly Met Ala Gly Ala Arg Gly Thr Ser Gly Glu Arg Gly Pro Pro
   1145 1150 1155
   Gly Thr Val Gly Pro Thr Gly Leu Pro Gly Pro Lys Gly Glu Arg
   1160 1165 1170
   Gly Glu Lys Gly Glu Pro Gln Ser Leu Ala Thr Leu Tyr Gln Leu
   1175 1180 1185
   Val Ser Gln Ala Cys Glu Ser Ala Ile Gln Thr His Val Ser Lys
   1190 1195 1200
   Phe Asp Ser Phe His Glu Asn Thr Arg Pro Pro Met Pro Ile Leu
   1205 1210 1215
   Glu Gln Lys Leu Glu Pro Gly Thr Glu Pro Leu Gly Ser Pro Gly
   1220 1225 1230
   Thr Arg Ser Lys Ala Leu Val Pro Gly Glu Trp Gly Arg Gly Gly
   1235 1240 1245
   Arg His Leu Glu Gly Arg Gly Glu Pro Gly Ala Val Gly Gln Met
   1250 1255 1260
   Gly Ser Pro Gly Gln Gln Gly Ala Ser Thr Gln Gly Leu Trp Glu
   1265 1270 1275
<210> 37
   <211> 1283
   <212> PRT
   <213> Homo sapiens
<400> 37
   Met Ser Ser Gly Asp Pro Ala His Leu Gly Leu Cys Leu Trp Leu Trp
   1 5 10 15
   Leu Gly Ala Thr Leu Gly Arg Glu Gln Val Gln Ala Ser Gly Leu Leu
   20 25 30
   Arg Leu Ala Val Leu Pro Glu Asp Arg Leu Gln Met Lys Trp Arg Glu
   35 40 45
   Ser Glu Gly Ser Gly Leu Gly Tyr Leu Val Gln Val Lys Pro Met Ala
   50 55 60
   Gly Asp Ser Glu Gln Glu Val Ile Leu Thr Thr Lys Thr Pro Lys Ala
   65 70 75 80
   Thr Val Gly Gly Leu Ser Pro Ser Lys Gly Tyr Thr Leu Gln Ile Phe
   85 90 95
   Glu Leu Thr Gly Ser Gly Arg Phe Leu Leu Ala Arg Arg Glu Phe Val
   100 105 110
   Ile Glu Asp Leu Lys Ser Ser Ser Leu Asp Arg Ser Ser Gln Arg Pro
   115 120 125
   Leu Gly Ser Gly Ala Pro Glu Pro Thr Pro Ser His Thr Gly Ser Pro
   130 135 140
   Asp Pro Glu Gln Ala Ser Glu Pro Gln Val Ala Phe Thr Pro Ser Gln
   145                 150                 155                 160
   Asp Pro Arg Thr Pro Ala Gly Pro Gln Phe Arg Cys Leu Pro Pro Val
                   165                 170                 175
   Pro Ala Asp Met Val Phe Leu Val Asp Gly Ser Trp Ser Ile Gly His
               180                 185                 190
   Ser His Phe Gln Gln Val Lys Asp Phe Leu Ala Ser Val Ile Ala Pro
           195                 200                 205
   Phe Glu Ile Gly Pro Asp Lys Val Gln Val Gly Leu Thr Gln Tyr Ser
       210                 215                 220
   Gly Asp Ala Gln Thr Glu Trp Asp Leu Asn Ser Leu Ser Thr Lys Glu
   225                 230                 235                 240
   Gln Val Leu Ala Ala Val Arg Arg Leu Arg Tyr Lys Gly Gly Asn Thr
                   245                 250                 255
   Phe Thr Gly Leu Ala Leu Thr His Val Leu Gly Gln Asn Leu Gln Pro
               260                 265                 270
   Ala Ala Gly Leu Arg Pro Glu Ala Ala Lys Val Val Ile Leu Val Thr
           275                 280                 285
   Asp Gly Lys Ser Gln Asp Asp Val His Thr Ala Ala Arg Val Leu Lys
       290                 295                 300
   Asp Leu Gly Val Asn Val Phe Ala Val Gly Val Lys Asn Ala Asp Glu
   305                 310                 315                 320
   Ala Glu Leu Arg Leu Leu Ala Ser Pro Pro Arg Asp Ile Thr Val His
                   325                 330                 335
   Ser Val Leu Asp Phe Leu Gln Leu Gly Ala Leu Ala Gly Leu Leu Ser
               340                 345                 350
   Arg Leu Ile Cys Gln Arg Leu Gln Gly Gly Ser Pro Arg Gln Gly Pro
           355                 360                 365
   Ala Ala Ala Pro Ala Leu Asp Thr Leu Pro Ala Pro Thr Ser Leu Val
       370                 375                 380
   Leu Ser Gln Val Thr Ser Ser Ser Ile Arg Leu Ser Trp Thr Pro Ala
   385                 390                 395                 400
   Pro Arg His Pro Leu Lys Tyr Leu Ile Val Trp Arg Ala Ser Arg Gly
   405 410 415
   Gly Thr Pro Arg Glu Val Val Val Glu Gly Pro Ala Ala Ser Thr Glu
   420 425 430
   Leu His Asn Leu Ala Ser Arg Thr Glu Tyr Leu Val Ser Val Phe Pro
   435 440 445
   Ile Tyr Glu Gly Gly Val Gly Glu Gly Leu Arg Gly Leu Val Thr Thr
   450 455 460
   Ala Pro Leu Pro Pro Pro Arg Ala Leu Thr Leu Ala Ala Val Thr Pro
   465 470 475 480
   Arg Thr Val His Leu Thr Trp Gln Pro Ser Ala Gly Ala Thr His Tyr
   485 490 495
   Leu Val Arg Cys Ser Pro Ala Ser Pro Lys Gly Glu Glu Glu Glu Arg
   500 505 510
   Glu Val Gln Val Gly Arg Pro Glu Val Leu Leu Asp Gly Leu Glu Pro
   515 520 525
   Gly Arg Asp Tyr Glu Val Ser Val Gln Ser Leu Arg Gly Pro Glu Gly
   530 535 540
   Ser Glu Ala Arg Gly Ile Arg Ala Arg Thr Pro Thr Leu Ala Pro Pro
   545 550 555 560
   Arg His Leu Gly Phe Ser Asp Val Ser His Asp Ala Ala Arg Val Phe
   565 570 575
   Trp Glu Gly Ala Pro Arg Pro Val Arg Leu Val Arg Val Thr Tyr Val
   580 585 590
   Ser Ser Glu Gly Gly His Ser Gly Gln Thr Glu Ala Pro Gly Asn Ala
   595 600 605
   Thr Ser Ala Thr Leu Gly Pro Leu Ser Ser Ser Thr Thr Tyr Thr Val
   610 615 620
   Arg Val Thr Cys Leu Tyr Pro Gly Gly Gly Ser Ser Thr Leu Thr Gly
   625 630 635 640
   Arg Val Thr Thr Lys Lys Ala Pro Ser Pro Ser Gln Leu Ser Met Thr
                   645                 650                 655
   Glu Leu Pro Gly Asp Ala Val Gln Leu Ala Trp Val Ala Ala Ala Pro
               660                 665                 670
   Ser Gly Val Leu Val Tyr Gln Ile Thr Trp Thr Pro Leu Gly Glu Gly
           675                 680                 685
   Lys Ala His Glu Ile Ser Val Pro Gly Asn Leu Gly Thr Ala Val Leu
       690                 695                 700
   Pro Gly Leu Gly Arg His Thr Glu Tyr Asp Val Thr Ile Leu Ala Tyr
   705                 710                 715                 720
   Tyr Arg Asp Gly Ala Arg Ser Asp Pro Val Ser Leu Arg Tyr Thr Pro
                   725                 730                 735
   Ser Thr Val Ser Arg Ser Pro Pro Ser Asn Leu Ala Leu Ala Ser Glu
               740                 745                 750
   Thr Pro Asp Ser Leu Gln Val Ser Trp Thr Pro Pro Leu Gly Arg Val
           755                 760                 765
   Leu His Tyr Trp Leu Thr Tyr Ala Pro Ala Ser Gly Leu Gly Pro Glu
       770                 775                 780
   Lys Ser Val Ser Val Pro Gly Ala Arg Ser His Val Thr Leu Pro Asp
   785                 790                 795                 800
   Leu Gln Ala Ala Thr Lys Tyr Arg Val Leu Val Ser Ala Ile Tyr Ala
                   805                 810                 815
   Ala Gly Arg Ser Glu Ala Val Ser Ala Thr Gly Gln Thr Ala Cys Pro
               820                 825                 830
   Ala Leu Arg Pro Asp Gly Ser Leu Pro Gly Phe Asp Leu Met Val Ala
           835                 840                 845
   Phe Ser Leu Val Glu Lys Ala Tyr Ala Ser Ile Arg Gly Val Ala Met
       850                 855                 860
   Glu Pro Ser Ala Phe Gly Gly Thr Pro Thr Phe Thr Leu Phe Lys Asp
   865                 870                 875                 880
   Ala Gln Leu Thr Arg Arg Val Ser Asp Val Tyr Pro Ala Pro Leu Pro
                   885                 890                 895
   Pro Glu His Thr Ile Val Phe Leu Val Arg Leu Leu Pro Glu Thr Pro
               900                 905                 910
   Arg Glu Ala Phe Ala Leu Trp Gln Met Thr Ala Glu Asp Phe Gln Pro
           915                 920                 925
   Leu Leu Gly Val Leu Leu Asp Ala Gly Lys Lys Ser Leu Thr Tyr Phe
       930                 935                 940
   His Arg Asp Pro Arg Ala Ala Leu Gln Glu Ala Thr Phe Asp Pro Gln
   945                 950                 955                 960
   Glu Val Arg Lys Ile Phe Phe Gly Ser Phe His Lys Val His Val Ala
                   965                 970                 975
   Val Gly Arg Ser Lys Val Arg Leu Tyr Val Asp Cys Arg Lys Val Ala
               980                 985                 990
   Glu Arg Pro Leu Gly Glu Met Gly  Ser Pro Pro Ala Ala  Gly Phe Val
           995                 1000                 1005
   Thr Leu  Gly Arg Leu Ala Lys  Ala Arg Gly Pro Arg  Ser Ser Ser
       1010                 1015                 1020
   Ala Ala  Phe Gln Leu Gln Met  Leu Gln Ile Val Cys  Ser Asp Thr
       1025                 1030                 1035
   Trp Ala  Asp Glu Asp Arg Cys  Cys Glu Leu Pro Ala  Ser Arg Asp
       1040                 1045                 1050
   Gly Glu  Thr Cys Pro Ala Phe  Val Ser Ala Cys Ser  Cys Ser Ser
       1055                 1060                 1065
   Glu Thr  Pro Gly Pro Pro Gly  Pro Gln Gly Pro Pro  Gly Leu Pro
       1070                 1075                 1080
   Gly Arg  Asn Gly Thr Pro Gly  Glu Gln Gly Phe Pro  Gly Pro Arg
       1085                 1090                 1095
   Gly Pro  Pro Gly Val Lys Gly  Glu Lys Gly Asp His  Gly Leu Pro
       1100                 1105                 1110
   Gly Leu  Gln Gly His Pro Gly  His Gln Gly Ile Pro  Gly Arg Val
       1115                 1120                 1125
   Gly Leu  Gln Gly Pro Lys Gly  Met Arg Gly Leu Glu  Gly Thr Ala
   1130 1135 1140
   Gly Leu Pro Gly Pro Gly Pro Arg Gly Phe Gln Gly Met Ala Gly
   1145 1150 1155
   Ala Arg Gly Thr Ser Gly Glu Arg Gly Pro Pro Gly Thr Val Gly
   1160 1165 1170
   Pro Thr Gly Leu Pro Gly Pro Lys Gly Glu Arg Gly Glu Lys Gly
   1175 1180 1185
   Glu Pro Gln Ser Leu Ala Thr Leu Tyr Gln Leu Val Ser Gln Ala
   1190 1195 1200
   Ser His Val Ser Lys Phe Asp Ser Phe His Glu Asn Thr Arg Pro
   1205 1210 1215
   Pro Met Pro Ile Leu Glu Gln Lys Leu Glu Pro Gly Thr Glu Pro
   1220 1225 1230
   Leu Gly Ser Pro Gly Thr Arg Ser Lys Ala Leu Val Pro Gly Glu
   1235 1240 1245
   Trp Gly Arg Gly Gly Arg His Leu Glu Gly Arg Gly Glu Pro Gly
   1250 1255 1260
   Ala Val Gly Gln Met Gly Ser Pro Gly Gln Gln Gly Ala Ser Thr
   1265 1270 1275
   Gln Gly Leu Trp Glu
   1280
<210> 38
   <211> 3987
   <212> DNA
   <213> Homo sapiens
<400> 38
   atgagctccg gagaccctgc acacctcggc ctctgcctct ggctgtggct gggcgccacc      60
   ctgggaagag agcaagttca agcaagcggt ctcctgaggc tggctgtgct gcctgaggac     120
   cggctgcaga tgaagtggag agagtcggag gggagcggcc tcggctacct ggtgcaggtg     180
   aagcccatgg caggggactc ggaacaggag gtgatactga ccaccaagac ccctaaggcc     240
   acagtggggg gcctgagccc ctccaagggc tacaccttgc agatcttcga gctcactggc     300
   tctgggcgct tcctgctagc tcggagggag tttgtgattg aggatctgaa gagtagctcc     360
   ctggacagga gcagccagag gcccctcggc tctggagccc cggagcccac cccctcccac     420
   acggggagcc cagaccctga gcaggcttct gagccccaag ttgccttcac accaagccag 480
   gatccgcgca ctcctggtgg gtcagagtgg agagagaccg gcccccagtt ccgctgcctg 540
   ccccccgtgc ctgctgacat ggtcttcctg gtggacgggt cctggagcat tggccacagt 600
   cacttccagc aggtcaagga cttcctggcc agtgtcatcg caccctttga aatcgggccg 660
   gataaggtcc aagtaggcct gactcagtac agcggggatg ctcagactga gtgggacctg 720
   aactccctca gcaccaagga acaggtgctg gcagctgtgc gccgcctccg ctacaagggg 780
   gggaacacgt tcacaggcct tgccctgacc cacgtgctgg ggcagaacct gcagccggcg 840
   gctggcctcc gtccagaggc agccaaggtg gtgattctgg tgacggacgg caagtcccag 900
   gacgatgtgc acactgctgc ccgtgtcctc aaggacctgg gcgtgaacgt cttcgctgtg 960
   ggtgtgaaga acgccgatga ggctgagctg aggctcctgg cgtccccgcc gagggacatc 1020
   accgtccaca gcgtgctgga cttcctgcag ctcggcgcgc tggctggcct gctcagccgt 1080
   ctcatctgcc agaggctcca gggtgggagc ccgcggcagg gcccagcagc ggctccagcc 1140
   ctggacaccc tccctgcccc caccagcctg gtcctgagcc aggtgacctc ctccagcatc 1200
   cgcctgtcct ggactccagc cccccggcac cccctcaagt atctgatcgt ttggcgagcc 1260
   tctagaggtg gcacccccag ggaggtggtg gtggagggac ccgccgcctc cacggagctg 1320
   cacaacctgg cctcccgcac agagtacctg gtctccgtgt tccccatcta tgagggcggg 1380
   gttggcgaag gcctgcgggg cctggtgacc acagcacctc tgcctccgcc ccgggcgctg 1440
   accctggccg cagtgacgcc cagaaccgtc cacctcacct ggcagccctc ggccggggcc 1500
   acccactacc tggtgcgatg ttctcctgct tcccccaagg gtgaagagga ggagcgagag 1560
   gtgcaggtcg ggcggcccga ggtgctgctg gatggcctgg aacctggcag ggactatgag 1620
   gtctcggtgc agagcctgcg aggccctgag ggcagcgagg cccggggcat ccgtgccagg 1680
   acccccaccc tggccccccc gagacacctg ggcttctcag acgtgagcca cgacgcggca 1740
   cgagtgttct gggagggtgc cccgaggcct gtgcgcctgg tcagggtcac ctatgtgtcc 1800
   agcgagggtg gacactcggg gcagacagag gctcctggga acgccacctc ggccacgctg 1860
   gggcctctct cttcctccac cacctacact gtccgtgtca cctgcctcta ccctgggggt 1920
   ggctcctcta cgctgactgg ccgggtgacc accaagaaag ctcccagccc aagccagctg 1980
   tccatgacgg agctgccagg ggatgcagtc cagctggcgt gggtggccgc agccccgtct 2040
   ggcgtgcttg tctaccagat cacgtggacg cccctgggag aggggaaggc tcacgagatc 2100
   tctgtcccag ggaacctcgg cacggccgtc ctgcctggcc tagggaggca cacagagtac 2160
   gacgtcacca tcttggccta ctacagggac ggggcccgca gtgaccctgt gtccctccgc 2220
   tataccccct ccacggtgag caggagccca ccctccaacc tggccctggc ctcggagacc 2280
   cccgacagcc tgcaggtcag ctggacgccc ccgcttggcc gcgtgctcca ttactggctc 2340
   acctacgccc ccgcctctgg cttgggaccc gagaaatccg tctctgtgcc aggagccagg 2400
   agccacgtga cactgcccga cctgcaggca gccacgaagt acagggtcct ggtctcagct 2460
   atctatgcag caggcaggag tgaggctgtg tctgccacgg gccagacagc ctgcccagcc 2520
   ctccgccctg acggctccct cccagggttt gacctgatgg tggccttcag cctggtggaa 2580
   aaggcttatg cgtccatccg gggcgtggcc atggagccct ctgccttcgg tgggaccccg 2640
   accttcacgc tcttcaagga cgcccagctg acaagacggg tcagtgacgt ctacccagcc 2700
   cccctacctc cagagcacac catcgtcttc cttgtgcgcc tacttcccga gacaccccgt 2760
   gaggccttcg cgctgtggca gatgacagcc gaggacttcc agcccctcct tggggttctg 2820
   ctggatgccg ggaagaagtc cctgacctac ttccaccgtg accccagggc tgccttgcag 2880
   gaggccacct tcgacccgca ggaagtgagg aagattttct tcgggagctt ccacaaggtg 2940
   cacgtggctg tgggccgctc caaggtcagg ctctatgtgg actgccggaa ggtggctgag 3000
   cggccccttg gggagatggg cagcccaccc gctgcgggct tcgtcacgct ggggaggctg 3060
   gccaaggcca ggggcccccg gagcagttcg gccgcgtttc agctccagat gctgcagatc 3120
   gtgtgcagtg acacctgggc cgatgaggac cggtgctgtg agctccctgc ctcgagggat 3180
   ggagagacct gccccgcctt cgtgtctgcc tgttcctgtt cctcagagac ccctgggccc 3240
   ccaggacctc aaggaccccc aggcctccct gggaggaatg gcaccccagg agagcagggc 3300
   ttcccagggc ccaggggaga gcccgggcca cccggacaga tgggaccaga aggtcctgga 3360
   ggccagcagg gctcgccggg gacccagggc cgtgcagtcc aggggcctgt gggtccacca 3420
   ggggtcaaag gagagaaggg agaccatggg cttccaggct tgcagggcca ccccggccac 3480
   cagggcatcc ccgggagagt tggcctccag ggaccaaagg gaatgagagg cctggaggga 3540
   actgctggcc tgcctggacc cggccccagg gggttccagg gcatggcagg ggccaggggc 3600
   actagtggag agcgaggacc tccagggacc gtggggccca caggactgcc agggcccaaa 3660
   ggggaacgag gagagaaggg cgagccgcag tcccttgcca ccctctacca gcttgtgagc 3720
   caggcctgtg agtctgccat tcagacacac gtgtcaaagt tcgactcctt ccacgagaac 3780
   accaggcccc ccatgcccat cttggagcag aagctggagc cgggcactga gcccctgggg 3840
   tcccctggca cccgcagcaa ggccctggtt cctggagaat gggggcgtgg tggccgccac 3900
   cttgagggca gaggggagcc tggagctgtt ggtcagatgg gcagccctgg gcagcagggg 3960
   gctagcaccc agggcctctg ggagtga 3987
<210> 39
   <211> 3837
   <212> DNA
   <213> Homo sapiens
<400> 39
   atgagctccg gagaccctgc acacctcggc ctctgcctct ggctgtggct gggcgccacc     60
   ctgggaagag agcaagttca agcaagcggt ctcctgaggc tggctgtgct gcctgaggac    120
   cggctgcaga tgaagtggag agagtcggag gggagcggcc tcggctacct ggtgcaggtg    180
   aagcccatgg caggggactc ggaacaggag gtgatactga ccaccaagac ccctaaggcc    240
   acagtggggg gcctgagccc ctccaagggc tacaccttgc agatcttcga gctcactggc    300
   tctgggcgct tcctgctagc tcggagggag tttgtgattg aggatctgaa gagtagctcc    360
   ctggacagga gcagccagag gcccctcggc tctggagccc cggagcccac cccctcccac    420
   acggggagcc cagaccctga gcaggcttct gagccccaag ttgccttcac accaagccag    480
   gatccgcgca ctcctggtgg gtcagagtgg agagagaccg gcccccagtt ccgctgcctg    540
   ccccccgtgc ctgctgacat ggtcttcctg gtggacgggt cctggagcat tggccacagt    600
   cacttccagc aggtcaagga cttcctggcc agtgtcatcg caccctttga aatcgggccg    660
   gataaggtcc aagtaggcct gactcagtac agcggggatg ctcagactga gtgggacctg    720
   aactccctca gcaccaagga acaggtgctg gcagctgtgc gccgcctccg ctacaagggg    780
   gggaacacgt tcacaggcct tgccctgacc cacgtgctgg ggcagaacct gcagccggcg    840
   gctggcctcc gtccagaggc agccaaggtg gtgattctgg tgacggacgg caagtcccag    900
   gacgatgtgc acactgctgc ccgtgtcctc aaggacctgg gcgtgaacgt cttcgctgtg    960
   ggtgtgaaga acgccgatga ggctgagctg aggctcctgg cgtccccgcc gagggacatc   1020
   accgtccaca gcgtgctgga cttcctgcag ctcggcgcgc tggctggcct gctcagccgt   1080
   ctcatctgcc agaggctcca gggtgggagc ccgcggcagg gcccagcagc ggctccagcc   1140
   ctggacaccc tccctgcccc caccagcctg gtcctgagcc aggtgacctc ctccagcatc   1200
   cgcctgtcct ggactccagc cccccggcac cccctcaagt atctgatcgt ttggcgagcc   1260
   tctagaggtg gcacccccag ggaggtggtg gtggagggac ccgccgcctc cacggagctg   1320
   cacaacctgg cctcccgcac agagtacctg gtctccgtgt tccccatcta tgagggcggg   1380
   gttggcgaag gcctgcgggg cctggtgacc acagcacctc tgcctccgcc ccgggcgctg   1440
   accctggccg cagtgacgcc cagaaccgtc cacctcacct ggcagccctc ggccggggcc   1500
   acccactacc tggtgcgatg ttctcctgct tcccccaagg gtgaagagga ggagcgagag   1560
   gtgcaggtcg ggcggcccga ggtgctgctg gatggcctgg aacctggcag ggactatgag   1620
   gtctcggtgc agagcctgcg aggccctgag ggcagcgagg cccggggcat ccgtgccagg   1680
   acccccaccc tggccccccc gagacacctg ggcttctcag acgtgagcca cgacgcggca   1740
   cgagtgttct gggagggtgc cccgaggcct gtgcgcctgg tcagggtcac ctatgtgtcc 1800
   agcgagggtg gacactcggg gcagacagag gctcctggga acgccacctc ggccacgctg 1860
   gggcctctct cttcctccac cacctacact gtccgtgtca cctgcctcta ccctgggggt 1920
   ggctcctcta cgctgactgg ccgggtgacc accaagaaag ctcccagccc aagccagctg 1980
   tccatgacgg agctgccagg ggatgcagtc cagctggcgt gggtggccgc agccccgtct 2040
   ggcgtgcttg tctaccagat cacgtggacg cccctgggag aggggaaggc tcacgagatc 2100
   tctgtcccag ggaacctcgg cacggccgtc ctgcctggcc tagggaggca cacagagtac 2160
   gacgtcacca tcttggccta ctacagggac ggggcccgca gtgaccctgt gtccctccgc 2220
   tataccccct ccacggtgag caggagccca ccctccaacc tggccctggc ctcggagacc 2280
   cccgacagcc tgcaggtcag ctggacgccc ccgcttggcc gcgtgctcca ttactggctc 2340
   acctacgccc ccgcctctgg cttgggaccc gagaaatccg tctctgtgcc aggagccagg 2400
   agccacgtga cactgcccga cctgcaggca gccacgaagt acagggtcct ggtctcagct 2460
   atctatgcag caggcaggag tgaggctgtg tctgccacgg gccagacagc ctgcccagcc 2520
   ctccgccctg acggctccct cccagggttt gacctgatgg tggccttcag cctggtggaa 2580
   aaggcttatg cgtccatccg gggcgtggcc atggagccct ctgccttcgg tgggaccccg 2640
   accttcacgc tcttcaagga cgcccagctg acaagacggg tcagtgacgt ctacccagcc 2700
   cccctacctc cagagcacac catcgtcttc cttgtgcgcc tacttcccga gacaccccgt 2760
   gaggccttcg cgctgtggca gatgacagcc gaggacttcc agcccctcct tggggttctg 2820
   ctggatgccg ggaagaagtc cctgacctac ttccaccgtg accccagggc tgccttgcag 2880
   gaggccacct tcgacccgca ggaagtgagg aagattttct tcgggagctt ccacaaggtg 2940
   cacgtggctg tgggccgctc caaggtcagg ctctatgtgg actgccggaa ggtggctgag 3000
   cggccccttg gggagatggg cagcccaccc gctgcgggct tcgtcacgct ggggaggctg 3060
   gccaaggcca ggggcccccg gagcagttcg gccgcgtttc agctccagat gctgcagatc 3120
   gtgtgcagtg acacctgggc cgatgaggac cggtgctgtg agctccctgc ctcgagggat 3180
   ggagagacct gccccgcctt cgtgtctgcc tgttcctgtt cctcagagac ccctgggccc 3240
   ccaggacctc aaggaccccc aggtccacca ggggtcaaag gagagaaggg agaccatggg 3300
   cttccaggct tgcagggcca ccccggccac cagggcatcc ccgggagagt tggcctccag 3360
   ggaccaaagg gaatgagagg cctggaggga actgctggcc tgcctggacc tggccccagg 3420
   gggttccagg gcatggcagg ggccaggggc actagtggag agcgaggacc tccagggacc 3480
   gtggggccca caggactgcc agggcccaaa ggggaacgag gagagaaggg cgagccgcag 3540
   tcccttgcca ccctctacca gcttgtgagc caggcctgtg agtctgccat tcagacacac 3600
   gtgtcaaagt tcgactcctt ccacgagaac accaggcccc ccatgcccat cttggagcag    3660
   aagctggagc cgggcactga gcccctgggg tcccctggca cccgcagcaa ggccctggtt    3720
   cctggagaat gggggcgtgg tggccgccac cttgagggca gaggggagcc tggagctgtt    3780
   ggtcagatgg gcagccctgg gcagcagggg gctagcaccc agggcctctg ggagtga       3837
<210> 40
   <211> 4168
   <212> DNA
   <213> Homo sapiens
<400> 40
   ataagctcca gccttcctgt ggccacagca ggaccagagt ggaccagcac accccaggag    60
   agaggactgg ggtcccagga gtaggaggag cccgagcacc atgagctccg gagaccctgc   120
   acacctcggc ctctgcctct ggctgtggct gggcgccacc ctgggaagag agcaagttca   180
   agcaagcggt ctcctgaggc tggctgtgct gcctgaggac cggctgcaga tgaagtggag   240
   agagtcggag gggagcggcc tcggctacct ggtgcaggtg aagcccatgg caggggactc   300
   ggaacaggag gtgatactga ccaccaagac ccctaaggcc acagtggggg gcctgagccc   360
   ctccaagggc tacaccttgc agatcttcga gctcactggc tctgggcgct tcctgctagc   420
   tcggagggag tttgtgattg aggatctgaa gagtagctcc ctggacagga gcagccagag   480
   gcccctcggc tctggagccc cggagcccac cccctcccac acggggagcc cagaccctga   540
   gcaggcttct gagccccaag ttgccttcac accaagccag gatccgcgca ctcctgccgg   600
   cccccagttc cgctgcctgc cccccgtgcc tgctgacatg gtcttcctgg tggacgggtc   660
   ctggagcatt ggccacagtc acttccagca ggtcaaggac ttcctggcca gtgtcatcgc   720
   accctttgaa atcgggccgg ataaggtcca agtaggcctg actcagtaca gcggggatgc   780
   tcagactgag tgggacctga actccctcag caccaaggaa caggtgctgg cagctgtgcg   840
   ccgcctccgc tacaaggggg ggaacacgtt cacaggcctt gccctgaccc acgtgctggg   900
   gcagaacctg cagccggcgg ctggcctccg tccagaggca gccaaggtgg tgattctggt   960
   gacggacggc aagtcccagg acgatgtgca cactgctgcc cgtgtcctca aggacctggg  1020
   cgtgaacgtc ttcgctgtgg gtgtgaagaa cgccgatgag gctgagctga ggctcctggc  1080
   gtccccgccg agggacatca ccgtccacag cgtgctggac ttcctgcagc tcggcgcgct  1140
   ggctggcctg ctcagccgtc tcatctgcca gaggctccag ggtgggagcc cgcggcaggg  1200
   cccagcagcg gctccagccc tggacaccct ccctgccccc accagcctgg tcctgagcca  1260
   ggtgacctcc tccagcatcc gcctgtcctg gactccagcc ccccggcacc ccctcaagta  1320
   tctgatcgtt tggcgagcct ctagaggtgg cacccccagg gaggtggtgg tggagggacc  1380
   cgccgcctcc acggagctgc acaacctggc ctcccgcaca gagtacctgg tctccgtgtt  1440
   ccccatctat gagggcgggg ttggcgaagg cctgcggggc ctggtgacca cagcacctct 1500
   gcctccgccc cgggcgctga ccctggccgc agtgacgccc agaaccgtcc acctcacctg 1560
   gcagccctcg gccggggcca cccactacct ggtgcgatgt tctcctgctt cccccaaggg 1620
   tgaagaggag gagcgagagg tgcaggtcgg gcggcccgag gtgctgctgg atggcctgga 1680
   acctggcagg gactatgagg tctcggtgca gagcctgcga ggccctgagg gcagcgaggc 1740
   ccggggcatc cgtgccagga cccccaccct ggcccccccg agacacctgg gcttctcaga 1800
   cgtgagccac gacgcggcac gagtgttctg ggagggtgcc ccgaggcctg tgcgcctggt 1860
   cagggtcacc tatgtgtcca gcgagggtgg acactcgggg cagacagagg ctcctgggaa 1920
   cgccacctcg gccacgctgg ggcctctctc ttcctccacc acctacactg tccgtgtcac 1980
   ctgcctctac cctgggggtg gctcctctac gctgactggc cgggtgacca ccaagaaagc 2040
   tcccagccca agccagctgt ccatgacgga gctgccaggg gatgcagtcc agctggcgtg 2100
   ggtggccgca gccccgtctg gcgtgcttgt ctaccagatc acgtggacgc ccctgggaga 2160
   ggggaaggct cacgagatct ctgtcccagg gaacctcggc acggccgtcc tgcctggcct 2220
   agggaggcac acagagtacg acgtcaccat cttggcctac tacagggacg gggcccgcag 2280
   tgaccctgtg tccctccgct ataccccctc cacggtgagc aggagcccac cctccaacct 2340
   ggccctggcc tcggagaccc ccgacagcct gcaggtcagc tggacgcccc cgcttggccg 2400
   cgtgctccat tactggctca cctacgcccc cgcctctggc ttgggacccg agaaatccgt 2460
   ctctgtgcca ggagccagga gccacgtgac actgcccgac ctgcaggcag ccacgaagta 2520
   cagggtcctg gtctcagcta tctatgcagc aggcaggagt gaggctgtgt ctgccacggg 2580
   ccagacagcc tgcccagccc tccgccctga cggctccctc ccagggtttg acctgatggt 2640
   ggccttcagc ctggtggaaa aggcttatgc gtccatccgg ggcgtggcca tggagccctc 2700
   tgccttcggt gggaccccga ccttcacgct cttcaaggac gcccagctga caagacgggt 2760
   cagtgacgtc tacccagccc ccctacctcc agagcacacc atcgtcttcc ttgtgcgcct 2820
   acttcccgag acaccccgtg aggccttcgc gctgtggcag atgacagccg aggacttcca 2880
   gcccctcctt ggggttctgc tggatgccgg gaagaagtcc ctgacctact tccaccgtga 2940
   ccccagggct gccttgcagg aggccacctt cgacccgcag gaagtgagga agattttctt 3000
   cgggagcttc cacaaggtgc acgtggctgt gggccgctcc aaggtcaggc tctatgtgga 3060
   ctgccggaag gtggctgagc ggccccttgg ggagatgggc agcccacccg ctgcgggctt 3120
   cgtcacgctg gggaggctgg ccaaggccag gggcccccgg agcagttcgg ccgcgtttca 3180
   gctccagatg ctgcagatcg tgtgcagtga cacctgggcc gatgaggacc ggtgctgtga 3240
   gctccctgcc tcgagggatg gagagacctg ccccgccttc gtgtctgcct gttcctgttc 3300
   ctcagagacc cctgggcccc caggacctca aggaccccca ggcctccctg ggaggaatgg    3360
   caccccagga gagcagggct tcccagggcc caggggtcca ccaggggtca aaggagagaa    3420
   gggagaccat gggcttccag gcttgcaggg ccaccccggc caccagggca tccccgggag    3480
   agttggcctc cagggaccaa agggaatgag aggcctggag ggaactgctg gcctgcctgg    3540
   acctggcccc agggggttcc agggcatggc aggggccagg ggcactagtg gagagcgagg    3600
   acctccaggg accgtggggc ccacaggact gccagggccc aaaggggaac gaggagagaa    3660
   gggcgagccg cagtcccttg ccaccctcta ccagcttgtg agccaggcct cacacgtgtc    3720
   aaagttcgac tccttccacg agaacaccag gccccccatg cccatcttgg agcagaagct    3780
   ggagccgggc actgagcccc tggggtcccc tggcacccgc agcaaggccc tggttcctgg    3840
   agaatggggg cgtggtggcc gccaccttga gggcagaggg gagcctggag ctgttggtca    3900
   gatgggcagc cctgggcagc agggggctag cacccagggc ctctgggagt gacaggacat    3960
   tttctgcact gccccgagga acgctgagcc ttcctccctg ggtttgtctg gacaccgaga    4020
   gcgaccacat cctggagaag ccaggagaaa agctcaggaa gagcctgcag gtggaaggag    4080
   agggaagcag cggcctcggc caaggcccac cccatactct tggctctgta gcatttccaa    4140
   gttcagataa acccctgagt gctcaccc                                       4168
<210> 42
   <211> 2319
   <212> PRT
   <213> Homo sapiens
<400> 42
   Met Val Val Tyr Gly Gly Asn Val His Thr His Tyr Gln Glu Glu Lys
   1 5 10 15
   Cys Tyr Glu Asp Gly Ile Phe Phe Tyr His Leu Gly Cys His Gln Trp
   20 25 30
   Val Ser Gly Ala Glu Leu Ala Pro Pro Gly Thr Pro Glu Gly Arg Ala
   35 40 45
   Ala Pro Pro Ser Gly Arg Tyr Ser His Val Ala Ala Val Leu Gly Gly
   50 55 60
   Ser Val Leu Leu Val Ala Gly Gly Tyr Ser Gly Arg Pro Arg Gly Asp
   65 70 75 80
   Leu Met Ala Tyr Lys Val Pro Pro Phe Val Phe Gln Ala Pro Ala Pro
   85 90 95
   Asp Tyr His Leu Asp Tyr Cys Ser Met Tyr Thr Asp His Ser Val Cys
   100 105 110
   Ser Arg Asp Pro Glu Cys Ser Trp Cys Gln Gly Ala Cys Gln Ala Ala
   115 120 125
   Pro Pro Pro Gly Thr Pro Leu Gly Ala Cys Pro Ala Ala Ser Cys Leu
   130 135 140
   Gly Leu Gly Arg Leu Leu Gly Asp Cys Gln Ala Cys Leu Ala Phe Ser
   145                 150                 155                 160
   Ser Pro Thr Ala Pro Pro Arg Gly Pro Gly Thr Leu Gly Trp Cys Val
                   165                 170                 175
   His Asn Glu Ser Cys Leu Pro Arg Pro Glu Gln Ala Arg Cys Arg Gly
               180                 185                 190
   Glu Gln Ile Ser Gly Thr Val Gly Trp Trp Gly Pro Ala Pro Val Phe
           195                 200                 205
   Val Thr Ser Leu Glu Ala Cys Val Thr Gln Ser Phe Leu Pro Gly Leu
       210                 215                 220
   His Leu Leu Thr Phe Gln Gln Pro Pro Asn Thr Ser Gln Pro Asp Lys
   225                 230                 235                 240
   Glu Glu Val Gly Arg Trp Val Ala His Gln Glu Lys Glu Thr Arg Arg
                   245                 250                 255
   Leu Gln Arg Pro Gly Ser Ala Arg Leu Phe Pro Leu Pro Gly Arg Asp
               260                 265                 270
   His Lys Tyr Ala Val Glu Ile Gln Gly Gln Leu Asn Gly Ser Ala Gly
           275                 280                 285
   Pro Gly His Ser Glu Leu Thr Leu Leu Trp Asp Arg Thr Gly Val Pro
       290                 295                 300
   Gly Gly Ser Glu Ile Ser Phe Phe Phe Leu Glu Pro Tyr Arg Ser Ser
   305                 310                 315                 320
   Ser Cys Thr Ser Tyr Ser Ser Cys Leu Gly Cys Leu Ala Asp Gln Gly
                   325                 330                 335
   Cys Gly Trp Cys Leu Thr Ser Ala Thr Cys His Leu Arg Gln Gly Gly
               340                 345                 350
   Ala His Cys Gly Asp Asp Gly Ala Gly Gly Ser Leu Leu Val Leu Val
           355                 360                 365
   Pro Thr Leu Cys Pro Leu Cys Glu Glu His Arg Asp Cys His Ala Cys
       370                 375                 380
   Thr Gln Asp Pro Phe Cys Glu Trp His Gln Ser Thr Ser Arg Lys Gly
   385                 390                 395                 400
   Asp Ala Ala Cys Ser Arg Arg Gly Arg Gly Arg Gly Ala Leu Lys Ser
   405 410 415
   Pro Glu Glu Cys Pro Pro Leu Cys Ser Gln Arg Leu Thr Cys Glu Asp
   420 425 430
   Cys Leu Ala Asn Ser Ser Gln Cys Ala Trp Cys Gln Ser Thr His Thr
   435 440 445
   Cys Phe Leu Phe Ala Ala Tyr Leu Ala Arg Tyr Pro His Gly Gly Cys
   450 455 460
   Arg Gly Trp Asp Asp Ser Val His Ser Glu Pro Arg Cys Arg Ser Cys
   465 470 475 480
   Asp Gly Phe Leu Thr Cys His Glu Cys Leu Gln Ser His Glu Cys Gly
   485 490 495
   Trp Cys Gly Asn Glu Asp Asn Pro Thr Leu Gly Arg Cys Leu Gln Gly
   500 505 510
   Asp Phe Ser Gly Pro Leu Gly Gly Gly Asn Cys Ser Leu Trp Val Gly
   515 520 525
   Glu Gly Leu Gly Leu Pro Val Ala Leu Pro Ala Arg Trp Ala Tyr Ala
   530 535 540
   Arg Cys Pro Asp Val Asp Glu Cys Arg Leu Gly Leu Ala Arg Cys His
   545 550 555 560
   Pro Arg Ala Thr Cys Leu Asn Thr Pro Leu Ser Tyr Glu Cys His Cys
   565 570 575
   Gln Arg Gly Tyr Gln Gly Asp Gly Ile Ser His Cys Asn Arg Thr Cys
   580 585 590
   Leu Glu Asp Cys Gly His Gly Val Cys Ser Gly Pro Pro Asp Phe Thr
   595 600 605
   Cys Val Cys Asp Leu Gly Trp Thr Ser Asp Leu Pro Pro Pro Thr Pro
   610 615 620
   Ala Pro Gly Pro Pro Ala Pro Arg Cys Ser Arg Asp Cys Gly Cys Ser
   625 630 635 640
   Phe His Ser His Cys Arg Lys Arg Gly Pro Gly Phe Cys Asp Glu Cys
                   645                 650                 655
   Gln Asp Trp Thr Trp Gly Glu His Cys Glu Arg Cys Arg Pro Gly Ser
               660                 665                 670
   Phe Gly Asn Ala Thr Gly Ser Arg Gly Cys Arg Pro Cys Gln Cys Asn
           675                 680                 685
   Gly His Gly Asp Pro Arg Arg Gly His Cys Asp Asn Leu Ser Gly Leu
       690                 695                 700
   Cys Phe Cys Gln Asp His Thr Glu Gly Ala His Cys Gln Leu Cys Ser
   705                 710                 715                 720
   Pro Gly Tyr Tyr Gly Asp Pro Arg Ala Gly Gly Ser Cys Phe Arg Glu
                   725                 730                 735
   Cys Gly Gly Arg Ala Leu Leu Thr Asn Val Ser Ser Val Ala Leu Gly
               740                 745                 750
   Ser Arg Arg Val Gly Gly Leu Leu Pro Pro Gly Gly Gly Ala Ala Arg
           755                 760                 765
   Ala Gly Pro Gly Leu Ser Tyr Cys Val Trp Val Val Ser Ala Thr Glu
       770                 775                 780
   Glu Leu Gln Pro Cys Ala Pro Gly Thr Leu Cys Pro Pro Leu Thr Leu
   785                 790                 795                 800
   Thr Phe Ser Pro Asp Ser Ser Thr Pro Cys Thr Leu Ser Tyr Val Leu
                   805                 810                 815
   Ala Phe Asp Gly Phe Pro Arg Phe Leu Asp Thr Gly Val Val Gln Ser
               820                 825                 830
   Asp Arg Ser Leu Ile Ala Ala Phe Cys Gly Gln Arg Arg Asp Arg Pro
           835                 840                 845
   Leu Thr Val Gln Ala Leu Ser Gly Leu Leu Val Leu His Trp Glu Ala
       850                 855                 860
   Asn Gly Ser Ser Ser Trp Gly Phe Asn Ala Ser Val Gly Ser Ala Arg
   865                 870                 875                 880
   Cys Gly Ser Gly Gly Pro Gly Ser Cys Pro Val Pro Gln Glu Cys Val
                   885                 890                 895
   Pro Gln Asp Gly Ala Ala Gly Ala Gly Leu Cys Arg Cys Pro Gln Gly
               900                 905                 910
   Trp Ala Gly Pro His Cys Arg Met Ala Leu Cys Pro Glu Asn Cys Asn
           915                 920                 925
   Ala His Thr Gly Ala Gly Thr Cys Asn Gln Ser Leu Gly Val Cys Ile
       930                 935                 940
   Cys Ala Glu Gly Phe Gly Gly Pro Asp Cys Ala Thr Lys Leu Asp Gly
   945                 950                 955                 960
   Gly Gln Leu Val Trp Glu Thr Leu Met Asp Ser Arg Leu Ser Ala Asp
                   965                 970                 975
   Thr Ala Ser Arg Phe Leu His Arg Leu Gly His Thr Met Val Asp Gly
               980                 985                 990
   Pro Asp Ala Thr Leu Trp Met Phe  Gly Gly Leu Gly Leu  Pro Gln Gly
           995                 1000                1005
   Leu Leu  Gly Asn Leu Tyr Arg  Tyr Ser Val Ser Glu  Arg Arg Trp
       1010                 1015                 1020
   Thr Gln  Met Leu Ala Gly Ala  Glu Asp Gly Gly Pro  Gly Pro Ser
       1025                 1030                 1035
   Pro Arg  Ser Phe His Ala Ala  Ala Tyr Val Pro Ala  Gly Arg Gly
       1040                 1045                 1050
   Ala Met  Tyr Leu Leu Gly Gly  Leu Thr Ala Gly Gly  Val Thr Arg
       1055                 1060                 1065
   Asp Phe  Trp Val Leu Asn Leu  Thr Thr Leu Gln Trp  Arg Gln Glu
       1070                 1075                 1080
   Lys Ala  Pro Gln Thr Val Glu  Leu Pro Ala Val Ala  Gly His Thr
       1085                 1090                 1095
   Leu Thr  Ala Arg Arg Gly Leu  Ser Leu Leu Leu Val  Gly Gly Tyr
       1100                 1105                 1110
   Ser Pro  Glu Asn Gly Phe Asn  Gln Gln Leu Leu Glu  Tyr Gln Leu
       1115                 1120                 1125
   Ala Thr Gly Thr Trp Val Ser Gly Ala Gln Ser Gly Thr Pro Pro
   1130 1135 1140
   Thr Gly Leu Tyr Gly His Ser Ala Val Tyr His Glu Ala Thr Asp
   1145 1150 1155
   Ser Leu Tyr Val Phe Gly Gly Phe Arg Phe His Val Glu Leu Ala
   1160 1165 1170
   Ala Pro Ser Pro Glu Leu Tyr Ser Leu His Cys Pro Asp Arg Thr
   1175 1180 1185
   Trp Ser Leu Leu Ala Pro Ser Gln Gly Ala Lys Arg Asp Arg Met
   1190 1195 1200
   Arg Asn Val Arg Gly Ser Ser Arg Gly Leu Gly Gln Val Pro Gly
   1205 1210 1215
   Glu Gln Pro Gly Ser Trp Gly Phe Arg Glu Val Arg Lys Lys Met
   1220 1225 1230
   Ala Leu Trp Ala Ala Leu Ala Gly Thr Gly Gly Phe Leu Glu Glu
   1235 1240 1245
   Ile Ser Pro His Leu Lys Glu Pro Arg Pro Arg Leu Phe His Ala
   1250 1255 1260
   Ser Ala Leu Leu Gly Asp Thr Met Val Val Leu Gly Gly Arg Ser
   1265 1270 1275
   Asp Pro Asp Glu Phe Ser Ser Asp Val Leu Leu Tyr Gln Val Asn
   1280 1285 1290
   Cys Asn Ala Trp Leu Leu Pro Asp Leu Thr Arg Ser Ala Ser Val
   1295 1300 1305
   Gly Pro Pro Met Glu Glu Ser Val Ala His Ala Val Ala Ala Val
   1310 1315 1320
   Gly Ser Arg Leu Tyr Ile Ser Gly Gly Phe Gly Gly Val Ala Leu
   1325 1330 1335
   Gly Arg Leu Leu Ala Leu Thr Leu Pro Pro Asp Pro Cys Arg Leu
   1340 1345 1350
   Leu Ser Ser Pro Glu Ala Cys Asn Gln Ser Gly Ala Cys Thr Trp
   1355 1360 1365
   Cys His Gly Ala Cys Leu Ser Gly Asp Gln Ala His Arg Leu Gly
   1370 1375 1380
   Cys Gly Gly Ser Pro Cys Ser Pro Met Pro Arg Ser Pro Glu Glu
   1385 1390 1395
   Cys Arg Arg Leu Arg Thr Cys Ser Glu Cys Leu Ala Arg His Pro
   1400 1405 1410
   Arg Thr Leu Gln Pro Gly Asp Gly Glu Ala Ser Thr Pro Arg Cys
   1415 1420 1425
   Lys Trp Cys Thr Asn Cys Pro Glu Gly Ala Cys Ile Gly Arg Asn
   1430 1435 1440
   Gly Ser Cys Thr Ser Glu Asn Asp Cys Arg Ile Asn Gln Arg Glu
   1445 1450 1455
   Val Phe Trp Ala Gly Asn Cys Ser Glu Ala Ala Cys Gly Ala Ala
   1460 1465 1470
   Asp Cys Glu Gln Cys Thr Arg Glu Gly Lys Cys Met Trp Thr Arg
   1475 1480 1485
   Gln Phe Lys Arg Thr Gly Glu Thr Arg Arg Ile Leu Ser Val Gln
   1490 1495 1500
   Pro Thr Tyr Asp Trp Thr Cys Phe Ser His Ser Leu Leu Asn Val
   1505 1510 1515
   Ser Pro Met Pro Val Glu Ser Ser Pro Pro Leu Pro Cys Pro Thr
   1520 1525 1530
   Pro Cys His Leu Leu Pro Asn Cys Thr Ser Cys Leu Asp Ser Lys
   1535 1540 1545
   Gly Ala Asp Gly Gly Trp Gln His Cys Val Trp Ser Ser Ser Leu
   1550 1555 1560
   Gln Gln Cys Leu Ser Pro Ser Tyr Leu Pro Leu Arg Cys Met Ala
   1565 1570 1575
   Gly Gly Cys Gly Arg Leu Leu Arg Gly Pro Glu Ser Cys Ser Leu
   1580 1585 1590
   Gly Cys Ala Gln Ala Thr Gln Cys Ala Leu Cys Leu Arg Arg Pro
   1595 1600 1605
   His Cys Gly Trp Cys Ala Trp Gly Gly Gln Asp Gly Gly Gly Arg
   1610 1615 1620
   Cys Met Glu Gly Gly Leu Ser Gly Pro Arg Asp Gly Leu Thr Cys
   1625 1630 1635
   Gly Arg Pro Gly Ala Ser Trp Ala Phe Leu Ser Cys Pro Pro Glu
   1640 1645 1650
   Asp Glu Cys Ala Asn Gly His His Asp Cys Asn Glu Thr Gln Asn
   1655 1660 1665
   Cys His Asp Gln Pro His Gly Tyr Glu Cys Ser Cys Lys Thr Gly
   1670 1675 1680
   Tyr Thr Met Asp Asn Met Thr Gly Leu Cys Arg Pro Val Cys Ala
   1685 1690 1695
   Gln Gly Cys Val Asn Gly Ser Cys Val Glu Pro Asp His Cys Arg
   1700 1705 1710
   Cys His Phe Gly Phe Val Gly Arg Asn Cys Ser Thr Glu Cys Arg
   1715 1720 1725
   Cys Asn Arg His Ser Glu Cys Ala Gly Val Gly Ala Arg Asp His
   1730 1735 1740
   Cys Leu Leu Cys Arg Asn His Thr Lys Gly Ser His Cys Glu Gln
   1745 1750 1755
   Cys Leu Pro Leu Phe Val Gly Ser Ala Val Gly Gly Gly Thr Cys
   1760 1765 1770
   Arg Pro Cys His Ala Phe Cys Arg Gly Asn Ser His Ile Cys Ile
   1775 1780 1785
   Ser Arg Lys Glu Leu Gln Met Ser Lys Gly Glu Pro Lys Lys Tyr
   1790 1795 1800
   Ser Leu Asp Pro Glu Glu Ile Glu Asn Trp Val Thr Glu Gly Pro
   1805 1810 1815
   Ser Glu Asp Glu Ala Val Cys Val Asn Cys Gln Asn Asn Ser Tyr
   1820 1825 1830
   Gly Glu Lys Cys Glu Ser Cys Leu Gln Gly Tyr Phe Leu Leu Asp
   1835 1840 1845
   Gly Lys Cys Thr Lys Cys Gln Cys Asn Gly His Ala Asp Thr Cys
   1850 1855 1860
   Asn Glu Gln Asp Gly Thr Gly Cys Pro Cys Gln Asn Asn Thr Glu
   1865 1870 1875
   Thr Gly Thr Cys Gln Gly Ser Ser Pro Ser Asp Arg Arg Asp Cys
   1880 1885 1890
   Tyr Lys Tyr Gln Cys Ala Lys Cys Arg Glu Ser Phe His Gly Ser
   1895 1900 1905
   Pro Leu Gly Gly Gln Gln Cys Tyr Arg Leu Ile Ser Val Glu Gln
   1910 1915 1920
   Glu Cys Cys Leu Asp Pro Thr Ser Gln Thr Asn Cys Phe His Glu
   1925 1930 1935
   Pro Lys Arg Arg Ala Leu Gly Pro Gly Arg Thr Val Leu Phe Gly
   1940 1945 1950
   Val Gln Pro Lys Phe Thr Asn Val Asp Ile Arg Leu Thr Leu Asp
   1955 1960 1965
   Val Thr Phe Gly Ala Val Asp Leu Tyr Val Ser Thr Ser Tyr Asp
   1970 1975 1980
   Thr Phe Val Val Arg Val Ala Pro Asp Thr Gly Val His Thr Val
   1985 1990 1995
   His Ile Gln Pro Pro Pro Ala Pro Pro Pro Pro Pro Pro Pro Ala
   2000 2005 2010
   Asp Gly Gly Pro Arg Gly Ala Gly Asp Pro Gly Gly Ala Gly Ala
   2015 2020 2025
   Ser Ser Gly Pro Gly Ala Pro Ala Glu Pro Arg Val Arg Glu Val
   2030 2035 2040
   Trp Pro Arg Gly Leu Ile Thr Tyr Val Thr Val Thr Glu Pro Ser
   2045 2050 2055
   Ala Val Leu Val Val Arg Gly Val Arg Asp Arg Leu Val Ile Thr
   2060 2065 2070
   Tyr Pro His Glu His His Ala Leu Lys Ser Ser Arg Phe Tyr Leu
   2075 2080 2085
   Leu Leu Leu Gly Val Gly Asp Pro Ser Gly Pro Gly Ala Asn Gly
   2090 2095 2100
   Ser Ala Asp Ser Gln Gly Leu Leu Phe Phe Arg Gln Asp Gln Ala
   2105 2110 2115
   His Ile Asp Leu Phe Val Phe Phe Ser Val Phe Phe Ser Cys Phe
   2120 2125 2130
   Phe Leu Phe Leu Ser Leu Cys Val Leu Leu Trp Lys Ala Lys Gln
   2135 2140 2145
   Ala Leu Asp Gln Arg Gln Glu Gln Arg Arg His Leu Gln Glu Met
   2150 2155 2160
   Thr Lys Met Ala Ser Arg Pro Phe Ala Lys Val Thr Val Cys Phe
   2165 2170 2175
   Pro Pro Asp Pro Thr Ala Pro Ala Ser Ala Trp Lys Pro Ala Gly
   2180 2185 2190
   Leu Pro Pro Pro Ala Phe Arg Arg Ser Glu Pro Phe Leu Ala Pro
   2195 2200 2205
   Leu Leu Leu Thr Gly Ala Gly Gly Pro Trp Gly Pro Met Gly Gly
   2210 2215 2220
   Gly Cys Cys Pro Pro Ala Ile Pro Ala Thr Thr Ala Gly Leu Arg
   2225 2230 2235
   Ala Gly Pro Ile Thr Leu Glu Pro Thr Glu Asp Gly Met Ala Gly
   2240 2245 2250
   Val Ala Thr Leu Leu Leu Gln Leu Pro Gly Gly Pro His Ala Pro
   2255 2260 2265
   Asn Gly Ala Cys Leu Gly Ser Ala Leu Val Thr Leu Arg His Arg
   2270 2275 2280
   Leu His Glu Tyr Cys Gly Gly Gly Gly Gly Ala Gly Gly Ser Gly
   2285 2290 2295
   His Gly Thr Gly Ala Gly Arg Lys Gly Leu Leu Ser Gln Asp Asn
   2300 2305 2310
   Leu Thr Ser Met Ser Leu
   2315
<210> 43
   <211> 2386
   <212> PRT
   <213> Homo sapiens
<400> 43
   Met Val Val Tyr Gly Gly Asn Val His Thr His Tyr Gln Glu Glu Lys
   1 5 10 15
   Cys Tyr Glu Asp Gly Ile Phe Phe Tyr His Leu Gly Cys His Gln Trp
   20 25 30
   Val Ser Gly Ala Glu Leu Ala Pro Pro Gly Thr Pro Glu Gly Arg Ala
   35 40 45
   Ala Pro Pro Ser Gly Arg Tyr Ser His Val Ala Ala Val Leu Gly Gly
   50 55 60
   Ser Val Leu Leu Val Ala Gly Gly Tyr Ser Gly Arg Pro Arg Gly Asp
   65 70 75 80
   Leu Met Ala Tyr Lys Val Pro Pro Phe Val Phe Gln Ala Pro Ala Pro
   85 90 95
   Asp Tyr His Leu Asp Tyr Cys Ser Met Tyr Thr Asp His Ser Val Cys
   100 105 110
   Ser Arg Asp Pro Glu Cys Ser Trp Cys Gln Gly Ala Cys Gln Ala Ala
   115 120 125
   Pro Pro Pro Gly Thr Pro Leu Gly Ala Cys Pro Ala Ala Ser Cys Leu
   130 135 140
   Gly Leu Gly Arg Leu Leu Gly Asp Cys Gln Ala Cys Leu Ala Phe Ser
   145 150 155 160
   Ser Pro Thr Ala Pro Pro Arg Gly Pro Gly Thr Leu Gly Trp Cys Val
   165 170 175
   His Asn Glu Ser Cys Leu Pro Arg Pro Glu Gln Ala Arg Cys Arg Gly
   180 185 190
   Glu Gln Ile Ser Gly Thr Val Gly Trp Trp Gly Pro Ala Pro Val Phe
   195 200 205
   Val Thr Ser Leu Glu Ala Cys Val Thr Gln Ser Phe Leu Pro Gly Leu
   210 215 220
   His Leu Leu Thr Phe Gln Gln Pro Pro Asn Thr Ser Gln Pro Asp Lys
   225 230 235 240
   Val Ser Ile Val Arg Ser Thr Thr Ile Thr Leu Thr Pro Ser Ala Glu
   245 250 255
   Thr Asp Val Ser Leu Val Tyr Arg Gly Phe Ile Tyr Pro Met Leu Pro
   260 265 270
   Gly Gly Pro Gly Gly Pro Gly Ala Glu Asp Val Ala Val Trp Thr Arg
   275 280 285
   Ala Gln Arg Leu His Val Leu Ala Arg Met Ala Arg Gly Pro Asp Thr
   290 295 300
   Glu Asn Met Glu Glu Val Gly Arg Trp Val Ala His Gln Glu Lys Glu
   305 310 315 320
   Thr Arg Arg Leu Gln Arg Pro Gly Ser Ala Arg Leu Phe Pro Leu Pro
   325 330 335
   Gly Arg Asp His Lys Tyr Ala Val Glu Ile Gln Gly Gln Leu Asn Gly
   340 345 350
   Ser Ala Gly Pro Gly His Ser Glu Leu Thr Leu Leu Trp Asp Arg Thr
   355 360 365
   Gly Val Pro Gly Gly Ser Glu Ile Ser Phe Phe Phe Leu Glu Pro Tyr
   370 375 380
   Arg Ser Ser Ser Cys Thr Ser Tyr Ser Ser Cys Leu Gly Cys Leu Ala
   385 390 395 400
   Asp Gln Gly Cys Gly Trp Cys Leu Thr Ser Ala Thr Cys His Leu Arg
   405 410 415
   Gln Gly Gly Ala His Cys Gly Asp Asp Gly Ala Gly Gly Ser Leu Leu
   420 425 430
   Val Leu Val Pro Thr Leu Cys Pro Leu Cys Glu Glu His Arg Asp Cys
           435                 440                     445
   His Ala Cys Thr Gln Asp Pro Phe Cys Glu Trp His Gln Ser Thr Ser
       450                 455                     460
   Arg Lys Gly Asp Ala Ala Cys Ser Arg Arg Gly Arg Gly Arg Gly Ala
   465                 470                 475                     480
   Leu Lys Ser Pro Glu Glu Cys Pro Pro Leu Cys Ser Gln Arg Leu Thr
                   485                 490                     495
   Cys Glu Asp Cys Leu Ala Asn Ser Ser Gln Cys Ala Trp Cys Gln Ser
               500                 505                 510
   Thr His Thr Cys Phe Leu Phe Ala Ala Tyr Leu Ala Arg Tyr Pro His
           515                 520                     525
   Gly Gly Cys Arg Gly Trp Asp Asp Ser Val His Ser Glu Pro Arg Cys
       530                 535                     540
   Arg Ser Cys Asp Gly Phe Leu Thr Cys His Glu Cys Leu Gln Ser His
   545                 550                     555                 560
   Glu Cys Gly Trp Cys Gly Asn Glu Asp Asn Pro Thr Leu Gly Arg Cys
                   565                 570                     575
   Leu Gln Gly Asp Phe Ser Gly Pro Leu Gly Gly Gly Asn Cys Ser Leu
               580                 585                 590
   Trp Val Gly Glu Gly Leu Gly Leu Pro Val Ala Leu Pro Ala Arg Trp
           595                 600                     605
   Ala Tyr Ala Arg Cys Pro Asp Val Asp Glu Cys Arg Leu Gly Leu Ala
       610                 615                     620
   Arg Cys His Pro Arg Ala Thr Cys Leu Asn Thr Pro Leu Ser Tyr Glu
   625                 630                     635                 640
   Cys His Cys Gln Arg Gly Tyr Gln Gly Asp Gly Ile Ser His Cys Asn
                   645                 650                     655
   Arg Thr Cys Leu Glu Asp Cys Gly His Gly Val Cys Ser Gly Pro Pro
               660                 665                 670
   Asp Phe Thr Cys Val Cys Asp Leu Gly Trp Thr Ser Asp Leu Pro Pro
   675 680 685
   Pro Thr Pro Ala Pro Gly Pro Pro Ala Pro Arg Cys Ser Arg Asp Cys
   690 695 700
   Gly Cys Ser Phe His Ser His Cys Arg Lys Arg Gly Pro Gly Phe Cys
   705 710 715 720
   Asp Glu Cys Gln Asp Trp Thr Trp Gly Glu His Cys Glu Arg Cys Arg
   725 730 735
   Pro Gly Ser Phe Gly Asn Ala Thr Gly Ser Arg Gly Cys Arg Pro Cys
   740 745 750
   Gln Cys Asn Gly His Gly Asp Pro Arg Arg Gly His Cys Asp Asn Leu
   755 760 765
   Ser Gly Leu Cys Phe Cys Gln Asp His Thr Glu Gly Ala His Cys Gln
   770 775 780
   Leu Cys Ser Pro Gly Tyr Tyr Gly Asp Pro Arg Ala Gly Gly Ser Cys
   785 790 795 800
   Phe Arg Glu Cys Gly Gly Arg Ala Leu Leu Thr Asn Val Ser Ser Val
   805 810 815
   Ala Leu Gly Ser Arg Arg Val Gly Gly Leu Leu Pro Pro Gly Gly Gly
   820 825 830
   Ala Ala Arg Ala Gly Pro Gly Leu Ser Tyr Cys Val Trp Val Val Ser
   835 840 845
   Ala Thr Glu Glu Leu Gln Pro Cys Ala Pro Gly Thr Leu Cys Pro Pro
   850 855 860
   Leu Thr Leu Thr Phe Ser Pro Asp Ser Ser Thr Pro Cys Thr Leu Ser
   865 870 875 880
   Tyr Val Leu Ala Phe Asp Gly Phe Pro Arg Phe Leu Asp Thr Gly Val
   885 890 895
   Val Gln Ser Asp Arg Ser Leu Ile Ala Ala Phe Cys Gly Gln Arg Arg
   900 905 910
   Asp Arg Pro Leu Thr Val Gln Ala Leu Ser Gly Leu Leu Val Leu His
   915 920 925
   Trp Glu Ala Asn Gly Ser Ser Ser Trp Gly Phe Asn Ala Ser Val Gly
   930 935 940
   Ser Ala Arg Cys Gly Ser Gly Gly Pro Gly Ser Cys Pro Val Pro Gln
   945 950 955 960
   Glu Cys Val Pro Gln Asp Gly Ala Ala Gly Ala Gly Leu Cys Arg Cys
   965 970 975
   Pro Gln Gly Trp Ala Gly Pro His Cys Arg Met Ala Leu Cys Pro Glu
   980 985 990
   Asn Cys Asn Ala His Thr Gly Ala Gly Thr Cys Asn Gln Ser Leu Gly
   995 1000 1005
   Val Cys Ile Cys Ala Glu Gly Phe Gly Gly Pro Asp Cys Ala Thr
   1010 1015 1020
   Lys Leu Asp Gly Gly Gln Leu Val Trp Glu Thr Leu Met Asp Ser
   1025 1030 1035
   Arg Leu Ser Ala Asp Thr Ala Ser Arg Phe Leu His Arg Leu Gly
   1040 1045 1050
   His Thr Met Val Asp Gly Pro Asp Ala Thr Leu Trp Met Phe Gly
   1055 1060 1065
   Gly Leu Gly Leu Pro Gln Gly Leu Leu Gly Asn Leu Tyr Arg Tyr
   1070 1075 1080
   Ser Val Ser Glu Arg Arg Trp Thr Gln Met Leu Ala Gly Ala Glu
   1085 1090 1095
   Asp Gly Gly Pro Gly Pro Ser Pro Arg Ser Phe His Ala Ala Ala
   1100 1105 1110
   Tyr Val Pro Ala Gly Arg Gly Ala Met Tyr Leu Leu Gly Gly Leu
   1115 1120 1125
   Thr Ala Gly Gly Val Thr Arg Asp Phe Trp Val Leu Asn Leu Thr
   1130 1135 1140
   Thr Leu Gln Trp Arg Gln Glu Lys Ala Pro Gln Thr Val Glu Leu
   1145 1150 1155
   Pro Ala Val Ala Gly His Thr Leu Thr Ala Arg Arg Gly Leu Ser
   1160 1165 1170
   Leu Leu Leu Val Gly Gly Tyr Ser Pro Glu Asn Gly Phe Asn Gln
   1175 1180 1185
   Gln Leu Leu Glu Tyr Gln Leu Ala Thr Gly Thr Trp Val Ser Gly
   1190 1195 1200
   Ala Gln Ser Gly Thr Pro Pro Thr Gly Leu Tyr Gly His Ser Ala
   1205 1210 1215
   Val Tyr His Glu Ala Thr Asp Ser Leu Tyr Val Phe Gly Gly Phe
   1220 1225 1230
   Arg Phe His Val Glu Leu Ala Ala Pro Ser Pro Glu Leu Tyr Ser
   1235 1240 1245
   Leu His Cys Pro Asp Arg Thr Trp Ser Leu Leu Ala Pro Ser Gln
   1250 1255 1260
   Gly Ala Lys Arg Asp Arg Met Arg Asn Val Arg Gly Ser Ser Arg
   1265 1270 1275
   Gly Leu Gly Gln Val Pro Gly Glu Gln Pro Gly Ser Trp Gly Phe
   1280 1285 1290
   Arg Glu Val Arg Lys Lys Met Ala Leu Trp Ala Ala Leu Ala Gly
   1295 1300 1305
   Thr Gly Gly Phe Leu Glu Glu Ile Ser Pro His Leu Lys Glu Pro
   1310 1315 1320
   Arg Pro Arg Leu Phe His Ala Ser Ala Leu Leu Gly Asp Thr Met
   1325 1330 1335
   Val Val Leu Gly Gly Arg Ser Asp Pro Asp Glu Phe Ser Ser Asp
   1340 1345 1350
   Val Leu Leu Tyr Gln Val Asn Cys Asn Ala Trp Leu Leu Pro Asp
   1355 1360 1365
   Leu Thr Arg Ser Ala Ser Val Gly Pro Pro Met Glu Glu Ser Val
   1370 1375 1380
   Ala His Ala Val Ala Ala Val Gly Ser Arg Leu Tyr Ile Ser Gly
   1385 1390 1395
   Gly Phe Gly Gly Val Ala Leu Gly Arg Leu Leu Ala Leu Thr Leu
   1400 1405 1410
   Pro Pro Asp Pro Cys Arg Leu Leu Ser Ser Pro Glu Ala Cys Asn
   1415 1420 1425
   Gln Ser Gly Ala Cys Thr Trp Cys His Gly Ala Cys Leu Ser Gly
   1430 1435 1440
   Asp Gln Ala His Arg Leu Gly Cys Gly Gly Ser Pro Cys Ser Pro
   1445 1450 1455
   Met Pro Arg Ser Pro Glu Glu Cys Arg Arg Leu Arg Thr Cys Ser
   1460 1465 1470
   Glu Cys Leu Ala Arg His Pro Arg Thr Leu Gln Pro Gly Asp Gly
   1475 1480 1485
   Glu Ala Ser Thr Pro Arg Cys Lys Trp Cys Thr Asn Cys Pro Glu
   1490 1495 1500
   Gly Ala Cys Ile Gly Arg Asn Gly Ser Cys Thr Ser Glu Asn Asp
   1505 1510 1515
   Cys Arg Ile Asn Gln Arg Glu Val Phe Trp Ala Gly Asn Cys Ser
   1520 1525 1530
   Glu Ala Ala Cys Gly Ala Ala Asp Cys Glu Gln Cys Thr Arg Glu
   1535 1540 1545
   Gly Lys Cys Met Trp Thr Arg Gln Phe Lys Arg Thr Gly Glu Thr
   1550 1555 1560
   Arg Arg Ile Leu Ser Val Gln Pro Thr Tyr Asp Trp Thr Cys Phe
   1565 1570 1575
   Ser His Ser Leu Leu Asn Val Ser Pro Met Pro Val Glu Ser Ser
   1580 1585 1590
   Pro Pro Leu Pro Cys Pro Thr Pro Cys His Leu Leu Pro Asn Cys
   1595 1600 1605
   Thr Ser Cys Leu Asp Ser Lys Gly Ala Asp Gly Gly Trp Gln His
   1610 1615 1620
   Cys Val Trp Ser Ser Ser Leu Gln Gln Cys Leu Ser Pro Ser Tyr
   1625 1630 1635
   Leu Pro Leu Arg Cys Met Ala Gly Gly Cys Gly Arg Leu Leu Arg
   1640 1645 1650
   Gly Pro Glu Ser Cys Ser Leu Gly Cys Ala Gln Ala Thr Gln Cys
   1655 1660 1665
   Ala Leu Cys Leu Arg Arg Pro His Cys Gly Trp Cys Ala Trp Gly
   1670 1675 1680
   Gly Gln Asp Gly Gly Gly Arg Cys Met Glu Gly Gly Leu Ser Gly
   1685 1690 1695
   Pro Arg Asp Gly Leu Thr Cys Gly Arg Pro Gly Ala Ser Trp Ala
   1700 1705 1710
   Phe Leu Ser Cys Pro Pro Glu Asp Glu Cys Ala Asn Gly His His
   1715 1720 1725
   Asp Cys Asn Glu Thr Gln Asn Cys His Asp Gln Pro His Gly Tyr
   1730 1735 1740
   Glu Cys Ser Cys Lys Thr Gly Tyr Thr Met Asp Asn Met Thr Gly
   1745 1750 1755
   Leu Cys Arg Pro Val Cys Ala Gln Gly Cys Val Asn Gly Ser Cys
   1760 1765 1770
   Val Glu Pro Asp His Cys Arg Cys His Phe Gly Phe Val Gly Arg
   1775 1780 1785
   Asn Cys Ser Thr Glu Cys Arg Cys Asn Arg His Ser Glu Cys Ala
   1790 1795 1800
   Gly Val Gly Ala Arg Asp His Cys Leu Leu Cys Arg Asn His Thr
   1805 1810 1815
   Lys Gly Ser His Cys Glu Gln Cys Leu Pro Leu Phe Val Gly Ser
   1820 1825 1830
   Ala Val Gly Gly Gly Thr Cys Arg Pro Cys His Ala Phe Cys Arg
   1835 1840 1845
   Gly Asn Ser His Ile Cys Ile Ser Arg Lys Glu Leu Gln Met Ser
   1850 1855 1860
   Lys Gly Glu Pro Lys Lys Tyr Ser Leu Asp Pro Glu Glu Ile Glu
   1865 1870 1875
   Asn Trp Val Thr Glu Gly Pro Ser Glu Asp Glu Ala Val Cys Val
   1880 1885 1890
   Asn Cys Gln Asn Asn Ser Tyr Gly Glu Lys Cys Glu Ser Cys Leu
   1895 1900 1905
   Gln Gly Tyr Phe Leu Leu Asp Gly Lys Cys Thr Lys Cys Gln Cys
   1910 1915 1920
   Asn Gly His Ala Asp Thr Cys Asn Glu Gln Asp Gly Thr Gly Cys
   1925 1930 1935
   Pro Cys Gln Asn Asn Thr Glu Thr Gly Thr Cys Gln Gly Ser Ser
   1940 1945 1950
   Pro Ser Asp Arg Arg Asp Cys Tyr Lys Tyr Gln Cys Ala Lys Cys
   1955 1960 1965
   Arg Glu Ser Phe His Gly Ser Pro Leu Gly Gly Gln Gln Cys Tyr
   1970 1975 1980
   Arg Leu Ile Ser Val Glu Gln Glu Cys Cys Leu Asp Pro Thr Ser
   1985 1990 1995
   Gln Thr Asn Cys Phe His Glu Pro Lys Arg Arg Ala Leu Gly Pro
   2000 2005 2010
   Gly Arg Thr Val Leu Phe Gly Val Gln Pro Lys Phe Thr Asn Val
   2015 2020 2025
   Asp Ile Arg Leu Thr Leu Asp Val Thr Phe Gly Ala Val Asp Leu
   2030 2035 2040
   Tyr Val Ser Thr Ser Tyr Asp Thr Phe Val Val Arg Val Ala Pro
   2045 2050 2055
   Asp Thr Gly Val His Thr Val His Ile Gln Pro Pro Pro Ala Pro
   2060 2065 2070
   Pro Pro Pro Pro Pro Pro Ala Asp Gly Gly Pro Arg Gly Ala Gly
   2075 2080 2085
   Asp Pro Gly Gly Ala Gly Ala Ser Ser Gly Pro Gly Ala Pro Ala
   2090 2095 2100
   Glu Pro Arg Val Arg Glu Val Trp Pro Arg Gly Leu Ile Thr Tyr
   2105 2110 2115
   Val Thr Val Thr Glu Pro Ser Ala Val Leu Val Val Arg Gly Val
   2120 2125 2130
   Arg Asp Arg Leu Val Ile Thr Tyr Pro His Glu His His Ala Leu
   2135 2140 2145
   Lys Ser Ser Arg Phe Tyr Leu Leu Leu Leu Gly Val Gly Asp Pro
   2150 2155 2160
   Ser Gly Pro Gly Ala Asn Gly Ser Ala Asp Ser Gln Gly Leu Leu
   2165 2170 2175
   Phe Phe Arg Gln Asp Gln Ala His Ile Asp Leu Phe Val Phe Phe
   2180 2185 2190
   Ser Val Phe Phe Ser Cys Phe Phe Leu Phe Leu Ser Leu Cys Val
   2195 2200 2205
   Leu Leu Trp Lys Ala Lys Gln Ala Leu Asp Gln Arg Gln Glu Gln
   2210 2215 2220
   Arg Arg His Leu Gln Glu Met Thr Lys Met Ala Ser Arg Pro Phe
   2225 2230 2235
   Ala Lys Val Thr Val Cys Phe Pro Pro Asp Pro Thr Ala Pro Ala
   2240 2245 2250
   Ser Ala Trp Lys Pro Ala Gly Leu Pro Pro Pro Ala Phe Arg Arg
   2255 2260 2265
   Ser Glu Pro Phe Leu Ala Pro Leu Leu Leu Thr Gly Ala Gly Gly
   2270 2275 2280
   Pro Trp Gly Pro Met Gly Gly Gly Cys Cys Pro Pro Ala Ile Pro
   2285 2290 2295
   Ala Thr Thr Ala Gly Leu Arg Ala Gly Pro Ile Thr Leu Glu Pro
   2300 2305 2310
   Thr Glu Asp Gly Met Ala Gly Val Ala Thr Leu Leu Leu Gln Leu
   2315 2320 2325
   Pro Gly Gly Pro His Ala Pro Asn Gly Ala Cys Leu Gly Ser Ala
       2330                2335                2340
   Leu Val Thr Leu Arg His Arg Leu His Glu Tyr Cys Gly Gly Gly
       2345                2350                2355
   Gly Gly Ala Gly Gly Ser Gly His Gly Thr Gly Ala Gly Arg Lys
       2360                2365                2370
   Gly Leu Leu Ser Gln Asp Asn Leu Thr Ser Met Ser Leu
       2375                2380                2385
<210> 44
   <211> 10966
   <212> DNA
   <213> Homo sapiens
<400> 44
   ggccgctctg gcccaagcgt cccctcgtct ctctgatctg gcccatccgg cttcggaggg 60
   aggcgagggt gggtgagcaa agggattggg tctgtggggt ccaggcccga acccctgaag 120
   acgggctccg cccccggcac ccgctcgcgc cccgcccccg gctggaggag tctctcctgg 180
   accatccgaa cctagcctgt cccggcccgc agcctctatg gaggctcctg ccgggccgta 240
   gagcccttcg ccccctgggg acccacccgt ctataaggtc cgtttggcct gcagcagcct 300
   gagtccgtaa tgctgggcac tgttcatggg atcggccccc tatggagccc tgtgtctata 360
   ggggactcct acggtcccta gggttcggcc ccgtccataa tgactccata tacagggcct 420
   tccatcgctc tatagggctc agccctcggc ttccagagcc tgtcagcagt ggccgtaccc 480
   ttcgccggga ctgccgggtc tccgggacct cttcgatcta caaggtcatg ttatgcctat 540
   agaggtcgca tttgcagggc ctcaccccgg gtagagggtc ctctccaggt ttttacggcc 600
   tgtccccgct ctaagggtca gtgcaggagg cggcgatggc cctgggcaag gttctggcca 660
   tggcactggt tttggccttg gccgtgctgg ggtcgctgtc ccctggggcc cgggcggggg 720
   actgcaaggg gcagcggcag gtgctgcggg aggcgccagg cttcgtgacg gatggtgcgg 780
   gcaactacag cgtcaatggc aactgcgagt ggctcatcga ggccccaagc ccccagcacc 840
   ggatcctgct ggacttcctt ttcctggaca cagagtgcac gtatgactac ctgttcgtgt 900
   atgacggtga ctccccgcga gggccgctgc ttgccagtct aagtgggagc acccgacctc 960
   cgcccatcga agcttcctca ggcaagatgc tgctgcacct cttcagtgat gccaactaca 1020
   acctgctggg ctttaacgcc tcattccgct tctccctgtg cccgggtggc tgccagagcc 1080
   acgggcagtg ccagccaccg ggtgtgtgtg cctgcgagcc gggctggggg ggtcctgact 1140
   gtggcctgca ggagtgctca gcctactgtg gcagccacgg cacctgcgcc tcgcccctgg 1200
   gaccatgccg ctgtgagcct ggcttcttgg gacgtgcctg tgacctgcac ctgtgggaga 1260
   accagggggc tgggtggtgg cacaacgtga gtgccaggga ccctgccttc tctgcccgta 1320
   ttggggcagc tggcgccttc ctgtccccac cagggctgct ggcagttttc ggaggccagg 1380
   acctcaacaa tgccctgggt gacctcgtcc tatacaactt ctccgccaac acctgggagt 1440
   cttgggacct gagtcctgcc ccggctgccc gtcactccca tgtggccgtg gcctgggccg 1500
   gctccctggt actgatgggt ggtgagctgg ctgacggctc gctcaccaac gacgtgtggg 1560
   ccttcagtcc actgggcagg ggccactggg agctcctggc accacctgcc tccagctcct 1620
   cggggccccc aggcctggca ggtcacgcgg ctgccctggt ggatgatgtc tggctatatg 1680
   tgtctggagg ccgcaccccg cacgacctct tctcctctgg cctcttccgt ttccgccttg 1740
   acagcaccag cgggggctat tgggagcagg tgattccggc aggcggacgg ccccctgctg 1800
   ccactggcca ctccatggtg ttccatgccc cctcccgtgc cctgctggtc catggtggac 1860
   accggccctc cactgcccgg ttctctgtgc gagtgaactc cactgagctt ttccacgtgg 1920
   atcggcatgt gtggacgacg ctgaaggggc gggatgggct tcagggccca agggagcgag 1980
   ccttccacac agccagtgtt ctgggcaatt acatggtggt ctatgggggc aatgtgcaca 2040
   cccattacca ggaggaaaag tgctacgaag atggcatctt cttctaccac cttggctgcc 2100
   atcaatgggt gtcaggagct gagcttgccc cgccaggaac ccctgagggc cgagcagcgc 2160
   ctcccagtgg tcggtactca catgtagctg cggtgcttgg tggcagcgtc ctgttggtgg 2220
   ctggggggta cagcggccgg ccccgtgggg acttgatggc gtacaaggtg cccccctttg 2280
   tgttccaggc acctgcccct gactaccact tggactactg ctccatgtac acagaccaca 2340
   gcgtctgctc ccgggacccg gaatgcagtt ggtgccaagg agcctgccaa gctgcacccc 2400
   ctcctgggac ccccttgggg gcttgtccag ccgccagctg cctgggcctg ggccgcctcc 2460
   tgggtgactg ccaggcctgc ctggccttca gcagccccac agcccctcca cggggacctg 2520
   gcaccctggg ctggtgcgtg cacaatgaga gctgcctccc taggcctgag caggcccgct 2580
   gccgagggga gcagatctca ggcactgtgg gctggtgggg gcctgcgcct gtcttcgtca 2640
   cgtccctgga ggcctgcgtc acccagagct tcctgcctgg cctgcacttg ctcacctttc 2700
   agcagccgcc caatacctcc cagcctgaca aggaggaggt ggggcgctgg gtggctcatc 2760
   aggagaagga gacgcggcgg ctgcagcgcc ctgggtctgc tcgcctcttc cctctgcctg 2820
   ggcgggacca caagtatgca gtagagatcc agggccagct caatggctcg gcaggccctg 2880
   ggcacagcga gctaactctg ctgtgggatc ggactggtgt gccaggaggc agcgagatct 2940
   ccttcttctt cctggagccc taccgctcgt cgtcctgcac ctcctattct tcctgcctgg 3000
   gctgcttggc agaccagggc tgtggctggt gcctgaccag tgccacctgc cacctgcgcc 3060
   agggcggagc ccattgcggg gatgacgggg ctggtgggtc cctgctggtg ctggtgccta 3120
   ccctctgccc actctgcgag gagcatcggg actgccacgc ctgcacccag gaccccttct 3180
   gtgagtggca tcagagcacc agccgcaaag gggacgcggc atgcagccgg cggggccggg 3240
   gtcggggtgc cctgaagagt ccagaggagt gtcccccgct ctgcagccag cgactgacct 3300
   gtgaggactg cctggccaac tctagccagt gcgcctggtg ccagtccacc cacacctgct 3360
   tcctgtttgc tgcctacttg gcccggtacc cacacggggg ctgtcgaggc tgggacgaca 3420
   gtgtacactc ggagccacgg tgccggagct gcgatggctt cctgacctgc catgagtgtc 3480
   tgcagagcca cgagtgtggc tggtgtggca atgaggacaa ccccacactg ggacggtgcc 3540
   tacaggggga cttctcaggg cccctcggtg ggggtaactg ctccctgtgg gtgggggagg 3600
   gcctggggct tcccgtggcc ctccctgccc gctgggcata cgcccgctgt cctgacgtgg 3660
   atgagtgtcg cctgggcctg gcccggtgcc acccgcgggc gacctgcctg aacacgcccc 3720
   tcagctacga gtgtcactgc cagcggggct accagggtga tggcatctca cactgcaacc 3780
   gcacgtgctt ggaggactgt ggccatggtg tgtgcagtgg ccccccggac tttacctgcg 3840
   tgtgtgacct aggctggaca tcagacctgc cccctcccac acccgccccg ggtccgccag 3900
   ccccccgctg ctcccgggac tgtggctgca gcttccacag ccactgccgc aagcggggcc 3960
   ctggcttctg cgacgagtgc caggactgga catgggggga gcactgcgaa cgatgccggc 4020
   ccggcagctt cggcaacgcc acaggctcta ggggctgccg gccctgccag tgcaacgggc 4080
   acggggaccc acgccgtggc cactgcgaca acctcagtgg gctctgcttc tgccaggacc 4140
   acaccgaggg tgcccactgc cagctctgct ccccaggcta ttatggggat ccccgggccg 4200
   gtggttcctg ctttcgggag tgtgggggtc gcgccctcct caccaacgtg tcctcagtgg 4260
   cactgggctc acgccgggtc ggggggctgc tgcctccagg tggcggggct gcaagagccg 4320
   ggcctggcct gtcctactgt gtgtgggttg tctcggccac tgaggagcta cagccctgtg 4380
   ctcccgggac cctctgtccc ccactcaccc tcaccttctc ccccgacagc agcaccccct 4440
   gcacgctgag ctacgtcctg gcgtttgatg gattcccacg cttcctggac actggtgttg 4500
   tccagtcgga ccgcagcctc atagctgcct tctgcggcca gcgacgggac aggcccctca 4560
   ctgttcaggc cctgtctggg ctgctcgtgc tgcactggga ggccaatggc tcctcatcct 4620
   ggggcttcaa tgcttcggtg ggctctgccc gctgtgggtc agggggcccc gggagctgtc 4680
   ccgtccccca ggaatgcgtg ccccaggacg gtgctgcagg tgcggggctc tgccgatgtc 4740
   ctcagggctg ggctggccca cactgccgca tggctctgtg tcctgagaac tgcaatgccc 4800
   acactggggc aggaacttgt aaccagagcc tgggtgtgtg catctgtgcc gagggcttcg 4860
   ggggccccga ctgcgccacc aagctggatg gcgggcagct ggtctgggag accctcatgg 4920
   acagccgcct ctcagccgac actgccagcc gcttcctgca ccgcctgggc cacaccatgg 4980
   tggatggacc cgatgccacc ttgtggatgt ttgggggcct gggcctgccc caggggctgc 5040
   tgggaaacct gtacaggtac tcagtgagtg agcggcggtg gacacagatg ctggcgggag 5100
   ccgaggacgg gggcccaggc ccatcgcccc gctccttcca tgcagccgca tatgtgcccg 5160
   ctggccgtgg tgccatgtat ctgctggggg gacttaccgc tggaggcgtc acccgtgatt 5220
   tctgggtcct caacctcacc accctgcaat ggcggcagga gaaggccccc cagaccgtgg 5280
   agctgccagc cgttgctggt cacaccctta ctgcccgccg aggcctgtct ctgctcctgg 5340
   tgggcggtta ctccccggaa aatggcttca accagcagct gctggagtac cagctggcaa 5400
   ccggcacctg ggtgtcagga gcccagagtg ggacaccccc cacaggtctc tatggtcact 5460
   ctgctgtcta ccacgaggcc accgactccc tctacgtgtt tggggggttc cgattccatg 5520
   tggagctggc ggccccatcc cccgagctct actccctgca ctgtcctgac cgcacctgga 5580
   gtctgctggc cccttctcag ggggcaaagc gagatcgtat gaggaatgtg cgtggctcat 5640
   ctcggggtct gggccaagtt cctggggagc agcctgggtc atgggggttc cgggaagtca 5700
   ggaagaagat ggctctgtgg gctgctcttg ctggtacagg aggtttcctg gaggaaatct 5760
   cacctcacct gaaggagccc cgcccccggc ttttccacgc ctcagccctg ttaggggaca 5820
   ccatggtggt tcttgggggg cgctcggacc ctgacgagtt cagcagcgac gttctgctct 5880
   accaggtcaa ctgcaatgcc tggcttctgc ccgacctcac ccgctcggcc tctgtggggc 5940
   ccccaatgga ggagtctgtg gcccatgctg tggcagcagt cgggagccgc ctgtatatct 6000
   ctgggggttt cgggggagtg gccctgggcc gcctgctggc actgaccctg ccccctgacc 6060
   cctgccgcct gctgtcctca cctgaagctt gtaaccagtc tggggcctgc acctggtgcc 6120
   atggggcctg cttgtccggg gatcaggccc acaggctggg ctgcgggggc tccccctgct 6180
   ccccaatgcc tcgctccccg gaggaatgtc gacgtctccg gacctgcagt gagtgcctgg 6240
   cccgccatcc tcggaccctg caacctggag atggagaggc gtccaccccc cgctgtaagt 6300
   ggtgtaccaa ctgccccgaa ggtgcttgca ttggacgcaa tgggtcctgc acctctgaga 6360
   atgactgtcg gatcaaccag cgagaggtct tctgggcagg gaactgctcc gaggctgcgt 6420
   gcggggctgc tgactgcgag cagtgcacgc gggagggcaa gtgcatgtgg acgcggcagt 6480
   tcaagaggac aggggagacc cgccgcatcc tctccgtgca gcccacctat gactggacgt 6540
   gcttcagcca ctctctgctg aatgtgtccc ccatgccggt ggaatcatca cccccactgc 6600
   cctgccccac cccttgtcac ctcctaccca actgtacctc ctgcctggac tctaagggag 6660
   cagatggggg ctggcagcac tgtgtttgga gcagcagcct gcagcagtgt ctgagccctt 6720
   cctacctgcc cctgcgatgt atggccggag gctgtgggcg gctgctccgg ggacctgaga 6780
   gctgctccct gggctgtgct caggcaactc agtgcgcctt gtgcctgcgg cgcccccatt 6840
   gcggctggtg tgcctggggg ggccaggatg ggggtggccg ctgcatggag ggtggactca 6900
   gcggcccccg tgatgggctg acatgtgggc gtccgggggc ctcctgggcc ttcctgtcct 6960
   gcccccctga ggacgagtgt gcaaacgggc accacgactg caacgagacg cagaattgcc 7020
   acgaccagcc ccacggctat gagtgcagct gcaagaccgg ctataccatg gacaacatga 7080
   cagggctgtg ccgccctgtg tgcgcccagg gctgcgtgaa cggctcatgt gtggagcccg 7140
   accactgccg ctgccacttt ggctttgtgg gccgcaactg ctccacggaa tgccgctgca 7200
   accgccacag tgaatgcgct ggtgttgggg cgcgtgacca ctgcttgctc tgccgcaacc 7260
   acaccaaggg cagccactgt gagcagtgcc tcccgctgtt tgtgggttca gctgtcggag 7320
   gcgggacctg ccggccctgc cacgcctttt gtcgtggaaa tagccacatc tgcatctcca 7380
   ggaaggagtt acaaatgtcc aagggagagc caaagaagta ctcactggac ccagaggaga 7440
   ttgaaaactg ggtgacagag ggtcctagtg aagacgaggc cgtgtgcgtg aactgccaga 7500
   ataacagcta tggggagaaa tgcgagagct gcctgcaggg ctacttcctc ctggacggga 7560
   agtgcaccaa atgccagtgt aatggccacg cggacacatg taacgagcag gatgggacgg 7620
   gctgtccatg tcagaataac acagagacgg gcacatgcca gggcagctcc cccagtgacc 7680
   gtcgagactg ctacaagtac cagtgcgcca agtgccggga atcatttcac gggagtccgc 7740
   tgggcggcca gcagtgctac cgcctcatct cggtggagca ggagtgctgc ctggacccca 7800
   cgtcccagac caactgcttc catgagccca aacgccgggc gctaggcccc ggccgcactg 7860
   tcctctttgg cgtgcagccc aaattcacca acgtggacat ccgcctgacg ctggacgtga 7920
   ccttcggggc cgtggacctc tatgtctcca cctcctatga caccttcgtg gtccgtgtgg 7980
   cccctgacac tggcgtccat actgtacaca tccagccacc cccagcccca ccacctccac 8040
   caccccctgc agatggtggg ccccgggggg ctggggatcc aggaggagca ggggccagca 8100
   gtgggccggg cgccccagca gagccacggg tacgggaggt atggccgcgg ggcctgatta 8160
   cctacgtgac ggtgacggag ccgtcggcag tgctggtggt ccgcggcgtg cgggaccggc 8220
   tggtcatcac ctacccacac gagcaccatg ccctcaagtc gagccgcttc tacctgctgc 8280
   tgctgggcgt gggagaccca agtgggcccg gcgccaacgg ctcagccgac tcgcagggcc 8340
   tgctcttctt ccggcaggac caggcccaca ttgacctgtt tgtcttcttc tccgtcttct 8400
   tctcctgctt cttcctcttc ctctcactct gtgtgctcct ctggaaggcc aagcaggctc 8460
   tggaccagcg gcaggagcag cgccggcact tgcaggagat gaccaagatg gccagccgcc 8520
   ccttcgccaa ggtcaccgtc tgcttcccac ctgaccctac tgccccggcc tccgcctgga 8580
   agccggctgg gctcccacct cccgccttcc gccgctctga gcccttcctg gcacccctgc 8640
   tgctgacagg ggccggtggg ccctggggac ccatgggagg gggctgctgc ccaccagcca 8700
   tccccgccac cactgctggg ctgcgagctg ggcccatcac tctcgagccc acagaagatg 8760
   gcatggctgg cgtggccaca ctgctgctcc agctgcctgg cgggccccat gcacccaacg 8820
   gcgcctgcct ggggtcagcc ctcgtcacac tgcggcacag gctgcacgag tactgtgggg 8880
   gtggtggggg tgctgggggc agtgggcatg ggactggtgc gggccggaag ggactgttga 8940
   gccaggacaa cctcaccagc atgtccctct gacatgccca gggttctcat ccacagcagc 9000
   tgggtcacct gatagggccg ccctggactt ggggtccctc cacctggggg cccctggaca 9060
   ctgtctactt ggagaccact ggcccccttc ccccagggtt gcccagatgg ggcctccttt 9120
   gttctgcatt cagcagctat ttatcgagta cctactctgt caggcactgt cataggcgtg 9180
   gggcaaagca ggaaccaaga gacgaggttc cctgatctca tgggacttag gttctggtga 9240
   agggagacaa tcagtgcaca tgcacacacc ccacacgcat acacacatga acacatgcac 9300
   atgtgcacac acaagtaaga tggcttcaga gagggagaag cactgtgagg cctccagagg 9360
   atgtggcagt gagggacgat ggggtgaagt cagctgggca ttcaaagaag ctagactgag 9420
   aacgcctgag aagaaccagc tacgggaaga gctttgggaa gcaaaggcag aggccctggg 9480
   gtgggagcag gcttgtttta ttggaaggac cagaaaactg gtaagtgtga cccagatcaa 9540
   gtgtgaggag atgaggctgg ggatagtcag gggctggatc acccagggcc ttgtgggccc 9600
   cacatagggt tttgggtttt attctcaggg caatgggaag ctgttggatg gtttgatgaa 9660
   ggggagtgac aggatccgat gtacctattt aagaatttaa gagggtcggg tgcggtggct 9720
   catgcctata atcctagcac tttgagaggc caaggtgggc tgatcacaag gtcaagagtt 9780
   cgtgaccagc ctggccaata tggtgaaacc ccatgtctac taaaaataca aaaattagct 9840
   gggcatggtg gcacacactt gtagtccgag ctactcggga ggctgaggca gaagaatcgc 9900
   ttgaacccag gaggcggagg ttgcagtgag ctcagattgc gtcactgcac tccaacctgg 9960
   gtgacagagc gagactccac ctcaaaaaaa aaaaaaaaat ttaagaggtc actcagttgt 10020
   gctgtggaga atggaccgga gggacaagag gggcagcagg gatggtgggc tggagtaggg 10080
   tgctggcaat gagggagtct ggctcagatg tgggatgtgt atggaagaat ataaatgatg 10140
   gtgtggatgt cagggtgagg gaggagacaa aaccacgatg acccctagct ttgtggcctg 10200
   aactgtgggt ggctgagggg atcgttaatt gaatggggca gactgaggct tgtgaggaag 10260
   atcagagtct ggttcttgac atgagatgcc cttcagacat ctcttcactc aggtccaact 10320
   agggatacag aaacactgaa tatttcaaca gcagaaattg aatgggggga ttgatagcgc 10380
   tggcgaggga agcagctgga aagagacaga tggcaccctg agacagccca gaggtgaata 10440
   ggacccccag gctgcaggga taaagctcag tggtggtgtt acctcaccgg ggaccagggt 10500
   cacacagcaa agctggaaca acagaggcgt gttgtggggg agcctcagag gggacaaaac   10560
   ctctgcctga gatcccaccc caggtgggca tgggggccac tgaggttggg gatgaaaatg   10620
   ccggtaccgt cagtgcacag ccctgttcca gacagtgctg cctggaagat ttctgggctc   10680
   tcctgaggcg ccaccccgca cctgagccac ctccttggac tcctgtcctc taccccttga   10740
   ggacctccct cccttctacc ctagctgtct tcttgaactt gggactctcc tttcccaaga   10800
   cttccatcac tagctcctgg agggactgga ctttgcatct tcccttcgcg tggagcctca   10860
   gtgtgagagg ccctagccaa tgcgtgcatg tcagaggtgg tggggaccac atcagaagaa   10920
   gaggggggtg atgaaattaa caaataaaaa gtatggggaa aaccca                   10966
<210> 45
   <211> 10966
   <212> DNA
   <213> Homo sapiens
<400> 45
   ggccgctctg gcccaagcgt cccctcgtct ctctgatctg gcccatccgg cttcggaggg      60
   aggcgagggt gggtgagcaa agggattggg tctgtggggt ccaggcccga acccctgaag     120
   acgggctccg cccccggcac ccgctcgcgc cccgcccccg gctggaggag tctctcctgg     180
   accatccgaa cctagcctgt cccggcccgc agcctctatg gaggctcctg ccgggccgta     240
   gagcccttcg ccccctgggg acccacccgt ctataaggtc cgtttggcct gcagcagcct     300
   gagtccgtaa tgctgggcac tgttcatggg atcggccccc tatggagccc tgtgtctata     360
   ggggactcct acggtcccta gggttcggcc ccgtccataa tgactccata tacagggcct     420
   tccatcgctc tatagggctc agccctcggc ttccagagcc tgtcagcagt ggccgtaccc     480
   ttcgccggga ctgccgggtc tccgggacct cttcgatcta caaggtcatg ttatgcctat     540
   agaggtcgca tttgcagggc ctcaccccgg gtagagggtc ctctccaggt ttttacggcc     600
   tgtccccgct ctaagggtca gtgcaggagg cggcgatggc cctgggcaag gttctggcca     660
   tggcactggt tttggccttg gccgtgctgg ggtcgctgtc ccctggggcc cgggcggggg     720
   actgcaaggg gcagcggcag gtgctgcggg aggcgccagg cttcgtgacg gatggtgcgg     780
   gcaactacag cgtcaatggc aactgcgagt ggctcatcga ggccccaagc ccccagcacc     840
   ggatcctgct ggacttcctt ttcctggaca cagagtgcac gtatgactac ctgttcgtgt     900
   atgacggtga ctccccgcga gggccgctgc ttgccagtct aagtgggagc acccgacctc     960
   cgcccatcga agcttcctca ggcaagatgc tgctgcacct cttcagtgat gccaactaca    1020
   acctgctggg ctttaacgcc tcattccgct tctccctgtg cccgggtggc tgccagagcc    1080
   acgggcagtg ccagccaccg ggtgtgtgtg cctgcgagcc gggctggggg ggtcctgact    1140
   gtggcctgca ggagtgctca gcctactgtg gcagccacgg cacctgcgcc tcgcccctgg    1200
   gaccatgccg ctgtgagcct ggcttcttgg gacgtgcctg tgacctgcac ctgtgggaga    1260
   accagggggc tgggtggtgg cacaacgtga gtgccaggga ccctgccttc tctgcccgta    1320
   ttggggcagc tggcgccttc ctgtccccac cagggctgct ggcagttttc ggaggccagg    1380
   acctcaacaa tgccctgggt gacctcgtcc tatacaactt ctccgccaac acctgggagt    1440
   cttgggacct gagtcctgcc ccggctgccc gtcactccca tgtggccgtg gcctgggccg    1500
   gctccctggt actgatgggt ggtgagctgg ctgacggctc gctcaccaac gacgtgtggg    1560
   ccttcagtcc actgggcagg ggccactggg agctcctggc accacctgcc tccagctcct    1620
   cggggccccc aggcctggca ggtcacgcgg ctgccctggt ggatgatgtc tggctatatg    1680
   tgtctggagg ccgcaccccg cacgacctct tctcctctgg cctcttccgt ttccgccttg    1740
   acagcaccag cgggggctat tgggagcagg tgattccggc aggcggacgg ccccctgctg    1800
   ccactggcca ctccatggtg ttccatgccc cctcccgtgc cctgctggtc catggtggac    1860
   accggccctc cactgcccgg ttctctgtgc gagtgaactc cactgagctt ttccacgtgg    1920
   atcggcatgt gtggacgacg ctgaaggggc gggatgggct tcagggccca agggagcgag    1980
   ccttccacac agccagtgtt ctgggcaatt acatggtggt ctatgggggc aatgtgcaca    2040
   cccattacca ggaggaaaag tgctacgaag atggcatctt cttctaccac cttggctgcc    2100
   atcaatgggt gtcaggagct gagcttgccc cgccaggaac ccctgagggc cgagcagcgc    2160
   ctcccagtgg tcggtactca catgtagctg cggtgcttgg tggcagcgtc ctgttggtgg    2220
   ctggggggta cagcggccgg ccccgtgggg acttgatggc gtacaaggtg cccccctttg    2280
   tgttccaggc acctgcccct gactaccact tggactactg ctccatgtac acagaccaca    2340
   gcgtctgctc ccgggacccg gaatgcagtt ggtgccaagg agcctgccaa gctgcacccc    2400
   ctcctgggac ccccttgggg gcttgtccag ccgccagctg cctgggcctg ggccgcctcc    2460
   tgggtgactg ccaggcctgc ctggccttca gcagccccac agcccctcca cggggacctg    2520
   gcaccctggg ctggtgcgtg cacaatgaga gctgcctccc taggcctgag caggcccgct    2580
   gccgagggga gcagatctca ggcactgtgg gctggtgggg gcctgcgcct gtcttcgtca    2640
   cgtccctgga ggcctgcgtc acccagagct tcctgcctgg cctgcacttg ctcacctttc    2700
   agcagccgcc caatacctcc cagcctgaca aggaggaggt ggggcgctgg gtggctcatc    2760
   aggagaagga gacgcggcgg ctgcagcgcc ctgggtctgc tcgcctcttc cctctgcctg    2820
   ggcgggacca caagtatgca gtagagatcc agggccagct caatggctcg gcaggccctg    2880
   ggcacagcga gctaactctg ctgtgggatc ggactggtgt gccaggaggc agcgagatct    2940
   ccttcttctt cctggagccc taccgctcgt cgtcctgcac ctcctattct tcctgcctgg    3000
   gctgcttggc agaccagggc tgtggctggt gcctgaccag tgccacctgc cacctgcgcc 3060
   agggcggagc ccattgcggg gatgacgggg ctggtgggtc cctgctggtg ctggtgccta 3120
   ccctctgccc actctgcgag gagcatcggg actgccacgc ctgcacccag gaccccttct 3180
   gtgagtggca tcagagcacc agccgcaaag gggacgcggc atgcagccgg cggggccggg 3240
   gtcggggtgc cctgaagagt ccagaggagt gtcccccgct ctgcagccag cgactgacct 3300
   gtgaggactg cctggccaac tctagccagt gcgcctggtg ccagtccacc cacacctgct 3360
   tcctgtttgc tgcctacttg gcccggtacc cacacggggg ctgtcgaggc tgggacgaca 3420
   gtgtacactc ggagccacgg tgccggagct gcgatggctt cctgacctgc catgagtgtc 3480
   tgcagagcca cgagtgtggc tggtgtggca atgaggacaa ccccacactg ggacggtgcc 3540
   tacaggggga cttctcaggg cccctcggtg ggggtaactg ctccctgtgg gtgggggagg 3600
   gcctggggct tcccgtggcc ctccctgccc gctgggcata cgcccgctgt cctgacgtgg 3660
   atgagtgtcg cctgggcctg gcccggtgcc acccgcgggc gacctgcctg aacacgcccc 3720
   tcagctacga gtgtcactgc cagcggggct accagggtga tggcatctca cactgcaacc 3780
   gcacgtgctt ggaggactgt ggccatggtg tgtgcagtgg ccccccggac tttacctgcg 3840
   tgtgtgacct aggctggaca tcagacctgc cccctcccac acccgccccg ggtccgccag 3900
   ccccccgctg ctcccgggac tgtggctgca gcttccacag ccactgccgc aagcggggcc 3960
   ctggcttctg cgacgagtgc caggactgga catgggggga gcactgcgaa cgatgccggc 4020
   ccggcagctt cggcaacgcc acaggctcta ggggctgccg gccctgccag tgcaacgggc 4080
   acggggaccc acgccgtggc cactgcgaca acctcagtgg gctctgcttc tgccaggacc 4140
   acaccgaggg tgcccactgc cagctctgct ccccaggcta ttatggggat ccccgggccg 4200
   gtggttcctg ctttcgggag tgtgggggtc gcgccctcct caccaacgtg tcctcagtgg 4260
   cactgggctc acgccgggtc ggggggctgc tgcctccagg tggcggggct gcaagagccg 4320
   ggcctggcct gtcctactgt gtgtgggttg tctcggccac tgaggagcta cagccctgtg 4380
   ctcccgggac cctctgtccc ccactcaccc tcaccttctc ccccgacagc agcaccccct 4440
   gcacgctgag ctacgtcctg gcgtttgatg gattcccacg cttcctggac actggtgttg 4500
   tccagtcgga ccgcagcctc atagctgcct tctgcggcca gcgacgggac aggcccctca 4560
   ctgttcaggc cctgtctggg ctgctcgtgc tgcactggga ggccaatggc tcctcatcct 4620
   ggggcttcaa tgcttcggtg ggctctgccc gctgtgggtc agggggcccc gggagctgtc 4680
   ccgtccccca ggaatgcgtg ccccaggacg gtgctgcagg tgcggggctc tgccgatgtc 4740
   ctcagggctg ggctggccca cactgccgca tggctctgtg tcctgagaac tgcaatgccc 4800
   acactggggc aggaacttgt aaccagagcc tgggtgtgtg catctgtgcc gagggcttcg 4860
   ggggccccga ctgcgccacc aagctggatg gcgggcagct ggtctgggag accctcatgg 4920
   acagccgcct ctcagccgac actgccagcc gcttcctgca ccgcctgggc cacaccatgg 4980
   tggatggacc cgatgccacc ttgtggatgt ttgggggcct gggcctgccc caggggctgc 5040
   tgggaaacct gtacaggtac tcagtgagtg agcggcggtg gacacagatg ctggcgggag 5100
   ccgaggacgg gggcccaggc ccatcgcccc gctccttcca tgcagccgca tatgtgcccg 5160
   ctggccgtgg tgccatgtat ctgctggggg gacttaccgc tggaggcgtc acccgtgatt 5220
   tctgggtcct caacctcacc accctgcaat ggcggcagga gaaggccccc cagaccgtgg 5280
   agctgccagc cgttgctggt cacaccctta ctgcccgccg aggcctgtct ctgctcctgg 5340
   tgggcggtta ctccccggaa aatggcttca accagcagct gctggagtac cagctggcaa 5400
   ccggcacctg ggtgtcagga gcccagagtg ggacaccccc cacaggtctc tatggtcact 5460
   ctgctgtcta ccacgaggcc accgactccc tctacgtgtt tggggggttc cgattccatg 5520
   tggagctggc ggccccatcc cccgagctct actccctgca ctgtcctgac cgcacctgga 5580
   gtctgctggc cccttctcag ggggcaaagc gagatcgtat gaggaatgtg cgtggctcat 5640
   ctcggggtct gggccaagtt cctggggagc agcctgggtc atgggggttc cgggaagtca 5700
   ggaagaagat ggctctgtgg gctgctcttg ctggtacagg aggtttcctg gaggaaatct 5760
   cacctcacct gaaggagccc cgcccccggc ttttccacgc ctcagccctg ttaggggaca 5820
   ccatggtggt tcttgggggg cgctcggacc ctgacgagtt cagcagcgac gttctgctct 5880
   accaggtcaa ctgcaatgcc tggcttctgc ccgacctcac ccgctcggcc tctgtggggc 5940
   ccccaatgga ggagtctgtg gcccatgctg tggcagcagt cgggagccgc ctgtatatct 6000
   ctgggggttt cgggggagtg gccctgggcc gcctgctggc actgaccctg ccccctgacc 6060
   cctgccgcct gctgtcctca cctgaagctt gtaaccagtc tggggcctgc acctggtgcc 6120
   atggggcctg cttgtccggg gatcaggccc acaggctggg ctgcgggggc tccccctgct 6180
   ccccaatgcc tcgctccccg gaggaatgtc gacgtctccg gacctgcagt gagtgcctgg 6240
   cccgccatcc tcggaccctg caacctggag atggagaggc gtccaccccc cgctgtaagt 6300
   ggtgtaccaa ctgccccgaa ggtgcttgca ttggacgcaa tgggtcctgc acctctgaga 6360
   atgactgtcg gatcaaccag cgagaggtct tctgggcagg gaactgctcc gaggctgcgt 6420
   gcggggctgc tgactgcgag cagtgcacgc gggagggcaa gtgcatgtgg acgcggcagt 6480
   tcaagaggac aggggagacc cgccgcatcc tctccgtgca gcccacctat gactggacgt 6540
   gcttcagcca ctctctgctg aatgtgtccc ccatgccggt ggaatcatca cccccactgc 6600
   cctgccccac cccttgtcac ctcctaccca actgtacctc ctgcctggac tctaagggag 6660
   cagatggggg ctggcagcac tgtgtttgga gcagcagcct gcagcagtgt ctgagccctt 6720
   cctacctgcc cctgcgatgt atggccggag gctgtgggcg gctgctccgg ggacctgaga 6780
   gctgctccct gggctgtgct caggcaactc agtgcgcctt gtgcctgcgg cgcccccatt 6840
   gcggctggtg tgcctggggg ggccaggatg ggggtggccg ctgcatggag ggtggactca 6900
   gcggcccccg tgatgggctg acatgtgggc gtccgggggc ctcctgggcc ttcctgtcct 6960
   gcccccctga ggacgagtgt gcaaacgggc accacgactg caacgagacg cagaattgcc 7020
   acgaccagcc ccacggctat gagtgcagct gcaagaccgg ctataccatg gacaacatga 7080
   cagggctgtg ccgccctgtg tgcgcccagg gctgcgtgaa cggctcatgt gtggagcccg 7140
   accactgccg ctgccacttt ggctttgtgg gccgcaactg ctccacggaa tgccgctgca 7200
   accgccacag tgaatgcgct ggtgttgggg cgcgtgacca ctgcttgctc tgccgcaacc 7260
   acaccaaggg cagccactgt gagcagtgcc tcccgctgtt tgtgggttca gctgtcggag 7320
   gcgggacctg ccggccctgc cacgcctttt gtcgtggaaa tagccacatc tgcatctcca 7380
   ggaaggagtt acaaatgtcc aagggagagc caaagaagta ctcactggac ccagaggaga 7440
   ttgaaaactg ggtgacagag ggtcctagtg aagacgaggc cgtgtgcgtg aactgccaga 7500
   ataacagcta tggggagaaa tgcgagagct gcctgcaggg ctacttcctc ctggacggga 7560
   agtgcaccaa atgccagtgt aatggccacg cggacacatg taacgagcag gatgggacgg 7620
   gctgtccatg tcagaataac acagagacgg gcacatgcca gggcagctcc cccagtgacc 7680
   gtcgagactg ctacaagtac cagtgcgcca agtgccggga atcatttcac gggagtccgc 7740
   tgggcggcca gcagtgctac cgcctcatct cggtggagca ggagtgctgc ctggacccca 7800
   cgtcccagac caactgcttc catgagccca aacgccgggc gctaggcccc ggccgcactg 7860
   tcctctttgg cgtgcagccc aaattcacca acgtggacat ccgcctgacg ctggacgtga 7920
   ccttcggggc cgtggacctc tatgtctcca cctcctatga caccttcgtg gtccgtgtgg 7980
   cccctgacac tggcgtccat actgtacaca tccagccacc cccagcccca ccacctccac 8040
   caccccctgc agatggtggg ccccgggggg ctggggatcc aggaggagca ggggccagca 8100
   gtgggccggg cgccccagca gagccacggg tacgggaggt atggccgcgg ggcctgatta 8160
   cctacgtgac ggtgacggag ccgtcggcag tgctggtggt ccgcggcgtg cgggaccggc 8220
   tggtcatcac ctacccacac gagcaccatg ccctcaagtc gagccgcttc tacctgctgc 8280
   tgctgggcgt gggagaccca agtgggcccg gcgccaacgg ctcagccgac tcgcagggcc 8340
   tgctcttctt ccggcaggac caggcccaca ttgacctgtt tgtcttcttc tccgtcttct 8400
   tctcctgctt cttcctcttc ctctcactct gtgtgctcct ctggaaggcc aagcaggctc 8460
   tggaccagcg gcaggagcag cgccggcact tgcaggagat gaccaagatg gccagccgcc 8520
   ccttcgccaa ggtcaccgtc tgcttcccac ctgaccctac tgccccggcc tccgcctgga 8580
   agccggctgg gctcccacct cccgccttcc gccgctctga gcccttcctg gcacccctgc 8640
   tgctgacagg ggccggtggg ccctggggac ccatgggagg gggctgctgc ccaccagcca 8700
   tccccgccac cactgctggg ctgcgagctg ggcccatcac tctcgagccc acagaagatg 8760
   gcatggctgg cgtggccaca ctgctgctcc agctgcctgg cgggccccat gcacccaacg 8820
   gcgcctgcct ggggtcagcc ctcgtcacac tgcggcacag gctgcacgag tactgtgggg 8880
   gtggtggggg tgctgggggc agtgggcatg ggactggtgc gggccggaag ggactgttga 8940
   gccaggacaa cctcaccagc atgtccctct gacatgccca gggttctcat ccacagcagc 9000
   tgggtcacct gatagggccg ccctggactt ggggtccctc cacctggggg cccctggaca 9060
   ctgtctactt ggagaccact ggccccttcc ccccagggtt gcccagatgg ggcctccttt 9120
   gttctgcatt cagcagctat ttatcgagta cctactctgt caggcactgt cataggcgtg 9180
   gggcaaagca ggaaccaaga gacgaggttc cctgatctca tgggacttag gttctggtga 9240
   agggagacaa tcagtgcaca tgcacacacc ccacacgcat acacacatga acacatgcac 9300
   atgtgcacac acaagtaaga tggcttcaga gagggagaag cactgtgagg cctccagagg 9360
   atgtggcagt gagggacgat ggggtgaagt cagctgggca ttcaaagaag ctagactgag 9420
   aacgcctgag aagaaccagc tacgggaaga gctttgggaa gcaaaggcag aggccctggg 9480
   gtgggagcag gcttgtttta ttggaaggac cagaaaactg gtaagtgtga cccagatcaa 9540
   gtgtgaggag atgaggctgg ggatagtcag gggctggatc acccagggcc ttgtgggccc 9600
   cacatagggt tttgggtttt attctcaggg caatgggaag ctgttggatg gtttgatgaa 9660
   ggggagtgac aggatccgat gtacctattt aagaatttaa gagggtcggg tgcggtggct 9720
   catgcctata atcctagcac tttgagaggc caaggtgggc tgatcacaag gtcaagagtt 9780
   cgtgaccagc ctggccaata tggtgaaacc ccatgtctac taaaaataca aaaattagct 9840
   gggcatggtg gcacacactt gtagtccgag ctactcggga ggctgaggca gaagaatcgc 9900
   ttgaacccag gaggcggagg ttgcagtgag ctcagattgc gtcactgcac tccaacctgg 9960
   gtgacagagc gagactccac ctcaaaaaaa aaaaaaaaat ttaagaggtc actcagttgt 10020
   gctgtggaga atggaccgga gggacaagag gggcagcagg gatggtgggc tggagtaggg 10080
   tgctggcaat gagggagtct ggctcagatg tgggatgtgt atggaagaat ataaatgatg 10140
   gtgtggatgt cagggtgagg gaggagacaa aaccacgatg acccctagct ttgtggcctg 10200
   aactgtgggt ggctgagggg atcgttaatt gaatggggca gactgaggct tgtgaggaag 10260
   atcagagtct ggttcttgac atgagatgcc cttcagacat ctcttcactc aggtccaact 10320
   agggatacag aaacactgaa tatttcaaca gcagaaattg aatgggggga ttgatagcgc 10380
   tggcgaggga agcagctgga aagagacaga tggcaccctg agacagccca gaggtgaata 10440
   ggacccccag gctgcaggga taaagctcag tggtggtgtt acctcaccgg ggaccagggt  10500
   cacacagcaa agctggaaca acagaggcgt gttgtggggg agcctcagag gggacaaaac  10560
   ctctgcctga gatcccaccc caggtgggca tgggggccac tgaggttggg gatgaaaatg  10620
   ccggtaccgt cagtgcacag ccctgttcca gacagtgctg cctggaagat ttctgggctc  10680
   tcctgaggcg ccaccccgca cctgagccac ctccttggac tcctgtcctc taccccttga  10740
   ggacctccct cccttctacc ctagctgtct tcttgaactt gggactctcc tttcccaaga  10800
   cttccatcac tagctcctgg agggactgga ctttgcatct tcccttcgcg tggagcctca  10860
   gtgtgagagg ccctagccaa tgcgtgcatg tcagaggtgg tggggaccac atcagaagaa  10920
   gaggggggtg atgaaattaa caaataaaaa gtatggggaa aaccca                  10966
<210> 46
   <211> 11167
   <212> DNA
   <213> Homo sapiens
<400> 46
   ggccgctctg gcccaagcgt cccctcgtct ctctgatctg gcccatccgg cttcggaggg      60
   aggcgagggt gggtgagcaa agggattggg tctgtggggt ccaggcccga acccctgaag     120
   acgggctccg cccccggcac ccgctcgcgc cccgcccccg gctggaggag tctctcctgg     180
   accatccgaa cctagcctgt cccggcccgc agcctctatg gaggctcctg ccgggccgta     240
   gagcccttcg ccccctgggg acccacccgt ctataaggtc cgtttggcct gcagcagcct     300
   gagtccgtaa tgctgggcac tgttcatggg atcggccccc tatggagccc tgtgtctata     360
   ggggactcct acggtcccta gggttcggcc ccgtccataa tgactccata tacagggcct     420
   tccatcgctc tatagggctc agccctcggc ttccagagcc tgtcagcagt ggccgtaccc     480
   ttcgccggga ctgccgggtc tccgggacct cttcgatcta caaggtcatg ttatgcctat     540
   agaggtcgca tttgcagggc ctcaccccgg gtagagggtc ctctccaggt ttttacggcc     600
   tgtccccgct ctaagggtca gtgcaggagg cggcgatggc cctgggcaag gttctggcca     660
   tggcactggt tttggccttg gccgtgctgg ggtcgctgtc ccctggggcc cgggcggggg     720
   actgcaaggg gcagcggcag gtgctgcggg aggcgccagg cttcgtgacg gatggtgcgg     780
   gcaactacag cgtcaatggc aactgcgagt ggctcatcga ggccccaagc ccccagcacc     840
   ggatcctgct ggacttcctt ttcctggaca cagagtgcac gtatgactac ctgttcgtgt     900
   atgacggtga ctccccgcga gggccgctgc ttgccagtct aagtgggagc acccgacctc     960
   cgcccatcga agcttcctca ggcaagatgc tgctgcacct cttcagtgat gccaactaca    1020
   acctgctggg ctttaacgcc tcattccgct tctccctgtg cccgggtggc tgccagagcc    1080
   acgggcagtg ccagccaccg ggtgtgtgtg cctgcgagcc gggctggggg ggtcctgact    1140
   gtggcctgca ggagtgctca gcctactgtg gcagccacgg cacctgcgcc tcgcccctgg 1200
   gaccatgccg ctgtgagcct ggcttcttgg gacgtgcctg tgacctgcac ctgtgggaga 1260
   accagggggc tgggtggtgg cacaacgtga gtgccaggga ccctgccttc tctgcccgta 1320
   ttggggcagc tggcgccttc ctgtccccac cagggctgct ggcagttttc ggaggccagg 1380
   acctcaacaa tgccctgggt gacctcgtcc tatacaactt ctccgccaac acctgggagt 1440
   cttgggacct gagtcctgcc ccggctgccc gtcactccca tgtggccgtg gcctgggccg 1500
   gctccctggt actgatgggt ggtgagctgg ctgacggctc gctcaccaac gacgtgtggg 1560
   ccttcagtcc actgggcagg ggccactggg agctcctggc accacctgcc tccagctcct 1620
   cggggccccc aggcctggca ggtcacgcgg ctgccctggt ggatgatgtc tggctatatg 1680
   tgtctggagg ccgcaccccg cacgacctct tctcctctgg cctcttccgt ttccgccttg 1740
   acagcaccag cgggggctat tgggagcagg tgattccggc aggcggacgg ccccctgctg 1800
   ccactggcca ctccatggtg ttccatgccc cctcccgtgc cctgctggtc catggtggac 1860
   accggccctc cactgcccgg ttctctgtgc gagtgaactc cactgagctt ttccacgtgg 1920
   atcggcatgt gtggacgacg ctgaaggggc gggatgggct tcagggccca agggagcgag 1980
   ccttccacac agccagtgtt ctgggcaatt acatggtggt ctatgggggc aatgtgcaca 2040
   cccattacca ggaggaaaag tgctacgaag atggcatctt cttctaccac cttggctgcc 2100
   atcaatgggt gtcaggagct gagcttgccc cgccaggaac ccctgagggc cgagcagcgc 2160
   ctcccagtgg tcggtactca catgtagctg cggtgcttgg tggcagcgtc ctgttggtgg 2220
   ctggggggta cagcggccgg ccccgtgggg acttgatggc gtacaaggtg cccccctttg 2280
   tgttccaggc acctgcccct gactaccact tggactactg ctccatgtac acagaccaca 2340
   gcgtctgctc ccgggacccg gaatgcagtt ggtgccaagg agcctgccaa gctgcacccc 2400
   ctcctgggac cccttggggg gcttgtccag ccgccagctg cctgggcctg ggccgcctcc 2460
   tgggtgactg ccaggcctgc ctggccttca gcagccccac agcccctcca cggggacctg 2520
   gcaccctggg ctggtgcgtg cacaatgaga gctgcctccc taggcctgag caggcccgct 2580
   gccgagggga gcagatctca ggcactgtgg gctggtgggg gcctgcgcct gtcttcgtca 2640
   cgtccctgga ggcctgcgtc acccagagct tcctgcctgg cctgcacttg ctcacctttc 2700
   agcagccgcc caatacctcc cagcctgaca aggtctcaat tgtccgcagc acgaccatca 2760
   ccctaacacc cagcgcagag acagatgtgt ccctggtcta ccgtggcttc atctacccaa 2820
   tgctgcctgg agggccaggt ggaccagggg ctgaggacgt ggccgtgtgg acgcgggccc 2880
   agcgcctaca cgtcctggcc cggatggccc gtggccctga cacggagaac atggaggagg 2940
   tggggcgctg ggtggctcat caggagaagg agacgcggcg gctgcagcgc cctgggtctg 3000
   ctcgcctctt ccctctgcct gggcgggacc acaagtatgc agtagagatc cagggccagc 3060
   tcaatggctc ggcaggccct gggcacagcg agctaactct gctgtgggat cggactggtg 3120
   tgccaggagg cagcgagatc tccttcttct tcctggagcc ctaccgctcg tcgtcctgca 3180
   cctcctattc ttcctgcctg ggctgcttgg cagaccaggg ctgtggctgg tgcctgacca 3240
   gtgccacctg ccacctgcgc cagggcggag cccattgcgg ggatgacggg gctggtgggt 3300
   ccctgctggt gctggtgcct accctctgcc cactctgcga ggagcatcgg gactgccacg    3360
   cctgcaccca ggaccccttc tgtgagtggc atcagagcac cagccgcaaa ggggacgcgg    3420
   catgcagccg gcggggccgg ggtcggggtg ccctgaagag tccagaggag tgtcccccgc 3480
   tctgcagcca gcgactgacc tgtgaggact gcctggccaa ctctagccag tgcgcctggt 3540
   gccagtccac ccacacctgc ttcctgtttg ctgcctactt ggcccggtac ccacacgggg 3600
   gctgtcgagg ctgggacgac agtgtacact cggagccacg gtgccggagc tgcgatggct 3660
   tcctgacctg ccatgagtgt ctgcagagcc acgagtgtgg ctggtgtggc aatgaggaca 3720
   accccacact gggacggtgc ctacaggggg acttctcagg gcccctcggt gggggtaact 3780
   gctccctgtg ggtgggggag ggcctggggc ttcccgtggc cctccctgcc cgctgggcat 3840
   acgcccgctg tcctgacgtg gatgagtgtc gcctgggcct ggcccggtgc cacccgcggg 3900
   cgacctgcct gaacacgccc ctcagctacg agtgtcactg ccagcggggc taccagggtg 3960
   atggcatctc acactgcaac cgcacgtgct tggaggactg tggccatggt gtgtgcagtg 4020
   gccccccgga ctttacctgc gtgtgtgacc taggctggac atcagacctg ccccctccca    4080
   cacccgcccc gggtccgcca gccccccgct gctcccggga ctgtggctgc agcttccaca    4140
   gccactgccg caagcggggc cctggcttct gcgacgagtg ccaggactgg acatgggggg 4200
   agcactgcga acgatgccgg cccggcagct tcggcaacgc cacaggctct aggggctgcc 4260
   ggccctgcca gtgcaacggg cacggggacc cacgccgtgg ccactgcgac aacctcagtg 4320
   ggctctgctt ctgccaggac cacaccgagg gtgcccactg ccagctctgc tccccaggct 4380
   attatgggga tccccgggcc ggtggttcct gctttcggga gtgtgggggt cgcgccctcc 4440
   tcaccaacgt gtcctcagtg gcactgggct cacgccgggt cggggggctg ctgcctccag 4500
   gtggcggggc tgcaagagcc gggcctggcc tgtcctactg tgtgtgggtt gtctcggcca 4560
   ctgaggagct acagccctgt gctcccggga ccctctgtcc cccactcacc ctcaccttct 4620
   cccccgacag cagcaccccc tgcacgctga gctacgtcct ggcgtttgat ggattcccac 4680
   gcttcctgga cactggtgtt gtccagtcgg accgcagcct catagctgcc ttctgcggcc 4740
   agcgacggga caggcccctc actgttcagg ccctgtctgg gctgctcgtg ctgcactggg 4800
   aggccaatgg ctcctcatcc tggggcttca atgcttcggt gggctctgcc cgctgtgggt 4860
   cagggggccc cgggagctgt cccgtccccc aggaatgcgt gccccaggac ggtgctgcag 4920
   gtgcggggct ctgccgatgt cctcagggct gggctggccc acactgccgc atggctctgt 4980
   gtcctgagaa ctgcaatgcc cacactgggg caggaacttg taaccagagc ctgggtgtgt 5040
   gcatctgtgc cgagggcttc gggggccccg actgcgccac caagctggat ggcgggcagc 5100
   tggtctggga gaccctcatg gacagccgcc tctcagccga cactgccagc cgcttcctgc 5160
   accgcctggg ccacaccatg gtggatggac ccgatgccac cttgtggatg tttgggggcc 5220
   tgggcctgcc ccaggggctg ctgggaaacc tgtacaggta ctcagtgagt gagcggcggt 5280
   ggacacagat gctggcggga gccgaggacg gggcccaggg cccatcgccc cgctccttcc 5340
   atgcagccgc atatgtgccc gctggccgtg gtgccatgta tctgctgggg ggacttaccg 5400
   ctggaggcgt cacccgtgat ttctgggtcc tcaacctcac caccctgcaa tggcggcagg 5460
   agaaggcccc ccagaccgtg gagctgccag ccgttgctgg tcacaccctt actgcccgcc 5520
   gaggcctgtc tctgctcctg gtgggcggtt actccccgga aaatggcttc aaccagcagc 5580
   tgctggagta ccagctggca accggcacct gggtgtcagg agcccagagt gggacacccc 5640
   ccacaggtct ctatggtcac tctgctgtct accacgaggc caccgactcc ctctacgtgt 5700
   ttggggggtt ccgattccat gtggagctgg cggccccatc ccccgagctc tactccctgc 5760
   actgtcctga ccgcacctgg agtctgctgg ccccttctca gggggcaaag cgagatcgta 5820
   tgaggaatgt gcgtggctca tctcggggtc tgggccaagt tcctggggag cagcctgggt 5880
   catgggggtt ccgggaagtc aggaagaaga tggctctgtg ggctgctctt gctggtacag 5940
   gaggtttcct ggaggaaatc tcacctcacc tgaaggagcc ccgcccccgg cttttccacg 6000
   cctcagccct gttaggggac accatggtgg ttcttggggg gcgctcggac cctgacgagt 6060
   tcagcagcga cgttctgctc taccaggtca actgcaatgc ctggcttctg cccgacctca 6120
   cccgctcggc ctctgtgggg cccccaatgg aggagtctgt ggcccatgct gtggcagcag 6180
   tcgggagccg cctgtatatc tctgggggtt tcgggggagt ggccctgggc cgcctgctgg 6240
   cactgaccct gccccctgac ccctgccgcc tgctgtcctc acctgaagct tgtaaccagt 6300
   ctggggcctg cacctggtgc catggggcct gcttgtccgg ggatcaggcc cacaggctgg 6360
   gctgcggggg ctccccctgc tccccaatgc ctcgctcccc ggaggaatgt cgacgtctcc 6420
   ggacctgcag tgagtgcctg gcccgccatc ctcggaccct gcaacctgga gatggagagg 6480
   cgtccacccc ccgctgtaag tggtgtacca actgccccga aggtgcttgc attggacgca 6540
   atgggtcctg cacctctgag aatgactgtc ggatcaacca gcgagaggtc ttctgggcag 6600
   ggaactgctc cgaggctgcg tgcggggctg ctgactgcga gcagtgcacg cgggagggca 6660
   agtgcatgtg gacgcggcag ttcaagagga caggggagac ccgccgcatc ctctccgtgc 6720
   agcccaccta tgactggacg tgcttcagcc actctctgct gaatgtgtcc cccatgccgg    6780
   tggaatcatc acccccactg ccctgcccca ccccttgtca cctcctaccc aactgtacct    6840
   cctgcctgga ctctaaggga gcagatgggg gctggcagca ctgtgtttgg agcagcagcc 6900
   tgcagcagtg tctgagccct tcctacctgc ccctgcgatg tatggccgga ggctgtgggc 6960
   ggctgctccg gggacctgag agctgctccc tgggctgtgc tcaggcaact cagtgcgcct 7020
   tgtgcctgcg gcgcccccat tgcggctggt gtgcctgggg gggccaggat gggggtggcc 7080
   gctgcatgga gggtggactc agcggccccc gtgatgggct gacatgtggg cgtccggggg 7140
   cctcctgggc cttcctgtcc tgcccccctg aggacgagtg tgcaaacggg caccacgact 7200
   gcaacgagac gcagaattgc cacgaccagc cccacggcta tgagtgcagc tgcaagaccg 7260
   gctataccat ggacaacatg acagggctgt gccgccctgt gtgcgcccag ggctgcgtga 7320
   acggctcatg tgtggagccc gaccactgcc gctgccactt tggctttgtg ggccgcaact 7380
   gctccacgga atgccgctgc aaccgccaca gtgaatgcgc tggtgttggg gcgcgtgacc 7440
   actgcttgct ctgccgcaac cacaccaagg gcagccactg tgagcagtgc ctcccgctgt 7500
   ttgtgggttc agctgtcgga ggcgggacct gccggccctg ccacgccttt tgtcgtggaa 7560
   atagccacat ctgcatctcc aggaaggagt tacaaatgtc caagggagag ccaaagaagt 7620
   actcactgga cccagaggag attgaaaact gggtgacaga gggtcctagt gaagacgagg 7680
   ccgtgtgcgt gaactgccag aataacagct atggggagaa atgcgagagc tgcctgcagg 7740
   gctacttcct cctggacggg aagtgcacca aatgccagtg taatggccac gcggacacat 7800
   gtaacgagca ggatgggacg ggctgtccat gtcagaataa cacagagacg ggcacatgcc 7860
   agggcagctc ccccagtgac cgtcgagact gctacaagta ccagtgcgcc aagtgccggg 7920
   aatcatttca cgggagtccg ctgggcggcc agcagtgcta ccgcctcatc tcggtggagc 7980
   aggagtgctg cctggacccc acgtcccaga ccaactgctt ccatgagccc aaacgccggg 8040
   cgctaggccc cggccgcact gtcctctttg gcgtgcagcc caaattcacc aacgtggaca 8100
   tccgcctgac gctggacgtg accttcgggg ccgtggacct ctatgtctcc acctcctatg 8160
   acaccttcgt ggtccgtgtg gcccctgaca ctggcgtcca tactgtacac atccagccac 8220
   ccccagcccc accacctcca ccaccccctg cagatggtgg gccccggggg gctggggatc 8280
   caggaggagc aggggccagc agtgggccgg gcgccccagc agagccacgg gtacgggagg 8340
   tatggccgcg gggcctgatt acctacgtga cggtgacgga gccgtcggca gtgctggtgg 8400
   tccgcggcgt gcgggaccgg ctggtcatca cctacccaca cgagcaccat gccctcaagt 8460
   cgagccgctt ctacctgctg ctgctgggcg tgggagaccc aagtgggccc ggcgccaacg 8520
   gctcagccga ctcgcagggc ctgctcttct tccggcagga ccaggcccac attgacctgt 8580
   ttgtcttctt ctccgtcttc ttctcctgct tcttcctctt cctctcactc tgtgtgctcc 8640
   tctggaaggc caagcaggct ctggaccagc ggcaggagca gcgccggcac ttgcaggaga 8700
   tgaccaagat ggccagccgc cccttcgcca aggtcaccgt ctgcttccca cctgacccta 8760
   ctgccccggc ctccgcctgg aagccggctg ggctcccacc tcccgccttc cgccgctctg 8820
   agcccttcct ggcacccctg ctgctgacag gggccggtgg gccctgggga cccatgggag 8880
   ggggctgctg cccaccagcc atccccgcca ccactgctgg gctgcgagct gggcccatca 8940
   ctctcgagcc cacagaagat ggcatggctg gcgtggccac actgctgctc cagctgcctg 9000
   gcgggcccca tgcacccaac ggcgcctgcc tggggtcagc cctcgtcaca ctgcggcaca 9060
   ggctgcacga gtactgtggg ggtggtgggg gtgctggggg cagtgggcat gggactggtg 9120
   cgggccggaa gggactgttg agccaggaca acctcaccag catgtccctc tgacatgccc 9180
   agggttctca tccacagcag ctgggtcacc tgatagggcc gccctggact tggggtccct 9240
   ccacctgggg gcccctggac actgtctact tggagaccac tggccccctt cccccagggt 9300
   tgcccagatg gggcctcctt tgttctgcat tcagcagcta tttatcgagt acctactctg 9360
   tcaggcactg tcataggcgt ggggcaaagc aggaaccaag agacgaggtt ccctgatctc 9420
   atgggactta ggttctggtg aagggagaca atcagtgcac atgcacacac cccacacgca 9480
   tacacacatg aacacatgca catgtgcaca cacaagtaag atggcttcag agagggagaa 9540
   gcactgtgag gcctccagag gatgtggcag tgagggacga tggggtgaag tcagctgggc 9600
   attcaaagaa gctagactga gaacgcctga gaagaaccag ctacgggaag agctttggga 9660
   agcaaaggca gaggccctgg ggtgggagca ggcttgtttt attggaagga ccagaaaact 9720
   ggtaagtgtg acccagatca agtgtgagga gatgaggctg gggatagtca ggggctggat 9780
   cacccagggc cttgtgggcc ccacataggg ttttgggttt tattctcagg gcaatgggaa 9840
   gctgttggat ggtttgatga aggggagtga caggatccga tgtacctatt taagaattta 9900
   agagggtcgg gtgcggtggc tcatgcctat aatcctagca ctttgagagg ccaaggtggg 9960
   ctgatcacaa ggtcaagagt tcgtgaccag cctggccaat atggtgaaac cccatgtcta 10020
   ctaaaaatac aaaaattagc tgggcatggt ggcacacact tgtagtccga gctactcggg 10080
   aggctgaggc agaagaatcg cttgaaccca ggaggcggag gttgcagtga gctcagattg 10140
   cgtcactgca ctccaacctg ggtgacagag cgagactcca cctcaaaaaa aaaaaaaaaa 10200
   tttaagaggt cactcagttg tgctgtggag aatggaccgg agggacaaga ggggcagcag 10260
   ggatggtggg ctggagtagg gtgctggcaa tgagggagtc tggctcagat gtgggatgtg 10320
   tatggaagaa tataaatgat ggtgtggatg tcagggtgag ggaggagaca aaaccacgat 10380
   gacccctagc tttgtggcct gaactgtggg tggctgaggg gatcgttaat tgaatggggc 10440
   agactgaggc ttgtgaggaa gatcagagtc tggttcttga catgagatgc ccttcagaca 10500
   tctcttcact caggtccaac tagggataca gaaacactga atatttcaac agcagaaatt 10560
   gaatgggggg attgatagcg ctggcgaggg aagcagctgg aaagagacag atggcaccct 10620
   gagacagccc agaggtgaat aggaccccca ggctgcaggg ataaagctca gtggtggtgt 10680
   tacctcaccg gggaccaggg tcacacagca aagctggaac aacagaggcg tgttgtgggg 10740
   gagcctcaga ggggacaaaa cctctgcctg agatcccacc ccaggtgggc atgggggcca 10800
   ctgaggttgg ggatgaaaat gccggtaccg tcagtgcaca gccctgttcc agacagtgct 10860
   gcctggaaga tttctgggct ctcctgaggc gccaccccgc acctgagcca cctccttgga 10920
   ctcctgtcct ctaccccttg aggacctccc tcccttctac cctagctgtc ttcttgaact 10980
   tgggactctc ctttcccaag acttccatca ctagctcctg gagggactgg actttgcatc 11040
   ttcccttcgc gtggagcctc agtgtgagag gccctagcca atgcgtgcat gtcagaggtg 11100
   gtggggacca catcagaaga agaggggggt gatgaaatta acaaataaaa agtatgggga 11160
   aaaccca 11167
<210> 47
   <211> 406
   <212> PRT
   <213> Homo sapiens
<400> 47
   Met Asp Cys Arg Thr Lys Ala Asn Pro Asp Arg Thr Phe Asp Leu Val
   1 5 10 15
   Leu Lys Val Lys Cys His Ala Ser Glu Asn Glu Asp Pro Val Val Leu
   20 25 30
   Trp Lys Phe Pro Glu Asp Phe Gly Asp Gln Glu Ile Leu Gln Ser Val
   35 40 45
   Pro Lys Phe Cys Phe Pro Phe Asp Val Glu Arg Val Ser Gln Asn Gln
   50 55 60
   Val Gly Gln His Phe Thr Phe Val Leu Thr Asp Ile Glu Ser Lys Gln
   65 70 75 80
   Arg Phe Gly Phe Cys Arg Leu Thr Ser Gly Gly Thr Ile Cys Leu Cys
   85 90 95
   Ile Leu Ser Tyr Leu Pro Trp Phe Glu Val Tyr Tyr Lys Leu Leu Asn
   100 105 110
   Thr Leu Ala Asp Tyr Leu Ala Lys Glu Leu Glu Asn Asp Leu Asn Glu
           115                 120                 125
   Thr Leu Arg Ser Leu Tyr Asn His Pro Val Pro Lys Ala Asn Thr Pro
       130                 135                 140
   Val Asn Leu Ser Val His Ser Tyr Phe Ile Ala Pro Asp Val Thr Gly
   145                 150                 155                 160
   Leu Pro Thr Ile Pro Glu Ser Arg Asn Leu Thr Glu Tyr Phe Val Ala
                   165                 170                 175
   Val Asp Val Asn Asn Met Leu Gln Leu Tyr Ala Ser Met Leu His Glu
               180                 185                 190
   Arg Arg Ile Val Ile Ile Ser Ser Lys Leu Ser Thr Leu Thr Ala Cys
           195                 200                 205
   Ile His Gly Ser Ala Ala Leu Leu Tyr Pro Met Tyr Trp Gln His Ile
       210                 215                 220
   Tyr Ile Pro Val Leu Pro Pro His Leu Leu Asp Tyr Cys Cys Ala Pro
   225                 230                 235                 240
   Met Pro Tyr Leu Ile Gly Ile His Ser Ser Leu Ile Glu Arg Val Lys
                   245                 250                 255
   Asn Lys Ser Leu Glu Asp Val Val Met Leu Asn Val Asp Thr Asn Thr
               260                 265                 270
   Leu Glu Ser Pro Phe Ser Asp Leu Asn Asn Leu Pro Ser Asp Val Val
           275                 280                 285
   Ser Ala Leu Lys Asn Lys Leu Lys Lys Gln Ser Thr Ala Thr Gly Asp
       290                 295                 300
   Gly Val Ala Arg Ala Phe Leu Arg Ala Gln Ala Ala Leu Phe Gly Ser
   305                 310                 315                 320
   Tyr Arg Asp Ala Leu Arg Tyr Lys Pro Gly Glu Pro Ile Thr Phe Cys
                   325                 330                 335
   Glu Glu Ser Phe Val Lys His Arg Ser Ser Val Met Lys Gln Phe Leu
               340                 345                 350
   Glu Thr Ala Ile Asn Leu Gln Leu Phe Lys Gln Phe Ile Asp Gly Arg
   355 360 365
   Leu Ala Lys Leu Asn Ala Gly Arg Gly Phe Ser Asp Val Phe Glu Glu
   370 375 380
   Glu Ile Thr Ser Gly Gly Phe Cys Gly Gly Lys Asp Lys Leu Gln Tyr
   385 390 395 400
   Asp Tyr Pro Phe Ser Gln
   405
<210> 48
   <211> 426
   <212> PRT
   <213> Homo sapiens
<400> 48
   Met Asp Cys Arg Thr Lys Ala Asn Pro Asp Arg Thr Phe Asp Leu Val
   1 5 10 15
   Leu Lys Val Lys Cys His Ala Ser Glu Asn Glu Asp Pro Val Val Leu
   20 25 30
   Trp Lys Phe Pro Glu Asp Phe Gly Asp Gln Glu Ile Leu Gln Ser Val
   35 40 45
   Pro Lys Phe Cys Phe Pro Phe Asp Val Glu Arg Val Ser Gln Asn Gln
   50 55 60
   Val Gly Gln His Phe Thr Phe Val Leu Thr Asp Ile Glu Ser Lys Gln
   65 70 75 80
   Arg Phe Gly Phe Cys Arg Leu Thr Ser Gly Gly Thr Ile Cys Leu Cys
   85 90 95
   Ile Leu Ser Tyr Leu Pro Trp Phe Glu Val Tyr Tyr Lys Leu Leu Asn
   100 105 110
   Thr Leu Ala Asp Tyr Leu Ala Lys Glu Leu Glu Asn Asp Leu Asn Glu
   115 120 125
   Thr Leu Arg Ser Leu Tyr Asn His Pro Val Pro Lys Ala Asn Thr Pro
   130 135 140
   Val Asn Leu Ser Val Asn Gln Glu Ile Phe Ile Ala Cys Glu Gln Val
   145 150 155 160
   Leu Lys Asp Gln Pro Ala Leu Val Pro His Ser Tyr Phe Ile Ala Pro
                   165                 170                 175
   Asp Val Thr Gly Leu Pro Thr Ile Pro Glu Ser Arg Asn Leu Thr Glu
               180                 185                 190
   Tyr Phe Val Ala Val Asp Val Asn Asn Met Leu Gln Leu Tyr Ala Ser
           195                 200                 205
   Met Leu His Glu Arg Arg Ile Val Ile Ile Ser Ser Lys Leu Ser Thr
       210                 215                 220
   Leu Thr Ala Cys Ile His Gly Ser Ala Ala Leu Leu Tyr Pro Met Tyr
   225                 230                 235                 240
   Trp Gln His Ile Tyr Ile Pro Val Leu Pro Pro His Leu Leu Asp Tyr
                   245                 250                 255
   Cys Cys Ala Pro Met Pro Tyr Leu Ile Gly Ile His Ser Ser Leu Ile
               260                 265                 270
   Glu Arg Val Lys Asn Lys Ser Leu Glu Asp Val Val Met Leu Asn Val
           275                 280                 285
   Asp Thr Asn Thr Leu Glu Ser Pro Phe Ser Asp Leu Asn Asn Leu Pro
       290                 295                 300
   Ser Asp Val Val Ser Ala Leu Lys Asn Lys Leu Lys Lys Gln Ser Thr
   305                 310                 315                 320
   Ala Thr Gly Asp Gly Val Ala Arg Ala Phe Leu Arg Ala Gln Ala Ala
                   325                 330                 335
   Leu Phe Gly Ser Tyr Arg Asp Ala Leu Arg Tyr Lys Pro Gly Glu Pro
               340                 345                 350
   Ile Thr Phe Cys Glu Glu Ser Phe Val Lys His Arg Ser Ser Val Met
           355                 360                 365
   Lys Gln Phe Leu Glu Thr Ala Ile Asn Leu Gln Leu Phe Lys Gln Phe
       370                 375                 380
   Ile Asp Gly Arg Leu Ala Lys Leu Asn Ala Gly Arg Gly Phe Ser Asp
   385                 390                 395                 400
   Val Phe Glu Glu Glu Ile Thr Ser Gly Gly Phe Cys Gly Gly Lys Asp
                   405                 410                 415
   Lys Leu Gln Tyr Asp Tyr Pro Phe Ser Gln
   420 425
<210> 49
   <211> 426
   <212> PRT
   <213> Homo sapiens
<400> 49
   Ile Glu Thr Lys Thr Arg Ala Asn Pro Asp Arg Thr Phe Asp Leu Val
   1 5 10 15
   Leu Lys Val Lys Cys His Ala Ser Glu Asn Glu Asp Pro Val Val Leu
   20 25 30
   Trp Lys Phe Pro Glu Asp Phe Gly Asp Gln Glu Ile Leu Gln Ser Val
   35 40 45
   Pro Lys Phe Cys Phe Pro Phe Asp Val Glu Arg Val Ser Gln Asn Gln
   50 55 60
   Val Gly Gln His Phe Thr Phe Val Leu Thr Asp Ile Glu Ser Lys Gln
   65 70 75 80
   Arg Phe Gly Phe Cys Arg Leu Thr Ser Gly Gly Thr Ile Cys Leu Cys
   85 90 95
   Ile Leu Ser Tyr Leu Pro Trp Phe Glu Val Tyr Tyr Lys Leu Leu Asn
   100 105 110
   Thr Leu Ala Asp Tyr Leu Ala Lys Glu Leu Glu Asn Asp Leu Asn Glu
   115 120 125
   Thr Leu Arg Ser Leu Tyr Asn His Pro Val Pro Lys Ala Asn Thr Pro
   130 135 140
   Val Asn Leu Ser Val Asn Gln Glu Ile Phe Ile Ala Cys Glu Gln Val
   145 150 155 160
   Leu Lys Asp Gln Pro Ala Leu Val Pro His Ser Tyr Phe Ile Ala Pro
   165 170 175
   Asp Val Thr Gly Leu Pro Thr Ile Pro Glu Ser Arg Asn Leu Thr Glu
   180 185 190
   Tyr Phe Val Ala Val Asp Val Asn Asn Met Leu Gln Leu Tyr Ala Ser
           195                 200                 205
   Met Leu His Glu Arg Arg Ile Val Ile Ile Ser Ser Lys Leu Ser Thr
       210                 215                 220
   Leu Thr Ala Cys Ile His Gly Ser Ala Ala Leu Leu Tyr Pro Met Tyr
   225                 230                 235                 240
   Trp Gln His Ile Tyr Ile Pro Val Leu Pro Pro His Leu Leu Asp Tyr
                   245                 250                 255
   Cys Cys Ala Pro Met Pro Tyr Leu Ile Gly Ile His Ser Ser Leu Ile
               260                 265                 270
   Glu Arg Val Lys Asn Lys Ser Leu Glu Asp Val Val Met Leu Asn Val
           275                 280                 285
   Asp Thr Asn Thr Leu Glu Ser Pro Phe Ser Asp Leu Asn Asn Leu Pro
       290                 295                 300
   Ser Asp Val Val Ser Ala Leu Lys Asn Lys Leu Lys Lys Gln Ser Thr
   305                 310                 315                 320
   Ala Thr Gly Asp Gly Val Ala Arg Ala Phe Leu Arg Ala Gln Ala Ala
                   325                 330                 335
   Leu Phe Gly Ser Tyr Arg Asp Ala Leu Arg Tyr Lys Pro Gly Glu Pro
               340                 345                 350
   Ile Thr Phe Cys Glu Glu Ser Phe Val Lys His Arg Ser Ser Val Met
           355                 360                 365
   Lys Gln Phe Leu Glu Thr Ala Ile Asn Leu Gln Leu Phe Lys Gln Phe
       370                 375                 380
   Ile Asp Gly Arg Leu Ala Lys Leu Asn Ala Gly Arg Gly Phe Ser Asp
   385                 390                 395                 400
   Val Phe Glu Glu Glu Ile Thr Ser Gly Gly Phe Cys Gly Gly Lys Asp
                   405                 410                 415
   Lys Leu Gln Tyr Asp Tyr Pro Phe Ser Gln
               420                 425
<210> 50
   <211> 396
   <212> PRT
   <213> Homo sapiens
<400> 50
   Met Ala Ala Ala Pro Arg Glu Glu Lys Arg Trp Pro Gln Pro Val Phe
   1               5                   10                  15
   Ser Asn Pro Val Val Leu Trp Lys Phe Pro Glu Asp Phe Gly Asp Gln
               20                  25                  30
   Glu Ile Leu Gln Ser Val Pro Lys Phe Cys Phe Pro Phe Asp Val Glu
           35                  40                  45
   Arg Val Ser Gln Asn Gln Val Gly Gln His Phe Thr Phe Val Leu Thr
       50                  55                  60
   Asp Ile Glu Ser Lys Gln Arg Phe Gly Phe Cys Arg Leu Thr Ser Gly
   65                  70                  75                  80
   Gly Thr Ile Cys Leu Cys Ile Leu Ser Tyr Leu Pro Trp Phe Glu Val
                   85                  90                  95
   Tyr Tyr Lys Leu Leu Asn Thr Leu Ala Asp Tyr Leu Ala Lys Glu Leu
               100                 105                 110
   Glu Asn Asp Leu Asn Glu Thr Leu Arg Ser Leu Tyr Asn His Pro Val
           115                 120                 125
   Pro Lys Ala Asn Thr Pro Val Asn Leu Ser Val His Ser Tyr Phe Ile
       130                 135                 140
   Ala Pro Asp Val Thr Gly Leu Pro Thr Ile Pro Glu Ser Arg Asn Leu
   145                 150                 155                 160
   Thr Glu Tyr Phe Val Ala Val Asp Val Asn Asn Met Leu Gln Leu Tyr
                   165                 170                 175
   Ala Ser Met Leu His Glu Arg Arg Ile Val Ile Ile Ser Ser Lys Leu
               180                 185                 190
   Ser Thr Leu Thr Ala Cys Ile His Gly Ser Ala Ala Leu Leu Tyr Pro
           195                 200                 205
   Met Tyr Trp Gln His Ile Tyr Ile Pro Val Leu Pro Pro His Leu Leu
       210                 215                 220
   Asp Tyr Cys Cys Ala Pro Met Pro Tyr Leu Ile Gly Ile His Ser Ser
   225                 230                 235                 240
   Leu Ile Glu Arg Val Lys Asn Lys Ser Leu Glu Asp Val Val Met Leu
   245 250 255
   Asn Val Asp Thr Asn Thr Leu Glu Ser Pro Phe Ser Asp Leu Asn Asn
   260 265 270
   Leu Pro Ser Asp Val Val Ser Ala Leu Lys Asn Lys Leu Lys Lys Gln
   275 280 285
   Ser Thr Ala Thr Gly Asp Gly Val Ala Arg Ala Phe Leu Arg Ala Gln
   290 295 300
   Ala Ala Leu Phe Gly Ser Tyr Arg Asp Ala Leu Arg Tyr Lys Pro Gly
   305 310 315 320
   Glu Pro Ile Thr Phe Cys Glu Glu Ser Phe Val Lys His Arg Ser Ser
   325 330 335
   Val Met Lys Gln Phe Leu Glu Thr Ala Ile Asn Leu Gln Leu Phe Lys
   340 345 350
   Gln Phe Ile Asp Gly Arg Leu Ala Lys Leu Asn Ala Gly Arg Gly Phe
   355 360 365
   Ser Asp Val Phe Glu Glu Glu Ile Thr Ser Gly Gly Phe Cys Gly Gly
   370 375 380
   Lys Asp Lys Leu Gln Tyr Asp Tyr Pro Phe Ser Gln
   385 390 395
<210> 51
   <211> 2117
   <212> DNA
   <213> Homo sapiens
<400> 51
   gccgggggcg cagccgacat gggcccgccg ccacggctgc tgtgagcagc ctctttccct 60
   gtgtggccgc cggcgtgggc ggggacggcg cgaccctcgc gcggccgggc tgcgggcttc 120
   caggccagcg cgcgggggcc ggacggacag ccccacaccg acatgtaacc atggactgca 180
   ggaccaaggc aaatccagac agaacctttg acttggtgtt gaaagtgaaa tgtcatgcct 240
   ctgaaaatga agatcctgtg gtattgtgga aattcccaga ggactttgga gaccaggaaa 300
   tactacagag tgtgccaaag ttctgttttc cctttgacgt tgaaagggtg tctcagaatc 360
   aagttggaca gcactttacc tttgtactga cagacattga aagtaaacag agatttggat 420
   tctgcagact gacgtcagga ggcacaattt gtttatgcat ccttagttac cttccctggt    480
   ttgaagtgta ttacaagctt ctaaatactc ttgcagatta cttggctaag gaactggaaa    540
   atgatttgaa tgaaactctc agatcactgt ataaccaccc agtaccaaag gcaaatactc    600
   ctgtaaattt gagtgtgcat tcctacttca ttgcccctga tgtaactgga ctcccaacaa    660
   tacccgagag tagaaatctt acagaatatt ttgttgccgt ggatgtgaac aacatgctgc    720
   agctgtatgc cagtatgctg catgaaaggc gcatcgtgat tatctcgagc aaattaagca    780
   ctttaactgc ctgtatccat ggatcagctg ctcttctata cccaatgtat tggcaacaca    840
   tatacatccc agtgcttcct ccacacctgc tggactactg ctgtgcccca atgccatacc    900
   tgattggaat acactccagc ctcatagaga gagtgaaaaa caaatcattg gaagatgttg    960
   ttatgttaaa tgttgataca aacacattag aatcaccatt tagtgacttg aacaacctac   1020
   caagtgatgt ggtctcggcc ttgaaaaata aactgaagaa gcagtctaca gctacgggtg   1080
   atggagtagc tagggccttt cttagagcac aggctgcttt gtttggatcc tacagagatg   1140
   cactgagata caaacctggt gagcccatca ctttctgtga ggagagtttt gtaaagcacc   1200
   gctcaagcgt gatgaaacag ttcctggaaa ctgccattaa cctccagctt tttaagcagt   1260
   ttatcgatgg tcgactggca aaactaaatg caggaagggg tttctctgat gtatttgaag   1320
   aagagatcac ttcaggtggc ttttgtggag gtaaagacaa gttacaatat gattatccat   1380
   tttctcaata acaattttct tggtctttgc acttgtgtct gataaaacct atttcataaa   1440
   caactaatga tttcctccta aatatgtaat gtcttaaata catttttcat cttataaaag   1500
   ctatggaatt agcttatttt gcctgatacc tgttactcaa ggcattaagt tggcctcctg   1560
   aattggcagc tgttggcctc gataatctct taatattgct ggaaattagt aatacagaaa   1620
   tccaatcaac tcatatcttc ctgtctttcc ttctgaatag tagtattctc tgctagaaaa   1680
   ctactagtga tggttattac tgagtatgaa tttaagaact gaggttatga ttggtaatac   1740
   aatccaaaaa gaagggtctg aacaccaaaa ttctttatac atatttaagt aactgtatta   1800
   ttattataca gatgtcttta cctttttgac tttatagatc actgcagcat taagaaagtt   1860
   tccagtttac cattccataa gtacaattaa tccttctagt gtaaatgttc aaatactgtt   1920
   ataattatct aggcaattaa taatttacaa actgatattt ttgcacgatt gtagtggtgt   1980
   atagtcttga cttgcagagc attttgcttg agtccttgaa atgtcgtgtt cattcattat   2040
   ttgctgagtg cttacaatgt attaggcact gttctaaata ttaagtgtac taaataaaca   2100
   aaaatccttg tattctg                                                  2117
<210> 52
   <211> 2177
   <212> DNA
   <213> Homo sapiens
<400> 52
   gccgggggcg cagccgacat gggcccgccg ccacggctgc tgtgagcagc ctctttccct      60
   gtgtggccgc cggcgtgggc ggggacggcg cgaccctcgc gcggccgggc tgcgggcttc     120
   caggccagcg cgcgggggcc ggacggacag ccccacaccg acatgtaacc atggactgca     180
   ggaccaaggc aaatccagac agaacctttg acttggtgtt gaaagtgaaa tgtcatgcct     240
   ctgaaaatga agatcctgtg gtattgtgga aattcccaga ggactttgga gaccaggaaa     300
   tactacagag tgtgccaaag ttctgttttc cctttgacgt tgaaagggtg tctcagaatc     360
   aagttggaca gcactttacc tttgtactga cagacattga aagtaaacag agatttggat     420
   tctgcagact gacgtcagga ggcacaattt gtttatgcat ccttagttac cttccctggt     480
   ttgaagtgta ttacaagctt ctaaatactc ttgcagatta cttggctaag gaactggaaa     540
   atgatttgaa tgaaactctc agatcactgt ataaccaccc agtaccaaag gcaaatactc     600
   ctgtaaattt gagtgtgaac caagagatat ttattgcctg tgagcaagtt ctgaaagatc     660
   agcctgctct agtaccgcat tcctacttca ttgcccctga tgtaactgga ctcccaacaa     720
   tacccgagag tagaaatctt acagaatatt ttgttgccgt ggatgtgaac aacatgctgc     780
   agctgtatgc cagtatgctg catgaaaggc gcatcgtgat tatctcgagc aaattaagca     840
   ctttaactgc ctgtatccat ggatcagctg ctcttctata cccaatgtat tggcaacaca     900
   tatacatccc agtgcttcct ccacacctgc tggactactg ctgtgcccca atgccatacc     960
   tgattggaat acactccagc ctcatagaga gagtgaaaaa caaatcattg gaagatgttg    1020
   ttatgttaaa tgttgataca aacacattag aatcaccatt tagtgacttg aacaacctac    1080
   caagtgatgt ggtctcggcc ttgaaaaata aactgaagaa gcagtctaca gctacgggtg    1140
   atggagtagc tagggccttt cttagagcac aggctgcttt gtttggatcc tacagagatg    1200
   cactgagata caaacctggt gagcccatca ctttctgtga ggagagtttt gtaaagcacc    1260
   gctcaagcgt gatgaaacag ttcctggaaa ctgccattaa cctccagctt tttaagcagt    1320
   ttatcgatgg tcgactggca aaactaaatg caggaagggg tttctctgat gtatttgaag    1380
   aagagatcac ttcaggtggc ttttgtggag gtaaagacaa gttacaatat gattatccat    1440
   tttctcaata acaattttct tggtctttgc acttgtgtct gataaaacct atttcataaa    1500
   caactaatga tttcctccta aatatgtaat gtcttaaata catttttcat cttataaaag    1560
   ctatggaatt agcttatttt gcctgatacc tgttactcaa ggcattaagt tggcctcctg    1620
   aattggcagc tgttggcctc gataatctct taatattgct ggaaattagt aatacagaaa    1680
   tccaatcaac tcatatcttc ctgtctttcc ttctgaatag tagtattctc tgctagaaaa    1740
   ctactagtga tggttattac tgagtatgaa tttaagaact gaggttatga ttggtaatac    1800
   aatccaaaaa gaagggtctg aacaccaaaa ttctttatac atatttaagt aactgtatta   1860
   ttattataca gatgtcttta cctttttgac tttatagatc actgcagcat taagaaagtt   1920
   tccagtttac cattccataa gtacaattaa tccttctagt gtaaatgttc aaatactgtt   1980
   ataattatct aggcaattaa taatttacaa actgatattt ttgcacgatt gtagtggtgt   2040
   atagtcttga cttgcagagc attttgcttg agtccttgaa atgtcgtgtt cattcattat   2100
   ttgctgagtg cttacaatgt attaggcact gttctaaata ttaagtgtac taaataaaca   2160
   aaaatccttg tattctg                                                  2177
<210> 53
   <211> 2007
   <212> DNA
   <213> Homo sapiens
<400> 53
   attgagacaa aaacaagggc aaatccagac agaacctttg acttggtgtt gaaagtgaaa     60
   tgtcatgcct ctgaaaatga agatcctgtg gtattgtgga aattcccaga ggactttgga    120
   gaccaggaaa tactacagag tgtgccaaag ttctgttttc cctttgacgt tgaaagggtg    180
   tctcagaatc aagttggaca gcactttacc tttgtactga cagacattga aagtaaacag    240
   agatttggat tctgcagact gacgtcagga ggcacaattt gtttatgcat ccttagttac    300
   cttccctggt ttgaagtgta ttacaagctt ctaaatactc ttgcagatta cttggctaag    360
   gaactggaaa atgatttgaa tgaaactctc agatcactgt ataaccaccc agtaccaaag    420
   gcaaatactc ctgtaaattt gagtgtgaac caagagatat ttattgcctg tgagcaagtt    480
   ctgaaagatc agcctgctct agtaccgcat tcctacttca ttgcccctga tgtaactgga    540
   ctcccaacaa tacccgagag tagaaatctt acagaatatt ttgttgccgt ggatgtgaac    600
   aacatgctgc agctgtatgc cagtatgctg catgaaaggc gcatcgtgat tatctcgagc    660
   aaattaagca ctttaactgc ctgtatccat ggatcagctg ctcttctata cccaatgtat    720
   tggcaacaca tatacatccc agtgcttcct ccacacctgc tggactactg ctgtgcccca    780
   atgccatacc tgattggaat acactccagc ctcatagaga gagtgaaaaa caaatcattg    840
   gaagatgttg ttatgttaaa tgttgataca aacacattag aatcaccatt tagtgacttg    900
   aacaacctac caagtgatgt ggtctcggcc ttgaaaaata aactgaagaa gcagtctaca    960
   gctacgggtg atggagtagc tagggccttt cttagagcac aggctgcttt gtttggatcc   1020
   tacagagatg cactgagata caaacctggt gagcccatca ctttctgtga ggagagtttt   1080
   gtaaagcacc gctcaagcgt gatgaaacag ttcctggaaa ctgccattaa cctccagctt   1140
   tttaagcagt ttatcgatgg tcgactggca aaactaaatg caggaagggg tttctctgat   1200
   gtatttgaag aagagatcac ttcaggtggc ttttgtggag gtaaagacaa gttacaatat 1260
   gattatccat tttctcaata acaattttct tggtctttgc acttgtgtct gataaaacct 1320
   atttcataaa caactaatga tttcctccta aatatgtaat gtcttaaata catttttcat 1380
   cttataaaag ctatggaatt agcttatttt gcctgatacc tgttactcaa ggcattaagt 1440
   tggcctcctg aattggcagc tgttggcctc gataatctct taatattgct ggaaattagt 1500
   aatacagaaa tccaatcaac tcatatcttc ctgtctttcc ttctgaatag tagtattctc 1560
   tgctagaaaa ctactagtga tggttattac tgagtatgaa tttaagaact gaggttatga 1620
   ttggtaatac aatccaaaaa gaagggtctg aacaccaaaa ttctttatac atatttaagt 1680
   aactgtatta ttattataca gatgtcttta cctttttgac tttatagatc actgcagcat 1740
   taagaaagtt tccagtttac cattccataa gtacaattaa tccttctagt gtaaatgttc 1800
   aaatactgtt ataattatct aggcaattaa taatttacaa actgatattt ttgcacgatt 1860
   gtagtggtgt atagtcttga cttgcagagc attttgcttg agtccttgaa atgtcgtgtt 1920
   cattcattat ttgctgagtg cttacaatgt attaggcact gttctaaata ttaagtgtac 1980
   taaataaaca aaaatccttg tattctg 2007
<210> 54
   <211> 2197
   <212> DNA
   <213> Homo sapiens
<400> 54
   gcgggggccg gacggacagc cccacaccga catgtaacca tggactgcag gaccaaggca     60
   aatccagaca gaacctttga cttggtgttg aaagtgaaat gtcatgcctc tgaaaatgaa    120
   gaggacagtc cagcttatct gccgaggatt ccccctggaa aagtacgccg attcgcattt    180
   tgcattaaga aactggaaaa ctttcctgtc ggtcctggcg tagcgcctcc cgtgtccggg    240
   gtagaccttg taccggctga aaccgcatag ctcgaccttc atggcggcag ctccacggga    300
   ggagaaaaga tggccccaac ctgtattttc gaatcctgtg gtattgtgga aattcccaga    360
   ggactttgga gaccaggaaa tactacagag tgtgccaaag ttctgttttc cctttgacgt    420
   tgaaagggtg tctcagaatc aagttggaca gcactttacc tttgtactga cagacattga    480
   aagtaaacag agatttggat tctgcagact gacgtcagga ggcacaattt gtttatgcat    540
   ccttagttac cttccctggt ttgaagtgta ttacaagctt ctaaatactc ttgcagatta    600
   cttggctaag gaactggaaa atgatttgaa tgaaactctc agatcactgt ataaccaccc    660
   agtaccaaag gcaaatactc ctgtaaattt gagtgtgcat tcctacttca ttgcccctga    720
   tgtaactgga ctcccaacaa tacccgagag tagaaatctt acagaatatt ttgttgccgt    780
   ggatgtgaac aacatgctgc agctgtatgc cagtatgctg catgaaaggc gcatcgtgat    840
   tatctcgagc aaattaagca ctttaactgc ctgtatccat ggatcagctg ctcttctata 900
   cccaatgtat tggcaacaca tatacatccc agtgcttcct ccacacctgc tggactactg 960
   ctgtgcccca atgccatacc tgattggaat acactccagc ctcatagaga gagtgaaaaa 1020
   caaatcattg gaagatgttg ttatgttaaa tgttgataca aacacattag aatcaccatt 1080
   tagtgacttg aacaacctac caagtgatgt ggtctcggcc ttgaaaaata aactgaagaa 1140
   gcagtctaca gctacgggtg atggagtagc tagggccttt cttagagcac aggctgcttt 1200
   gtttggatcc tacagagatg cactgagata caaacctggt gagcccatca ctttctgtga 1260
   ggagagtttt gtaaagcacc gctcaagcgt gatgaaacag ttcctggaaa ctgccattaa 1320
   cctccagctt tttaagcagt ttatcgatgg tcgactggca aaactaaatg caggaagggg 1380
   tttctctgat gtatttgaag aagagatcac ttcaggtggc ttttgtggag gtaaagacaa 1440
   gttacaatat gattatccat tttctcaata acaattttct tggtctttgc acttgtgtct 1500
   gataaaacct atttcataaa caactaatga tttcctccta aatatgtaat gtcttaaata 1560
   catttttcat cttataaaag ctatggaatt agcttatttt gcctgatacc tgttactcaa 1620
   ggcattaagt tggcctcctg aattggcagc tgttggcctc gataatctct taatattgct 1680
   ggaaattagt aatacagaaa tccaatcaac tcatatcttc ctgtctttcc ttctgaatag 1740
   tagtattctc tgctagaaaa ctactagtga tggttattac tgagtatgaa tttaagaact 1800
   gaggttatga ttggtaatac aatccaaaaa gaagggtctg aacaccaaaa ttctttatac 1860
   atatttaagt aactgtatta ttattataca gatgtcttta cctttttgac tttatagatc 1920
   actgcagcat taagaaagtt tccagtttac cattccataa gtacaattaa tccttctagt 1980
   gtaaatgttc aaatactgtt ataattatct aggcaattaa taatttacaa actgatattt 2040
   ttgcacgatt gtagtggtgt atagtcttga cttgcagagc attttgcttg agtccttgaa 2100
   atgtcgtgtt cattcattat ttgctgagtg cttacaatgt attaggcact gttctaaata 2160
   ttaagtgtac taaataaaca aaaatccttg tattctg 2197
<210> 55
   <211> 211
   <212> PRT
   <213> Homo sapiens
<400> 55
   Met Gly Pro Gln His Leu Arg Leu Val Gln Leu Phe Cys Leu Leu Gly
   1 5 10 15
   Ala Ile Ser Thr Leu Pro Arg Met Ser Cys Gly Ala Gly Cys Tyr Lys
   20 25 30
   Thr Gln Lys Gly Thr Ala Arg Gly Val Val Gly Phe Lys Gly Cys Ser
   35 40 45
   Ser Ser Ser Ser Tyr Pro Ala Gln Ile Ser Tyr Leu Val Ser Pro Pro
   50 55 60
   Gly Val Ser Ile Ala Ser Tyr Ser Arg Val Cys Arg Ser Tyr Leu Cys
   65 70 75 80
   Asn Asn Leu Thr Asn Leu Glu Pro Phe Val Lys Leu Lys Ala Ser Thr
   85 90 95
   Pro Lys Ser Ile Thr Ser Ala Ser Cys Ser Cys Pro Thr Cys Val Gly
   100 105 110
   Glu His Met Lys Asp Cys Leu Pro Asn Phe Val Thr Thr Asn Ser Cys
   115 120 125
   Pro Leu Ala Ala Ser Thr Cys Tyr Ser Ser Thr Leu Lys Phe Gln Ala
   130 135 140
   Gly Phe Leu Asn Thr Thr Phe Leu Leu Met Gly Cys Ala Arg Glu His
   145 150 155 160
   Asn Gln Leu Leu Ala Asp Phe His His Ile Gly Ser Ile Lys Val Thr
   165 170 175
   Glu Val Leu Asn Ile Leu Glu Lys Ser Gln Ile Val Gly Ala Ala Ser
   180 185 190
   Ser Arg Gln Asp Pro Ala Trp Gly Val Val Leu Gly Leu Leu Phe Ala
   195 200 205
   Phe Arg Asp
   210
<210> 56
   <211> 246
   <212> PRT
   <213> Homo sapiens
<400> 56
   Met Gly Pro Gln His Leu Arg Leu Val Gln Leu Phe Cys Leu Leu Gly
   1 5 10 15
   Ala Ile Ser Thr Leu Pro Arg Ala Gly Ala Leu Leu Cys Tyr Glu Ala
   20 25 30
   Thr Ala Ser Arg Phe Arg Ala Val Ala Phe His Asn Trp Lys Trp Leu
           35                  40                  45
   Leu Met Arg Asn Met Val Cys Lys Leu Gln Glu Gly Cys Glu Glu Thr
       50                  55                  60
   Leu Val Phe Ile Glu Thr Gly Thr Ala Arg Gly Val Val Gly Phe Lys
   65                  70                  75                  80
   Gly Cys Ser Ser Ser Ser Ser Tyr Pro Ala Gln Ile Ser Tyr Leu Val
                   85                  90                  95
   Ser Pro Pro Gly Val Ser Ile Ala Ser Tyr Ser Arg Val Cys Arg Ser
               100                 105                 110
   Tyr Leu Cys Asn Asn Leu Thr Asn Leu Glu Pro Phe Val Lys Leu Lys
           115                 120                 125
   Ala Ser Thr Pro Lys Ser Ile Thr Ser Ala Ser Cys Ser Cys Pro Thr
       130                 135                 140
   Cys Val Gly Glu His Met Lys Asp Cys Leu Pro Asn Phe Val Thr Thr
   145                 150                 155                 160
   Asn Ser Cys Pro Leu Ala Ala Ser Thr Cys Tyr Ser Ser Thr Leu Lys
                   165                 170                 175
   Phe Gln Ala Gly Phe Leu Asn Thr Thr Phe Leu Leu Met Gly Cys Ala
               180                 185                 190
   Arg Glu His Asn Gln Leu Leu Ala Asp Phe His His Ile Gly Ser Ile
           195                 200                 205
   Lys Val Thr Glu Val Leu Asn Ile Leu Glu Lys Ser Gln Ile Val Gly
       210                 215                 220
   Ala Ala Ser Ser Arg Gln Asp Pro Ala Trp Gly Val Val Leu Gly Leu
   225                 230                 235                 240
   Leu Phe Ala Phe Arg Asp
                   245
<210> 57
   <211> 705
   <212> DNA
   <213> Homo sapiens
<400> 57
   atgggacccc agcatttgag acttgtgcag ctgttctgcc ttctaggggc catctccact 60
   ctgcctcgta tgtcctgtgg ggctggatgc tataagaccc agaaagggac tgcaagggga 120
   gttgtgggct ttaaaggctg cagctcgtct tcgtcttacc ctgcgcaaat ctcctacctt 180
   gtttccccac ccggagtgtc cattgcctcc tacagtcgcg tctgccggtc ttatctctgc 240
   aacaacctca ccaatttgga gccttttgtg aaactcaagg ccagcactcc taagtctatc 300
   acatctgcgt cctgtagctg cccgacctgt gtgggcgagc acatgaagga ttgcctccca 360
   aattttgtca ccactaattc ttgccccttg gctgcttcta cgtgttacag ttccacctta 420
   aaatttcagg cagggtttct caataccacc ttcctcctca tggggtgtgc tcgtgaacat 480
   aaccagcttt tagcagattt tcatcatatt gggagcatca aagtgactga ggtcctcaac 540
   atcttagaga agtctcagat tgttggtgca gcatcctcca ggcaagatcc tgcttggggt 600
   gtcgtcttag gcctcctgtt tgccttcagg gactgaccat ctagctgcac ccgacaagca 660
   cccagactct ttcacataac aaataaaata gcagagttcc ctttc 705
<210> 58
   <211> 2022
   <212> DNA
   <213> Homo sapiens
<400> 58
   ggtctgtgca cgggttgcct cgtttctggc cttcccttgc cttgtacctc gacttcgcgc 60
   gtggcttgtt tttgctgagt ggaggcctgc gttttctgtt atgtctcttc cggtgcccca 120
   caactcctga gctacccttc ttttatgcgt tctctcccag cgctgtgatt accgggttgc 180
   ttaccgggcc ctccttcctc agattgcacc cctttccttg tgtctcttct tgtctttgtg 240
   tcggttgtga ttttcctaat ctctgatttt ccttttctct cggacgctct ccctcttcgg 300
   acccattttc tcccgtgctt catgccctga tagcctggcc ccttcccggc ttccttcgct 360
   accggggacg cctctagttt ttctgaattt ctggctggct ccaccctccg cgttcatctt 420
   cctcaagagt tcgcccctct gggggctcct ctgtgtaatc gtcgccttct ctgggtattt 480
   ctgtgaactc cgtctcacac catcccgcca tcttctctgc cttggcccct tttctctgta 540
   cagccagctc tgtgtccttt tcttctcccc ctctaaaatc gactcctctt ctccctgaga 600
   gccccacctt tgtgccccac tcctcatttt cctacgcctc cctctctctg ctggtcctct 660
   ctctccctgc aaggttccat tccatcaatt tgtttgtctt ttgtaggggt ggcatcccct 720
   ctgactactg ctccatcctt tttttttttt tttttttttt tttgcttgag gatttcactt 780
   caatcttttc tggttgcgtc tccacttgta ctcagcttgt taggtccagg tccagttgtt 840
   ctgcatctga ggctggcgtg tgctgtcttc tctgattggc ctaatctccc tcacccccgt 900
   gagatctgtt gtcagccttc gtttctcttt cctgtgtccc agcttttctg cgggtcttgg 960
   cacctttctt ggccacagat ttctgggtta cagagcatgt gtgtctgagg cattgcaggc 1020
   agaaaagggt ggccgacgtg acctctagct ggactgctgg gcaggggagc tgtcctagat 1080
   aaaattggaa agaaacagtg acccagagac aggtggacaa agaattcggg gactgatggg 1140
   aactgagctt gggatccaga ctgaaactga ttccagactg acctctagca cccaggaccc 1200
   agacacaggg ccatgggacc ccagcatttg agacttgtgc agctgttctg ccttctaggg 1260
   gccatctcca ctctgcctcg ggctggagct cttttgtgct atgaagcaac agcctcaaga 1320
   ttcagagctg ttgctttcca taactggaag tggcttctga tgaggaacat ggtgtgtaag 1380
   ctgcaagagg gctgcgagga gacgctagtg ttcattgaga cagggactgc aaggggagtt 1440
   gtgggcttta aaggctgcag ctcgtcttcg tcttaccctg cgcaaatctc ctaccttgtt 1500
   tccccacccg gagtgtccat tgcctcctac agtcgcgtct gccggtctta tctctgcaac 1560
   aacctcacca atttggagcc ttttgtgaaa ctcaaggcca gcactcctaa gtctatcaca 1620
   tctgcgtcct gtagctgccc gacctgtgtg ggcgagcaca tgaaggattg cctcccaaat 1680
   tttgtcacca ctaattcttg ccccttggct gcttctacgt gttacagttc caccttaaaa 1740
   tttcaggcag ggtttctcaa taccaccttc ctcctcatgg ggtgtgctcg tgaacataac 1800
   cagcttttag cagattttca tcatattggg agcatcaaag tgactgaggt cctcaacatc 1860
   ttagagaagt ctcagattgt tggtgcagca tcctccaggc aagatcctgc ttggggtgtc 1920
   gtcttaggcc tcctgtttgc cttcagggac tgaccatcta gctgcacccg acaagcaccc 1980
   agactctttc acataacaaa taaaatagca gagttccctt tc 2022
<210> 59
   <211> 542
   <212> PRT
   <213> Homo sapiens
<400> 59
   Met Ala Gln Glu Ile Asp Leu Ser Ala Leu Lys Glu Leu Glu Arg Glu
   1 5 10 15
   Ala Ile Leu Gln Val Leu Tyr Arg Asp Gln Ala Val Gln Asn Thr Glu
   20 25 30
   Glu Glu Arg Thr Arg Lys Leu Lys Thr His Leu Gln His Leu Arg Trp
   35 40 45
   Lys Gly Ala Lys Asn Thr Asp Trp Glu His Lys Glu Lys Cys Cys Ala
   50 55 60
   Arg Cys Gln Gln Val Leu Gly Phe Leu Leu His Arg Gly Ala Val Cys
   65 70 75 80
   Arg Gly Cys Ser His Arg Val Cys Ala Gln Cys Arg Val Phe Leu Arg
   85 90 95
   Gly Thr His Ala Trp Lys Cys Thr Val Cys Phe Glu Asp Arg Asn Val
   100 105 110
   Lys Ile Lys Thr Gly Glu Trp Phe Tyr Glu Glu Arg Ala Lys Lys Phe
   115 120 125
   Pro Thr Gly Gly Lys His Glu Thr Val Gly Gly Gln Leu Leu Gln Ser
   130 135 140
   Tyr Gln Lys Leu Ser Lys Ile Ser Val Val Pro Pro Thr Pro Pro Pro
   145 150 155 160
   Val Ser Glu Ser Gln Cys Ser Arg Ser Pro Gly Arg Lys Val Ser Ala
   165 170 175
   Pro Asp Ile Leu Lys Pro Leu Asn Gln Glu Asp Pro Lys Cys Ser Thr
   180 185 190
   Asn Pro Ile Leu Lys Gln Gln Asn Leu Pro Ser Ser Pro Ala Pro Ser
   195 200 205
   Thr Ile Phe Ser Gly Gly Phe Arg His Gly Ser Leu Ile Ser Ile Asp
   210 215 220
   Ser Thr Cys Thr Glu Met Gly Asn Phe Asp Asn Ala Asn Val Thr Gly
   225 230 235 240
   Glu Ile Glu Phe Ala Ile His Tyr Cys Phe Lys Thr His Ser Leu Glu
   245 250 255
   Ile Cys Ile Lys Ala Cys Lys Asn Leu Ala Tyr Gly Glu Glu Lys Lys
   260 265 270
   Lys Lys Cys Asn Pro Tyr Val Lys Thr Tyr Leu Leu Pro Asp Arg Ser
   275 280 285
   Ser Gln Gly Lys Arg Lys Thr Gly Val Gln Arg Asn Thr Val Asp Pro
   290 295 300
   Thr Phe Gln Glu Thr Leu Lys Tyr Gln Val Ala Pro Ala Gln Leu Val
   305                 310                 315                 320
   Thr Arg Gln Leu Gln Val Ser Val Trp His Leu Gly Thr Leu Ala Arg
                   325                 330                 335
   Arg Val Phe Leu Gly Glu Val Ile Ile Pro Leu Ala Thr Trp Asp Phe
               340                 345                 350
   Glu Asp Ser Thr Thr Gln Ser Phe Arg Trp His Pro Leu Arg Ala Lys
           355                 360                 365
   Ala Glu Lys Tyr Glu Asp Ser Val Pro Gln Ser Asn Gly Glu Leu Thr
       370                 375                 380
   Val Arg Ala Lys Leu Val Leu Pro Ser Arg Pro Arg Lys Leu Gln Glu
   385                 390                 395                 400
   Ala Gln Glu Gly Thr Asp Gln Pro Ser Leu His Gly Gln Leu Cys Leu
                   405                 410                 415
   Val Val Leu Gly Ala Lys Asn Leu Pro Val Arg Pro Asp Gly Thr Leu
               420                 425                 430
   Asn Ser Phe Val Lys Gly Cys Leu Thr Leu Pro Asp Gln Gln Lys Leu
           435                 440                 445
   Arg Leu Lys Ser Pro Val Leu Arg Lys Gln Ala Cys Pro Gln Trp Lys
       450                 455                 460
   His Ser Phe Val Phe Ser Gly Val Thr Pro Ala Gln Leu Arg Gln Ser
   465                 470                 475                 480
   Ser Leu Glu Leu Thr Val Trp Asp Gln Ala Leu Phe Gly Met Asn Asp
                   485                 490                 495
   Arg Leu Leu Gly Gly Thr Arg Leu Gly Ser Lys Gly Asp Thr Ala Val
               500                 505                 510
   Gly Gly Asp Ala Cys Ser Leu Ser Lys Leu Gln Trp Gln Lys Val Leu
           515                 520                 525
   Ser Ser Pro Asn Leu Trp Thr Asp Met Thr Leu Val Leu His
       530                 535                 540
<210> 60
   <211> 610
   <212> PRT
   <213> Homo sapiens
<400> 60
   Met Ala Gln Glu Ile Asp Leu Ser Ala Leu Lys Glu Leu Glu Arg Glu
   1               5                   10                  15
   Ala Ile Leu Gln Val Leu Tyr Arg Asp Gln Ala Val Gln Asn Thr Glu
               20                  25                  30
   Glu Glu Arg Thr Arg Lys Leu Lys Thr His Leu Gln His Leu Arg Trp
           35                  40                  45
   Lys Gly Ala Lys Asn Thr Asp Trp Glu His Lys Glu Lys Cys Cys Ala
       50                  55                  60
   Arg Cys Gln Gln Val Leu Gly Phe Leu Leu His Arg Gly Ala Val Cys
   65                  70                  75                  80
   Arg Gly Cys Ser His Arg Val Cys Ala Gln Cys Arg Val Phe Leu Arg
                   85                  90                  95
   Gly Thr His Ala Trp Lys Cys Thr Val Cys Phe Glu Asp Arg Asn Val
               100                 105                 110
   Lys Ile Lys Thr Gly Glu Trp Phe Tyr Glu Glu Arg Ala Lys Lys Phe
           115                 120                 125
   Pro Thr Gly Gly Lys His Glu Thr Val Gly Gly Gln Leu Leu Gln Ser
       130                 135                 140
   Tyr Gln Lys Leu Ser Lys Ile Ser Val Val Pro Pro Thr Pro Pro Pro
   145                 150                 155                 160
   Val Ser Glu Ser Gln Cys Ser Arg Ser Pro Gly Arg Leu Gln Glu Phe
                   165                 170                 175
   Gly Gln Phe Arg Gly Phe Asn Lys Ser Val Glu Asn Leu Phe Leu Ser
               180                 185                 190
   Leu Ala Thr His Val Lys Lys Leu Ser Lys Ser Gln Asn Asp Met Thr
           195                 200                 205
   Ser Glu Lys His Leu Leu Ala Thr Gly Pro Arg Gln Cys Val Gly Gln
       210                 215                 220
   Thr Glu Arg Arg Ser Gln Ser Asp Thr Ala Val Asn Val Thr Thr Arg
   225                 230                 235                 240
   Lys Val Ser Ala Pro Asp Ile Leu Lys Pro Leu Asn Gln Glu Asp Pro
   245 250 255
   Lys Cys Ser Thr Asn Pro Ile Leu Lys Gln Gln Asn Leu Pro Ser Ser
   260 265 270
   Pro Ala Pro Ser Thr Ile Phe Ser Gly Gly Phe Arg His Gly Ser Leu
   275 280 285
   Ile Ser Ile Asp Ser Thr Cys Thr Glu Met Gly Asn Phe Asp Asn Ala
   290 295 300
   Asn Val Thr Gly Glu Ile Glu Phe Ala Ile His Tyr Cys Phe Lys Thr
   305 310 315 320
   His Ser Leu Glu Ile Cys Ile Lys Ala Cys Lys Asn Leu Ala Tyr Gly
   325 330 335
   Glu Glu Lys Lys Lys Lys Cys Asn Pro Tyr Val Lys Thr Tyr Leu Leu
   340 345 350
   Pro Asp Arg Ser Ser Gln Gly Lys Arg Lys Thr Gly Val Gln Arg Asn
   355 360 365
   Thr Val Asp Pro Thr Phe Gln Glu Thr Leu Lys Tyr Gln Val Ala Pro
   370 375 380
   Ala Gln Leu Val Thr Arg Gln Leu Gln Val Ser Val Trp His Leu Gly
   385 390 395 400
   Thr Leu Ala Arg Arg Val Phe Leu Gly Glu Val Ile Ile Pro Leu Ala
   405 410 415
   Thr Trp Asp Phe Glu Asp Ser Thr Thr Gln Ser Phe Arg Trp His Pro
   420 425 430
   Leu Arg Ala Lys Ala Glu Lys Tyr Glu Asp Ser Val Pro Gln Ser Asn
   435 440 445
   Gly Glu Leu Thr Val Arg Ala Lys Leu Val Leu Pro Ser Arg Pro Arg
   450 455 460
   Lys Leu Gln Glu Ala Gln Glu Gly Thr Asp Gln Pro Ser Leu His Gly
   465 470 475 480
   Gln Leu Cys Leu Val Val Leu Gly Ala Lys Asn Leu Pro Val Arg Pro
                   485                 490                 495
   Asp Gly Thr Leu Asn Ser Phe Val Lys Gly Cys Leu Thr Leu Pro Asp
               500                 505                 510
   Gln Gln Lys Leu Arg Leu Lys Ser Pro Val Leu Arg Lys Gln Ala Cys
           515                 520                 525
   Pro Gln Trp Lys His Ser Phe Val Phe Ser Gly Val Thr Pro Ala Gln
       530                 535                 540
   Leu Arg Gln Ser Ser Leu Glu Leu Thr Val Trp Asp Gln Ala Leu Phe
   545                 550                 555                 560
   Gly Met Asn Asp Arg Leu Leu Gly Gly Thr Arg Leu Gly Ser Lys Gly
                   565                 570                 575
   Asp Thr Ala Val Gly Gly Asp Ala Cys Ser Leu Ser Lys Leu Gln Trp
               580                 585                 590
   Gln Lys Val Leu Ser Ser Pro Asn Leu Trp Thr Asp Met Thr Leu Val
           595                 600                 605
   Leu His
       610
<210> 61
   <211> 542
   <212> PRT
   <213> Homo sapiens
<400> 61
   Met Ala Gln Glu Ile Asp Leu Ser Ala Leu Lys Glu Leu Glu Arg Glu
   1               5                   10                  15
   Ala Ile Leu Gln Val Leu Tyr Arg Asp Gln Ala Val Gln Asn Thr Glu
               20                  25                  30
   Glu Glu Arg Thr Arg Lys Leu Lys Thr His Leu Gln His Leu Arg Trp
           35                  40                  45
   Lys Gly Ala Lys Asn Thr Asp Trp Glu His Lys Glu Lys Cys Cys Ala
       50                  55                  60
   Arg Cys Gln Gln Val Leu Gly Phe Leu Leu His Arg Gly Ala Val Cys
   65                  70                  75                  80
   Arg Gly Cys Ser His Arg Val Cys Ala Gln Cys Arg Val Phe Leu Arg
                   85                  90                  95
   Gly Thr His Ala Trp Lys Cys Thr Val Cys Phe Glu Asp Arg Asn Val
               100                 105                 110
   Lys Ile Lys Thr Gly Glu Trp Phe Tyr Glu Glu Arg Ala Lys Lys Phe
           115                 120                 125
   Pro Thr Gly Gly Lys His Glu Thr Val Gly Gly Gln Leu Leu Gln Ser
       130                 135                 140
   Tyr Gln Lys Leu Ser Lys Ile Ser Val Val Pro Pro Thr Pro Pro Pro
   145                 150                 155                 160
   Val Ser Glu Ser Gln Cys Ser Arg Ser Pro Gly Arg Lys Val Ser Ala
                   165                 170                 175
   Pro Asp Ile Leu Lys Pro Leu Asn Gln Glu Asp Pro Lys Cys Ser Thr
               180                 185                 190
   Asn Pro Ile Leu Lys Gln Gln Asn Leu Pro Ser Ser Pro Ala Pro Ser
           195                 200                 205
   Thr Ile Phe Ser Gly Gly Phe Arg His Gly Ser Leu Ile Ser Ile Asp
       210                 215                 220
   Ser Thr Cys Thr Glu Met Gly Asn Phe Asp Asn Ala Asn Val Thr Gly
   225                 230                 235                 240
   Glu Ile Glu Phe Ala Ile His Tyr Cys Phe Lys Thr His Ser Leu Glu
                   245                 250                 255
   Ile Cys Ile Lys Ala Cys Lys Asn Leu Ala Tyr Gly Glu Glu Lys Lys
               260                 265                 270
   Lys Lys Cys Asn Pro Tyr Val Lys Thr Tyr Leu Leu Pro Asp Arg Ser
           275                 280                 285
   Ser Gln Gly Lys Arg Lys Thr Gly Val Gln Arg Asn Thr Val Asp Pro
       290                 295                 300
   Thr Phe Gln Glu Thr Leu Lys Tyr Gln Val Ala Pro Ala Gln Leu Val
   305                 310                 315                 320
   Thr Arg Gln Leu Gln Val Ser Val Trp His Leu Gly Thr Leu Ala Arg
   325 330 335
   Arg Val Phe Leu Gly Glu Val Ile Ile Pro Leu Ala Thr Trp Asp Phe
   340 345 350
   Glu Asp Ser Thr Thr Gln Ser Phe Arg Trp His Pro Leu Arg Ala Lys
   355 360 365
   Ala Glu Lys Tyr Glu Asp Ser Val Pro Gln Ser Asn Gly Glu Leu Thr
   370 375 380
   Val Arg Ala Lys Leu Val Leu Pro Ser Arg Pro Arg Lys Leu Gln Glu
   385 390 395 400
   Ala Gln Glu Gly Thr Asp Gln Pro Ser Leu His Gly Gln Leu Cys Leu
   405 410 415
   Val Val Leu Gly Ala Lys Asn Leu Pro Val Arg Pro Asp Gly Thr Leu
   420 425 430
   Asn Ser Phe Val Lys Gly Cys Leu Thr Leu Pro Asp Gln Gln Lys Leu
   435 440 445
   Arg Leu Lys Ser Pro Val Leu Arg Lys Gln Ala Cys Pro Gln Trp Lys
   450 455 460
   His Ser Phe Val Phe Ser Gly Val Thr Pro Ala Gln Leu Arg Gln Ser
   465 470 475 480
   Ser Leu Glu Leu Thr Val Trp Asp Gln Ala Leu Phe Gly Met Asn Asp
   485 490 495
   Arg Leu Leu Gly Gly Thr Arg Leu Gly Ser Lys Gly Asp Thr Ala Val
   500 505 510
   Gly Gly Asp Ala Cys Ser Leu Ser Lys Leu Gln Trp Gln Lys Val Leu
   515 520 525
   Ser Ser Pro Asn Leu Trp Thr Asp Met Thr Leu Val Leu His
   530 535 540
<210> 62
   <211> 2277
   <212> DNA
   <213> Homo sapiens
<400> 62
   aagaagaatt gacttccttc tctgcagagc cggctctggt ctcttctctt gaagcagatg 60
   cgaaggctcc ccgaatgaga aagaatactc ggaatcagcg gtgaattgca gtgatctttc 120
   agagaaagcg cctgttcaac tttgtcctct ctcagagact cagagccttg gggcactgag 180
   ggatgccagt tctgcctgtt catctggaac ctggatctaa ggagggaaga ggcgttgccc 240
   ctgctggcat agtcaggtat tgaacgggct gagcttttca tgatggttcc tgctgacctg 300
   gaaacatctt aaatggaagg gcgtgagcgc ttggtccatg cagtgaagct cttccaacct 360
   gggtcaacga aaacggagaa gaaatggccc aagaaataga tctgagtgct ctcaaggagt 420
   tagaacgcga ggccattctc caggtcctgt accgagacca ggcggttcaa aacacagagg 480
   aggagaggac acggaaactg aaaacacacc tgcagcatct ccggtggaaa ggagcgaaga 540
   acacggactg ggagcacaaa gagaagtgct gtgcgcgctg ccagcaggtg ctggggttcc 600
   tgctgcaccg gggcgccgtg tgccggggct gcagccaccg cgtgtgtgcc cagtgccgag 660
   tgttcctgag ggggacccat gcctggaagt gcacggtgtg cttcgaggac aggaatgtca 720
   aaataaaaac tggagaatgg ttctatgagg aacgagccaa gaaatttcca actggaggca 780
   aacatgagac agttggaggg cagctcttgc aatcttatca gaagctgagc aaaatttctg 840
   tggttcctcc tactccacct cctgtcagcg agagccagtg cagccgcagt cctggcagga 900
   aggtcagtgc accagatatt ctgaaacctc tcaatcaaga ggatcccaaa tgctctacta 960
   accctatttt gaagcaacag aatctcccat ccagtccggc acccagtacc atattctctg 1020
   gaggttttag acacggaagt ttaattagca ttgacagcac ctgtacagag atgggcaatt 1080
   ttgacaatgc taatgtcact ggagaaatag aatttgccat tcattattgc ttcaaaaccc 1140
   attctttaga aatatgcatc aaggcctgta agaaccttgc ctatggagaa gaaaagaaga 1200
   aaaagtgcaa tccgtatgtg aagacctacc tgttgcccga cagatcctcc cagggaaagc 1260
   gcaagactgg agtccaaagg aacaccgtgg acccgacctt tcaggagacc ttgaagtatc 1320
   aggtggcccc tgcccagctg gtgacccggc agctgcaggt ctcggtgtgg catctgggca 1380
   cgctggcccg gagagtgttt cttggagaag tgatcattcc tctggccacg tgggactttg 1440
   aagacagcac aacacagtcc ttccgctggc atccgctccg ggccaaggcg gagaaatacg 1500
   aagacagcgt tcctcagagt aatggagagc tcacagtccg ggctaagctg gttctccctt 1560
   cacggcccag aaaactccaa gaggctcaag aagggacaga tcagccatca cttcatggtc 1620
   aactttgttt ggtagtgcta ggagccaaga atttacctgt gcggccagat ggcaccttga 1680
   actcatttgt taagggctgt ctcactctgc cagaccaaca aaaactgaga ctgaagtcgc 1740
   cagtcctgag gaagcaggct tgcccccagt ggaaacactc atttgtcttc agtggcgtaa 1800
   ccccagctca gctgaggcag tcaagcttgg agttaactgt ctgggatcag gccctctttg 1860
   gaatgaacga ccgcttgctt ggaggaacca gacttggttc aaagggagac acagctgttg 1920
   gcggggatgc atgctcacta tcgaagctcc agtggcagaa agtcctttcc agccccaatc   1980
   tatggacaga catgactctt gtcctgcact gacatgaagg cctcaaggtt ccaggttgca   2040
   gcaggcgtga ggcactgtgc gtctgcagag gggctacgaa ccaggtgcag ggtcccagct   2100
   ggagacccct ttgaccttga gcagtctcca tctgcggccc tgtcccatgg cttaaccgcc   2160
   tattggtatc tgtgtatatt tacgttaaac acaattatgt tacctaagcc tctggtgggt   2220
   tatctcctct ttgagatgta gaaaatggcc agattttaat aaacgttgtt acccatg      2277
<210> 63
   <211> 2131
   <212> DNA
   <213> Homo sapiens
<400> 63
   aggagggaag aggcgttgcc cctgctggca tagtcaggta ccagcccagc caggtattga 60
   acgggctgag cttttcatga tggttcctgc tgacctggaa acatcttaaa tggaagggcg 120
   tgagcgcttg gtccatgcag tgaagctctt ccaacctggg tcaacgaaaa cggagaagaa 180
   atggcccaag aaatagatct gagtgctctc aaggagttag aacgcgaggc cattctccag 240
   gtcctgtacc gagaccaggc ggttcaaaac acagaggagg agaggacacg gaaactgaaa 300
   acacacctgc agcatctccg gtggaaagga gcgaagaaca cggactggga gcacaaagag 360
   aagtgctgtg cgcgctgcca gcaggtgctg gggttcctgc tgcaccgggg cgccgtgtgc 420
   cggggctgca gccaccgcgt gtgtgcccag tgccgagtgt tcctgagggg gacccatgcc 480
   tggaagtgca cggtgtgctt cgaggacagg aatgtcaaaa taaaaactgg agaatggttc 540
   tatgaggaac gagccaagaa atttccaact ggaggcaaac atgagacagt tggagggcag 600
   ctcttgcaat cttatcagaa gctgagcaaa atttctgtgg ttcctcctac tccacctcct 660
   gtcagcgaga gccagtgcag ccgcagtcct ggcaggttac aggaatttgg tcagtttaga 720
   ggatttaata agtccgtgga aaatttgttt ctgtctcttg ctacccacgt gaaaaagctc 780
   tccaaatccc agaatgatat gacttctgag aagcatcttc tcgccacggg ccccaggcag 840
   tgtgtgggac agacagagag acggagccag tctgacactg cggtcaacgt caccaccagg 900
   aaggtcagtg caccagatat tctgaaacct ctcaatcaag aggatcccaa atgctctact 960
   aaccctattt tgaagcaaca gaatctccca tccagtccgg cacccagtac catattctct 1020
   ggaggtttta gacacggaag tttaattagc attgacagca cctgtacaga gatgggcaat 1080
   tttgacaatg ctaatgtcac tggagaaata gaatttgcca ttcattattg cttcaaaacc 1140
   cattctttag aaatatgcat caaggcctgt aagaaccttg cctatggaga agaaaagaag 1200
   aaaaagtgca atccgtatgt gaagacctac ctgttgcccg acagatcctc ccagggaaag 1260
   cgcaagactg gagtccaaag gaacaccgtg gacccgacct ttcaggagac cttgaagtat 1320
   caggtggccc ctgcccagct ggtgacccgg cagctgcagg tctcggtgtg gcatctgggc 1380
   acgctggccc ggagagtgtt tcttggagaa gtgatcattc ctctggccac gtgggacttt 1440
   gaagacagca caacacagtc cttccgctgg catccgctcc gggccaaggc ggagaaatac 1500
   gaagacagcg ttcctcagag taatggagag ctcacagtcc gggctaagct ggttctccct 1560
   tcacggccca gaaaactcca agaggctcaa gaagggacag atcagccatc acttcatggt 1620
   caactttgtt tggtagtgct aggagccaag aatttacctg tgcggccaga tggcaccttg 1680
   aactcatttg ttaagggctg tctcactctg ccagaccaac aaaaactgag actgaagtcg 1740
   ccagtcctga ggaagcaggc ttgcccccag tggaaacact catttgtctt cagtggcgta 1800
   accccagctc agctgaggca gtcaagcttg gagttaactg tctgggatca ggccctcttt 1860
   ggaatgaacg accgcttgct tggaggaacc agacttggtt caaagggaga cacagctgtt 1920
   ggcggggatg catgctcact atcgaagctc cagtggcaga aagtcctttc cagccccaat 1980
   ctatggacag acatgactct tgtcctgcac tgacatgaag gcctcaaggt tccaggttgc 2040
   agcaggcgtg aggcactgtg cgtctgcaga ggggctacga accaggtgca gggtcccagc 2100
   tggagacccc tttgaccttg agcagtctcc a 2131
<210> 64
   <211> 1927
   <212> DNA
   <213> Homo sapiens
<400> 64
   aggagggaag aggcgttgcc cctgctggca tagtcaggta ccagcccagc caggtattga 60
   acgggctgag cttttcatga tggttcctgc tgacctggaa acatcttaaa tggaagggcg 120
   tgagcgcttg gtccatgcag tgaagctctt ccaacctggg tcaacgaaaa cggagaagaa 180
   atggcccaag aaatagatct gagtgctctc aaggagttag aacgcgaggc cattctccag 240
   gtcctgtacc gagaccaggc ggttcaaaac acagaggagg agaggacacg gaaactgaaa 300
   acacacctgc agcatctccg gtggaaagga gcgaagaaca cggactggga gcacaaagag 360
   aagtgctgtg cgcgctgcca gcaggtgctg gggttcctgc tgcaccgggg cgccgtgtgc 420
   cggggctgca gccaccgcgt gtgtgcccag tgccgagtgt tcctgagggg gacccatgcc 480
   tggaagtgca cggtgtgctt cgaggacagg aatgtcaaaa taaaaactgg agaatggttc 540
   tatgaggaac gagccaagaa atttccaact ggaggcaaac atgagacagt tggagggcag 600
   ctcttgcaat cttatcagaa gctgagcaaa atttctgtgg ttcctcctac tccacctcct 660
   gtcagcgaga gccagtgcag ccgcagtcct ggcaggaagg tcagtgcacc agatattctg 720
   aaacctctca atcaagagga tcccaaatgc tctactaacc ctattttgaa gcaacagaat 780
   ctcccatcca gtccggcacc cagtaccata ttctctggag gttttagaca cggaagttta 840
   attagcattg acagcacctg tacagagatg ggcaattttg acaatgctaa tgtcactgga 900
   gaaatagaat ttgccattca ttattgcttc aaaacccatt ctttagaaat atgcatcaag 960
   gcctgtaaga accttgccta tggagaagaa aagaagaaaa agtgcaatcc gtatgtgaag 1020
   acctacctgt tgcccgacag atcctcccag ggaaagcgca agactggagt ccaaaggaac 1080
   accgtggacc cgacctttca ggagaccttg aagtatcagg tggcccctgc ccagctggtg 1140
   acccggcagc tgcaggtctc ggtgtggcat ctgggcacgc tggcccggag agtgtttctt 1200
   ggagaagtga tcattcctct ggccacgtgg gactttgaag acagcacaac acagtccttc 1260
   cgctggcatc cgctccgggc caaggcggag aaatacgaag acagcgttcc tcagagtaat 1320
   ggagagctca cagtccgggc taagctggtt ctcccttcac ggcccagaaa actccaagag 1380
   gctcaagaag ggacagatca gccatcactt catggtcaac tttgtttggt agtgctagga 1440
   gccaagaatt tacctgtgcg gccagatggc accttgaact catttgttaa gggctgtctc 1500
   actctgccag accaacaaaa actgagactg aagtcgccag tcctgaggaa gcaggcttgc 1560
   ccccagtgga aacactcatt tgtcttcagt ggcgtaaccc cagctcagct gaggcagtca 1620
   agcttggagt taactgtctg ggatcaggcc ctctttggaa tgaacgaccg cttgcttgga 1680
   ggaaccagac ttggttcaaa gggagacaca gctgttggcg gggatgcatg ctcactatcg 1740
   aagctccagt ggcagaaagt cctttccagc cccaatctat ggacagacat gactcttgtc 1800
   ctgcactgac atgaaggcct caaggttcca ggttgcagca ggcgtgaggc actgtgcgtc 1860
   tgcagagggg ctacgaacca ggtgcagggt cccagctgga gacccctttg accttgagca 1920
   gtctcca 1927
<210> 65
   <211> 173
   <212> PRT
   <213> Homo sapiens
<400> 65
   Met His Trp Lys Met Leu Leu Leu Leu Leu Leu Tyr Tyr Asn Ala Glu
   1 5 10 15
   Ala Ser Met Cys His Arg Trp Ser Arg Ala Val Leu Phe Pro Ala Ala
   20 25 30
   His Arg Pro Lys Arg Ser Ser Ser Leu Pro Leu Asn Pro Val Leu Gln
   35 40 45
   Thr Ser Leu Glu Glu Val Glu Leu Leu Tyr Glu Phe Leu Leu Ala Glu
   50 55 60
   Leu Glu Ile Ser Pro Asp Leu Gln Ile Ser Ile Lys Asp Glu Glu Leu
   65 70 75 80
   Ala Ser Leu Arg Lys Ala Ser Asp Phe Arg Thr Val Cys Asn Asn Val
   85 90 95
   Ile Pro Lys Ser Ile Pro Asp Ile Arg Arg Leu Ser Ala Ser Leu Ser
   100 105 110
   Ser His Pro Gly Ile Leu Lys Lys Glu Asp Phe Glu Arg Thr Val Leu
   115 120 125
   Thr Leu Ala Tyr Thr Ala Tyr Arg Thr Ala Leu Ser His Gly His Gln
   130 135 140
   Lys Asp Ile Trp Ala Gln Ser Leu Val Ser Leu Phe Gln Ala Leu Arg
   145 150 155 160
   His Asp Leu Met Arg Ser Ser Gln Pro Gly Val Pro Pro
   165 170
<210> 66
   <211> 1645
   <212> DNA
   <213> Homo sapiens
<400> 66
   gcaaaaggaa gggagggaag cactccatca tctcactggg aagaacggca cgggcatacc     60
   tgcagctact ggggttccac tgggcttgag ggtcgatttt tcaccttttg aaggacaaga     120
   tgcattggaa gatgttgctg cttctgctgt tgtattacaa tgctgaggct tctatgtgcc    180
   acaggtggag cagggctgtg ctcttccctg ccgcccaccg gccaaagagg tcctcatcac    240
   tgccattgaa cccagtcctg cagacctccc tggaggaggt ggagctgctc tacgagttcc    300
   tgctggccga acttgagatc agccctgacc tgcagatctc catcaaggac gaggagctgg    360
   cctccttgcg gaaggcctca gacttccgca ccgtctgcaa caacgtcatc cccaagagca    420
   tcccagacat ccgccggctc agcgccagcc tctccagcca ccctggcatc ctcaagaaag    480
   aagactttga aaggacagtg ctgaccctgg cctacacagc ctaccgcaca gccctgtccc    540
   acggccatca gaaggacatc tgggcgcagt ccctcgttag cctcttccag gccctgaggc    600
   acgacttgat gcgctcctca cagccgggag tacctccctg agagactggc ccacaccagg    660
   acctcagagc agggaccagc acagtaatcc agaaagtctt cattctctac tccatttaca    720
   gagaccagca acaaaactct taccgctgac acagagcagc agagatcaaa cagtaacccc    780
   gatgctcttt tctccttgta gtttcctgga agacacatct gattcatgcc atcatgtgac    840
   ctgggctgga agaaagggct ggaatggtca ttcaagacgc ctccatgggc agaatggttt    900
   gcctatggca ggcagaattc tgatatgctt caacccagag cagtggccac acactcaaga    960
   gtgagaacag gcgtgagcca ccgtgcctgg cccaggatct aaaaactttc taagtttcct   1020
   ccatcgttgg catcctcaca gctatctcca atgtcactca agagacatca acagacattt   1080
   aactgctgca gacttcattg ctctgtcacc tcaccttgaa tctaacaaat caaagtattt   1140
   ctgcaggtcc aatggtctaa aatcaaatgc ttgttaaatg actttttaca acacccctta    1200
   ctttcctaat ccatttcaat cttatttttt ttattgtggt aaaaaacaca tcaggtaaaa    1260
   tgtaccatct taaccatttt taagcatatg gtacagcagt gttaactcca tgcatgttgt   1320
   gaaacagacc cccggaactt tctcatcttg taattctgaa gttctatacc caccgaacaa   1380
   ctcctctttt ccccttcccc ctgcctgccc cagctcttgg caccattatt ctgctttctg   1440
   tttttgagag tctgactact taagatacct catacaagcg ggatctggct tacatttctt   1500
   gagcattgta ttctggaaaa gtgtttcctt cctctgaaaa atgggtagag ttctgaagga   1560
   gaactactgg tcttattgta cacttgctgt acctattttt atttaacaaa tattcatcta   1620
   tggtataata aagatgtcat gattg                                         1645
<210> 67
   <211> 552
   <212> PRT
   <213> Homo sapiens
<400> 67
   Met Ala Ala Leu Ala Pro Val Gly Ser Pro Ala Ser Arg Gly Pro Arg
   1               5                   10                  15
   Leu Ala Ala Gly Leu Arg Leu Leu Pro Met Leu Gly Leu Leu Gln Leu
               20                  25                  30
   Leu Ala Glu Pro Gly Leu Gly Arg Val His His Leu Ala Leu Lys Asp
           35                  40                  45
   Asp Val Arg His Lys Val His Leu Asn Thr Phe Gly Phe Phe Lys Asp
       50                  55                  60
   Gly Tyr Met Val Val Asn Val Ser Ser Leu Ser Leu Asn Glu Pro Glu
   65                  70                  75                  80
   Asp Lys Asp Val Thr Ile Gly Phe Ser Leu Asp Arg Thr Lys Asn Asp
                   85                  90                  95
   Gly Phe Ser Ser Tyr Leu Asp Glu Asp Val Asn Tyr Cys Ile Leu Lys
               100                 105                 110
   Lys Gln Ser Val Ser Val Thr Leu Leu Ile Leu Asp Ile Ser Arg Ser
           115                 120                 125
   Glu Val Arg Val Lys Ser Pro Pro Glu Ala Gly Thr Gln Leu Pro Lys
       130                 135                 140
   Ile Ile Phe Ser Arg Asp Glu Lys Val Leu Gly Gln Ser Gln Glu Pro
   145                 150                 155                 160
   Asn Val Asn Pro Ala Ser Ala Gly Asn Gln Thr Gln Lys Thr Gln Asp
                   165                 170                 175
   Gly Gly Lys Ser Lys Arg Ser Thr Val Asp Ser Lys Ala Met Gly Glu
               180                 185                 190
   Lys Ser Phe Ser Val His Asn Asn Gly Gly Ala Val Ser Phe Gln Phe
           195                 200                 205
   Phe Phe Asn Ile Ser Thr Asp Asp Gln Glu Gly Leu Tyr Ser Leu Tyr
       210                 215                 220
   Phe His Lys Cys Leu Gly Lys Glu Leu Pro Ser Asp Lys Phe Thr Phe
   225                 230                 235                 240
   Ser Leu Asp Ile Glu Ile Thr Glu Lys Asn Pro Asp Ser Tyr Leu Ser
                   245                 250                 255
   Ala Gly Glu Ile Pro Leu Pro Lys Leu Tyr Ile Ser Met Ala Phe Phe
               260                 265                 270
   Phe Phe Leu Ser Gly Thr Ile Trp Ile His Ile Leu Arg Lys Arg Arg
           275                 280                 285
   Asn Asp Val Phe Lys Ile His Trp Leu Met Ala Ala Leu Pro Phe Thr
       290                 295                 300
   Lys Ser Leu Ser Leu Val Phe His Ala Ile Asp Tyr His Tyr Ile Ser
   305                 310                 315                 320
   Ser Gln Gly Phe Pro Ile Glu Gly Trp Ala Val Val Tyr Tyr Ile Thr
                   325                 330                 335
   His Leu Leu Lys Gly Ala Leu Leu Phe Ile Thr Ile Ala Leu Ile Gly
   340 345 350
   Thr Gly Trp Ala Phe Ile Lys His Ile Leu Ser Asp Lys Asp Lys Lys
   355 360 365
   Ile Phe Met Ile Val Ile Pro Leu Gln Val Leu Ala Asn Val Ala Tyr
   370 375 380
   Ile Ile Ile Glu Ser Thr Glu Glu Gly Thr Thr Glu Tyr Gly Leu Trp
   385 390 395 400
   Lys Asp Ser Leu Phe Leu Val Asp Leu Leu Cys Cys Gly Ala Ile Leu
   405 410 415
   Phe Pro Val Val Trp Ser Ile Arg His Leu Gln Glu Ala Ser Ala Thr
   420 425 430
   Asp Gly Lys Ala Ala Ile Asn Leu Ala Lys Leu Lys Leu Phe Arg His
   435 440 445
   Tyr Tyr Val Leu Ile Val Cys Tyr Ile Tyr Phe Thr Arg Ile Ile Ala
   450 455 460
   Phe Leu Leu Lys Leu Ala Val Pro Phe Gln Trp Lys Trp Leu Tyr Gln
   465 470 475 480
   Leu Leu Asp Glu Thr Ala Thr Leu Val Phe Phe Val Leu Thr Gly Tyr
   485 490 495
   Lys Phe Arg Pro Ala Ser Asp Asn Pro Tyr Leu Gln Leu Ser Gln Glu
   500 505 510
   Glu Glu Asp Leu Glu Met Glu Ser Val Val Thr Thr Ser Gly Val Met
   515 520 525
   Glu Ser Met Lys Lys Val Lys Lys Val Thr Asn Gly Ser Val Glu Pro
   530 535 540
   Gln Gly Glu Trp Glu Gly Ala Val
   545 550
<210> 68
   <211> 600
   <212> PRT
   <213> Homo sapiens
<400> 68
   Met Ala Ala Leu Ala Pro Val Gly Ser Pro Ala Ser Arg Gly Pro Arg
   1               5                   10                  15
   Leu Ala Ala Gly Leu Arg Leu Leu Pro Met Leu Gly Leu Leu Gln Leu
               20                  25                  30
   Leu Ala Glu Pro Gly Leu Gly Arg Val His His Leu Ala Leu Lys Asp
           35                  40                  45
   Asp Val Arg His Lys Val His Leu Asn Thr Phe Gly Phe Phe Lys Asp
       50                  55                  60
   Gly Tyr Met Val Val Asn Val Ser Ser Leu Ser Leu Asn Glu Pro Glu
   65                  70                  75                  80
   Asp Lys Asp Val Thr Ile Gly Phe Ser Leu Asp Arg Thr Lys Asn Asp
                   85                  90                  95
   Gly Phe Ser Ser Tyr Leu Asp Glu Asp Val Asn Tyr Cys Ile Leu Lys
               100                 105                 110
   Lys Gln Ser Val Ser Val Thr Leu Leu Ile Leu Asp Ile Ser Arg Ser
           115                 120                 125
   Glu Val Arg Val Lys Ser Pro Pro Glu Ala Gly Thr Gln Leu Pro Lys
       130                 135                 140
   Ile Ile Phe Ser Arg Asp Glu Lys Val Leu Gly Gln Ser Gln Glu Pro
   145                 150                 155                 160
   Asn Val Asn Pro Ala Ser Ala Gly Asn Gln Thr Gln Lys Thr Gln Asp
                   165                 170                 175
   Gly Gly Lys Ser Lys Arg Ser Thr Val Asp Ser Lys Ala Met Gly Glu
               180                 185                 190
   Lys Ser Phe Ser Val His Asn Asn Gly Gly Ala Val Ser Phe Gln Phe
           195                 200                 205
   Phe Phe Asn Ile Ser Thr Asp Asp Gln Glu Gly Leu Tyr Ser Leu Tyr
       210                 215                 220
   Phe His Lys Cys Leu Gly Lys Glu Leu Pro Ser Asp Lys Phe Thr Phe
   225                 230                 235                 240
   Ser Leu Asp Ile Glu Ile Thr Glu Lys Asn Pro Asp Ser Tyr Leu Ser
                   245                 250                 255
   Ala Gly Glu Ile Pro Leu Pro Lys Leu Tyr Ile Ser Met Ala Phe Phe
               260                 265                 270
   Phe Phe Leu Ser Gly Thr Ile Trp Ile His Ile Leu Arg Lys Arg Arg
           275                 280                 285
   Asn Asp Val Phe Lys Ile His Trp Leu Met Ala Ala Leu Pro Phe Thr
       290                 295                 300
   Lys Ser Leu Ser Leu Val Phe His Ala Ile Asp Tyr His Tyr Ile Ser
   305                 310                 315                 320
   Ser Gln Gly Phe Pro Ile Glu Gly Trp Ala Val Val Tyr Tyr Ile Thr
                   325                 330                 335
   His Leu Leu Lys Gly Ala Leu Leu Phe Ile Thr Ile Ala Leu Ile Gly
               340                 345                 350
   Thr Gly Trp Ala Phe Ile Lys His Ile Leu Ser Asp Lys Asp Lys Lys
           355                 360                 365
   Ile Phe Met Ile Val Ile Pro Leu Gln Val Leu Ala Asn Val Ala Tyr
       370                 375                 380
   Ile Ile Ile Glu Ser Thr Glu Glu Gly Thr Thr Glu Tyr Gly Leu Trp
   385                 390                 395                 400
   Lys Asp Ser Leu Phe Leu Val Asp Leu Leu Cys Cys Gly Ala Ile Leu
                   405                 410                 415
   Phe Pro Val Val Trp Ser Ile Arg His Leu Gln Glu Ala Ser Ala Thr
               420                 425                 430
   Asp Gly Lys Gly Asp Ser Met Gly Pro Leu Gln Gln Arg Ala Asn Leu
           435                 440                 445
   Arg Ala Gly Ser Arg Ile Glu Ser His His Phe Ala Gln Ala Asp Leu
       450                 455                 460
   Glu Leu Leu Ala Ser Ser Cys Pro Pro Ala Ser Val Ser Gln Arg Ala
   465                 470                 475                 480
   Gly Ile Thr Ala Ala Ile Asn Leu Ala Lys Leu Lys Leu Phe Arg His
                   485                 490                 495
   Tyr Tyr Val Leu Ile Val Cys Tyr Ile Tyr Phe Thr Arg Ile Ile Ala
   500 505 510
   Phe Leu Leu Lys Leu Ala Val Pro Phe Gln Trp Lys Trp Leu Tyr Gln
   515 520 525
   Leu Leu Asp Glu Thr Ala Thr Leu Val Phe Phe Val Leu Thr Gly Tyr
   530 535 540
   Lys Phe Arg Pro Ala Ser Asp Asn Pro Tyr Leu Gln Leu Ser Gln Glu
   545 550 555 560
   Glu Glu Asp Leu Glu Met Glu Ser Val Val Thr Thr Ser Gly Val Met
   565 570 575
   Glu Ser Met Lys Lys Val Lys Lys Val Thr Asn Gly Ser Val Glu Pro
   580 585 590
   Gln Gly Glu Trp Glu Gly Ala Val
   595 600
<210> 69
   <211> 600
   <212> PRT
   <213> Homo sapiens
<400> 69
   Met Ala Ala Leu Ala Pro Val Gly Ser Pro Ala Ser Arg Gly Pro Arg
   1 5 10 15
   Leu Ala Ala Gly Leu Arg Leu Leu Pro Met Leu Gly Leu Leu Gln Leu
   20 25 30
   Leu Ala Glu Pro Gly Leu Gly Arg Val His His Leu Ala Leu Lys Asp
   35 40 45
   Asp Val Arg His Lys Val His Leu Asn Thr Phe Gly Phe Phe Lys Asp
   50 55 60
   Gly Tyr Met Val Val Asn Val Ser Ser Leu Ser Leu Asn Glu Pro Glu
   65 70 75 80
   Asp Lys Asp Val Thr Ile Gly Phe Ser Leu Asp Arg Thr Lys Asn Asp
   85 90 95
   Gly Phe Ser Ser Tyr Leu Asp Glu Asp Val Asn Tyr Cys Ile Leu Lys
   100 105 110
   Lys Gln Ser Val Ser Val Thr Leu Leu Ile Leu Asp Ile Ser Arg Ser
           115                 120                 125
   Glu Val Arg Val Lys Ser Pro Pro Glu Ala Gly Thr Gln Leu Pro Lys
       130                 135                 140
   Ile Ile Phe Ser Arg Asp Glu Lys Val Leu Gly Gln Ser Gln Glu Pro
   145                 150                 155                 160
   Asn Val Asn Pro Ala Ser Ala Gly Asn Gln Thr Gln Lys Thr Gln Asp
                   165                 170                 175
   Gly Gly Lys Ser Lys Arg Ser Thr Val Asp Ser Lys Ala Met Gly Glu
               180                 185                 190
   Lys Ser Phe Ser Val His Asn Asn Gly Gly Ala Val Ser Phe Gln Phe
           195                 200                 205
   Phe Phe Asn Ile Ser Thr Asp Asp Gln Glu Gly Leu Tyr Ser Leu Tyr
       210                 215                 220
   Phe His Lys Cys Leu Gly Lys Glu Leu Pro Ser Asp Lys Phe Thr Phe
   225                 230                 235                 240
   Ser Leu Asp Ile Glu Ile Thr Glu Lys Asn Pro Asp Ser Tyr Leu Ser
                   245                 250                 255
   Ala Gly Glu Ile Pro Leu Pro Lys Leu Tyr Ile Ser Met Ala Phe Phe
               260                 265                 270
   Phe Phe Leu Ser Gly Thr Ile Trp Ile His Ile Leu Arg Lys Arg Arg
           275                 280                 285
   Asn Asp Val Phe Lys Ile His Trp Leu Met Ala Ala Leu Pro Phe Thr
       290                 295                 300
   Lys Ser Leu Ser Leu Val Phe His Ala Ile Asp Tyr His Tyr Ile Ser
   305                 310                 315                 320
   Ser Gln Gly Phe Pro Ile Glu Gly Trp Ala Val Val Tyr Tyr Ile Thr
                   325                 330                 335
   His Leu Leu Lys Gly Ala Leu Leu Phe Ile Thr Ile Ala Leu Ile Gly
               340                 345                 350
   Thr Gly Trp Ala Phe Ile Lys His Ile Leu Ser Asp Lys Asp Lys Lys
   355 360 365
   Ile Phe Met Ile Val Ile Pro Leu Gln Val Leu Ala Asn Val Ala Tyr
   370 375 380
   Ile Ile Ile Glu Ser Thr Glu Glu Gly Thr Thr Glu Tyr Gly Leu Trp
   385 390 395 400
   Lys Asp Ser Leu Phe Leu Val Asp Leu Leu Cys Cys Gly Ala Ile Leu
   405 410 415
   Phe Pro Val Val Trp Ser Ile Arg His Leu Gln Glu Ala Ser Ala Thr
   420 425 430
   Asp Gly Lys Gly Asp Ser Met Gly Pro Leu Gln Gln Arg Ala Asn Leu
   435 440 445
   Arg Ala Gly Ser Arg Ile Glu Ser His His Phe Ala Gln Ala Asp Leu
   450 455 460
   Glu Leu Leu Ala Ser Ser Cys Pro Pro Ala Ser Val Ser Gln Arg Ala
   465 470 475 480
   Gly Ile Thr Ala Ala Ile Asn Leu Ala Lys Leu Lys Leu Phe Arg His
   485 490 495
   Tyr Tyr Val Leu Ile Val Cys Tyr Ile Tyr Phe Thr Arg Ile Ile Ala
   500 505 510
   Phe Leu Leu Lys Leu Ala Val Pro Phe Gln Trp Lys Trp Leu Tyr Gln
   515 520 525
   Leu Leu Asp Glu Thr Ala Thr Leu Val Phe Phe Val Leu Thr Gly Tyr
   530 535 540
   Lys Phe Arg Pro Ala Ser Asp Asn Pro Tyr Leu Gln Leu Ser Gln Glu
   545 550 555 560
   Glu Glu Asp Leu Glu Met Glu Ser Val Val Thr Thr Ser Gly Val Met
   565 570 575
   Glu Ser Met Lys Lys Val Lys Lys Val Thr Asn Gly Ser Val Glu Pro
   580 585 590
   Gln Gly Glu Trp Glu Gly Ala Val
   595 600
<210> 70
   <211> 6874
   <212> DNA
   <213> Homo sapiens
<400> 70
   ccgaggtggg cagcacaggc tcctcgacga cttcctaggt cgcaatctcc aggaaaacga      60
   ccacagggtc agcggagcta gccgccgagc cccgctcccc gggcccttcc ggcggctgcg     120
   ccctttcacc ccggacgtgg gcgggagagg aagcggctgg tgatgctgga acaaacatgg     180
   ccgctctggc gcccgtcggc tcccccgcct cccgcggtcc taggctggcc gcgggcctcc     240
   ggctgctccc aatgctgggt ttgctgcagt tgctggccga gcctggcctg ggccgcgtcc     300
   atcacctggc actcaaggat gatgtgaggc ataaagttca tctgaacacc tttggcttct     360
   tcaaggatgg gtacatggtg gtgaatgtca gtagcctctc actgaatgag cctgaagaca     420
   aggatgtgac tattggattt agcctagacc gtacaaagaa tgatggcttt tcttcttacc     480
   tggatgaaga tgtgaattac tgtattttaa agaaacagtc tgtctctgtc acccttttaa     540
   tcctagacat ctccagaagt gaggtaagag taaagtctcc accagaagct ggtacccagt     600
   taccaaagat catcttcagc agggatgaga aagtccttgg tcagagccag gagcctaatg     660
   ttaaccctgc ttcagcaggc aaccagaccc agaagacaca agatggtgga aagtctaaaa     720
   gaagtacagt ggattcaaag gccatgggag agaaatcctt ttctgttcat aataatggtg     780
   gggcagtgtc atttcagttt ttctttaaca tcagcactga tgaccaagaa ggcctttaca     840
   gtctttattt tcataaatgc cttggaaaag aattgccaag tgacaagttt acattcagcc     900
   ttgatattga gatcacagag aagaatcctg acagctacct ctcagcagga gaaattcctc     960
   tccccaaatt atacatctca atggcctttt tcttctttct ttctgggacc atctggattc    1020
   atatccttcg aaaacgacgg aatgatgtat ttaaaatcca ctggctgatg gcggcccttc    1080
   ctttcaccaa gtctctttcc ttggtgttcc atgcaattga ctaccactac atctcctccc    1140
   agggcttccc tatcgaaggc tgggctgttg tgtactacat aactcacctt ttgaaagggg    1200
   cgctactctt catcaccatt gcactcattg gcactggctg ggctttcatt aagcacatcc    1260
   tttctgataa agacaaaaag atcttcatga ttgtcattcc actccaggtc ctggcaaatg    1320
   tagcctacat catcatagag tccaccgagg agggcacgac tgaatatggc ttgtggaagg    1380
   actctctatt tctggtcgac ctgttgtgtt gtggtgccat cctcttccca gtggtgtggt    1440
   caatcagaca tttacaagaa gcatcagcaa cagatggaaa agctgctatt aacttagcaa    1500
   agctgaaact tttcagacat tattacgtct tgattgtgtg ttacatatac ttcactagga    1560
   tcattgcatt tctcctcaaa ctcgctgttc cattccagtg gaagtggctc taccagctcc    1620
   tggatgaaac ggccacactg gtcttctttg ttctaacggg gtataaattc cgtccggctt    1680
   cagataaccc ctacctacaa ctttctcagg aagaagaaga cttggaaatg gagtccgttg 1740
   tgacaacatc tggggtgatg gaaagtatga agaaagtcaa gaaggtgacc aacggctccg 1800
   tggagcccca gggcgagtgg gaaggcgccg tgtgacagag ccgaccctga ggatggcact 1860
   gtccaaggaa actgttaact tattcatagt cctattggac agcaggagca gctcctacag 1920
   tgaactattg gcaccaccga cagtgacacc agggcacatg gctggagcac agtgccgcgg 1980
   aaacctgatt ttgtactctc ttttatggaa acgatctgtg gctgtttaga ggcagctgga 2040
   tcctctttca ggcgggaatg ggagggcggg cacagggagg aggagaggaa gagaaaagga 2100
   agaattcatt tttaatttag gtttcttttt ttcttcttca tttcggagct ctaaggtgta 2160
   tgcagttgtg accccatgtg tggggaagtg tagcaaggac ggctggtgga gggggaagga 2220
   gggtgcgagg tgtctgtctg atgctttagg aaatgtctac tgaggaccct gggacttaag 2280
   aagaagggcg gggagagtgc cattgcctgt ttgggagaca aaaatgaacg aaaacaggtg 2340
   actttggaaa gcaaagtcaa aacccagttt aggatgtagc acctgcccca ggattcctgc 2400
   cctcggcttt gccccagacc cttattccag atgctgagag tgaccaggac agcagctcct 2460
   gaggcccagt ggtcttcttt ccaacaggaa aagaaggctg tgatgtcgct gtcaggatca 2520
   tgccctgtgg cacagcacag gtggtgggag gtggttttct gactgagatg ttgcctgatg 2580
   gatggaaaga aatgtatttt taagttcaaa aagcattatc ctgtggcgtt gcctggacat 2640
   ccactccctg acagcccaga gcagcactgt ctggcttccc ttcatgcttg tggctttgtt 2700
   gtgtttgatc agaattttgg gggaaatgga aagttttcct caaggagcag ctgggggcag 2760
   aataggtagt atttaagcaa atacttaagt ccaagcaaat catccccatt aaaaagcttt 2820
   tcctgtaggc tagtaggatt tctaaataga tgaattcaac agacttggtc cccatagtcc 2880
   aagagtatgt atgtgaagaa agtgagcatg attcaacagt ttcactctca gggattttag 2940
   gatggcaaaa tacttcacag aaactcaatg attaagttcc cttccacact tccagagctt 3000
   gaatgaacac aggtagccac ctaaattgag cagtattgca actcagagag aaaatcatct 3060
   gaatagtagg acaagctcag aaggtacatt gtgactgagg gcttaaaagg agaccaaaac 3120
   atggccccat cagggaagct tcttaatgct tggggggcca gctaggtagg gttgcttcca 3180
   aaagctggag cccacccctg cctaggggtt gtcagagagc cacacctgca ggggaacagg 3240
   tacctccgag ggtgagagtc gtggtctctg ggagttgttt tctcacctct ggcttagaag 3300
   ggtcaggcag aaaccacagg atgtggggtc acactcactg tcccaagttt gggaacctga 3360
   aaaagtctcc attcagaaca tggttgttct ccctgtccca tgctatctta tcttcctaaa 3420
   tgactaatga ggaagcgggt gttctttttc tgcactttga ttcgccatct gggttctgta 3480
   gggtgctctg aaggtgtgat ctgccttctg gctgatgtgg aggaagagca agcgccttcc 3540
   caggccacag ctgctcacct ctcggcagat attttaggca agcatccgtg tgtcttccca 3600
   tcttcaggag aaaggtaaat gcaccctaag tgttcacttc tggacctttt tcaagttcac 3660
   ttgggactgt gtgacagaag ggagttggag ggaggatggg aatattttta acactttgtt 3720
   ttcctgtgca gaaacataat accagttttc gcagaaatgt gtctcaatct gtgactacca 3780
   aagccctcct cagtccttcc ctcagaggga cacatttgct gtttctcccg caagcagatg 3840
   ttgtggatga ggcgatagac tccttggcaa gaacgaaagg tgtgatgaaa cctccctgct 3900
   cggaagggtc tccgtggagg tgtcctcatt tcacatgctg ggttttgcaa gcgaggaagc 3960
   caggcagtgg aggaactaga gagaggcagg cgtgtgtgtg gacaagcgct ggagccgcag 4020
   ccctcagact ggcacgggaa cgccagcgtt gggtgttcag attccacgcg tatgtctggg 4080
   ctcactcaca gcatggccga gtgtctgcag tgctggtcct gacccttcca gagcagcagt 4140
   ggacagatga gataagactg tttcagaaac aaagatggcc acagccttcc taacaagcag 4200
   gtcatctggc catgtctgta ttgtaactgg taaaaggctt caagtcagat tgatgatcaa 4260
   gaaaagtcaa aaccccagcc caagattggg aaagcaggtg gtggttccaa gcttttaaaa 4320
   aattattgaa gctctccatc ctgttctgtg agtgtgtctt ctctttctcc ttcacgtcat 4380
   agccgtgacc caccgttcat ctctgctctt gcgtaaagat gaccgatgga gtccaaagcc 4440
   aagtggcttc accagctgac aagccaccct cctgcagcct gagtttcaca gtccactggg 4500
   ttcgttgtca tgcggtgttt gaatggttaa gcccttgcag tatttcagat cgggcaaaaa 4560
   atatcggatg cacatagcag aaccattggt ggtatttata gctttgcttt gtactcctca 4620
   ctgtttctgc ctacgcaaaa tatccatgtt tcctctgaga aatctgttgt ggactgaaag 4680
   cgctgctggc tgtgaaattt aataaagtgt gtatgctttg ctagaaaatt atttcttgga 4740
   caataggaac agtcattgat ctgtaaatcc tggctcttaa cagtgagtgg ccaaggactt 4800
   gatcagccca tttcttggtc cctcagtgct ttaaaattta agtagcactg cattttgtaa 4860
   tgttgaatat gactctagtg acttgtagga ggcacttgtg aggagatgct tgcttcagtg 4920
   taaaagatgc tcatggcctg agtcagttga gttttctttc aagaaaccac ttcagagtga 4980
   aatatccagg gtttccccgc cctggacatg tccagcctgc ccaggcagca cacagccctg 5040
   taagtccacc tcgtgtgggt gagatttcct cctgcgtgat gacctcatcg ccatctctgc 5100
   tgtctcattc cacagcctcc ctccctcttc tctcctcctc tgccctcgcc cttccccctt 5160
   ccccatcccc tccccctcct cctctgccct cgcccttacc cctccccctt ccccttccgc 5220
   tcctcctccc tcctccacct ctttctcctc ctccttccct cctcctccct cctcttccct 5280
   tctctgccat ctttctcccc gtgcctattg atcccacata ggctcattct gggtacaccg 5340
   gctaaaggct ttggtgcatt gcagcgtttt ctcccagcag ctgtgtgaaa gatgcatttt 5400
   ctaagctaag gagaattttc tcaagagtgg catactcatg ccaaatatta ttgctctggg 5460
   ccatataggc tggtcttcct ccacactaaa atgggtgtct tgttttggta cttaaaacag 5520
   tctactccag gcatccagtc cttacagacc aaggaagagc atagcgatgc ctgttggaat 5580
   tgcagatgca ttctggcctt ctcccccgtc ctgaaacatt ttctttgagg aaggctctta 5640
   gaacattaga tagtctgctg aggttgttgg cccagctcca tacacccagt agaacagtgg 5700
   aacaactcat gcttcatgct gccaagctgc tgtacttcaa aggaaacaga tctagcacac 5760
   tgctgcaccc ctgcttccac actccacact tcaccccgct gcttttctct gacccgcccc 5820
   tggccttgta agactcacgt aagctaagtc caggatgcct gtggcctgcg gcttgattct 5880
   tccctttagg attcagcaag ttaatggctt cctcgctata gaagtgagac tttgacttga 5940
   tgcctcttgg tatatcaaaa agatattcat ccagaaagta ccaaatgttc tgaaagaccc 6000
   gctcttcact ccagttttcc ctagggtgtt tctggcaggg cgtttttaaa aggcatctac 6060
   ctgagttgac gctaatactt gtcaccacct ggaacgtagt tatcggtcgg caggctgaac 6120
   atactccaga ttccccagag gccacttctg tagcccagcg atgcatctga gcctctctgc 6180
   gtggtttatg cttgaaaaat agataatgct tttagatggt tcactgccag gccatgggcc 6240
   ccacacatct caggccctgt gtgagggagc acactgagat ggtgcaggag tgaatgggca 6300
   tggcttggcc tcgctacctc ggggacctgt tggagttctg gcagcagggt gtctgcaggt 6360
   gggacggcgt tctgggcaga gtcagaatgg tcagaatgaa acagaacagc caactcaccc 6420
   acaggacagc ttattttgag gcaaggtttt ggattttgga ggaagcagcc agatgaggcg 6480
   gtgagcctcc agaaggtcag cctttggagc acgtaagata ctgttacagg gtccagaaat 6540
   cgtgttcaca tgggggcttt gactcttcaa acagcttttg cagatcgtaa attgcatttg 6600
   cctagtcgtg tgacctcaaa agaagtcaga catatttaat ccagaaatag tttcgtttga 6660
   gggagggctt gcaggtctgt aaatagcatt tgctttcctg gttagagatt gggatgcaga 6720
   aggagttttc agtatttttt ttaaaacact aatgatcatt gaagagtatt tatgtaaaca 6780
   tacaacgtat aatgggtggg ggatccgatc atggtgatgt acggggtgaa ttctcttgcc 6840
   gtgttgcaaa tgtgtaaaat aaagattatc tggc 6874
<210> 71
   <211> 7018
   <212> DNA
   <213> Homo sapiens
<400> 71
   ccgaggtggg cagcacaggc tcctcgacga cttcctaggt cgcaatctcc aggaaaacga 60
   ccacagggtc agcggagcta gccgccgagc cccgctcccc gggcccttcc ggcggctgcg 120
   ccctttcacc ccggacgtgg gcgggagagg aagcggctgg tgatgctgga acaaacatgg 180
   ccgctctggc gcccgtcggc tcccccgcct cccgcggtcc taggctggcc gcgggcctcc 240
   ggctgctccc aatgctgggt ttgctgcagt tgctggccga gcctggcctg ggccgcgtcc 300
   atcacctggc actcaaggat gatgtgaggc ataaagttca tctgaacacc tttggcttct 360
   tcaaggatgg gtacatggtg gtgaatgtca gtagcctctc actgaatgag cctgaagaca 420
   aggatgtgac tattggattt agcctagacc gtacaaagaa tgatggcttt tcttcttacc 480
   tggatgaaga tgtgaattac tgtattttaa agaaacagtc tgtctctgtc acccttttaa 540
   tcctagacat ctccagaagt gaggtaagag taaagtctcc accagaagct ggtacccagt 600
   taccaaagat catcttcagc agggatgaga aagtccttgg tcagagccag gagcctaatg 660
   ttaaccctgc ttcagcaggc aaccagaccc agaagacaca agatggtgga aagtctaaaa 720
   gaagtacagt ggattcaaag gccatgggag agaaatcctt ttctgttcat aataatggtg 780
   gggcagtgtc atttcagttt ttctttaaca tcagcactga tgaccaagaa ggcctttaca 840
   gtctttattt tcataaatgc cttggaaaag aattgccaag tgacaagttt acattcagcc 900
   ttgatattga gatcacagag aagaatcctg acagctacct ctcagcagga gaaattcctc 960
   tccccaaatt atacatctca atggcctttt tcttctttct ttctgggacc atctggattc 1020
   atatccttcg aaaacgacgg aatgatgtat ttaaaatcca ctggctgatg gcggcccttc 1080
   ctttcaccaa gtctctttcc ttggtgttcc atgcaattga ctaccactac atctcctccc 1140
   agggcttccc tatcgaaggc tgggctgttg tgtactacat aactcacctt ttgaaagggg 1200
   cgctactctt catcaccatt gcactcattg gcactggctg ggctttcatt aagcacatcc 1260
   tttctgataa agacaaaaag atcttcatga ttgtcattcc actccaggtc ctggcaaatg 1320
   tagcctacat catcatagag tccaccgagg agggcacgac tgaatatggc ttgtggaagg 1380
   actctctatt tctggtcgac ctgttgtgtt gtggtgccat cctcttccca gtggtgtggt 1440
   caatcagaca tttacaagaa gcatcagcaa cagatggaaa aggtgacagc atgggacctc 1500
   ttcagcagag agcgaatcta agagcaggaa gtcgcataga gtctcaccat tttgcccagg 1560
   ctgatcttga actcctggcc tctagctgtc ctcctgcctc agtctcccaa agggctggga 1620
   ttacagctgc tattaactta gcaaagctga aacttttcag acattattac gtcttgattg 1680
   tgtgttacat atacttcact aggatcattg catttctcct caaactcgct gttccattcc 1740
   agtggaagtg gctctaccag ctcctggatg aaacggccac actggtcttc tttgttctaa 1800
   cggggtataa attccgtccg gcttcagata acccctacct acaactttct caggaagaag 1860
   aagacttgga aatggagtcc gttgtgacaa catctggggt gatggaaagt atgaagaaag 1920
   tcaagaaggt gaccaacggc tccgtggagc cccagggcga gtgggaaggc gccgtgtgac 1980
   agagccgacc ctgaggatgg cactgtccaa ggaaactgtt aacttattca tagtcctatt 2040
   ggacagcagg agcagctcct acagtgaact attggcacca ccgacagtga caccagggca 2100
   catggctgga gcacagtgcc gcggaaacct gattttgtac tctcttttat ggaaacgatc 2160
   tgtggctgtt tagaggcagc tggatcctct ttcaggcggg aatgggaggg cgggcacagg 2220
   gaggaggaga ggaagagaaa aggaagaatt catttttaat ttaggtttct ttttttcttc 2280
   ttcatttcgg agctctaagg tgtatgcagt tgtgacccca tgtgtgggga agtgtagcaa 2340
   ggacggctgg tggaggggga aggagggtgc gaggtgtctg tctgatgctt taggaaatgt 2400
   ctactgagga ccctgggact taagaagaag ggcggggaga gtgccattgc ctgtttggga 2460
   gacaaaaatg aacgaaaaca ggtgactttg gaaagcaaag tcaaaaccca gtttaggatg 2520
   tagcacctgc cccaggattc ctgccctcgg ctttgcccca gacccttatt ccagatgctg 2580
   agagtgacca ggacagcagc tcctgaggcc cagtggtctt ctttccaaca ggaaaagaag 2640
   gctgtgatgt cgctgtcagg atcatgccct gtggcacagc acaggtggtg ggaggtggtt 2700
   ttctgactga gatgttgcct gatggatgga aagaaatgta tttttaagtt caaaaagcat 2760
   tatcctgtgg cgttgcctgg acatccactc cctgacagcc cagagcagca ctgtctggct 2820
   tcccttcatg cttgtggctt tgttgtgttt gatcagaatt ttgggggaaa tggaaagttt 2880
   tcctcaagga gcagctgggg gcagaatagg tagtatttaa gcaaatactt aagtccaagc 2940
   aaatcatccc cattaaaaag cttttcctgt aggctagtag gatttctaaa tagatgaatt 3000
   caacagactt ggtccccata gtccaagagt atgtatgtga agaaagtgag catgattcaa 3060
   cagtttcact ctcagggatt ttaggatggc aaaatacttc acagaaactc aatgattaag 3120
   ttcccttcca cacttccaga gcttgaatga acacaggtag ccacctaaat tgagcagtat 3180
   tgcaactcag agagaaaatc atctgaatag taggacaagc tcagaaggta cattgtgact 3240
   gagggcttaa aaggagacca aaacatggcc ccatcaggga agcttcttaa tgcttggggg 3300
   gccagctagg tagggttgct tccaaaagct ggagcccacc cctgcctagg ggttgtcaga 3360
   gagccacacc tgcaggggaa caggtacctc cgagggtgag agtcgtggtc tctgggagtt 3420
   gttttctcac ctctggctta gaagggtcag gcagaaacca caggatgtgg ggtcacactc 3480
   actgtcccaa gtttgggaac ctgaaaaagt ctccattcag aacatggttg ttctccctgt 3540
   cccatgctat cttatcttcc taaatgacta atgaggaagc gggtgttctt tttctgcact 3600
   ttgattcgcc atctgggttc tgtagggtgc tctgaaggtg tgatctgcct tctggctgat 3660
   gtggaggaag agcaagcgcc ttcccaggcc acagctgctc acctctcggc agatatttta 3720
   ggcaagcatc cgtgtgtctt cccatcttca ggagaaaggt aaatgcaccc taagtgttca 3780
   cttctggacc tttttcaagt tcacttggga ctgtgtgaca gaagggagtt ggagggagga 3840
   tgggaatatt tttaacactt tgttttcctg tgcagaaaca taataccagt tttcgcagaa 3900
   atgtgtctca atctgtgact accaaagccc tcctcagtcc ttccctcaga gggacacatt 3960
   tgctgtttct cccgcaagca gatgttgtgg atgaggcgat agactccttg gcaagaacga 4020
   aaggtgtgat gaaacctccc tgctcggaag ggtctccgtg gaggtgtcct catttcacat 4080
   gctgggtttt gcaagcgagg aagccaggca gtggaggaac tagagagagg caggcgtgtg 4140
   tgtggacaag cgctggagcc gcagccctca gactggcacg ggaacgccag cgttgggtgt 4200
   tcagattcca cgcgtatgtc tgggctcact cacagcatgg ccgagtgtct gcagtgctgg 4260
   tcctgaccct tccagagcag cagtggacag atgagataag actgtttcag aaacaaagat 4320
   ggccacagcc ttcctaacaa gcaggtcatc tggccatgtc tgtattgtaa ctggtaaaag 4380
   gcttcaagtc agattgatga tcaagaaaag tcaaaacccc agcccaagat tgggaaagca 4440
   ggtggtggtt ccaagctttt aaaaaattat tgaagctctc catcctgttc tgtgagtgtg 4500
   tcttctcttt ctccttcacg tcatagccgt gacccaccgt tcatctctgc tcttgcgtaa 4560
   agatgaccga tggagtccaa agccaagtgg cttcaccagc tgacaagcca ccctcctgca 4620
   gcctgagttt cacagtccac tgggttcgtt gtcatgcggt gtttgaatgg ttaagccctt 4680
   gcagtatttc agatcgggca aaaaatatcg gatgcacata gcagaaccat tggtggtatt 4740
   tatagctttg ctttgtactc ctcactgttt ctgcctacgc aaaatatcca tgtttcctct 4800
   gagaaatctg ttgtggactg aaagcgctgc tggctgtgaa atttaataaa gtgtgtatgc 4860
   tttgctagaa aattatttct tggacaatag gaacagtcat tgatctgtaa atcctggctc 4920
   ttaacagtga gtggccaagg acttgatcag cccatttctt ggtccctcag tgctttaaaa 4980
   tttaagtagc actgcatttt gtaatgttga atatgactct agtgacttgt aggaggcact 5040
   tgtgaggaga tgcttgcttc agtgtaaaag atgctcatgg cctgagtcag ttgagttttc 5100
   tttcaagaaa ccacttcaga gtgaaatatc cagggtttcc ccgccctgga catgtccagc 5160
   ctgcccaggc agcacacagc cctgtaagtc cacctcgtgt gggtgagatt tcctcctgcg 5220
   tgatgacctc atcgccatct ctgctgtctc attccacagc ctccctccct cttctctcct 5280
   cctctgccct cgcccttccc ccttccccat cccctccccc tcctcctctg ccctcgccct 5340
   tacccctccc ccttcccctt ccgctcctcc tccctcctcc acctctttct cctcctcctt 5400
   ccctcctcct ccctcctctt cccttctctg ccatctttct ccccgtgcct attgatccca 5460
   cataggctca ttctgggtac accggctaaa ggctttggtg cattgcagcg ttttctccca 5520
   gcagctgtgt gaaagatgca ttttctaagc taaggagaat tttctcaaga gtggcatact 5580
   catgccaaat attattgctc tgggccatat aggctggtct tcctccacac taaaatgggt 5640
   gtcttgtttt ggtacttaaa acagtctact ccaggcatcc agtccttaca gaccaaggaa 5700
   gagcatagcg atgcctgttg gaattgcaga tgcattctgg ccttctcccc cgtcctgaaa 5760
   cattttcttt gaggaaggct cttagaacat tagatagtct gctgaggttg ttggcccagc 5820
   tccatacacc cagtagaaca gtggaacaac tcatgcttca tgctgccaag ctgctgtact 5880
   tcaaaggaaa cagatctagc acactgctgc acccctgctt ccacactcca cacttcaccc 5940
   cgctgctttt ctctgacccg cccctggcct tgtaagactc acgtaagcta agtccaggat 6000
   gcctgtggcc tgcggcttga ttcttccctt taggattcag caagttaatg gcttcctcgc 6060
   tatagaagtg agactttgac ttgatgcctc ttggtatatc aaaaagatat tcatccagaa 6120
   agtaccaaat gttctgaaag acccgctctt cactccagtt ttccctaggg tgtttctggc 6180
   agggcgtttt taaaaggcat ctacctgagt tgacgctaat acttgtcacc acctggaacg 6240
   tagttatcgg tcggcaggct gaacatactc cagattcccc agaggccact tctgtagccc 6300
   agcgatgcat ctgagcctct ctgcgtggtt tatgcttgaa aaatagataa tgcttttaga 6360
   tggttcactg ccaggccatg ggccccacac atctcaggcc ctgtgtgagg gagcacactg 6420
   agatggtgca ggagtgaatg ggcatggctt ggcctcgcta cctcggggac ctgttggagt 6480
   tctggcagca gggtgtctgc aggtgggacg gcgttctggg cagagtcaga atggtcagaa 6540
   tgaaacagaa cagccaactc acccacagga cagcttattt tgaggcaagg ttttggattt 6600
   tggaggaagc agccagatga ggcggtgagc ctccagaagg tcagcctttg gagcacgtaa 6660
   gatactgtta cagggtccag aaatcgtgtt cacatggggg ctttgactct tcaaacagct 6720
   tttgcagatc gtaaattgca tttgcctagt cgtgtgacct caaaagaagt cagacatatt 6780
   taatccagaa atagtttcgt ttgagggagg gcttgcaggt ctgtaaatag catttgcttt 6840
   cctggttaga gattgggatg cagaaggagt tttcagtatt ttttttaaaa cactaatgat 6900
   cattgaagag tatttatgta aacatacaac gtataatggg tgggggatcc gatcatggtg 6960
   atgtacgggg tgaattctct tgccgtgttg caaatgtgta aaataaagat tatctggc 7018
<210> 72
   <211> 7353
   <212> DNA
   <213> Homo sapiens
<400> 72
   attattgcaa ccatttcgct tgtatttgag tgtgaagcgc ctagaaaacc acaggacccc      60
   tacggcgagc cgggaatttt tagatatttt cctccgagtc aacgctcagt gaaatcagtt     120
   caatcagtgg cccgacaccg tgcggctgac acagcttatc ccccgaccct gagatcaggg     180
   gtccccggag cccaaggtcg cttccaaagc tcagcgaggc ggaggtgcgg cccgggctgg     240
   tctggttcgg ccaccgttgt tatggcaacc gccaataggt tggcttcatc tctaactgaa     300
   agtctgcaca ggagcggccg atcgaagggc cccgaggtgg gcagcacagg ctcctcgacg     360
   acttcctagg tcgcaatctc caggaaaacg accacagggt cagcggagct agccgccgag    420
   ccccgctccc cgggcccttc cggcggctgc gccctttcac cccggacgtg ggcgggagag    480
   gaagcggctg gtgatgctgg aacaaacatg gccgctctgg cgcccgtcgg ctcccccgcc    540
   tcccgcggtc ctaggctggc cgcgggcctc cggctgctcc caatgctggg tttgctgcag    600
   ttgctggccg agcctggcct gggccgcgtc catcacctgg cactcaagga tgatgtgagg    660
   cataaagttc atctgaacac ctttggcttc ttcaaggatg ggtacatggt ggtgaatgtc    720
   agtagcctct cactgaatga gcctgaagac aaggatgtga ctattggatt tagcctagac    780
   cgtacaaaga atgatggctt ttcttcttac ctggatgaag atgtgaatta ctgtatttta    840
   aagaaacagt ctgtctctgt caccctttta atcctagaca tctccagaag tgaggtaaga    900
   gtaaagtctc caccagaagc tggtacccag ttaccaaaga tcatcttcag cagggatgag    960
   aaagtccttg gtcagagcca ggagcctaat gttaaccctg cttcagcagg caaccagacc   1020
   cagaagacac aagatggtgg aaagtctaaa agaagtacag tggattcaaa ggccatggga   1080
   gagaaatcct tttctgttca taataatggt ggggcagtgt catttcagtt tttctttaac   1140
   atcagcactg atgaccaaga aggcctttac agtctttatt ttcataaatg ccttggaaaa   1200
   gaattgccaa gtgacaagtt tacattcagc cttgatattg agatcacaga gaagaatcct   1260
   gacagctacc tctcagcagg agaaattcct ctccccaaat tatacatctc aatggccttt   1320
   ttcttctttc tttctgggac catctggatt catatccttc gaaaacgacg gaatgatgta   1380
   tttaaaatcc actggctgat ggcggccctt cctttcacca agtctctttc cttggtgttc   1440
   catgcaattg actaccacta catctcctcc cagggcttcc ctatcgaagg ctgggctgtt   1500
   gtgtactaca taactcacct tttgaaaggg gcgctactct tcatcaccat tgcactcatt   1560
   ggcactggct gggctttcat taagcacatc ctttctgata aagacaaaaa gatcttcatg   1620
   attgtcattc cactccaggt cctggcaaat gtagcctaca tcatcataga gtccaccgag   1680
   gagggcacga ctgaatatgg cttgtggaag gactctctat ttctggtcga cctgttgtgt   1740
   tgtggtgcca tcctcttccc agtggtgtgg tcaatcagac atttacaaga agcatcagca   1800
   acagatggaa aaggtgacag catgggacct cttcagcaga gagcgaatct aagagcagga   1860
   agtcgcatag agtctcacca ttttgcccag gctgatcttg aactcctggc ctctagctgt   1920
   cctcctgcct cagtctccca aagggctggg attacagctg ctattaactt agcaaagctg   1980
   aaacttttca gacattatta cgtcttgatt gtgtgttaca tatacttcac taggatcatt   2040
   gcatttctcc tcaaactcgc tgttccattc cagtggaagt ggctctacca gctcctggat   2100
   gaaacggcca cactggtctt ctttgttcta acggggtata aattccgtcc ggcttcagat   2160
   aacccctacc tacaactttc tcaggaagaa gaagacttgg aaatggagtc cgttgtgaca   2220
   acatctgggg tgatggaaag tatgaagaaa gtcaagaagg tgaccaacgg ctccgtggag 2280
   ccccagggcg agtgggaagg cgccgtgtga cagagccgac cctgaggatg gcactgtcca 2340
   aggaaactgt taacttattc atagtcctat tggacagcag gagcagctcc tacagtgaac 2400
   tattggcacc accgacagtg acaccagggc acatggctgg agcacagtgc cgcggaaacc 2460
   tgattttgta ctctctttta tggaaacgat ctgtggctgt ttagaggcag ctggatcctc 2520
   tttcaggcgg gaatgggagg gcgggcacag ggaggaggag aggaagagaa aaggaagaat 2580
   tcatttttaa tttaggtttc tttttttctt cttcatttcg gagctctaag gtgtatgcag 2640
   ttgtgacccc atgtgtgggg aagtgtagca aggacggctg gtggaggggg aaggagggtg 2700
   cgaggtgtct gtctgatgct ttaggaaatg tctactgagg accctgggac ttaagaagaa 2760
   gggcggggag agtgccattg cctgtttggg agacaaaaat gaacgaaaac aggtgacttt 2820
   ggaaagcaaa gtcaaaaccc agtttaggat gtagcacctg ccccaggatt cctgccctcg 2880
   gctttgcccc agacccttat tccagatgct gagagtgacc aggacagcag ctcctgaggc 2940
   ccagtggtct tctttccaac aggaaaagaa ggctgtgatg tcgctgtcag gatcatgccc 3000
   tgtggcacag cacaggtggt gggaggtggt tttctgactg agatgttgcc tgatggatgg 3060
   aaagaaatgt atttttaagt tcaaaaagca ttatcctgtg gcgttgcctg gacatccact 3120
   ccctgacagc ccagagcagc actgtctggc ttcccttcat gcttgtggct ttgttgtgtt 3180
   tgatcagaat tttgggggaa atggaaagtt ttcctcaagg agcagctggg ggcagaatag 3240
   gtagtattta agcaaatact taagtccaag caaatcatcc ccattaaaaa gcttttcctg 3300
   taggctagta ggatttctaa atagatgaat tcaacagact tggtccccat agtccaagag 3360
   tatgtatgtg aagaaagtga gcatgattca acagtttcac tctcagggat tttaggatgg 3420
   caaaatactt cacagaaact caatgattaa gttcccttcc acacttccag agcttgaatg 3480
   aacacaggta gccacctaaa ttgagcagta ttgcaactca gagagaaaat catctgaata 3540
   gtaggacaag ctcagaaggt acattgtgac tgagggctta aaaggagacc aaaacatggc 3600
   cccatcaggg aagcttctta atgcttgggg ggccagctag gtagggttgc ttccaaaagc 3660
   tggagcccac ccctgcctag gggttgtcag agagccacac ctgcagggga acaggtacct 3720
   ccgagggtga gagtcgtggt ctctgggagt tgttttctca cctctggctt agaagggtca 3780
   ggcagaaacc acaggatgtg gggtcacact cactgtccca agtttgggaa cctgaaaaag 3840
   tctccattca gaacatggtt gttctccctg tcccatgcta tcttatcttc ctaaatgact 3900
   aatgaggaag cgggtgttct ttttctgcac tttgattcgc catctgggtt ctgtagggtg 3960
   ctctgaaggt gtgatctgcc ttctggctga tgtggaggaa gagcaagcgc cttcccaggc 4020
   cacagctgct cacctctcgg cagatatttt aggcaagcat ccgtgtgtct tcccatcttc 4080
   aggagaaagg taaatgcacc ctaagtgttc acttctggac ctttttcaag ttcacttggg 4140
   actgtgtgac agaagggagt tggagggagg atgggaatat ttttaacact ttgttttcct 4200
   gtgcagaaac ataataccag ttttcgcaga aatgtgtctc aatctgtgac taccaaagcc 4260
   ctcctcagtc cttccctcag agggacacat ttgctgtttc tcccgcaagc agatgttgtg 4320
   gatgaggcga tagactcctt ggcaagaacg aaaggtgtga tgaaacctcc ctgctcggaa 4380
   gggtctccgt ggaggtgtcc tcatttcaca tgctgggttt tgcaagcgag gaagccaggc 4440
   agtggaggaa ctagagagag gcaggcgtgt gtgtggacaa gcgctggagc cgcagccctc 4500
   agactggcac gggaacgcca gcgttgggtg ttcagattcc acgcgtatgt ctgggctcac 4560
   tcacagcatg gccgagtgtc tgcagtgctg gtcctgaccc ttccagagca gcagtggaca 4620
   gatgagataa gactgtttca gaaacaaaga tggccacagc cttcctaaca agcaggtcat 4680
   ctggccatgt ctgtattgta actggtaaaa ggcttcaagt cagattgatg atcaagaaaa 4740
   gtcaaaaccc cagcccaaga ttgggaaagc aggtggtggt tccaagcttt taaaaaatta 4800
   ttgaagctct ccatcctgtt ctgtgagtgt gtcttctctt tctccttcac gtcatagccg 4860
   tgacccaccg ttcatctctg ctcttgcgta aagatgaccg atggagtcca aagccaagtg 4920
   gcttcaccag ctgacaagcc accctcctgc agcctgagtt tcacagtcca ctgggttcgt 4980
   tgtcatgcgg tgtttgaatg gttaagccct tgcagtattt cagatcgggc aaaaaatatc 5040
   ggatgcacat agcagaacca ttggtggtat ttatagcttt gctttgtact cctcactgtt 5100
   tctgcctacg caaaatatcc atgtttcctc tgagaaatct gttgtggact gaaagcgctg 5160
   ctggctgtga aatttaataa agtgtgtatg ctttgctaga aaattatttc ttggacaata 5220
   ggaacagtca ttgatctgta aatcctggct cttaacagtg agtggccaag gacttgatca 5280
   gcccatttct tggtccctca gtgctttaaa atttaagtag cactgcattt tgtaatgttg 5340
   aatatgactc tagtgacttg taggaggcac ttgtgaggag atgcttgctt cagtgtaaaa 5400
   gatgctcatg gcctgagtca gttgagtttt ctttcaagaa accacttcag agtgaaatat 5460
   ccagggtttc cccgccctgg acatgtccag cctgcccagg cagcacacag ccctgtaagt 5520
   ccacctcgtg tgggtgagat ttcctcctgc gtgatgacct catcgccatc tctgctgtct 5580
   cattccacag cctccctccc tcttctctcc tcctctgccc tcgcccttcc cccttcccca 5640
   tcccctcccc ctcctcctct gccctcgccc ttacccctcc cccttcccct tccgctcctc 5700
   ctccctcctc cacctctttc tcctcctcct tccctcctcc tccctcctct tcccttctct 5760
   gccatctttc tccccgtgcc tattgatccc acataggctc attctgggta caccggctaa 5820
   aggctttggt gcattgcagc gttttctccc agcagctgtg tgaaagatgc attttctaag 5880
   ctaaggagaa ttttctcaag agtggcatac tcatgccaaa tattattgct ctgggccata 5940
   taggctggtc ttcctccaca ctaaaatggg tgtcttgttt tggtacttaa aacagtctac 6000
   tccaggcatc cagtccttac agaccaagga agagcatagc gatgcctgtt ggaattgcag 6060
   atgcattctg gccttctccc ccgtcctgaa acattttctt tgaggaaggc tcttagaaca 6120
   ttagatagtc tgctgaggtt gttggcccag ctccatacac ccagtagaac agtggaacaa 6180
   ctcatgcttc atgctgccaa gctgctgtac ttcaaaggaa acagatctag cacactgctg 6240
   cacccctgct tccacactcc acacttcacc ccgctgcttt tctctgaccc gcccctggcc 6300
   ttgtaagact cacgtaagct aagtccagga tgcctgtggc ctgcggcttg attcttccct 6360
   ttaggattca gcaagttaat ggcttcctcg ctatagaagt gagactttga cttgatgcct 6420
   cttggtatat caaaaagata ttcatccaga aagtaccaaa tgttctgaaa gacccgctct 6480
   tcactccagt tttccctagg gtgtttctgg cagggcgttt ttaaaaggca tctacctgag 6540
   ttgacgctaa tacttgtcac cacctggaac gtagttatcg gtcggcaggc tgaacatact 6600
   ccagattccc cagaggccac ttctgtagcc cagcgatgca tctgagcctc tctgcgtggt 6660
   ttatgcttga aaaatagata atgcttttag atggttcact gccaggccat gggccccaca 6720
   catctcaggc cctgtgtgag ggagcacact gagatggtgc aggagtgaat gggcatggct 6780
   tggcctcgct acctcgggga cctgttggag ttctggcagc agggtgtctg caggtgggac 6840
   ggcgttctgg gcagagtcag aatggtcaga atgaaacaga acagccaact cacccacagg 6900
   acagcttatt ttgaggcaag gttttggatt ttggaggaag cagccagatg aggcggtgag 6960
   cctccagaag gtcagccttt ggagcacgta agatactgtt acagggtcca gaaatcgtgt 7020
   tcacatgggg gctttgactc ttcaaacagc ttttgcagat cgtaaattgc atttgcctag 7080
   tcgtgtgacc tcaaaagaag tcagacatat ttaatccaga aatagtttcg tttgagggag 7140
   ggcttgcagg tctgtaaata gcatttgctt tcctggttag agattgggat gcagaaggag 7200
   ttttcagtat tttttttaaa acactaatga tcattgaaga gtatttatgt aaacatacaa 7260
   cgtataatgg gtgggggatc cgatcatggt gatgtacggg gtgaattctc ttgccgtgtt 7320
   gcaaatgtgt aaaataaaga ttatctggca gaa 7353
<210> 73
   <211> 431
   <212> PRT
   <213> Homo sapiens
<400> 73
   Met Arg Arg Leu Arg Arg Leu Ala His Leu Val Leu Phe Cys Pro Phe
   1 5 10 15
   Ser Lys Arg Leu Gln Gly Arg Leu Pro Gly Leu Arg Val Arg Cys Ile
                   20                  25                  30
   Phe Leu Ala Trp Leu Gly Val Phe Ala Gly Ser Trp Leu Val Tyr Val
           35                  40                  45
   His Tyr Ser Ser Tyr Ser Glu Arg Cys Arg Gly His Val Cys Gln Val
       50                  55                  60
   Val Ile Cys Asp Gln Tyr Arg Lys Gly Ile Ile Ser Gly Ser Val Cys
   65                  70                  75                  80
   Gln Asp Leu Cys Glu Leu His Met Val Glu Trp Arg Thr Cys Leu Ser
                   85                  90                  95
   Val Ala Pro Gly Gln Gln Val Tyr Ser Gly Leu Trp Arg Asp Lys Asp
               100                 105                 110
   Val Thr Ile Lys Cys Gly Ile Glu Glu Thr Leu Asp Ser Lys Ala Arg
           115                 120                 125
   Ser Asp Ala Ala Pro Arg Arg Glu Leu Val Leu Phe Asp Lys Pro Thr
       130                 135                 140
   Arg Gly Thr Ser Ile Lys Glu Phe Arg Glu Met Thr Leu Ser Phe Leu
   145                 150                 155                 160
   Lys Ala Asn Leu Gly Asp Leu Pro Ser Leu Pro Ala Leu Val Gly Gln
                   165                 170                 175
   Val Leu Leu Met Ala Asp Phe Asn Lys Asp Asn Arg Val Ser Leu Ala
               180                 185                 190
   Glu Ala Lys Ser Val Trp Ala Leu Leu Gln Arg Asn Glu Phe Leu Leu
           195                 200                 205
   Leu Leu Ser Leu Gln Glu Lys Glu His Ala Ser Arg Leu Leu Gly Tyr
       210                 215                 220
   Cys Gly Asp Leu Tyr Leu Thr Glu Gly Val Pro His Gly Ala Trp His
   225                 230                 235                 240
   Ala Ala Ala Leu Pro Pro Leu Leu Arg Pro Leu Leu Pro Pro Ala Leu
                   245                 250                 255
   Gln Gly Ala Leu Gln Gln Trp Leu Gly Pro Ala Trp Pro Trp Arg Ala
               260                 265                 270
   Lys Ile Ala Ile Gly Leu Leu Glu Phe Val Glu Glu Leu Phe His Gly
   275 280 285
   Ser Tyr Gly Thr Phe Tyr Met Cys Glu Thr Thr Leu Ala Asn Val Gly
   290 295 300
   Tyr Thr Ala Thr Tyr Asp Phe Lys Met Ala Asp Leu Gln Gln Val Ala
   305 310 315 320
   Pro Glu Ala Thr Val Arg Arg Phe Leu Gln Gly Arg Arg Cys Glu His
   325 330 335
   Ser Thr Asp Cys Thr Tyr Gly Arg Asp Cys Arg Ala Pro Cys Asp Arg
   340 345 350
   Leu Met Arg Gln Cys Lys Gly Asp Leu Ile Gln Pro Asn Leu Ala Lys
   355 360 365
   Val Cys Ala Leu Leu Arg Gly Tyr Leu Leu Pro Gly Ala Pro Ala Asp
   370 375 380
   Leu Arg Glu Glu Leu Gly Thr Gln Leu Arg Thr Cys Thr Thr Leu Ser
   385 390 395 400
   Gly Leu Ala Ser Gln Val Glu Ala His His Ser Leu Val Leu Ser His
   405 410 415
   Leu Lys Thr Leu Leu Trp Lys Lys Ile Ser Asn Thr Lys Tyr Ser
   420 425 430
<210> 74
   <211> 344
   <212> PRT
   <213> Homo sapiens
<400> 74
   Met Val Glu Trp Arg Thr Cys Leu Ser Val Ala Pro Gly Gln Gln Val
   1 5 10 15
   Tyr Ser Gly Leu Trp Arg Asp Lys Asp Val Thr Ile Lys Cys Gly Ile
   20 25 30
   Glu Glu Thr Leu Asp Ser Lys Ala Arg Ser Asp Ala Ala Pro Arg Arg
   35 40 45
   Glu Leu Val Leu Phe Asp Lys Pro Thr Arg Gly Thr Ser Ile Lys Glu
   50 55 60
   Phe Arg Glu Met Thr Leu Ser Phe Leu Lys Ala Asn Leu Gly Asp Leu
   65                  70                  75                  80
   Pro Ser Leu Pro Ala Leu Val Gly Gln Val Leu Leu Met Ala Asp Phe
                   85                  90                  95
   Asn Lys Asp Asn Arg Val Ser Leu Ala Glu Ala Lys Ser Val Trp Ala
               100                 105                 110
   Leu Leu Gln Arg Asn Glu Phe Leu Leu Leu Leu Ser Leu Gln Glu Lys
           115                 120                 125
   Glu His Ala Ser Arg Leu Leu Gly Tyr Cys Gly Asp Leu Tyr Leu Thr
       130                 135                 140
   Glu Gly Val Pro His Gly Ala Trp His Ala Ala Ala Leu Pro Pro Leu
   145                 150                 155                 160
   Leu Arg Pro Leu Leu Pro Pro Ala Leu Gln Gly Ala Leu Gln Gln Trp
                   165                 170                 175
   Leu Gly Pro Ala Trp Pro Trp Arg Ala Lys Ile Ala Ile Gly Leu Leu
               180                 185                 190
   Glu Phe Val Glu Glu Leu Phe His Gly Ser Tyr Gly Thr Phe Tyr Met
           195                 200                 205
   Cys Glu Thr Thr Leu Ala Asn Val Gly Tyr Thr Ala Thr Tyr Asp Phe
       210                 215                 220
   Lys Met Ala Asp Leu Gln Gln Val Ala Pro Glu Ala Thr Val Arg Arg
   225                 230                 235                 240
   Phe Leu Gln Gly Arg Arg Cys Glu His Ser Thr Asp Cys Thr Tyr Gly
                   245                 250                 255
   Arg Asp Cys Arg Ala Pro Cys Asp Arg Leu Met Arg Gln Cys Lys Gly
               260                 265                 270
   Asp Leu Ile Gln Pro Asn Leu Ala Lys Val Cys Ala Leu Leu Arg Gly
           275                 280                 285
   Tyr Leu Leu Pro Gly Ala Pro Ala Asp Leu Arg Glu Glu Leu Gly Thr
       290                 295                 300
   Gln Leu Arg Thr Cys Thr Thr Leu Ser Gly Leu Ala Ser Gln Val Glu
   305 310 315 320
   Ala His His Ser Leu Val Leu Ser His Leu Lys Thr Leu Leu Trp Lys
   325 330 335
   Lys Ile Ser Asn Thr Lys Tyr Ser
   340
<210> 75
   <211> 1645
   <212> DNA
   <213> Homo sapiens
<400> 75
   agggagcggc ggccgctgcg ggccgggccg ggccggggct gaggccgagc gagccgcggg 60
   gcccgcgcag ccccggccgg agcccaccat gcggcggctg cggcgcctgg cgcacctggt 120
   gctcttctgc cccttctcca agcgcctgca gggccggctc ccaggcctca gggtccgctg 180
   catcttcctg gcctggctgg gcgtctttgc aggcagctgg ctggtgtacg tgcactactc 240
   gtcctactcg gagcgctgtc gcggccatgt ctgccaggtg gtcatttgtg accagtaccg 300
   caaggggatc atctcgggct ccgtctgcca ggacctgtgt gagctgcata tggtggagtg 360
   gaggacctgc ctctcggtgg ccccgggcca gcaggtgtac agcgggctct ggcgggacaa 420
   ggatgtaacc atcaagtgtg gcattgagga gaccctcgac tccaaggccc ggtcggatgc 480
   ggcccccggg cgggagctgg tactgtttga caagcccacc cggggcacct ccatcaagga 540
   attccgggag atgaccctca gcttcctcaa ggcgaacctg ggagacctgc cttccctgcc 600
   ggcgctggtt ggccaggtcc tgctcatggc tgacttcaac aaggacaacc gggtgtccct 660
   ggcggaagcc aagtccgtgt gggccctgct gcagcgtaac gagttcctgc tgctgctgtc 720
   cctgcaggag aaggagcacg cctccagact gctgggctac tgtggggacc tctacctcac 780
   cgagggcgtg ccgcatggcg cctggcacgc ggccgccctt ccacccctgt tgcgcccact 840
   gctgccgcct gccctgcagg gtgctctcca gcagtggctg gggcctgcgt ggccttggcg 900
   ggccaagatc gccatcggcc tgctggagtt cgtggaggag ctcttccacg gctcttacgg 960
   gactttctac atgtgtgaga ccacactggc caacgtgggc tacacagcca cctacgactt 1020
   caagatggcc gacctgcagc aggtggcacc cgaggccacc gtgcgccgct tcctgcaggg 1080
   ccgccgctgc gagcacagca ccgactgcac ctacgggcgc gactgcaggg ccccgtgtga 1140
   caggctcatg aggcagtgca agggcgacct catccagccc aacctggcca aggtgtgcgc 1200
   actgctacgg ggctacctgc tgcctggcgc gcccgccgac ctccgcgagg agctgggcac 1260
   acagctgcgc acctgtacca cgctgagcgg gctggccagc caggtggagg cccatcactc 1320
   gctggtgctc agccacctca agactctgct ctggaagaag atctccaaca ccaagtactc 1380
   ttgatggggc agtgaggggc ctggccaccc ttcctggagc tggccaggtg ccagggtcca 1440
   accctccctc aaggaatcct gtcagaagat gtgaaatgca actgtgttgc aaaatcactc 1500
   ccctaccgtc agggctctgg attccagcac cacagacatg agaccccagc tcggagcaaa 1560
   ggcggacatg gacatcccgg caggagagtc ctccaagggg gtttgttact ctgaagaacg 1620
   taatgtcaat aaacagcttt tatgt 1645
<210> 76
   <211> 2410
   <212> DNA
   <213> Homo sapiens
<400> 76
   ggccgcagcc tgtcctcctg cctcagcctg gggaaggctg ggccgggcca aagagggagg     60
   ccaagtcctt gggacaggag gagacccaca cctgagatta gtggaaaccc agccagaagc    120
   tgccatgcag gtgtggctct gggcatcagg atctgtcgtg agagccccca tgagtgccca    180
   gtgcagaatg gctggcagcc cgccctgacg gggagcagag gggctggacg cggtgccctt    240
   cacgggatga cacccagctg tttgccctgt gtccaggggg ttgcttctct gacagaggcc    300
   ctatggctcg tgtctgactc ctgtccaggt tctgccagcc tgaccatcca tcgctctggc    360
   accaagagcc cacccttttg ttcttcctgg cgtcccaggg aaagccctgc ctgggtgggg     420
   cagctcctgg cccttcagat ggaagacgca gtccagtcag caccatcata ggaaacaagt    480
   tcagaaatgt ctcacttact attccgggca gggagggcgc catgagtcag ggggtgcatc     540
   ctccctcctg gcgtcacccg aggcaggaat gaagagtcag gcagagagcg cgcgtgtggc    600
   agctggtggt gtagatatta gggactagtg tgaattctag ttcaccggcc aatgcctgga    660
   tggtccagag ctgggtcggc tgggcggaga gctgcctcca ggttcctgcc tctggccctg    720
   gcgtggggtc gacactgggt gtggtgtgtg tctcatgtcc aggcagtggc ctttgctgtg    780
   ccgtcctgtt acaggagcca ggatggtggg gacgggaccg gaccggaggg ttggcggggc    840
   tgcccctgca gccgacagcc ccatcctgca gccaccaatg gcatgaccca gggccccggc     900
   actgcctgtg tgaggggctg gcagctttcc aactgcagca agtggaggcc cctgccagct     960
   tcgggcctgt gggcaggggc tcagtggggc aggggtgtgg ctgccccgcc cggcacgcct   1020
   gcacctgtct cctcagtgtg accagtaccg caaggggatc atctcgggct ccgtctgcca   1080
   ggacctgtgt gagctgcata tggtggagtg gaggacctgc ctctcggtgg ccccgggcca   1140
   gcaggtgtac agcgggctct ggcgggacaa ggatgtaacc atcaagtgtg gcattgagga   1200
   gaccctcgac tccaaggccc ggtcggatgc ggccccccgg cgggagctgg tactgtttga   1260
   caagcccacc cggggcacct ccatcaagga attccgggag atgaccctca gcttcctcaa   1320
   ggcgaacctg ggagacctgc cttccctgcc ggcgctggtt ggccaggtcc tgctcatggc 1380
   tgacttcaac aaggacaacc gggtgtccct ggcggaagcc aagtccgtgt gggccctgct 1440
   gcagcgtaac gagttcctgc tgctgctgtc cctgcaggag aaggagcacg cctccagact 1500
   gctgggctac tgtggggacc tctacctcac cgagggcgtg ccgcatggcg cctggcacgc 1560
   ggccgccctt ccacccctgt tgcgcccact gctgccgcct gccctgcagg gtgctctcca 1620
   gcagtggctg gggcctgcgt ggccttggcg ggccaagatc gccatcggcc tgctggagtt 1680
   cgtggaggag ctcttccacg gctcttacgg gactttctac atgtgtgaga ccacactggc 1740
   caacgtgggc tacacagcca cctacgactt caagatggcc gacctgcagc aggtggcacc 1800
   cgaggccacc gtgcgccgct tcctgcaggg ccgccgctgc gagcacagca ccgactgcac 1860
   ctacgggcgc gactgcaggg ccccgtgtga caggctcatg aggcagtgca agggcgacct 1920
   catccagccc aacctggcca aggtgtgcgc actgctacgg ggctacctgc tgcctggcgc 1980
   gcccgccgac ctccgcgagg agctgggcac acagctgcgc acctgtacca cgctgagcgg 2040
   gctggccagc caggtggagg cccatcactc gctggtgctc agccacctca agactctgct 2100
   ctggaagaag atctccaaca ccaagtactc ttgatggggc agtgaggggc ctggccaccc 2160
   ttcctggagc tggccaggtg ccagggtcca accctccctc aaggaatcct gtcagaagat 2220
   gtgaaatgca actgtgttgc aaaatcactc ccctaccgtc agggctctgg attccagcac 2280
   cacagacatg agaccccagc tcggagcaaa ggcggacatg gacatcccgg caggagagtc 2340
   ctccaagggg gtttgttact ctgaagaacg taatgtcaat aaacagcttt tatgtaatgc 2400
   ccagggctga 2410
<210> 77
   <211> 409
   <212> PRT
   <213> Homo sapiens
<400> 77
   Met Glu Glu Ser Trp Glu Ala Ala Pro Gly Gly Gln Ala Gly Ala Glu
   1               5                   10                  15
   Leu Pro Met Glu Pro Val Gly Ser Leu Val Pro Thr Leu Glu Gln Pro
               20                  25                  30
   Gln Val Pro Ala Lys Val Arg Gln Pro Glu Gly Pro Glu Ser Ser Pro
           35                  40                  45
   Ser Pro Ala Gly Ala Val Glu Lys Ala Ala Gly Ala Gly Leu Glu Pro
       50                  55                  60
   Ser Ser Lys Lys Lys Pro Pro Ser Pro Arg Pro Gly Ser Pro Arg Val
   65                  70                  75                  80
   Pro Pro Leu Ser Leu Gly Tyr Gly Val Cys Pro Glu Pro Pro Ser Pro
                   85                  90                  95
   Gly Pro Ala Leu Val Lys Leu Pro Arg Asn Gly Glu Ala Pro Gly Ala
               100                 105                 110
   Glu Pro Ala Pro Ser Ala Trp Ala Pro Met Glu Leu Gln Val Asp Val
           115                 120                 125
   Arg Val Lys Pro Val Gly Ala Ala Gly Gly Ser Ser Thr Pro Ser Pro
       130                 135                 140
   Arg Pro Ser Thr Arg Phe Leu Lys Val Pro Val Pro Glu Ser Pro Ala
   145                 150                 155                 160
   Phe Ser Arg His Ala Asp Pro Ala His Gln Leu Leu Leu Arg Ala Pro
                   165                 170                 175
   Ser Gln Gly Gly Thr Trp Gly Arg Arg Ser Pro Leu Ala Ala Ala Arg
               180                 185                 190
   Thr Glu Ser Gly Cys Asp Ala Glu Gly Arg Ala Ser Pro Ala Glu Gly
           195                 200                 205
   Ser Ala Gly Ser Pro Gly Ser Pro Thr Cys Cys Arg Cys Lys Glu Leu
       210                 215                 220
   Gly Leu Glu Lys Glu Asp Ala Ala Leu Leu Pro Arg Ala Gly Leu Asp
   225                 230                 235                 240
   Gly Asp Glu Lys Leu Pro Arg Ala Val Thr Leu Thr Gly Leu Pro Met
                   245                 250                 255
   Tyr Val Lys Ser Leu Tyr Trp Ala Leu Ala Phe Met Ala Val Leu Leu
               260                 265                 270
   Ala Val Ser Gly Val Val Ile Val Val Leu Ala Ser Arg Ala Gly Ala
           275                 280                 285
   Arg Cys Gln Gln Cys Pro Pro Gly Trp Val Leu Ser Glu Glu His Cys
       290                 295                 300
   Tyr Tyr Phe Ser Ala Glu Ala Gln Ala Trp Glu Ala Ser Gln Ala Phe
   305                 310                 315                 320
   Cys Ser Ala Tyr His Ala Thr Leu Pro Leu Leu Ser His Thr Gln Asp
                   325                 330                 335
   Phe Leu Gly Arg Tyr Pro Val Ser Arg His Ser Trp Val Gly Ala Trp
               340                 345                 350
   Arg Gly Pro Gln Gly Trp His Trp Ile Asp Glu Ala Pro Leu Pro Pro
           355                 360                 365
   Gln Leu Leu Pro Glu Asp Gly Glu Asp Asn Leu Asp Ile Asn Cys Gly
       370                 375                 380
   Ala Leu Glu Glu Gly Thr Leu Val Ala Ala Asn Cys Ser Thr Pro Arg
   385                 390                 395                 400
   Pro Trp Val Cys Ala Lys Gly Thr Gln
                   405
<210> 78
   <211> 314
   <212> PRT
   <213> Homo sapiens
<400> 78
   Met Glu Glu Ser Trp Glu Ala Ala Pro Gly Gly Gln Ala Gly Ala Glu
   1 5 10 15
   Leu Pro Met Glu Pro Val Gly Ser Leu Val Pro Thr Leu Glu Gln Pro
   20 25 30
   Gln Val Pro Ala Lys Val Arg Gln Pro Glu Gly Pro Glu Ser Ser Pro
   35 40 45
   Ser Pro Ala Gly Ala Val Glu Lys Ala Ala Gly Ala Gly Leu Glu Pro
   50 55 60
   Ser Ser Lys Lys Lys Pro Pro Ser Pro Arg Pro Gly Ser Pro Arg Val
   65 70 75 80
   Pro Pro Leu Ser Leu Gly Tyr Gly Val Cys Pro Glu Pro Pro Ser Pro
   85 90 95
   Gly Pro Ala Leu Val Lys Leu Pro Arg Asn Gly Glu Ala Pro Gly Ala
   100 105 110
   Glu Pro Ala Pro Ser Ala Trp Ala Pro Met Glu Leu Gln Val Asp Val
   115 120 125
   Arg Val Lys Pro Val Gly Ala Ala Gly Gly Ser Ser Thr Pro Ser Pro
   130 135 140
   Arg Pro Ser Thr Arg Phe Leu Lys Val Pro Val Pro Glu Ser Pro Ala
   145 150 155 160
   Phe Ser Arg His Ala Asp Pro Ala His Gln Leu Leu Leu Arg Ala Pro
   165 170 175
   Ser Gln Gly Gly Thr Trp Gly Arg Arg Ser Pro Leu Ala Ala Ala Arg
   180 185 190
   Thr Glu Ser Gly Cys Asp Ala Glu Gly Arg Ala Ser Pro Ala Glu Gly
   195 200 205
   Ser Ala Gly Ser Pro Gly Ser Pro Thr Cys Cys Arg Cys Lys Glu Leu
   210 215 220
   Gly Leu Glu Lys Glu Asp Ala Ala Leu Leu Pro Arg Ala Gly Leu Asp
   225 230 235 240
   Gly Asp Glu Lys Leu Pro Arg Ala Val Thr Leu Thr Asp Ser Leu Arg
   245 250 255
   Thr Ala Arg Thr Ile Trp Ile Ser Thr Val Gly Pro Trp Arg Lys Ala
   260 265 270
   Arg Trp Trp Leu Gln Thr Ala Ala Leu Gln Asp Pro Gly Ser Val Pro
   275 280 285
   Arg Gly Pro Ser Asp Leu Gly Ser Ala Trp Ser Ser Ala Cys Gln Ala
   290 295 300
   Asp Ala Ala Pro Pro Thr Gly Glu Ala Ser
   305 310
<210> 79
   <211> 1544
   <212> DNA
   <213> Homo sapiens
<400> 79
   gagagcgaag ctcctctgca ctgggcccag gtgcgctcct cagcgtctcc gggtggcggg 60
   gcgcgcggga tggaggagtc ttgggaggct gcgcccggag gccaagccgg ggcagagctc 120
   ccaatggagc ccgtgggaag cctggtcccc acgctggagc agccgcaggt gcccgcgaag 180
   gtgcgacaac ctgaaggtcc cgaaagcagc ccaagtccgg ccggggccgt ggagaaggcg 240
   gcgggcgcag gcctggagcc ctcgagcaag aaaaagccgc cttcgcctcg ccccgggtcc 300
   ccgcgcgtgc cgccgctcag cctgggctac ggggtctgcc ccgagccgcc gtcaccgggc 360
   cctgccttgg tcaagctgcc ccggaatggc gaggcgcccg gggctgagcc tgcgcccagc 420
   gcctgggcgc ccatggagct gcaggtagat gtgcgcgtga agcccgtggg cgcggccggt 480
   ggcagcagca cgccatcgcc caggccctcc acgcgcttcc tcaaggtgcc ggtgcccgag 540
   tcccctgcct tctcccgcca cgcggacccg gcgcaccagc tcctgctgcg cgcaccatcc 600
   cagggcggca cgtggggccg ccgctcgccg ctggctgcag cccggacgga gagcggctgc 660
   gacgcagagg gccgggccag ccccgcggaa ggaagcgccg gctccccggg ctcccccacg 720
   tgctgccgct gcaaggagct ggggctggag aaggaggatg cggcgctgtt gccccgcgcg 780
   gggttggacg gcgacgagaa gctgccccgg gccgtaacgc ttacggggct acccatgtac 840
   gtgaagtccc tgtactgggc cctggcgttc atggctgtgc tcctggcagt ctctggggtt 900
   gtcattgtgg tcctggcctc aagagcagga gccagatgcc agcagtgccc cccaggctgg 960
   gtgttgtccg aggagcactg ttactacttc tctgcagaag cgcaggcctg ggaagccagc 1020
   caggctttct gctcagccta ccacgctacc ctccccctgc taagccacac ccaggacttc 1080
   ctgggcagat acccagtctc caggcactcc tgggtggggg cctggcgagg cccccagggc 1140
   tggcactgga tcgacgaggc cccactcccg ccccagctac tccctgagga cggcgaggac 1200
   aatctggata tcaactgtgg ggccctggag gaaggcacgc tggtggctgc aaactgcagc 1260
   actccaagac cctgggtctg tgccaagggg acccagtgat ctgggctctg cctggtcctc 1320
   agcctgccag gcagatgcag caccccctac aggggaggcc agttgagagc ttgggcagcc 1380
   tcttcctgga cccagttatc caggtcttca tgctctgctc aagggggcca catgagcgag 1440
   cctaggagct ggacttcaac ccaggaagat gcatccgagg gaaaggagat tttctatggc 1500
   ctcaggcctg agtgccaata ttagtctcca gcttctgtgg atga 1544
<210> 80
   <211> 1192
   <212> DNA
   <213> Homo sapiens
<400> 80
   gagagcgaag ctcctctgca ctgggcccag gtgcgctcct cagcgtctcc gggtggcggg 60
   gcgcgcggga tggaggagtc ttgggaggct gcgcccggag gccaagccgg ggcagagctc 120
   ccaatggagc ccgtgggaag cctggtcccc acgctggagc agccgcaggt gcccgcgaag 180
   gtgcgacaac ctgaaggtcc cgaaagcagc ccaagtccgg ccggggccgt ggagaaggcg 240
   gcgggcgcag gcctggagcc ctcgagcaag aaaaagccgc cttcgcctcg ccccgggtcc 300
   ccgcgcgtgc cgccgctcag cctgggctac ggggtctgcc ccgagccgcc gtcaccgggc 360
   cctgccttgg tcaagctgcc ccggaatggc gaggcgcccg gggctgagcc tgcgcccagc 420
   gcctgggcgc ccatggagct gcaggtagat gtgcgcgtga agcccgtggg cgcggccggt 480
   ggcagcagca cgccatcgcc caggccctcc acgcgcttcc tcaaggtgcc ggtgcccgag 540
   tcccctgcct tctcccgcca cgcggacccg gcgcaccagc tcctgctgcg cgcaccatcc 600
   cagggcggca cgtggggccg ccgctcgccg ctggctgcag cccggacgga gagcggctgc 660
   gacgcagagg gccgggccag ccccgcggaa ggaagcgccg gctccccggg ctcccccacg 720
   tgctgccgct gcaaggagct ggggctggag aaggaggatg cggcgctgtt gccccgcgcg 780
   gggttggacg gcgacgagaa gctgccccgg gccgtaacgc ttacggactc cctgaggacg 840
   gcgaggacaa tctggatatc aactgtgggg ccctggagga aggcacgctg gtggctgcaa 900
   actgcagcac tccaagaccc tgggtctgtg ccaaggggac ccagtgatct gggctctgcc 960
   tggtcctcag cctgccaggc agatgcagca ccccctacag gggaggccag ttgagagctt 1020
   gggcagcctc ttcctggacc cagttatcca ggtcttcatg ctctgctcaa gggggccaca 1080
   tgagcgagcc taggagctgg acttcaaccc aggaagatgc atccgaggga aaggagattt 1140
   tctatggcct caggcctgag tgccaatatt agtctccagc ttctgtggat ga 1192
<210> 81
   <211> 518
   <212> PRT
   <213> Homo sapiens
<400> 81
   Met Leu Ala Ala Ala Ser Lys Tyr Arg His Gly Asn Met Val Phe Phe
   1               5                   10                  15
   Asp Val Leu Gly Leu Phe Val Ile Ala Tyr Pro Ser Arg Ile Gly Ser
               20                  25                  30
   Ile Ile Asn Tyr Met Val Val Met Gly Val Val Leu Tyr Leu Gly Lys
           35                  40                  45
   Lys Phe Leu Gln Pro Lys His Lys Thr Gly Asn Tyr Lys Lys Asp Phe
       50                  55                  60
   Leu Cys Gly Leu Gly Ile Thr Leu Ile Ser Trp Phe Thr Ser Leu Val
   65                  70                  75                  80
   Thr Val Leu Ile Ile Ala Val Phe Ile Ser Leu Ile Gly Gln Ser Leu
   85 90 95
   Ser Trp Tyr Asn His Phe Tyr Val Ser Val Cys Leu Tyr Gly Thr Ala
   100 105 110
   Thr Val Ala Lys Ile Ile Leu Ile His Thr Leu Ala Lys Arg Phe Tyr
   115 120 125
   Tyr Met Asn Ala Ser Ala Gln Tyr Leu Gly Glu Val Phe Phe Asp Ile
   130 135 140
   Ser Leu Phe Val His Cys Cys Phe Leu Val Thr Leu Thr Tyr Gln Gly
   145 150 155 160
   Leu Cys Ser Ala Phe Ile Ser Ala Val Trp Val Ala Phe Pro Leu Leu
   165 170 175
   Thr Lys Leu Cys Val His Lys Asp Phe Lys Gln His Gly Ala Gln Gly
   180 185 190
   Lys Phe Ile Ala Phe Tyr Leu Leu Gly Met Phe Ile Pro Tyr Leu Tyr
   195 200 205
   Ala Leu Tyr Leu Ile Trp Ala Val Phe Glu Met Phe Thr Pro Ile Leu
   210 215 220
   Gly Arg Ser Gly Ser Glu Ile Pro Pro Asp Val Val Leu Ala Ser Ile
   225 230 235 240
   Leu Ala Gly Cys Thr Met Ile Leu Ser Ser Tyr Phe Ile Asn Phe Ile
   245 250 255
   Tyr Leu Ala Lys Ser Thr Lys Lys Thr Met Leu Thr Leu Thr Leu Val
   260 265 270
   Cys Ala Ile Thr Phe Leu Leu Val Cys Ser Gly Thr Phe Phe Pro Tyr
   275 280 285
   Ser Ser Asn Pro Ala Asn Pro Lys Pro Lys Arg Val Phe Leu Gln His
   290 295 300
   Met Thr Arg Thr Phe His Asp Leu Glu Gly Asn Ala Val Lys Arg Asp
   305 310 315 320
   Ser Gly Ile Trp Ile Asn Gly Phe Asp Tyr Thr Gly Ile Ser His Ile
   325 330 335
   Thr Pro His Ile Pro Glu Ile Asn Asp Ser Ile Arg Ala His Cys Glu
               340                 345                 350
   Glu Asn Ala Pro Leu Cys Gly Phe Pro Trp Tyr Leu Pro Val His Phe
           355                 360                 365
   Leu Ile Arg Lys Asn Trp Tyr Leu Pro Ala Pro Glu Val Ser Pro Arg
       370                 375                 380
   Asn Pro Pro His Phe Arg Leu Ile Ser Lys Glu Gln Thr Pro Trp Asp
   385                 390                 395                 400
   Ser Ile Lys Leu Thr Phe Glu Ala Thr Gly Pro Ser His Met Ser Phe
                   405                 410                 415
   Tyr Val Arg Ala His Lys Gly Ser Thr Leu Ser Gln Trp Ser Leu Gly
               420                 425                 430
   Asn Gly Thr Pro Val Thr Ser Lys Gly Gly Asp Tyr Phe Val Phe Tyr
           435                 440                 445
   Ser His Gly Leu Gln Ala Ser Ala Trp Gln Phe Trp Ile Glu Val Gln
       450                 455                 460
   Val Ser Glu Glu His Pro Glu Gly Met Val Thr Val Ala Ile Ala Ala
   465                 470                 475                 480
   His Tyr Leu Ser Gly Glu Asp Lys Arg Ser Pro Gln Leu Asp Ala Leu
                   485                 490                 495
   Lys Glu Lys Phe Pro Asp Trp Thr Phe Pro Ser Ala Trp Val Cys Thr
               500                 505                 510
   Tyr Asp Leu Phe Val Phe
           515
<210> 82
   <211> 904
   <212> PRT
   <213> Homo sapiens
<400> 82
   Met Glu Trp Gly Ser Glu Ser Ala Ala Val Arg Arg His Arg Val Gly
   1               5                   10                  15
   Val Glu Arg Arg Glu Gly Ala Ala Ala Ala Pro Pro Pro Glu Arg Glu
               20                  25                  30
   Ala Arg Ala Gln Glu Pro Leu Val Asp Gly Cys Ser Gly Gly Gly Arg
   35 40 45
   Thr Arg Lys Arg Ser Pro Gly Gly Ser Gly Gly Ala Ser Arg Gly Ala
   50 55 60
   Gly Thr Gly Leu Ser Glu Val Arg Ala Ala Leu Gly Leu Ala Leu Tyr
   65 70 75 80
   Leu Ile Ala Leu Arg Thr Leu Val Gln Leu Ser Leu Gln Gln Leu Val
   85 90 95
   Leu Arg Gly Ala Ala Gly His Arg Gly Glu Phe Asp Ala Leu Gln Ala
   100 105 110
   Arg Asp Tyr Leu Glu His Ile Thr Ser Ile Gly Pro Arg Thr Thr Gly
   115 120 125
   Ser Pro Glu Asn Glu Ile Leu Thr Val His Tyr Leu Leu Glu Gln Ile
   130 135 140
   Lys Leu Ile Glu Val Gln Ser Asn Ser Leu His Lys Ile Ser Val Asp
   145 150 155 160
   Val Gln Arg Pro Thr Gly Ser Phe Ser Ile Asp Phe Leu Gly Gly Phe
   165 170 175
   Thr Ser Tyr Tyr Asp Asn Ile Thr Asn Val Val Val Lys Leu Glu Pro
   180 185 190
   Arg Asp Gly Ala Gln His Ala Val Leu Ala Asn Cys His Phe Asp Ser
   195 200 205
   Val Ala Asn Ser Pro Gly Ala Ser Asp Asp Ala Val Ser Cys Ser Val
   210 215 220
   Met Leu Glu Val Leu Arg Val Leu Ser Thr Ser Ser Glu Ala Leu His
   225 230 235 240
   His Ala Val Ile Phe Leu Phe Asn Gly Ala Glu Glu Asn Val Leu Gln
   245 250 255
   Ala Ser His Gly Phe Ile Thr Gln His Pro Trp Ala Ser Leu Ile Arg
   260 265 270
   Ala Phe Ile Asn Leu Glu Ala Ala Gly Val Gly Gly Lys Glu Leu Val
           275                 280                 285
   Phe Gln Thr Gly Pro Glu Asn Pro Trp Leu Val Gln Ala Tyr Val Ser
       290                 295                 300
   Ala Ala Lys His Pro Phe Ala Ser Val Val Ala Gln Glu Val Phe Gln
   305                 310                 315                 320
   Ser Gly Ile Ile Pro Ser Asp Thr Asp Phe Arg Ile Tyr Arg Asp Phe
                   325                 330                 335
   Gly Asn Ile Pro Gly Ile Asp Leu Ala Phe Ile Glu Asn Gly Tyr Ile
               340                 345                 350
   Tyr His Thr Lys Tyr Asp Thr Ala Asp Arg Ile Leu Thr Asp Ser Ile
           355                 360                 365
   Gln Arg Ala Gly Asp Asn Ile Leu Ala Val Leu Lys His Leu Ala Thr
       370                 375                 380
   Ser Asp Met Leu Ala Ala Ala Ser Lys Tyr Arg His Gly Asn Met Val
   385                 390                 395                 400
   Phe Phe Asp Val Leu Gly Leu Phe Val Ile Ala Tyr Pro Ser Arg Ile
                   405                 410                 415
   Gly Ser Ile Ile Asn Tyr Met Val Val Met Gly Val Val Leu Tyr Leu
               420                 425                 430
   Gly Lys Lys Phe Leu Gln Pro Lys His Lys Thr Gly Asn Tyr Lys Lys
           435                 440                 445
   Asp Phe Leu Cys Gly Leu Gly Ile Thr Leu Ile Ser Trp Phe Thr Ser
       450                 455                 460
   Leu Val Thr Val Leu Ile Ile Ala Val Phe Ile Ser Leu Ile Gly Gln
   465                 470                 475                 480
   Ser Leu Ser Trp Tyr Asn His Phe Tyr Val Ser Val Cys Leu Tyr Gly
                   485                 490                 495
   Thr Ala Thr Val Ala Lys Ile Ile Leu Ile His Thr Leu Ala Lys Arg
               500                 505                 510
   Phe Tyr Tyr Met Asn Ala Ser Ala Gln Tyr Leu Gly Glu Val Phe Phe
           515                 520                 525
   Asp Ile Ser Leu Phe Val His Cys Cys Phe Leu Val Thr Leu Thr Tyr
   530 535 540
   Gln Gly Leu Cys Ser Ala Phe Ile Ser Ala Val Trp Val Ala Phe Pro
   545 550 555 560
   Leu Leu Thr Lys Leu Cys Val His Lys Asp Phe Lys Gln His Gly Ala
   565 570 575
   Gln Gly Lys Phe Ile Ala Phe Tyr Leu Leu Gly Met Phe Ile Pro Tyr
   580 585 590
   Leu Tyr Ala Leu Tyr Leu Ile Trp Ala Val Phe Glu Met Phe Thr Pro
   595 600 605
   Ile Leu Gly Arg Ser Gly Ser Glu Ile Pro Pro Asp Val Val Leu Ala
   610 615 620
   Ser Ile Leu Ala Gly Cys Thr Met Ile Leu Ser Ser Tyr Phe Ile Asn
   625 630 635 640
   Phe Ile Tyr Leu Ala Lys Ser Thr Lys Lys Thr Met Leu Thr Leu Thr
   645 650 655
   Leu Val Cys Ala Ile Thr Phe Leu Leu Val Cys Ser Gly Thr Phe Phe
   660 665 670
   Pro Tyr Ser Ser Asn Pro Ala Asn Pro Lys Pro Lys Arg Val Phe Leu
   675 680 685
   Gln His Met Thr Arg Thr Phe His Asp Leu Glu Gly Asn Ala Val Lys
   690 695 700
   Arg Asp Ser Gly Ile Trp Ile Asn Gly Phe Asp Tyr Thr Gly Ile Ser
   705 710 715 720
   His Ile Thr Pro His Ile Pro Glu Ile Asn Asp Ser Ile Arg Ala His
   725 730 735
   Cys Glu Glu Asn Ala Pro Leu Cys Gly Phe Pro Trp Tyr Leu Pro Val
   740 745 750
   His Phe Leu Ile Arg Lys Asn Trp Tyr Leu Pro Ala Pro Glu Val Ser
   755 760 765
   Pro Arg Asn Pro Pro His Phe Arg Leu Ile Ser Lys Glu Gln Thr Pro
   770 775 780
   Trp Asp Ser Ile Lys Leu Thr Phe Glu Ala Thr Gly Pro Ser His Met
   785 790 795 800
   Ser Phe Tyr Val Arg Ala His Lys Gly Ser Thr Leu Ser Gln Trp Ser
   805 810 815
   Leu Gly Asn Gly Thr Pro Val Thr Ser Lys Gly Gly Asp Tyr Phe Val
   820 825 830
   Phe Tyr Ser His Gly Leu Gln Ala Ser Ala Trp Gln Phe Trp Ile Glu
   835 840 845
   Val Gln Val Ser Glu Glu His Pro Glu Gly Met Val Thr Val Ala Ile
   850 855 860
   Ala Ala His Tyr Leu Ser Gly Glu Asp Lys Arg Ser Pro Gln Leu Asp
   865 870 875 880
   Ala Leu Lys Glu Lys Phe Pro Asp Trp Thr Phe Pro Ser Ala Trp Val
   885 890 895
   Cys Thr Tyr Asp Leu Phe Val Phe
   900
<210> 83
   <211> 419
   <212> PRT
   <213> Homo sapiens
<400> 83
   Met Val Val Met Gly Val Val Leu Tyr Leu Gly Lys Lys Phe Leu Gln
   1 5 10 15
   Pro Lys His Lys Thr Gly Asn Tyr Lys Lys Asp Phe Leu Cys Gly Leu
   20 25 30
   Gly Ile Thr Leu Ile Ser Trp Phe Thr Ser Leu Val Thr Val Leu Ile
   35 40 45
   Ile Ala Val Phe Ile Ser Leu Ile Gly Gln Ser Leu Ser Trp Tyr Asn
   50 55 60
   His Phe Tyr Val Ser Val Cys Leu Tyr Gly Thr Ala Thr Val Ala Lys
   65 70 75 80
   Ile Ile Leu Ile His Thr Leu Ala Lys Arg Phe Tyr Tyr Met Asn Ala
                   85                  90                  95
   Ser Ala Gln Tyr Leu Gly Glu Val Phe Phe Asp Ile Ser Leu Phe Val
               100                 105                 110
   His Cys Cys Phe Leu Val Thr Leu Thr Tyr Gln Gly Leu Cys Ser Ala
           115                 120                 125
   Phe Ile Ser Ala Val Trp Val Ala Phe Pro Leu Leu Thr Lys Leu Cys
       130                 135                 140
   Val His Lys Asp Phe Lys Gln His Gly Ala Gln Gly Lys Phe Ile Ala
   145                 150                 155                 160
   Phe Tyr Leu Leu Gly Met Phe Ile Pro Tyr Leu Tyr Ala Leu Tyr Leu
                   165                 170                 175
   Ile Trp Ala Val Phe Glu Met Phe Thr Pro Ile Leu Gly Arg Ser Gly
               180                 185                 190
   Ser Glu Ile Pro Pro Asp Val Val Leu Ala Ser Ile Leu Ala Gly Cys
           195                 200                 205
   Thr Met Ile Leu Ser Ser Tyr Phe Ile Asn Phe Ile Tyr Leu Ala Lys
       210                 215                 220
   Ser Thr Lys Lys Thr Met Leu Thr Leu Thr Leu Val Cys Ala Ile Thr
   225                 230                 235                 240
   Phe Leu Leu Val Cys Ser Gly Thr Phe Phe Pro Tyr Ser Ser Asn Pro
                   245                 250                 255
   Ala Asn Pro Lys Pro Lys Arg Val Phe Leu Gln His Met Thr Arg Thr
               260                 265                 270
   Phe His Asp Leu Glu Gly Asn Ala Val Lys Arg Asp Ser Gly Ile Trp
           275                 280                 285
   Ile Asn Gly Phe Asp Tyr Thr Gly Ile Ser His Ile Thr Pro His Ile
       290                 295                 300
   Pro Glu Ile Asn Asp Ser Ile Arg Ala His Cys Glu Glu Asn Ala Pro
   305                 310                 315                 320
   Leu Cys Gly Phe Pro Trp Tyr Leu Pro Val His Phe Leu Ile Arg Lys
   325 330 335
   Asn Trp Tyr Leu Pro Ala Pro Glu Val Ser Pro Arg Asn Pro Pro His
   340 345 350
   Phe Arg Leu Ile Ser Lys Glu Gln Thr Pro Trp Asp Ser Ile Lys Leu
   355 360 365
   Thr Phe Glu Ala Thr Ala Cys Leu Pro Ile Leu Gln Ile Leu Asp Leu
   370 375 380
   Pro Ala Ser Thr Ile Met Thr Lys Pro Tyr Val Leu Leu Cys Ser Ser
   385 390 395 400
   Pro Gln Arg Val Asn Thr Phe Ser Val Val Ser Trp Gln Trp His Pro
   405 410 415
   Ser His Lys
<210> 84
   <211> 4974
   <212> DNA
   <213> Homo sapiens
<400> 84
   ggcgcgggga ccgggctgtc tgaggtgcgc gccgcgctgg ggctcgcgct ctacctgatc     60
   gcgctgcgga cgctggtgca gctctcgctg cagcagctcg tgctacgcgg ggccgctgga    120
   caccgcgggg agttcgacgc gctccaagcc agggattatc ttgaacacat aacctccatt    180
   ggccccagga ctacaggaag tccagaaaat gaaattctga ccgtgcacta ccttttggaa    240
   cagattaaac tgattgaagt gcaaagcaac agccttcata agatttcagt agatgtacaa    300
   cggcccacag gctcttttag cattgatttc ttgggaggtt ttacaagcta ttatgacaac    360
   atcaccaatg ttgtggtaaa gctggaaccc agagatggag cccagcatgc tgtcttggct    420
   aattgtcatt ttgactcagt agcaaactca ccaggccagt catggtttca ttactcagca    480
   cccctgggct agcttgattc gtgcattcat taacctagag gcagcaggtg taggagggaa    540
   agaacttgta ttccaaacag gtcctgaaaa tccttggttg gttcaagctt atgtttcagc    600
   agctaaacac ccttttgctt ctgtggtggc tcaggaggtt tttcagagtg gaatcattcc    660
   ttcagatact gactttcgta tctacaggga ttttgggaac attccaggaa tagacttagc    720
   ttttattgag aatggataca tttatcacac caagtatgac acagcggaca gaattctaac    780
   agattccatt cagagagcag gtgacaacat tttagcagtt cttaagcatc tagctacatc    840
   tgatatgctg gctgctgctt ctaagtatcg acatggaaac atggtcttct ttgatgtgct    900
   gggcctgttt gtcattgcct acccctctcg tattggctca atcataaact acatggtggt    960
   aatgggtgtt gttttgtacc tgggcaaaaa atttttgcag cccaaacata agactggtaa   1020
   ctacaagaag gacttcttgt gtggacttgg catcactttg atcagctggt tcactagcct   1080
   tgttaccgtt ctcattatag cagtgttcat ctctcttatt ggacagtctc tctcatggta   1140
   taaccacttc tatgtctccg tttgtctgta tggaactgca actgtagcca aaataatact   1200
   tatacatact cttgcgaaaa gattttatta catgaatgcc agtgcccagt atctgggaga   1260
   agtatttttt gacatttcgc tgtttgtcca ttgctgtttt cttgttaccc tcacttacca   1320
   aggactttgc tcggcgttta ttagtgctgt ctgggtagca ttcccattgc tcacaaagct   1380
   ctgtgtgcat aaggacttca agcagcatgg tgcccaagga aaatttattg ctttttacct   1440
   tttggggatg tttattcctt atctttatgc attgtacctc atctgggcag tatttgagat   1500
   gtttacccct atcctcggga gaagtggttc tgaaatccca cctgatgttg tgctggcatc   1560
   cattttggct ggctgtacaa tgattctctc gtcctatttt attaacttca tctaccttgc   1620
   caagagcaca aaaaaaacca tgctaacttt aactttggta tgtgcaatta cattcctcct   1680
   tgtttgcagt ggaacatttt ttccatatag ctccaatcct gctaatccga agccaaagag   1740
   agtgtttctt cagcatatga ctagaacatt ccatgacttg gaaggaaatg cagttaaacg   1800
   ggactctgga atatggatca atgggtttga ttatactgga atttctcaca taacccctca   1860
   cattcctgag atcaatgata gtatccgagc tcactgtgag gagaatgcac ctctttgtgg   1920
   ttttccttgg tatcttccag tgcactttct gatcaggaaa aactggtatc ttcctgcccc   1980
   agaagtttct ccaagaaatc ctcctcattt ccgactcata tccaaagaac agacaccttg   2040
   ggattctata aaattgactt ttgaagcaac aggaccaagc catatgtcct tctatgttcg   2100
   agcccacaaa gggtcaacac tttctcagtg gtctcttggc aatggcaccc cagtcacaag   2160
   taaaggagga gactactttg tcttttactc ccatggactc caggcctctg catggcagtt   2220
   ctggatagaa gtgcaggttt cagaagaaca tcctgaagga atggtcaccg tggccattgc   2280
   tgcccactat ctgtctgggg aagacaagag atcccctcaa ctggatgctc tgaaggaaaa   2340
   gttcccagat tggacatttc cctctgcctg ggtgtgcacc tacgatctct ttgtatttta   2400
   atcttgtgga tgagctctaa gtacatgccc agtggatact ccatgtgaca tggtttctcc   2460
   ctatgttacg tggatgtttg taacgtaagt caatgaattt taatgatcat atgttcaaag   2520
   agctttctgg gttaacgctt ttcagggcca agcactataa gggtttagct gtggcgcagt   2580
   gatgcatggc ctgttgacac ttgaaaatgc cagtcttttg gcacttcagc acatgtgggt   2640
   actgccacta cacacacgtc attttatatg accttaagga caaagccaac aatccacttc   2700
   aatagctgcc cctttaggat caagaaagat gtacactgtc agagcattgt taatgagaca   2760
   aaagttgttt ccaatttaag ccccaaaacc atttgttgta ttagtggatg gtgggtaaaa 2820
   tatcattcac tgaggtaatg attccccttg agaatataac tctgtgtagg tcactggaaa 2880
   gtgattgcca tagggctggg agagaagcat tgcactcttg aggctgtagc ctgtgtcaag 2940
   ctgtttcttc aggcagcctc tcaaatgtgc tttgtctctc tgtgctgagg cctggaccct 3000
   gtgctgagct ggtgactcac tgtcctgaca agtggacaca cagatgcact gctgtgctgc 3060
   tttcctgagg tggttttcta tgcctgtttt cctctgaaac atgtctgtta cccctctcca 3120
   tcttaccaag ttgaaaaggg gaatatttgg ccacataccc ctctggtttt cgtaggttct 3180
   tttggttcag aatattgttt gtgccagtac atgaccttaa cttccttcct cagagcactg 3240
   agctgccatc tgggctattc tggggtagaa ggaaggctgg gagtggtggg aattttataa 3300
   atatttattc tcttttcttt gtttcatagg agtcttgtgt tatacaaggt tagtccttca 3360
   tggtataatc ttactgatgc actgggccta tctttttgtt ttccagccag ttgaatagat 3420
   tagtttttct cagtaactta ctatccagca gactggcttt cctgagactt gaggttgtgg 3480
   cttatactgg aatgagacca ctgtacgtgt aggtggttca gatcctgcgt aatggcagca 3540
   tgaggactta aaaggtggtt ttcattttga agatggctat gtagcttgta aggtgtatca 3600
   cagcagtacc tctcatggct ttttggttcc agcagtgagg gcattggtga gatcaatggt 3660
   aaactgtgca agctttcttt ttatcattag gaaatgtgaa acgttggaca aattttgagt 3720
   tttaacaagg acaaaaagtt gaaagaaaag gcacagttaa caaaaaaggg tggctagatt 3780
   tatcttgggt gatggaggaa atgagagagg aatgctcttg aaaggtggtc tgtggatctg 3840
   tctgaataga aagagcacag taagtatgca ttgccggaga aaacgtcctt gaagctgctt 3900
   gtctcatgtg tatgatgtgc tttttaaatc atgcccctcg ttgcctgcct aatctgtgac 3960
   tccctaaaaa ctaactgggc ccatgtagat ggggctgcaa ccagagctga ataacatgtt 4020
   aggctcacac atgcatcagc actgcacact ggaatcattg ctcttcctgg actttgtaga 4080
   aatcagtctc aagtgcttca agagtctggc tcctgctact tttatctgtc aggtagcaca 4140
   taaggtttgc agggtttata ttttgtatag aatcacagtt gtggagaaaa agtaataatt 4200
   tctcaatgaa ttttaaaaat gggcctattt tctatccccg tggttcatct gatataatta 4260
   gtgttccctg tgaattcccc ccctctatgg gaaggatgcc tttactcttt atcagtaata 4320
   aattatgact gttttcatat tgccttaggg ttatttccct gtgtaaacca ttgtcttttg 4380
   ttttggtttt ctttagcatt atgaagcttt ggtattgtac aaggtcagta gtaagatgct 4440
   cactagtctc agggcttgtg taatattctg ggaggtcatt taaatgccag aaatggtcaa 4500
   gcaattatac acagtattta tgactctgtt aagcataccg tttgtctgtc acattagtag 4560
   attctgagat taaaaaaaat ttttaaagag tgatcattta aataatttct aaaagggtct 4620
   tttcaagctc taacaaagtc actaacaaat gcattatttt ctacagaatt agatgttagt 4680
   agtacagtac tgcatattca gggaaaaagt gtgaggaatt gatttcaaaa tagttcgttc 4740
   ttgtgtttga cctaagaatg attgtcgcat gaagtgtttg tttttacagt ttagcatata 4800
   taaacaaaca tgataggatt ccttaagatg ttaccaccca gggggccaca agccagcctg 4860
   ctgtctcagg aagctgtaga aggagtgttt gtcaatttct tgtcactggt ttgctgactt 4920
   actgaggatt aattgttgcc ttacaatgtt actgaaataa actgtttaat atac 4974
<210> 85
   <211> 5338
   <212> DNA
   <213> Homo sapiens
<400> 85
   ggccggggct gtcgcgggtt ggggcggttg ggctggcagc tgaggctcgt ggccatggag 60
   tggggttctg agtcggctgc tgtgaggcgg caccgcgtcg gagtagagcg tcgagaggga 120
   gcggcggccg cgccaccgcc ggagagggag gcccgagcgc aggagcctct ggtggatggg 180
   tgcagcggcg gcgggaggac gcggaagagg agccccgggg gtagcggcgg cgcgagcagg 240
   ggcgcgggga ccgggctgtc tgaggtgcgc gccgcgctgg ggctcgcgct ctacctgatc 300
   gcgctgcgga cgctggtgca gctctcgctg cagcagctcg tgctacgcgg ggccgctgga 360
   caccgcgggg agttcgacgc gctccaagcc agggattatc ttgaacacat aacctccatt 420
   ggccccagga ctacaggaag tccagaaaat gaaattctga ccgtgcacta ccttttggaa 480
   cagattaaac tgattgaagt gcaaagcaac agccttcata agatttcagt agatgtacaa 540
   cggcccacag gctcttttag cattgatttc ttgggaggtt ttacaagcta ttatgacaac 600
   atcaccaatg ttgtggtaaa gctggaaccc agagatggag cccagcatgc tgtcttggct 660
   aattgtcatt ttgactcagt agcaaactca ccaggtgcca gtgatgatgc agttagctgc 720
   tcagtgatgc tggaagtcct tcgcgtcttg tcaacatctt cagaagcctt gcatcatgct 780
   gtcatatttc tctttaatgg tgctgaggaa aatgtcttgc aagccagtca tggtttcatt 840
   actcagcacc cctgggctag cttgattcgt gcattcatta acctagaggc agcaggtgta 900
   ggagggaaag aacttgtatt ccaaacaggt cctgaaaatc cttggttggt tcaagcttat 960
   gtttcagcag ctaaacaccc ttttgcttct gtggtggctc aggaggtttt tcagagtgga 1020
   atcattcctt cagatactga ctttcgtatc tacagggatt ttgggaacat tccaggaata 1080
   gacttagctt ttattgagaa tggatacatt tatcacacca agtatgacac agcggacaga 1140
   attctaacag attccattca gagagcaggt gacaacattt tagcagttct taagcatcta 1200
   gctacatctg atatgctggc tgctgcttct aagtatcgac atggaaacat ggtcttcttt 1260
   gatgtgctgg gcctgtttgt cattgcctac ccctctcgta ttggctcaat cataaactac 1320
   atggtggtaa tgggtgttgt tttgtacctg ggcaaaaaat ttttgcagcc caaacataag 1380
   actggtaact acaagaagga cttcttgtgt ggacttggca tcactttgat cagctggttc 1440
   actagccttg ttaccgttct cattatagca gtgttcatct ctcttattgg acagtctctc 1500
   tcatggtata accacttcta tgtctccgtt tgtctgtatg gaactgcaac tgtagccaaa 1560
   ataatactta tacatactct tgcgaaaaga ttttattaca tgaatgccag tgcccagtat 1620
   ctgggagaag tattttttga catttcgctg tttgtccatt gctgttttct tgttaccctc 1680
   acttaccaag gactttgctc ggcgtttatt agtgctgtct gggtagcatt cccattgctc 1740
   acaaagctct gtgtgcataa ggacttcaag cagcatggtg cccaaggaaa atttattgct 1800
   ttttaccttt tggggatgtt tattccttat ctttatgcat tgtacctcat ctgggcagta 1860
   tttgagatgt ttacccctat cctcgggaga agtggttctg aaatcccacc tgatgttgtg 1920
   ctggcatcca ttttggctgg ctgtacaatg attctctcgt cctattttat taacttcatc 1980
   taccttgcca agagcacaaa aaaaaccatg ctaactttaa ctttggtatg tgcaattaca 2040
   ttcctccttg tttgcagtgg aacatttttt ccatatagct ccaatcctgc taatccgaag 2100
   ccaaagagag tgtttcttca gcatatgact agaacattcc atgacttgga aggaaatgca 2160
   gttaaacggg actctggaat atggatcaat gggtttgatt atactggaat ttctcacata 2220
   acccctcaca ttcctgagat caatgatagt atccgagctc actgtgagga gaatgcacct 2280
   ctttgtggtt ttccttggta tcttccagtg cactttctga tcaggaaaaa ctggtatctt 2340
   cctgccccag aagtttctcc aagaaatcct cctcatttcc gactcatatc caaagaacag 2400
   acaccttggg attctataaa attgactttt gaagcaacag gaccaagcca tatgtccttc 2460
   tatgttcgag cccacaaagg gtcaacactt tctcagtggt ctcttggcaa tggcacccca 2520
   gtcacaagta aaggaggaga ctactttgtc ttttactccc atggactcca ggcctctgca 2580
   tggcagttct ggatagaagt gcaggtttca gaagaacatc ctgaaggaat ggtcaccgtg 2640
   gccattgctg cccactatct gtctggggaa gacaagagat cccctcaact ggatgctctg 2700
   aaggaaaagt tcccagattg gacatttccc tctgcctggg tgtgcaccta cgatctcttt 2760
   gtattttaat cttgtggatg agctctaagt acatgcccag tggatactcc atgtgacatg 2820
   gtttctccct atgttacgtg gatgtttgta acgtaagtca atgaatttta atgatcatat 2880
   gttcaaagag ctttctgggt taacgctttt cagggccaag cactataagg gtttagctgt 2940
   ggcgcagtga tgcatggcct gttgacactt gaaaatgcca gtcttttggc acttcagcac 3000
   atgtgggtac tgccactaca cacacgtcat tttatatgac cttaaggaca aagccaacaa 3060
   tccacttcaa tagctgcccc tttaggatca agaaagatgt acactgtcag agcattgtta 3120
   atgagacaaa agttgtttcc aatttaagcc ccaaaaccat ttgttgtatt agtggatggt   3180
   gggtaaaata tcattcactg aggtaatgat tccccttgag aatataactc tgtgtaggtc   3240
   actggaaagt gattgccata gggctgggag agaagcattg cactcttgag gctgtagcct   3300
   gtgtcaagct gtttcttcag gcagcctctc aaatgtgctt tgtctctctg tgctgaggcc   3360
   tggaccctgt gctgagctgg tgactcactg tcctgacaag tggacacaca gatgcactgc   3420
   tgtgctgctt tcctgaggtg gttttctatg cctgttttcc tctgaaacat gtctgttacc   3480
   cctctccatc ttaccaagtt gaaaagggga atatttggcc acatacccct ctggttttcg   3540
   taggttcttt tggttcagaa tattgtttgt gccagtacat gaccttaact tccttcctca   3600
   gagcactgag ctgccatctg ggctattctg ggtagaaggg aaggctggga gtggtgggaa   3660
   ttttataaat atttattctc ttttctttgt ttcataggag tcttgtgtta tacaaggtta   3720
   gtccttcatg gtataatctt actgatgcac tgggcctatc tttttgtttt ccagccagtt   3780
   gaatagatta gtttttctca gtaacttact atccagcaga ctggctttcc tgagacttga   3840
   ggttgtggct tatactggaa tgagaccact gtacgtgtag gtggttcaga tcctgcgtaa   3900
   tggcagcatg aggacttaaa aggtggtttt cattttgaag atggctatgt agcttgtaag   3960
   gtgtatcaca gcagtacctc tcatggcttt ttggttccag cagtgagggc attggtgaga   4020
   tcaatggtaa actgtgcaag ctttcttttt atcattagga aatgtgaaac gttggacaaa   4080
   ttttgagttt taacaaggac aaaaagttga aagaaaaggc acagttaaca aaaaagggtg   4140
   gctagattta tcttgggtga tggaggaaat gagagaggaa tgctcttgaa aggtggtctg   4200
   tggatctgtc tgaatagaaa gagcacagta agtatgcatt gccggagaaa acgtccttga   4260
   agctgcttgt ctcatgtgta tgatgtgctt tttaaatcat gcccctcgtt gcctgcctaa   4320
   tctgtgactc cctaaaaact aactgggccc atgtagatgg ggctgcaacc agagctgaat   4380
   aacatgttag gctcacacat gcatcagcac tgcacactgg aatcattgct cttcctggac   4440
   tttgtagaaa tcagtctcaa gtgcttcaag agtctggctc ctgctacttt tatctgtcag   4500
   gtagcacata aggtttgcag ggtttatatt ttgtatagaa tcacagttgt ggagaaaaag   4560
   taataatttc tcaatgaatt ttaaaaatgg gcctattttc tatccccgtg gttcatctga   4620
   tataattagt gttccctgtg aattcccccc ctctatggga aggatgcctt tactctttat   4680
   cagtaataaa ttatgactgt tttcatattg ccttagggtt atttccctgt gtaaaccatt   4740
   gtcttttgtt ttggttttct ttagcattat gaagctttgg tattgtacaa ggtcagtagt   4800
   aagatgctca ctagtctcag ggcttgtgta atattctggg aggtcattta aatgccagaa   4860
   atggtcaagc aattatacac agtatttatg actctgttaa gcataccgtt tgtctgtcac   4920
   attagtagat tctgagatta aaaaaaattt ttaaagagtg atcatttaaa taatttctaa   4980
   aagggtcttt tcaagctcta acaaagtcac taacaaatgc attattttct acagaattag 5040
   atgttagtag tacagtactg catattcagg gaaaaagtgt gaggaattga tttcaaaata 5100
   gttcgttctt gtgtttgacc taagaatgat tgtcgcatga agtgtttgtt tttacagttt 5160
   agcatatata aacaaacatg ataggattcc ttaagatgtt accacccagg gggccacaag 5220
   ccagcctgct gtctcaggaa gctgtagaag gagtgtttgt caatttcttg tcactggttt 5280
   gctgacttac tgaggattaa ttgttgcctt acaatgttac tgaaataaac tgtttaat 5338
<210> 86
   <211> 5387
   <212> DNA
   <213> Homo sapiens
<400> 86
   ggccggggct gtcgcgggtt ggggcggttg ggctggcagc tgaggctcgt ggccatggag 60
   tggggttctg agtcggctgc tgtgaggcgg caccgcgtcg gagtagagcg tcgagaggga 120
   gcggcggccg cgccaccgcc ggagagggag gcccgagcgc aggagcctct ggtggatggg 180
   tgcagcggcg gcgggaggac gcggaagagg agccccgggg gtagcggcgg cgcgagcagg 240
   ggcgcgggga ccgggctgtc tgaggtgcgc gccgcgctgg ggctcgcgct ctacctgatc 300
   gcgctgcgga cgctggtgca gctctcgctg cagcagctcg tgctacgcgg ggccgctgga 360
   caccgcgggg agttcgacgc gctccaagcc agggattatc ttgaacacat aacctccatt 420
   ggccccagga ctacaggaag tccagaaaat gaaattctga ccgtgcacta ccttttggaa 480
   cagattaaac tgattgaagt gcaaagcaac agccttcata agatttcagt agatgtacaa 540
   cggcccacag gctcttttag cattgatttc ttgggaggtt ttacaagcta ttatgacaac 600
   atcaccaatg ttgtggtaaa gctggaaccc agagatggag cccagcatgc tgtcttggct 660
   aattgtcatt ttgactcagt agcaaactca ccaggtgcca gtgatgatgc agttagctgc 720
   tcagtgatgc tggaagtcct tcgcgtcttg tcaacatctt cagaagcctt gcatcatgct 780
   gtcatatttc tctttaatgg tgctgaggaa aatgtcttgc aagccagtca tggtttcatt 840
   actcagcacc cctgggctag cttgattcgt gcattcatta acctagaggc agcaggtgta 900
   ggagggaaag aacttgtatt ccaaacaggt cctgaaaatc cttggttggt tcaagcttat 960
   gtttcagcag ctaaacaccc ttttgcttct gtggtggctc aggaggtttt tcagagtgga 1020
   atcattcctt cagatactga ctttcgtatc tacagggatt ttgggaacat tccaggaata 1080
   gacttagctt ttattgagaa tggatacatt tatcacacca agtatgacac agcggacaga 1140
   attctaacag attccattca gagagcaggt gacaacattt tagcagttct taagcatcta 1200
   gctacatctg atatgctggc tgctgcttct aagtatcgac atggaaacat ggtcttcttt 1260
   gatgtgctgg gcctgtttgt cattgcctac ccctctcgta ttggctcaat cataaactac 1320
   atggtggtaa tgggtgttgt tttgtacctg ggcaaaaaat ttttgcagcc caaacataag 1380
   actggtaact acaagaagga cttcttgtgt ggacttggca tcactttgat cagctggttc 1440
   actagccttg ttaccgttct cattatagca gtgttcatct ctcttattgg acagtctctc 1500
   tcatggtata accacttcta tgtctccgtt tgtctgtatg gaactgcaac tgtagccaaa 1560
   ataatactta tacatactct tgcgaaaaga ttttattaca tgaatgccag tgcccagtat 1620
   ctgggagaag tattttttga catttcgctg tttgtccatt gctgttttct tgttaccctc 1680
   acttaccaag gactttgctc ggcgtttatt agtgctgtct gggtagcatt cccattgctc 1740
   acaaagctct gtgtgcataa ggacttcaag cagcatggtg cccaaggaaa atttattgct 1800
   ttttaccttt tggggatgtt tattccttat ctttatgcat tgtacctcat ctgggcagta 1860
   tttgagatgt ttacccctat cctcgggaga agtggttctg aaatcccacc tgatgttgtg 1920
   ctggcatcca ttttggctgg ctgtacaatg attctctcgt cctattttat taacttcatc 1980
   taccttgcca agagcacaaa aaaaaccatg ctaactttaa ctttggtatg tgcaattaca 2040
   ttcctccttg tttgcagtgg aacatttttt ccatatagct ccaatcctgc taatccgaag 2100
   ccaaagagag tgtttcttca gcatatgact agaacattcc atgacttgga aggaaatgca 2160
   gttaaacggg actctggaat atggatcaat gggtttgatt atactggaat ttctcacata 2220
   acccctcaca ttcctgagat caatgatagt atccgagctc actgtgagga gaatgcacct 2280
   ctttgtggtt ttccttggta tcttccagtg cactttctga tcaggaaaaa ctggtatctt 2340
   cctgccccag aagtttctcc aagaaatcct cctcatttcc gactcatatc caaagaacag 2400
   acaccttggg attctataaa attgactttt gaagcaacag cctgcctgcc tatccttcag 2460
   attttggact tgccagcctc aacaatcatg accaagccat atgtccttct atgttcgagc 2520
   ccacaaaggg tcaacacttt ctcagtggtc tcttggcaat ggcaccccag tcacaagtaa 2580
   aggaggagac tactttgtct tttactccca tggactccag gcctctgcat ggcagttctg 2640
   gatagaagtg caggtttcag aagaacatcc tgaaggaatg gtcaccgtgg ccattgctgc 2700
   ccactatctg tctggggaag acaagagatc ccctcaactg gatgctctga aggaaaagtt 2760
   cccagattgg acatttccct ctgcctgggt gtgcacctac gatctctttg tattttaatc 2820
   ttgtggatga gctctaagta catgcccagt ggatactcca tgtgacatgg tttctcccta 2880
   tgttacgtgg atgtttgtaa cgtaagtcaa tgaattttaa tgatcatatg ttcaaagagc 2940
   tttctgggtt aacgcttttc agggccaagc actataaggg tttagctgtg gcgcagtgat 3000
   gcatggcctg ttgacacttg aaaatgccag tcttttggca cttcagcaca tgtgggtact 3060
   gccactacac acacgtcatt ttatatgacc ttaaggacaa agccaacaat ccacttcaat 3120
   agctgcccct ttaggatcaa gaaagatgta cactgtcaga gcattgttaa tgagacaaaa 3180
   gttgtttcca atttaagccc caaaaccatt tgttgtatta gtggatggtg ggtaaaatat   3240
   cattcactga ggtaatgatt ccccttgaga atataactct gtgtaggtca ctggaaagtg   3300
   attgccatag ggctgggaga gaagcattgc actcttgagg ctgtagcctg tgtcaagctg   3360
   tttcttcagg cagcctctca aatgtgcttt gtctctctgt gctgaggcct ggaccctgtg   3420
   ctgagctggt gactcactgt cctgacaagt ggacacacag atgcactgct gtgctgcttt   3480
   cctgaggtgg ttttctatgc ctgttttcct ctgaaacatg tctgttaccc ctctccatct   3540
   taccaagttg aaaaggggaa tatttggcca catacccctc tggttttcgt aggttctttt   3600
   ggttcagaat attgtttgtg ccagtacatg accttaactt ccttcctcag agcactgagc   3660
   tgccatctgg gctattctgg ggtagaagga aggctgggag tggtgggaat tttataaata   3720
   tttattctct ttctttgttt tcataggagt cttgtgttat acaaggttag tccttcatgg   3780
   tataatctta ctgatgcact gggcctatct ttttgttttc cagccagttg aatagattag   3840
   tttttctcag taacttacta tccagcagac tggctttcct gagacttgag gttgtggctt   3900
   atactggaat gagaccactg tacgtgtagg tggttcagat cctgcgtaat ggcagcatga   3960
   ggacttaaaa ggtggttttc attttgaaga tggctatgta gcttgtaagg tgtatcacag   4020
   cagtacctct catggctttt tggttccagc agtgagggca ttggtgagat caatggtaaa   4080
   ctgtgcaagc tttcttttta tcattaggaa atgtgaaacg ttggacaaat tttgagtttt   4140
   aacaaggaca aaaagttgaa agaaaaggca cagttaacaa aaaagggtgg ctagatttat   4200
   cttgggtgat ggaggaaatg agagaggaat gctcttgaaa ggtggtctgt ggatctgtct   4260
   gaatagaaag agcacagtaa gtatgcattg ccggagaaaa cgtccttgaa gctgcttgtc   4320
   tcatgtgtat gatgtgcttt ttaaatcatg cccctcgttg cctgcctaat ctgtgactcc   4380
   ctaaaaacta actgggccca tgtagatggg gctgcaacca gagctgaata acatgttagg   4440
   ctcacacatg catcagcact gcacactgga atcattgctc ttcctggact ttgtagaaat   4500
   cagtctcaag tgcttcaaga gtctggctcc tgctactttt atctgtcagg tagcacataa   4560
   ggtttgcagg gtttatattt tgtatagaat cacagttgtg gagaaaaagt aataatttct   4620
   caatgaattt taaaaatggg cctattttct atccccgtgg ttcatctgat ataattagtg   4680
   ttccctgtga attccccccc tctatgggaa ggatgccttt actctttatc agtaataaat   4740
   tatgactgtt ttcatattgc cttagggtta tttccctgtg taaaccattg tcttttgttt   4800
   tggttttctt tagcattatg aagctttggt attgtacaag gtcagtagta agatgctcac   4860
   tagtctcagg gcttgtgtaa tattctggga ggtcatttaa atgccagaaa tggtcaagca   4920
   attatacaca gtatttatga ctctgttaag cataccgttt gtctgtcaca ttagtagatt   4980
   ctgagattaa aaaaaatttt taaagagtga tcatttaaat aatttctaaa agggtctttt   5040
   caagctctaa caaagtcact aacaaatgca ttattttcta cagaattaga tgttagtagt   5100
   acagtactgc atattcaggg aaaaagtgtg aggaattgat ttcaaaatag ttcgttcttg   5160
   tgtttgacct aagaatgatt gtcgcatgaa gtgtttgttt ttacagttta gcatatataa   5220
   acaaacatga taggattcct taagatgtta ccacccaggg ggccacaagc cagcctgctg   5280
   tctcaggaag ctgtagaagg agtgtttgtc aatttcttgt cactggtttg ctgacttact   5340
   gaggattaat tgttgcctta caatgttact gaaataaact gtttaat                 5387
<210> 87
   <211> 202
   <212> PRT
   <213> Homo sapiens
<400> 87
   Met Glu Arg Gly Ala Gly Ala Lys Leu Leu Pro Leu Leu Leu Leu Leu
   1               5                   10                  15
   Arg Ala Thr Gly Phe Thr Cys Ala Gln Thr Asp Gly Arg Asn Gly Tyr
               20                  25                  30
   Thr Ala Val Ile Glu Val Thr Ser Gly Gly Pro Trp Gly Asp Trp Ala
           35                  40                  45
   Trp Pro Glu Met Cys Pro Asp Gly Phe Phe Ala Ser Gly Phe Ser Leu
       50                  55                  60
   Lys Val Glu Pro Pro Gln Gly Ile Pro Gly Asp Asp Thr Ala Leu Asn
   65                  70                  75                  80
   Gly Ile Arg Leu His Cys Ala Arg Gly Asn Val Leu Gly Asn Thr His
                   85                  90                  95
   Val Val Glu Ser Gln Ser Gly Ser Trp Gly Glu Trp Ser Glu Pro Leu
               100                 105                 110
   Trp Cys Arg Gly Gly Ala Tyr Leu Val Ala Phe Ser Leu Arg Val Glu
           115                 120                 125
   Ala Pro Thr Thr Leu Gly Asp Asn Thr Ala Ala Asn Asn Val Arg Phe
       130                 135                 140
   Arg Cys Ser Asp Gly Glu Glu Leu Gln Gly Pro Gly Leu Ser Trp Gly
   145                 150                 155                 160
   Asp Phe Gly Asp Trp Ser Asp His Cys Pro Lys Gly Ala Cys Gly Leu
   165 170 175
   Gln Thr Lys Ile Gln Gly Pro Arg Gly Leu Gly Asp Asp Thr Ala Leu
   180 185 190
   Asn Asp Ala Arg Leu Phe Cys Cys Arg Ser
   195 200
<210> 88
   <211> 70
   <212> PRT
   <213> Homo sapiens
<400> 88
   Met Glu Arg Gly Ala Gly Ala Lys Leu Leu Pro Leu Leu Leu Leu Leu
   1 5 10 15
   Arg Ala Thr Gly Phe Thr Cys Ala Gln Thr Asp Gly Arg Asn Gly Tyr
   20 25 30
   Thr Ala Val Ile Glu Val Thr Ser Gly Gly Pro Trp Gly Asp Trp Ala
   35 40 45
   Trp Pro Glu Met Cys Pro Asp Gly Phe Phe Ala Ser Gly Phe Ser Leu
   50 55 60
   Lys Leu Gly Arg Met Glu
   65 70
<210> 89
   <211> 102
   <212> PRT
   <213> Homo sapiens
<400> 89
   Met Glu Arg Gly Ala Gly Ala Lys Leu Leu Pro Leu Leu Leu Leu Leu
   1 5 10 15
   Arg Ala Thr Gly Phe Thr Cys Ala Gln Thr Asp Gly Arg Asn Gly Tyr
   20 25 30
   Thr Ala Val Ile Glu Val Thr Ser Gly Gly Pro Trp Gly Asp Trp Ala
   35 40 45
   Trp Pro Glu Met Cys Pro Asp Gly Phe Phe Ala Ser Gly Phe Ser Leu
   50 55 60
   Lys Val Glu Pro Pro Gln Gly Ile Pro Gly Asp Asp Thr Ala Leu Asn
   65 70 75 80
   Gly Ile Arg Leu His Cys Ala Arg Gly Asn Val Leu Gly Asn Thr His
   85 90 95
   Val Leu Gly Arg Met Glu
   100
<210> 90
   <211> 114
   <212> PRT
   <213> Homo sapiens
<400> 90
   Met Glu Arg Gly Ala Gly Ala Lys Leu Leu Pro Leu Leu Leu Leu Leu
   1 5 10 15
   Arg Ala Thr Gly Phe Thr Cys Ala Gln Thr Asp Gly Arg Asn Gly Tyr
   20 25 30
   Thr Ala Val Ile Glu Val Thr Ser Gly Gly Pro Trp Gly Asp Trp Ala
   35 40 45
   Trp Pro Glu Met Cys Pro Asp Gly Phe Phe Ala Ser Gly Phe Ser Leu
   50 55 60
   Lys Val Glu Pro Pro Gln Gly Ile Pro Gly Asp Asp Thr Ala Leu Asn
   65 70 75 80
   Gly Ile Arg Leu His Cys Ala Arg Gly Asn Val Leu Gly Asn Thr His
   85 90 95
   Val Val Glu Ser Gln Ser Gly Arg Trp Gly Ala Gly Val Glu Asp Pro
   100 105 110
   Leu Gly
<210> 91
   <211> 769
   <212> DNA
   <213> Homo sapiens
<400> 91
   ggaggccctt ccgcaatcgg agccctcaca gaggccaaac tgatataaat ctgcttagga 60
   ggcctgattc acagacgcta caggatggag cggggcgcag gagccaagct gctgccgctg 120
   ctgctgcttc tgcgggcgac tggtttcaca tgtgcacaga cagatggccg gaacggctac 180
   acggcggtca tcgaagtgac cagcgggggt ccctggggcg actgggcctg gcctgagatg 240
   tgtcccgatg gattcttcgc cagcgggttc tcgctcaagg tggagcctcc ccaaggcatt 300
   cctggcgacg acactgcact gaatgggatc aggctgcact gcgcgcgcgg gaacgtccta 360
   ggcaatacgc acgtggtaga gtcccagtct ggaagctggg gcgaatggag tgagccgctg 420
   tggtgtcgcg gcggcgccta cctagtggct ttctcgcttc gcgtggaggc acccacgacc 480
   ctcggtgaca acacagcagc gaacaacgtg cgcttccgct gttcagacgg cgaggaactg 540
   caggggcctg ggctgagctg gggagacttt ggagactgga gtgaccattg ccccaagggc 600
   gcgtgcggcc tgcagaccaa gatccaggga cctagaggcc tcggcgatga cactgcgctg 660
   aacgacgcgc gcttattctg ctgccgcagt tgaacggcgc cgccgccgcc gctctctccc 720
   gggccaggag gctagtccca cctcttgcta ttaaagcttc tctgagttg 769
<210> 92
   <211> 653
   <212> DNA
   <213> Homo sapiens
<400> 92
   ggaggccctt ccgcaatcgg agccctcaca gaggccaaac tgatataaat ctgcttagga 60
   ggcctgattc acagacgcta caggatggag cggggcgcag gagccaagct gctgccgctg 120
   ctgctgcttc tgcgggcgac tggtttcaca tgtgcacaga cagatggccg gaacggctac 180
   acggcggtca tcgaagtgac cagcgggggt ccctggggcg actgggcctg gcctgagatg 240
   tgtcccgatg gattcttcgc cagcgggttc tcgctcaagc tggggcgaat ggagtgagcc 300
   gctgtggtgt cgcggcggcg cctacctagt ggctttctcg cttcgcgtgg aggcacccac 360
   gaccctcggt gacaacacag cagcgaacaa cgtgcgcttc cgctgttcag acggcgagga 420
   actgcagggg cctgggctga gctggggaga ctttggagac tggagtgacc attgccccaa 480
   gggcgcgtgc ggcctgcaga ccaagatcca gggacctaga ggcctcggcg atgacactgc 540
   gctgaacgac gcgcgcttat tctgctgccg cagttgaacg gcgccgccgc cgccgctctc 600
   tcccgggcca ggaggctagt cccacctctt gctattaaag cttctctgag ttg 653
<210> 93
   <211> 747
   <212> DNA
   <213> Homo sapiens
<400> 93
   aggcccttcc gcaatcggag ccctcacaga ggccaaactg atataaatct gcttaggagg 60
   cctgattcac agacgctaca ggatggagcg gggcgcagga gccaagctgc tgccgctgct 120
   gctgcttctg cgggcgactg gtttcacatg tgcacagaca gatggccgga acggctacac 180
   ggcggtcatc gaagtgacca gcgggggtcc ctggggcgac tgggcctggc ctgagatgtg 240
   tcccgatgga ttcttcgcca gcgggttctc gctcaaggtg gagcctcccc aaggcattcc 300
   tggcgacgac actgcactga atgggatcag gctgcactgc gcgcgcggga acgtcctagg 360
   caatacgcac gtgctggggc gaatggagtg agccgctgtg gtgtcgcggc ggcgcctacc 420
   tagtggcttt ctcgcttcgc gtggaggcac ccacgaccct cggtgacaac acagcagcga 480
   acaacgtgcg cttccgctgt tcagacggcg aggaactgca ggggcctggg ctgagctggg 540
   gagactttgg agactggagt gaccattgcc ccaagggcgc gtgcggcctg cagaccaaga 600
   tccagggacc tagaggcctc ggcgatgaca ctgcgctgaa cgacgcgcgc ttattctgct 660
   gccgcagttg aacggcgccg ccgccgccgc tctctcccgg gccaggaggc tagtcccacc 720
   tcttgctatt aaagcttctc tgagttg 747
<210> 94
   <211> 803
   <212> DNA
   <213> Homo sapiens
<400> 94
   aggcccttcc gcaatcggag ccctcacaga ggccaaactg atataaatct gcttaggagg 60
   cctgattcac agacgctaca ggatggagcg gggcgcagga gccaagctgc tgccgctgct 120
   gctgcttctg cgggcgactg gtttcacatg tgcacagaca gatggccgga acggctacac 180
   ggcggtcatc gaagtgacca gcgggggtcc ctggggcgac tgggcctggc ctgagatgtg 240
   tcccgatgga ttcttcgcca gcgggttctc gctcaaggtg gagcctcccc aaggcattcc 300
   tggcgacgac actgcactga atgggatcag gctgcactgc gcgcgcggga acgtcctagg 360
   caatacgcac gtggtagagt cccagtctgg aaggtggggc gcaggggtcg aggatccctt 420
   ggggtgatgc tggggcgaat ggagtgagcc gctgtggtgt cgcggcggcg cctacctagt 480
   ggctttctcg cttcgcgtgg aggcacccac gaccctcggt gacaacacag cagcgaacaa 540
   cgtgcgcttc cgctgttcag acggcgagga actgcagggg cctgggctga gctggggaga 600
   ctttggagac tggagtgacc attgccccaa gggcgcgtgc ggcctgcaga ccaagatcca 660
   gggacctaga ggcctcggcg atgacactgc gctgaacgac gcgcgcttat tctgctgccg 720
   cagttgaacg gcgccgccgc cgccgctctc tcccgggcca ggaggctagt cccacctctt 780
   gctattaaag cttctctgag ttg 803
<210> 95
   <211> 21
   <212> DNA
   <213> Homo sapiens
<400> 95
   cgaggacaat ctggatatca a 21
<210> 96
   <211> 21
   <212> DNA
   <213> Homo sapiens
<400> 96
   ctggagccct cgagcaagaa a 21
<210> 97
   <211> 21
   <212> DNA
   <213> Homo sapiens
<400> 97
   cccgtggttc atctgatata a 21
<210> 98
   <211> 21
   <212> DNA
   <213> Homo sapiens
<400> 98
   aaggactttg ctcggcgttt a 21
<210> 99
   <211> 21
   <212> DNA
   <213> Homo sapiens
<400> 99
   tacgtggatg tttgtaacgt a 21
<210> 100
   <211> 21
   <212> DNA
   <213> Homo sapiens
<400> 100
   ctcgtattgg ctcaatcata a 21